(19) Europäisches Patentamt
European Patent Office
Office européen des brevets

(11) EP 2 712 894 A1

(12) EUROPEAN PATENT APPLICATION

(43) Date of publication:
02.04.2014 Bulletin 2014/14

(51) Int Cl.:
*C09B 31/153* (2006.01) *C09D 11/00* (2014.01)

(21) Application number: 13185960.5

(22) Date of filing: 25.09.2013

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(30) Priority: 26.09.2012 JP 2012212754
14.03.2013 JP 2013051807
20.09.2013 JP 2013196181

(71) Applicant: Fujifilm Corporation
Minato-ku
Tokyo (JP)

(72) Inventors:
- Yagi, Kazunari
Kanagawa 258-8577 (JP)
- Hayashi, Shinya
Kanagawa 258-8577 (JP)
- Iizumi, Takashi
Kanagawa 258-8577 (JP)
- Yamada, Hiroshi
Kanagawa 258-8577 (JP)
- Norizuki, Yutaro
Kanagawa 258-8577 (JP)
- Amasaki, Ichiro
Kanagawa 258-8577 (JP)
- Tanaka, Shigeaki
Kanagawa 258-8577 (JP)

(74) Representative: Morpeth, Fraser Forrest
Fujifilm Imaging Colorants Limited
Intellectual Property Group
P.O. Box 42
Hexagon Tower
Blackley, Manchester M9 8ZS (GB)

(54) **Azo compound, aqueous solution, ink composition, ink for inkjet recording, inkjet recording method, ink cartridge for inkjet recording, and inkjet recorded material**

(57) There is provided a compound represented by the following formula (1):

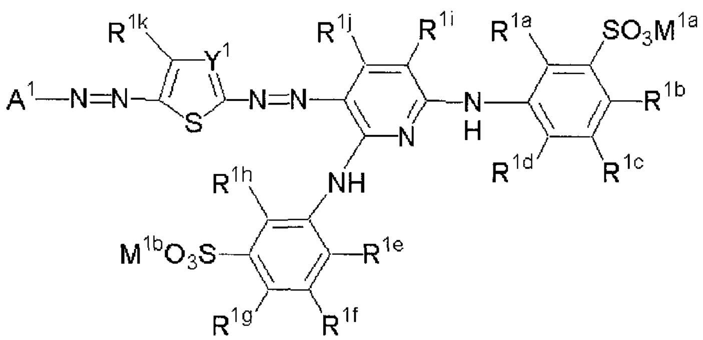

(1)

wherein each of $R^{1a}$ to $R^{1k}$ independently represents a hydrogen atom or a monovalent substituent, the substituents may combine with each other to form a ring, each of $M^{1a}$ and $M^{1b}$ independently represents a hydrogen atom or a monovalent counter cation, $Y^1$ represents a nitrogen atom or a carbon atom having a hydrogen atom or monovalent substituent, $A^1$ represents an aromatic group, and the aromatic group represented by $A^1$ may contain a heteroatom or may have a substituent.

EP 2 712 894 A1

## Description

## BACKGROUND OF THE INVENTION

### 1. **Field of the Invention**

The present invention relates to an azo compound, an aqueous solution, an ink composition, an ink for inkjet recording, an inkjet recording method, an ink cartridge for inkjet recording, and an inkjet recorded material.

### 2. **Description of the Related Art**

An inkjet recording method enjoys a low material cost and a capability of highspeed recording, low-noise recording and easy color recording and therefore, is rapidly spread and further growing.

The inkjet recording method includes a continuous system of continuously flying a liquid droplet and an on-demand system of flying a liquid droplet according to image information signals, and the ejection system therefor includes a system of ejecting a liquid droplet by applying a pressure from a piezoelectric element, a system of ejecting a liquid droplet by generating a bubble in the ink by heat, a system using an ultrasonic wave, and a system of suctioning and ejecting a liquid droplet by an electrostatic force. As the ink for inkjet recording, an aqueous ink, an oil-based ink or a solid (fusion-type) ink is used.

The coloring agent used in such an ink for inkjet recording is required to exhibit good solubility or dispersibility for a solvent, allow for high-density recording, provide a good hue, be fast to light, heat and active gases in the environment (for example, an oxidative gas such as NOx and ozone, and SOx), be excellent in the resistance against water and chemicals, ensure good fixing and less blurring on an image-receiving material, give an ink with excellent storability, have high purity and no toxicity, and be available at a low cost.

In particular, there is a strong demand for an ink composition having a good black hue, being fast to light, humidity and heat, and ensuring that the molar extinction coefficient is high, the storage stability is excellent, the letter quality in document printing is high, bronze gloss is suppressed, and at the printing on an image-receiving material having an ink-receiving layer containing a porous white inorganic pigment particle, resistance to an oxidative gas such as ozone in the environment is developed.

In International Publication No. 2012/014954 and International Publication No. 2012/014955, an ink composition containing an azo compound having a specific structure capable of resolving the above-described task is described.

## Summary of the Invention

The ink composition described in International Publication No. 2012/014954 and International Publication No. 2012/014955 exhibits an excellent performance as stated above, but with respect to the color tone and suppression of bronze gloss in a recorded image when various recording materials are used, a higher level of performance is sometimes required.

An object of the present invention is to provide a compound capable of providing for an ink having a good color tone as an ink for black color and exerting that the color tone is little changed when observed under various light sources (the dependency on observation light source is small) and exerting excellent suppression of bronze gloss in a recorded image when various recording materials are used. Also, the present invention relates to an aqueous solution, an ink composition, an ink for inkjet recording, each containing the compound, an inkjet recording method using the ink for inkjet recording, an ink cartridge for inkjet recording filled with the ink for inkjet recording, and an inkjet recorded material formed using the ink for inkjet recording.

The present inventors have made detailed studies with an aim to resolve the above-described task, as a result, it has been found that in a diazo compound having a specific structure containing a pyridine coupler substituted with a sulfophenylamino group, when the sulfo group is substituted on the meta-position with respect to the amino group, the solubility of the dye in water can be enhanced, making it possible to avoid dye precipitation on the printing paper surface and in turn, suppress bronze gloss, and also, the electron-withdrawing property of the sulfo group in the aniline moiety is reduced due to meta-substitution, leading to shift of the absorption wavelength to the long wavelength side and enhancement in the color tone as an ink for black color and the dependency on observation light source becomes good, whereby the task above can be resolved.

That is, the above-described object can be attained by the following techniques.

[1] A compound represented by the following formula (1):

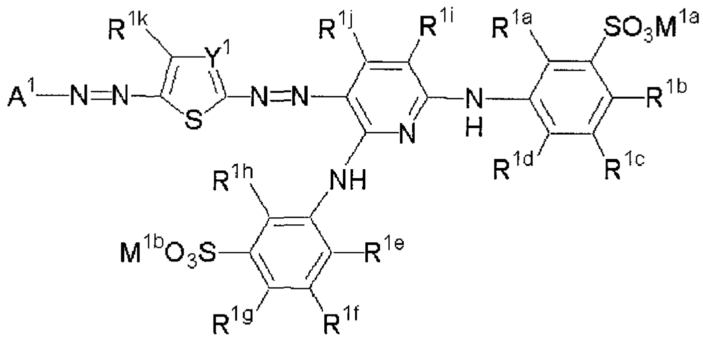

(1)

wherein
each of $R^{1a}$ to $R^{1k}$ independently represents a hydrogen atom or a monovalent substituent, the substituents may combine with each other to form a ring,
each of $M^{1a}$ and $M^{1b}$ independently represents a hydrogen atom or a monovalent counter cation,
$Y^1$ represents a nitrogen atom or a carbon atom having a hydrogen atom or monovalent substituent,
$A^1$ represents an aromatic group, and the aromatic group represented by $A^1$ may contain a heteroatom or may have a substituent.

[2] The compound as described in [1],
wherein the compound represented by formula (1) is a compound represented by the following formula (2):

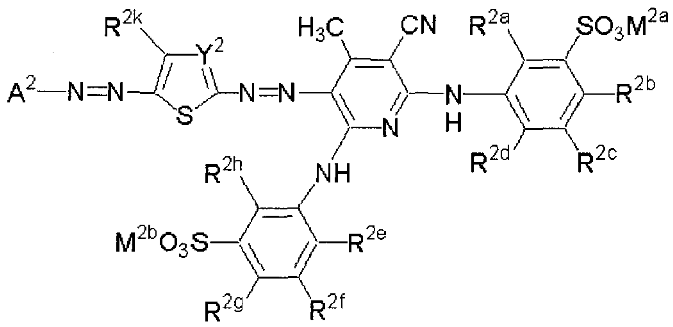

(2)

wherein
each of $R^{2a}$ to $R^{2h}$ and $R^{2k}$ independently represents a hydrogen atom or a monovalent substituent, the substituents may combine with each other to form a ring,
each of $M^{2a}$ and $M^{2b}$ independently represents a hydrogen atom or a monovalent counter cation,
$Y^2$ represents a nitrogen atom or a carbon atom having a hydrogen atom or a monovalent substituent,
$A^2$ represents an aromatic group, and the aromatic group represented by $A^2$ may contain a heteroatom or may have a substituent.

[3] The compound as described in [2],
wherein the compound represented by formula (2) is a compound represented by the following formula (3):

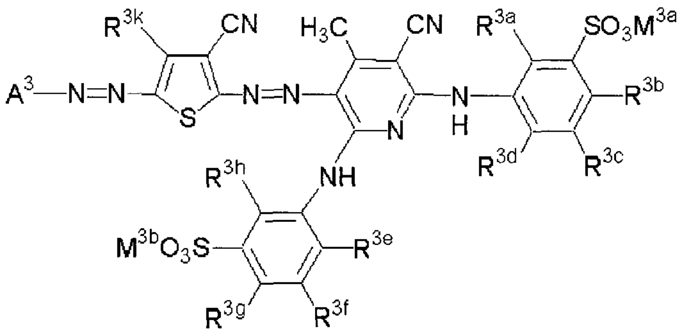

(3)

wherein
each of $R^{3a}$ to $R^{3h}$ and $R^{3k}$ independently represents a hydrogen atom or a monovalent substituent, the substituents may combine with each other to form a ring,
each of $M^{3a}$ and $M^{3b}$ independently represents a hydrogen atom or a monovalent counter cation,
$A^3$ represents an aromatic group, and the aromatic group represented by $A^3$ may contain a heteroatom or may have a substituent.
[4] The compound as described in [3],
wherein the compound represented by formula (3) is a compound represented by the following formula (4):

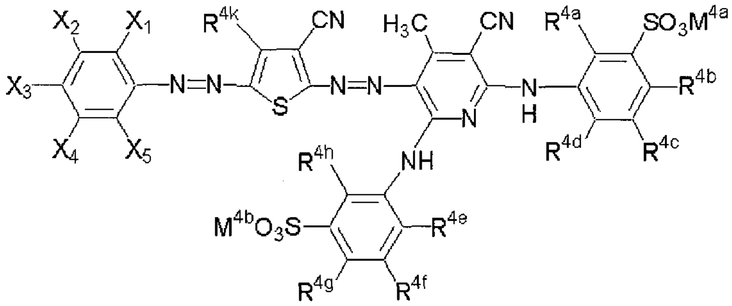

(4)

wherein
each of $R^{4a}$ to $R^{4h}$ and $R^{4k}$ independently represents a hydrogen atom or a monovalent substituent, the substituents may combine with each other to form a ring,
each of $M^{4a}$ and $M^{4b}$ independently represents a hydrogen atom or a monovalent counter cation, and
each of $X_1$ to $X_5$ independently represents a hydrogen atom or a monovalent substituent.
[5] The compound as described in [3],
wherein the compound represented by formula (3) is a compound represented by the following formula (5):

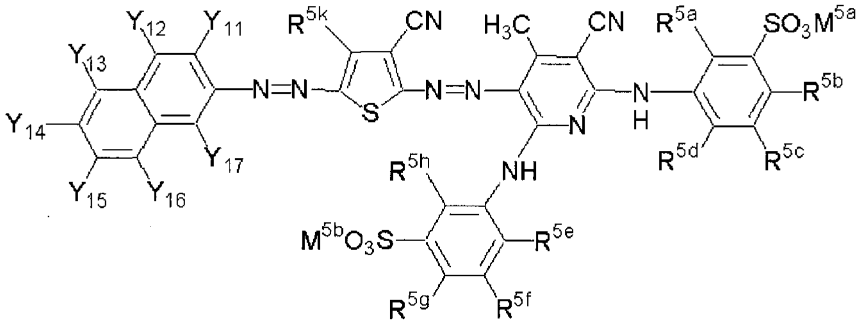

(5)

wherein
each of $R^{5a}$ to $R^{5h}$ and $R^{5k}$ independently represents a hydrogen atom or a monovalent substituent, the substituents may combine with each other to form a ring,
each of $M^{5a}$ and $M^{5b}$ independently represents a hydrogen atom or a monovalent counter cation, and
each of $Y_{11}$ to $Y_{17}$ independently represents a hydrogen atom or a monovalent substituent.
[6] The compound as described in [3],
wherein the compound represented by formula (3) is a compound represented by the following formula (6):

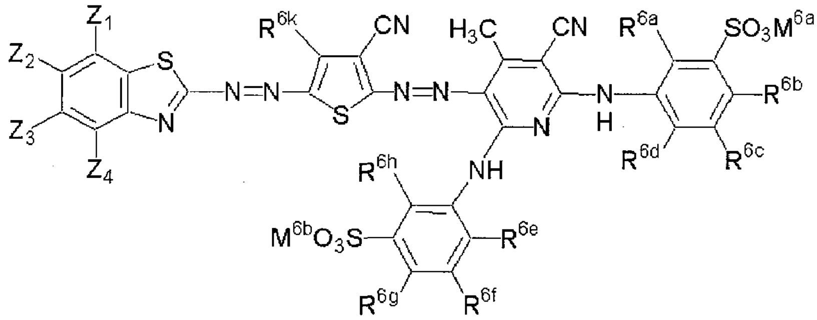

(6)

wherein
each of $R^{6a}$ to $R^{6h}$ and $R^{6k}$ independently represents a hydrogen atom or a monovalent substituent, the substituents may combine with each other to form a ring,
each of $M^{6a}$ and $M^{6b}$ independently represents a hydrogen atom or a monovalent counter cation, and
each of $Z_1$ to $Z_4$ independently represents a hydrogen atom or a monovalent substituent.
[7] The compound as described in any one of [1] to [6],
wherein $R^{1k}$, $R^{2k}$, $R^{3k}$, $R^{4k}$, $R^{5k}$ or $R^{6k}$ is a group represented by the following formula (7):

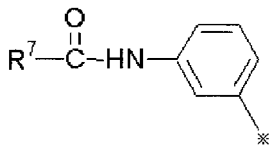

(7)

wherein $R^7$ represents a monovalent substituent, and
* represents a bond.
[8] The compound as described in any one of [1] to [6],
wherein $R^{1k}$, $R^{2k}$, $R^{3k}$, $R^{4k}$, $R^{5k}$ or $R^{6k}$ is a group represented by the following formula (8):

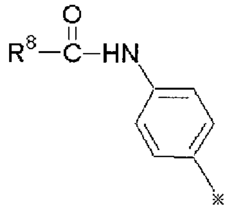

(8)

wherein $R^8$ represents a monovalent substituent, and
* represents a bond.

[9] The compound as described in any one of [1] to [6],
wherein $R^{1k}$, $R^{2k}$, $R^{3k}$, $R^{4k}$, $R^{5k}$ or $R^{6k}$ is a group represented by the following formula (9):

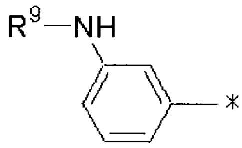

(9)

wherein $R^9$ represents a monovalent substituent, and
* represents a bond.

[10] The compound as described in any one of [1] to [6],
wherein $R^{1k}$, $R^{2k}$, $R^{3k}$, $R^{4k}$, $R^{5k}$ or $R^{6k}$ is a group represented by the following formula (10):

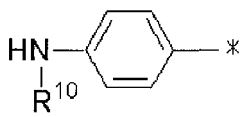

(10)

wherein $R^{10}$ represents a monovalent substituent, and
* represents a bond.

[11] The compound as described in any one of [1] to [6],
wherein $R^{1k}$, $R^{2k}$, $R^{3k}$, $R^{4k}$, $R^{5k}$ or $R^{6k}$ is a group represented by the following formula (11):

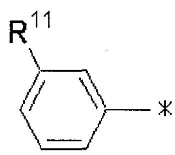

(11)

wherein $R^{11}$ represents a monovalent substituent, and
* represents a bond.

[12] The compound as described in any one of [1] to [6],
wherein $R^{1k}$, $R^{2k}$, $R^{3k}$, $R^{4k}$, $R^{5k}$ or $R^{6k}$ is a group represented by the following formula (12):

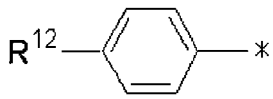

(12)

wherein $R^{12}$ represents a monovalent substituent, and
* represents a bond.
[13] The compound as described in any one of [1] to [12], having three or more ionic hydrophilic groups.
[14] An aqueous solution comprising:

(a) a preservative and
(b) at least one member of the compound claimed in any one of [1] to [12] and a salt thereof,

wherein the content of (b) is from 1 to 25 mass%.
[15] The aqueous solution as described in [14], further comprising:

(c) a pH adjusting agent.

[16] The aqueous solution as described in [14] or [15],
wherein the pH at 25°C is from 7.0 to 9.0.
[17] An ink composition comprising the aqueous solution described in any one of [14] to [16].
[18] An ink for inkjet recording, comprising the aqueous solution described in any one of [14] to [16] or the ink composition described in [17].
[19] An inkjet recording method, comprising:

forming a colored image on a recording material by using the ink for inkjet recording described in [18].

[20] An ink cartridge for inkjet recording, which is filled with the ink for inkjet recording described in [18].
[21] An inkjet recorded material, wherein a colored image is formed on a recording material by using the ink for inkjet recording described in [18].

According to the present invention, a compound capable of providing for an ink having a good color tone as an ink for black color and exerting that the color tone is little changed when observed under various light sources (the dependency on observation light source is small) and exerting excellent suppression of bronze gloss in a recorded image when various recording materials are used, can be provided.

DETAILED DESCRIPTION OF THE INVENTION

The present invention is described in detail below.
First, Substituent Group A', Substituent Group J, the ionic hydrophilic group, and the Hammett's substituent constant $\sigma p$ value in the present invention are defined.

(Substituent Group A')

This group includes a linear or branched alkyl group having a carbon number of 1 to 12, a linear or branched aralkyl group having a carbon number of 7 to 18, a linear or branched alkenyl group having a carbon number of 2 to 12, a linear or branched alkynyl group having a carbon number of 2 to 12, a cycloalkyl group having a carbon number of 3 to 12, a cycloalkenyl group having a carbon number of 3 to 12 (examples of these groups include methyl, ethyl, n-propyl, i-propyl, n-butyl, i-butyl, sec-butyl, tert-butyl, 2-ethylhexyl, 2-methylsulfonylethyl, 3-phenoxypropyl, trifluoromethyl and cyclopentyl), a halogen atom (e.g., chlorine, bromine), an aryl group (e.g., phenyl, 4-tert-butylphenyl, 2,4-di-tert-amylphenyl), a heterocyclic group (e.g., imidazolyl, pyrazolyl, triazolyl, 2-furyl, 2-thienyl, 2-pyrimidinyl, 2-benzothiazolyl), a cyano group, a hydroxyl group, a nitro group, a carboxy group, an amino group, an alkyloxy group (e.g., methoxy, ethoxy, 2-methoxyethoxy, 2-methylsulfonylethoxy), an aryloxy group (e.g., phenoxy, 2-methylphenoxy, 4-tert-butylphenoxy, 3-nitrophenoxy, 3-tert-butyloxycarbonylphenoxy, 3-methoxycarbonylphenyloxy), an acylamino group (e.g., acetamide, benzamide, 4-(3-tert-butyl-4-hydroxyphenoxy)butanamide), an alkylamino group (e.g., methylamino, butylamino, diethylamino, methylbutylamino), an arylamino group (e.g., phenylamino, 2-chloroanilino), a ureido group (e.g., phenylureido,

methylureido, N,N-dibutylureido), a sulfamoylamino group (e.g., N,N-dipropylsulfamoylamino), an alkylthio group (e.g., methylthio, octylthio, 2-phenoxyethylthio), an arylthio group (e.g., phenylthio, 2-butoxy-5-tert-octylphenylthio, 2-carboxyphenylthio), an alkyloxycarbonylamino group (e.g., methoxycarbonylamino), alkylsulfonylamino and arylsulfonylamino groups (e.g., methylsulfonylamino, phenylsulfonylamino, p-toluenesulfonylamino), a carbamoyl group (e.g., N-ethylcarbamoyl, N,N-dibutylcarbamoyl), a sulfamoyl group (e.g., N-ethylsulfamoyl, N,N-dipropylsulfamoyl, N-phenylsulfamoyl), an alkylsulfonyl group (e.g., methylsulfonyl, octylsulfonyl), an arylsulfonyl group (e.g., phenylsulfonyl, p-toluenesulfonyl), an alkyloxycarbonyl group (e.g., methoxycarbonyl, butyloxycarbonyl), a heterocyclic oxy group (e.g., 1-phenyltetrazol-5-oxy, 2-tetrahydropyranyloxy), an azo group (e.g., phenylazo, 4-methoxyphenylazo, 4-pivaloylaminophenylazo, 2-hydroxy-4-propanoylphenylazo), an acyloxy group (e.g., acetoxy), a carbamoyloxy group (e.g., N-methylcarbamoyloxy, N-phenylcarbamoyloxy), a silyloxy group (e.g., trimethylsilyloxy, dibutylmethylsilyloxy), an aryloxycarbonylamino group (e.g., phenoxycarbonylamino), an imide group (e.g., N-succinimide, N-phthalimide), a heterocyclic thio group (e.g., 2-benzothiazolylthio, 2,4-di-phenoxy-1,3,5-triazole-6-thio, 2-pyridylthio), a sulfinyl group (e.g., 3-phenoxypropylsulfinyl), a phosphonyl group (e.g., phenoxyphosphonyl, octyloxyphosphonyl, phenylphosphonyl), an aryloxycarbonyl group (e.g., phenoxycarbonyl), an acyl group (e.g., acetyl, 3-phenylpropanoyl, benzoyl), and an ionic hydrophilic group (e.g., carboxyl, sulfo). These substituents may be further substituted, and the further substituent includes a group selected from Substituent Group A' described above.

(Substituent Group J)

Examples of this substituent group include a halogen atom, an alkyl group, an aralkyl group, an alkenyl group, an alkynyl group, an aryl group, a heterocyclic group, a cyano group, a hydroxyl group, a nitro group, an alkoxy group, an aryloxy group, a silyloxy group, a heterocyclic oxy group, an acyloxy group, a carbamoyloxy group, an alkoxycarbonyloxy group, an aryloxycarbonyloxy group, an amino group, an acylamino group, an aminocarbonylamino group, an alkoxycarbonylamino group, an aryloxycarbonylamino group, a sulfamoylamino group, an alkylsulfonylamino group, an arylsulfonylamino group, a mercapto group, an alkylthio group, an arylthio group, a heterocyclic thio group, a sulfamoyl group, an alkylsulfinyl group, an arylsulfinyl group, an alkylsulfonyl group, an arylsulfonyl group, an acyl group, an aryloxycarbonyl group, an alkoxycarbonyl group, a carbamoyl group, an arylazo group, a heterocyclic azo group, an imido group, a phosphino group, a phosphinyl group, a phosphinyloxy group, a phosphinylamino group, a silyl group, and an ionic hydrophilic group. These substituents may be further substituted, and the further substituent includes a group selected from Substituent Group J.

More specifically, the halogen atom includes, for example, a fluorine atom, a chlorine atom, a bromine atom, and an iodine atom.

The alkyl group includes a linear, branched or cyclic, substituted or unsubstituted alkyl group and encompasses a cycloalkyl group, a bicycloalkyl group, and a tricyclo structure having many ring structures. The alkyl group in the substituents described hereinafter (for example, the alkyl group of an alkoxy group or an alkylthio group) indicates an alkyl group having such a concept. More specifically, the alkyl group is preferably an alkyl group having a carbon number of 1 to 30, and examples thereof include a methyl group, an ethyl group, an n-propyl group, an isopropyl group, a tert-butyl group, an n-octyl group, an eicosyl group, a 2-chloroethyl group, a 2-cyanoethyl group, and a 2-ethylhexyl group. The cycloalkyl group is preferably a substituted or unsubstituted cycloalkyl group having a carbon number of 3 to 30, and examples thereof include a cyclohexyl group, a cyclopentyl group and a 4-n-dodecylcyclohexyl group. The bicycloalkyl group is preferably a substituted or unsubstituted bicycloalkyl group having a carbon number of 5 to 30, that is, a monovalent group formed by removing one hydrogen atom from a bicycloalkane having a carbon number of 5 to 30, and examples thereof include a bicyclo[1,2,2] heptan-2-yl group and a bicyclo[2,2,2]octan-3-yl group.

The aralkyl group includes a substituted or unsubstituted aralkyl group. The substituted or unsubstituted aralkyl group is preferably an aralkyl group having a carbon number of 7 to 30, and examples thereof include a benzyl group and a 2-phenethyl group.

The alkenyl group includes a linear, branched or cyclic, substituted or unsubstituted alkenyl group and encompasses a cycloalkenyl group and a bicycloalkenyl group. Specifically, the alkenyl group is preferably a substituted or unsubstituted alkenyl group having a carbon number of 2 to 30, and examples thereof include a vinyl group, an allyl group, a prenyl group, a geranyl group, and an oleyl group. The cycloalkenyl group is preferably a substituted or unsubstituted cycloalkenyl group having a carbon number of 3 to 30, that is, a monovalent group formed by removing one hydrogen atom from a cycloalkene having a carbon number of 3 to 30, and examples thereof include a 2-cyclopenten-1-yl group and a 2-cyclohexen-1-yl group. The bicycloalkenyl group is a substituted or unsubstituted bicycloalkenyl group, preferably a substituted or unsubstituted bicycloalkenyl group having a carbon number of 5 to 30, that is, a monovalent group formed by removing one hydrogen atom from a bicycloalkene having one double bond, and examples thereof include a bicyclo[2,2,1]hept-2-en-1-yl group and a bicyclo[2,2,2]oct-2-en-4-yl group.

The alkynyl group is preferably a substituted or unsubstituted alkynyl group having a carbon number of 2 to 30, and examples thereof include an ethynyl group, a propargyl group, and a trimethylsilylethynyl group.

The aryl group is preferably a substituted or unsubstituted aryl group having a carbon number of 6 to 30, and examples thereof include a phenyl group, a p-tolyl group, a naphthyl group, an m-chlorophenyl group, and an o-hexadecanoylaminophenyl group.

The heterocyclic group is preferably a monovalent group formed by removing one hydrogen atom from a 5- or 6-membered substituted or unsubstituted, aromatic or non-aromatic heterocyclic compound, more preferably a 5- or 6-membered aromatic heterocyclic group having a carbon number of 3 to 30, and examples thereof include a 2-furyl group, a 2-thienyl group, a 2-pyrimidinyl group, and a 2-benzothiazolyl group.

The alkoxy group is preferably a substituted or unsubstituted alkoxy group having a carbon number of 1 to 30, and examples thereof include a methoxy group, an ethoxy group, an isopropoxy group, a tert-butoxy group, an n-octyloxy group, and a 2-methoxyethoxy group.

The aryloxy group is preferably a substituted or unsubstituted aryloxy group having a carbon number of 6 to 30, and examples thereof include a phenoxy group, a 2-methylphenoxy group, a 4-tert-butylphenoxy group, a 3-nitrophenoxy group, and a 2-tetradecanoylaminophenoxy group.

The silyloxy group is preferably a substituted or unsubstituted silyloxy group having a carbon number of 0 to 20, and examples thereof include a trimethylsilyloxy group and a diphenylmethylsilyloxy group.

The heterocyclic oxy group is preferably a substituted or unsubstituted heterocyclic oxy group having a carbon number of 2 to 30, and examples thereof include a 1-phenyltetrazol-5-oxy group and a 2-tetrahydropyranyloxy group.

The acyloxy group is preferably a formyloxy group, a substituted or unsubstituted alkylcarbonyloxy group having a carbon number of 2 to 30, or a substituted or unsubstituted arylcarbonyloxy group having a carbon number of 6 to 30, and examples thereof include an acetyloxy group, a pivaloyloxy group, a stearoyloxy group, a benzoyloxy group, and a p-methoxyphenycarbonyloxy group.

The carbamoyloxy group is preferably a substituted or unsubstituted carbamoyloxy group having a carbon number of 1 to 30, and examples thereof include an N,N-dimethylcarbamoyloxy group, an N,N-diethylcarbamoyloxy group, a morpholinocarbonyloxy group, an N,N-di-n-octylaminocarbonyloxy group, and an N-n-octylcarbamoyloxy group.

The alkoxycarbonyloxy group is preferably a substituted or unsubstituted alkoxycarbonyloxy group having a carbon number of 2 to 30, and examples thereof include a methoxycarbonyloxy group, an ethoxycarbonyloxy group, a tert-butoxycarbonyloxy group, and an n-octyloxycarbonyloxy group.

The aryloxycarbonyloxy group is preferably a substituted or unsubstituted aryloxycarbonyloxy group having a carbon number of 7 to 30, and examples thereof include a phenoxycarbonyloxy group, a p-methoxyphenoxycarbonyloxy group, and a p-n-hexadecyloxyphenoxycarbonyloxy group.

The amino group includes an alkylamino group, an arylamino group and a heterocyclic amino group and is preferably an amino group, a substituted or unsubstituted alkylamino group having a carbon number of 1 to 30, or a substituted or unsubstituted anilino group having a carbon number of 6 to 30, and examples thereof include a methylamino group, a dimethylamino group, an anilino group, an N-methyl-anilino group, a diphenylamino group, and a triazinylamino group.

The acylamino group is preferably a formylamino group, a substituted or unsubstituted alkylcarbonylamino group having a carbon number of 1 to 30, or a substituted or unsubstituted arylcarbonylamino group having a carbon number of 6 to 30, and examples thereof include an acetylamino group, a pivaloylamino group, a lauroylamino group, a benzoylamino group, and a 3,4,5-tri-n-octyloxyphenylcarbonylamino group.

The aminocarbonylamino group is preferably a substituted or unsubstituted aminocarbonylamino group having a carbon number of 1 to 30, and examples thereof include a carbamoylamino group, an N,N-dimethylaminocarbonylamino group, an N,N-diethylaminocarbonylamino group, and a morpholinocarbonylamino group.

The alkoxycarbonylamino group is preferably a substituted or unsubstituted alkoxycarbonylamino group having a carbon number of 2 to 30, and examples thereof include a methoxycarbonylamino group, an ethoxycarbonylamino group, a tert-butoxycarbonylamino group, an n-octadecyloxycarbonylamino group, and an N-methyl-methoxycarbonylamino group.

The aryloxycarbonylamino group is preferably a substituted or unsubstituted aryloxycarbonylamino group having a carbon number of 7 to 30, and examples thereof include a phenoxycarbonylamino group, a p-chlorophenoxycarbonylamino group, and an m-n-octyloxyphenoxycarbonylamino group.

The sulfamoylamino group is preferably a substituted or unsubstituted sulfamoylamino group having a carbon number of 0 to 30, and examples thereof include a sulfamoylamino group, an N,N-dimethylaminosulfonylamino group, and an N-n-octylaminosulfonylamino group.

The alkylsulfonylamino or arylsulfonylamino group is preferably a substituted or unsubstituted alkylsulfonylamino group having a carbon number of 1 to 30 or a substituted or unsubstituted arylsulfonylamino group having a carbon number of 6 to 30, and examples thereof include a methylsulfonylamino group, a butylsulfonylamino group, a phenylsulfonylamino group, a 2,3,5-trichlorophenylsulfonylamino group, and a p-methylphenylsulfonylamino group.

The alkylthio group is preferably a substituted or unsubstituted alkylthio group having a carbon number of 1 to 30, and examples thereof include a methylthio group, an ethylthio group, and an n-hexadecylthio group.

The arylthio group is preferably a substituted or unsubstituted arylthio group having a carbon number of 6 to 30, and examples thereof include a phenylthio group, a p-chlorophenylthio group, and an m-methoxyphenylthio group.

The heterocyclic thio group is preferably a substituted or unsubstituted heterocyclic thio group having a carbon number of 2 to 30, and examples thereof include a 2-benzothiazolylthio group and a 1-phenyltetrazol-5-ylthio group.

The sulfamoyl group is preferably a substituted or unsubstituted sulfamoyl group having a carbon number of 0 to 30, and examples thereof include an N-ethylsulfamoyl group, an N-(3-dodecyloxypropyl)sulfamoyl group, an N,N-dimethylsulfamoyl group, an N-acetylsulfamoyl group, an N-benzoylsulfamoyl group, and an N-(N'-phenylcarbamoyl)sulfamoyl group.

The alkylsulfinyl or arylsulfinyl group is preferably a substituted or unsubstituted alkylsulfinyl group having a carbon number of 1 to 30 or a substituted or unsubstituted arylsulfinyl group having a carbon number of 6 to 30, and examples thereof include a methylsulfinyl group, an ethylsulfinyl group, a phenylsulfinyl group, and a p-methylphenylsulfinyl group.

The alkylsulfonyl or arylsulfonyl group is preferably a substituted or unsubstituted alkylsulfonyl group having a carbon number of 1 to 30 or a substituted or unsubstituted arylsulfonyl group having a carbon number of 6 to 30, and examples thereof include a methylsulfonyl group, an ethylsulfonyl group, a phenylsulfonyl group, and a p-methylphenylsulfonyl group.

The acyl group is preferably a formyl group, a substituted or unsubstituted alkylcarbonyl group having a carbon number of 2 to 30, a substituted or unsubstituted arylcarbonyl group having a carbon number of 7 to 30, or a substituted or unsubstituted heterocyclic carbonyl group having a carbon number of 2 to 30, in which the carbonyl group is bonded through a carbon atom, and examples thereof include an acetyl group, a pivaloyl group, a 2-chloroacetyl group, a stearoyl group, a benzoyl group, a p-n-octyloxyphenylcarbonyl group, a 2-pyridylcarbonyl group, and a 2-furylcarbonyl group.

The aryloxycarbonyl group is preferably a substituted or unsubstituted aryloxycarbonyl group having a carbon number of 7 to 30, and examples thereof include a phenoxycarbonyl group, an o-chlorophenoxycarbonyl group, an m-nitrophenoxycarbonyl group, and a p-tert-butylphenoxycarbonyl group.

The alkoxycarbonyl group is preferably a substituted or unsubstituted alkoxycarbonyl group having a carbon number of 2 to 30, and examples thereof include a methoxycarbonyl group, an ethoxycarbonyl group, a tert-butoxycarbonyl group, and an n-octadecyloxycarbonyl group.

The carbamoyl group is preferably a substituted or unsubstituted carbamoyl group having a carbon number of 1 to 30, and examples thereof include a carbamoyl group, an N-methylcarbamoyl group, an N,N-dimethylcarbamoyl group, an N,N-di-n-octylcarbamoyl group, and an N-(methylsulfonyl)carbamoyl group.

The arylazo or heterocyclic azo group is preferably a substituted or unsubstituted arylazo group having a carbon number of 6 to 30 or a substituted or unsubstituted heterocyclic azo group having a carbon number of 3 to 30, and examples thereof include phenylazo, p-chlorophenylazo, and 5-ethylthio-1,3,4-thiadiazol-2-ylazo.

The imido group is preferably, for example, an N-succinimido group or an N-phthalimido group.

The phosphino group is preferably a substituted or unsubstituted phosphino group having a carbon number of 0 to 30, and examples thereof include a dimethylphosphino group, a diphenylphosphino group, and a methylphenoxyphosphino group.

The phosphinyl group is preferably a substituted or unsubstituted phosphinyl group having a carbon number of 0 to 30, and examples thereof include a phosphinyl group, a dioctyloxyphosphinyl group, and a diethoxyphosphinyl group.

The phosphinyloxy group is preferably a substituted or unsubstituted phosphinyloxy group having a carbon number of 0 to 30, and examples thereof include a diphenoxyphosphinyloxy group and a dioctyloxyphosphinyloxy group.

The phosphinylamino group is preferably a substituted or unsubstituted phosphinylamino group having a carbon number of 0 to 30, and examples thereof include a dimethoxyphosphinylamino group and a dimethylaminophosphinylamino group.

The silyl group is preferably a substituted or unsubstituted silyl group having a carbon number of 0 t o 30, and examples thereof include a trimethylsilyl group, a tertbutyldimethylsilyl group, and a phenyldimethylsilyl group.

(Ionic Hydrophilic Group)

Examples of the ionic hydrophilic group include a sulfo group, a carboxyl group, a thiocarboxyl group, a sulfino group, a phosphono group, and a dihydroxyphosphino group. Among these, a sulfo group and a carboxyl group are preferred. The carboxyl group, phosphono group and sulfo group may be in the salt state, and examples of the counter cation forming the salt include an ammonium ion, an alkali metal ion (e.g., lithium ion, sodium ion, potassium ion), and an organic cation (e.g., tetramethylammonium ion, tetramethylguanidium ion, tetramethylphosphonium). A lithium salt, a sodium salt, a potassium salt and an ammonium salt are preferred, a lithium salt and a mixed salt containing a lithium salt as a main component are more preferred, and a lithium salt is most preferred.

The counter cation (monovalent counter cation) of the ionic hydrophilic group contained in the azo compound

of the present invention preferably contains a lithium ion as a main component. The counter cation may not be all a lithium ion, but the lithium ion concentration in each ink composition is preferably 50 mass% or more, more preferably 75 mass% or more, still more preferably 80 mass% or more, yet still more preferably 95 mass% or more, based on all counter cations in each ink composition.

Under the condition of such an abundance ratio, a hydrogen ion, an alkali metal ion (e.g., sodium ion, potassium ion), an alkaline earth metal ion (e.g., magnesium ion, calcium ion), a quaternary ammonium ion, a quaternary phosphonium ion, a sulfonium ion, or the like can be contained as the counter cation.

As for the type and proportion of the counter cation in the coloring agent, details on analysis methods and elements are described in Shin Jikken Kagaku Koza 9, Bunseki Kagaku (Lecture 9 of New Experiment Chemistry, Analysis Chemistry), compiled by The Chemical Society of Japan (Maruzen, 1977), and Dai 4 Han, Jikken Kagaku Koza 15, Bunseki (4th Edition, Lecture 15 of Experiment Chemistry, Analysis), compiled by The Chemical Society of Japan (Maruzen, 1991). By referring to these publications, the analysis method may be selected, and the analysis and quantitative determination may be performed. Above all, the determination can be easily made by ion chromatography, atomic absorption method, inductively coupled plasma emission spectroscopy (ICP) or other analysis methods.

As the method to obtain the coloring agent for use in the present invention, in which the counter cation contains a lithium ion, any method may be used. Examples thereof include (1) a method of converting the counter cation into a lithium ion from a different cation by using an ion exchange resin, (2) a method by acid or salt precipitation from a system containing a lithium ion, (3) a method of forming a coloring agent by using a raw material or synthesis intermediate where the counter cation is a lithium ion, (4) a method of introducing an ionic hydrophilic group by conversion of a functional group of a coloring agent for each color by using a reactant in which the counter cation is a lithium ion, and (5) a method of synthesizing a compound where the counter cation of an ionic hydrophilic group in a coloring agent is a silver ion, reacting the compound with a lithium halide solution, and removing the precipitated silver halide, thereby forming a lithium ion as the counter cation.

The ionic hydrophilic group in the coloring agent for each color may be of any type as long as it is an ionic dissociative group. Preferred examples of the ionic hydrophilic group include a sulfo group (that may be a salt thereof), a carboxyl group (that may be a salt thereof), a hydroxyl group (that may be a salt thereof), a phosphono group (that may be a salt thereof), a quaternary ammonium group, an acylsulfamoyl group (that may be a salt thereof), a sulfonylcarbamoyl group (that may be a salt thereof), and a sulfonylsulfamoyl group (that may be a salt thereof).

The ionic hydrophilic group is preferably a sulfo group, a carboxyl group or a hydroxyl group (including salts thereof). In the case where the ionic hydrophilic group is a salt, the counter cation is preferably lithium or an alkali metal (e.g., sodium, potassium), ammonium or organic cation (e.g., pyridinium, tetramethylammonium, guanidium) mixed salt containing lithium as a main component, more preferably lithium or an alkali metal mixed salt containing lithium as a main component, still more preferably a lithium salt of sulfo group, a lithium salt of carboxy group, or a lithium salt of hydroxyl group.

(Hammett's Substituent Constant σp Value)

The Hammett's substituent constant σp value used in the description of the present invention is described below.

The Hammett's rule is an empirical rule advocated by L.P. Hammett in 1935 so as to quantitatively discuss the effect of a substituent on the reaction or equilibrium of a benzene derivative, and its propriety is widely admitted at present. The substituent constant determined by the Hammett's rule includes a σp value and a σm value, and these values can be found in a large number of general publications and are described in detail, for example, in J.A. Dean (compiler), Lange's Handbook of Chemistry, 12th ed., McGraw-Hill (1979), and Kagakuno Ryoiki (Chemistry Region), special number, No. 122, pp. 96-103, Nankodo (1979). In the present invention, each substituent is limited or described by using the Hammett's substituent constant σp, but this does not mean that the substituent is limited only to those having a known value which can be found in the above-described publications. Needless to say, the substituent includes a substituent of which value is not known in publications but when measured based on the Hammett's rule, falls in the specified range. Although the compounds of the present invention are not a benzene derivative, the σp value is used as a measure indicating the electron effect of the substituent, irrespective of the substitution position. In the present invention, hereinafter, the σp value is used in such a meaning. Incidentally, in the present invention, when the compound is a salt, the salt exists by dissociating into an ion in an aqueous solution and an ink composition, but for the sake of convenience, this is expressed as "contain a salt".

<Azo Compound>

The azo compound of the present invention is a compound represented by the following formula (1):

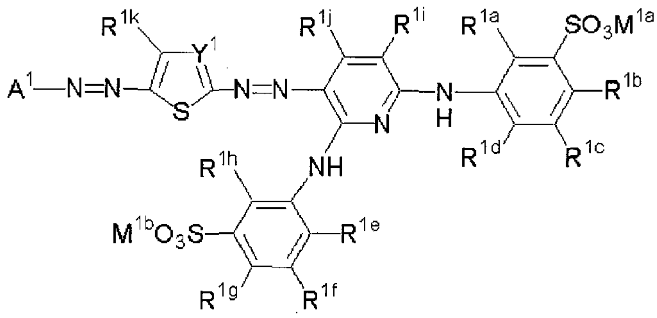

(1)

[In formula (1), each of $R^{1a}$ to $R^{1k}$ independently represents a hydrogen atom or a monovalent substituent, the substituents may combine with each other to form a ring, each of $M^{1a}$ and $M^{1b}$ independently represents a hydrogen atom or a monovalent counter cation, $Y^1$ represents a nitrogen atom, or a carbon atom having a hydrogen atom or a monovalent substituent, $A^1$ represents an aromatic group, and the aromatic group represented by $A^1$ may contain a heteroatom or may have a substituent.]

In formula (1), $Y^1$ represents a nitrogen atom, or a carbon atom having a hydrogen atom or a monovalent substituent. The carbon atom having a hydrogen atom or a monovalent substituent preferably represents $-C(R_1)=$. $R_1$ represents a hydrogen atom, a sulfo group, a carboxy group, a substituted or unsubstituted carbamoyl group or a cyano group, and when the carbamoyl group has a substituent, the substituent includes an alkyl group (preferably an alkyl group having a carbon number of 1 to 6, more preferably an alkyl group having a carbon number of 1 to 4, still more preferably a methyl group or an ethyl group), and an aryl group (preferably an aryl group having a carbon number of 6 to 8, more preferably a phenyl group). $R_1$ is preferably a carboxy group, a substituted or unsubstituted carbamoyl group or a cyano group, more preferably a cyano group.

In view of ozone fastness, $Y^1$ preferably represents a nitrogen atom or -C(CN)= and most preferably represents -C(CN)=.

In formula (1), each of $M^{1a}$ and $M^{1b}$ independently represents a hydrogen atom or a monovalent counter cation. Examples of the monovalent counter cation include an ammonium ion, an alkali metal ion (e.g., lithium ion, sodium ion, potassium ion), and an organic cation (e.g., tetramethylammonium ion, tetramethylguanidium ion, tetramethylphosphonium).

In view of suppression of bronze gloss, each of $M^{1a}$ and $M^{1b}$ preferably represents a monovalent counter cation, more preferably represents an alkali metal ion or an ammonium ion, still more preferably represents an alkali metal ion, yet still more preferably represents a lithium ion, a potassium ion or a sodium ion.

In the present invention, the compound represented by formula (1) may be in the salt form.

In the case where the azo compound represented by formula (1) is a mixed salt, in view of solubility for water, the viscosity and surface tension of aqueous solution and the storage stability of high-concentration aqueous solution, a mixed salt of a lithium salt and a sodium salt is preferred, and the mixed salt may be in an embodiment where a plurality of M partially represent a lithium ion and the remaining M represent a sodium ion, or in an embodiment where a compound in which all M in formula (1) represent a lithium ion and a compound in which all M in formula (1) represent a sodium ion are mixed.

The ratio of cations of the mixed salt can be measured by ion chromatography.

In formula (1), $A^1$ represents an aromatic group. The aromatic group represented by $A^1$ may contain a heteroatom or may have a substituent. $A^1$ preferably represents an aromatic group having a carbon number of 6 to 12, more preferably an aromatic group having a carbon number of 6 to 8. The heteroatom is preferably a nitrogen atom, a sulfur atom or an oxygen atom, more preferably a nitrogen atom or a sulfur atom. Above all, $A^1$ preferably represents a substituted or unsubstituted phenyl group, a substituted or unsubstituted naphthyl group, or a substituted or unsubstituted nitrogen-containing 5- or 6-membered heterocyclic group. The nitrogen-containing 5- or 6-membered heterocyclic group may further have a condensed ring structure.

In the case where $A^1$ represents a nitrogen-containing 5- or 6-membered heterocyclic group, the nitrogen-containing 5- or 6-membered heterocyclic group is preferably a monovalent group formed by removing one hydrogen atom from a 5-membered aromatic or non-aromatic heterocyclic compound, more preferably a 5-membered aromatic heterocyclic group having a carbon number of 2 to 4. The nitrogen-containing 5-membered heterocyclic group includes, without limiting the substitution position, a pyrrole ring, a pyrazole ring, an imidazole ring, a triazole ring, a thiazole ring, an isothiazole ring, and a thiadiazole ring, and a thiazole ring is preferred. The nitrogen-containing 6-membered heterocyclic group includes, without limiting the substitution position, a pyridine ring, a pyrazine ring, a pyrimidine ring, and

a triazine ring, and a pyridine ring is preferred.

The nitrogen-containing 5- or 6-membered heterocyclic group may further have a condensed ring structure and preferably, may be condensed with a benzene ring. In the case of having a condensed ring structure, $A^1$ is preferably a benzothiazole ring.

The substituent which $A^1$ may have includes a substituent selected from Substituent Group J and is preferably an ionic hydrophilic group or an electron-withdrawing group having a Hammett's σp value of 0.3 or more, more preferably a halogen atom (preferably a chlorine atom), a nitro group, $-SO_3M$ or $-CO_2M$ (wherein M represents a hydrogen atom or a monovalent counter cation, and specific examples and preferred range thereof are the same as those of $M^{1a}$ and $M^{1b}$ above), still more preferably $-SO_3Li$ or $-CO_2Li$.

Specific examples of the electron-withdrawing group having a Hammett's σp value of 0.3 or more include an acyl group, an acyloxy group, a carbamoyl group, an alkyloxycarbonyl group, an aryloxycarbonyl group, a cyano group, a nitro group, a dialkylphosphono group, a diarylphosphono group, a diaryl phosphinyl group, an alkylsulfinyl group, an arylsulfinyl group, an alkylsulfonyl group, an arylsulfonyl group, a sulfonyloxy group, an acylthio group, a sulfamoyl group, a thiocyanate group, a thiocarbonyl group, a halogenated alkyl group, a halogenated alkoxy group, a halogenated aryloxy group, a halogenated alkylamino group, a halogenated alkylthio group, an aryl group substituted with another electron-withdrawing group having a σp value of 0.3 or more, a nitro group, a heterocyclic group, a halogen atom, an azo group, and a selenocyanate group. A cyano group, a methylsulfonyl group, a phenylsulfonyl group, a methoxycarbonyl group, a carbamoyl group and a nitro group are preferred, and a cyano group, a methylsulfonyl group and a nitro group are more preferred. When the substituent is an electron-withdrawing group having a σp value in this range, the hue adjustment and increase in the light fastness and ozone gas fastness of the azo compound can be achieved, and this can produce an effect in terms of use as a water-soluble dye for a black ink in inkjet recording. As for the upper limit of the Hammett's substituent constant σp value, an electron-withdrawing group of 1.0 or less is preferred.

Specific preferred examples of $A^1$ are illustrated below, but the present invention is not limited thereto. In the following specific examples, * represents a bond.

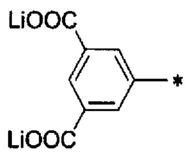
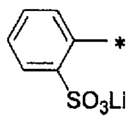
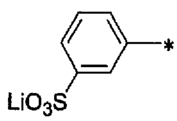
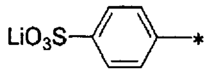
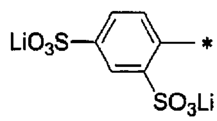

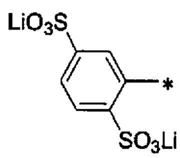
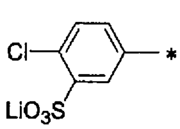
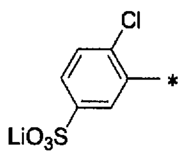
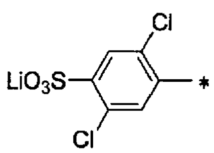
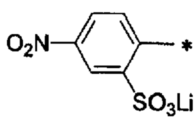

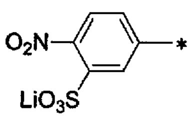
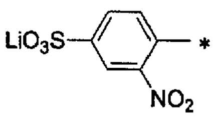
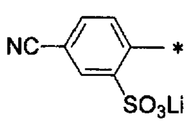
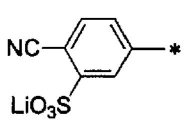
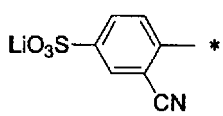

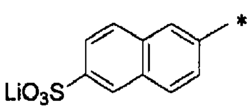
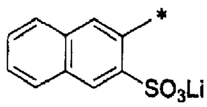
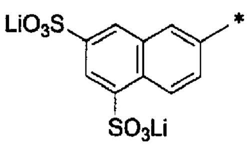
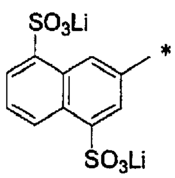
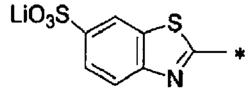

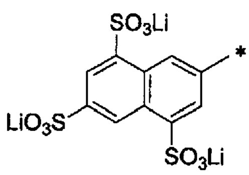
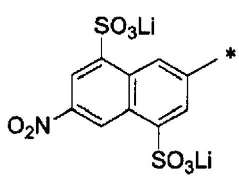
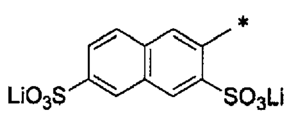
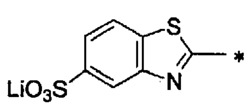

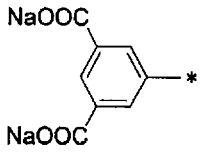
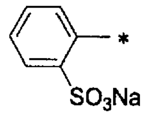
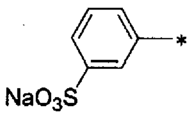
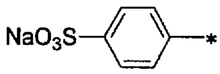
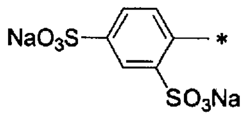

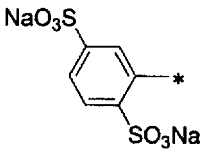
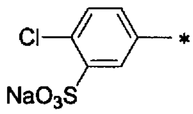
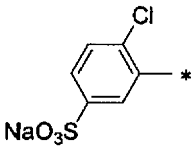
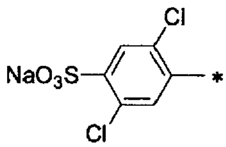
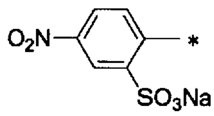

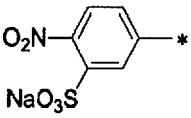
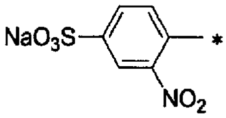
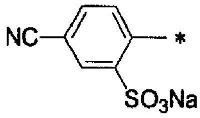
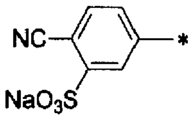
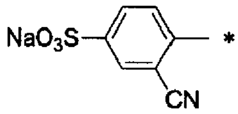

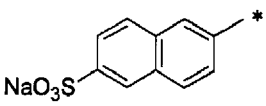
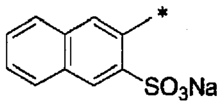
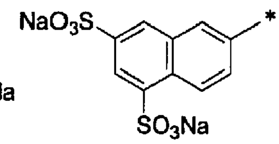
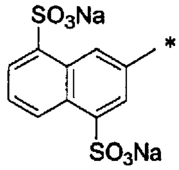

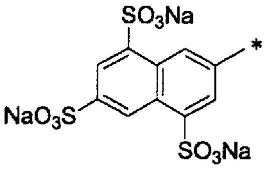
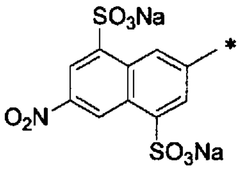
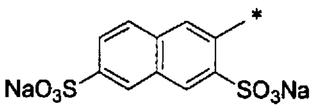
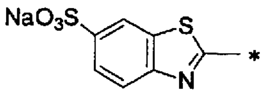

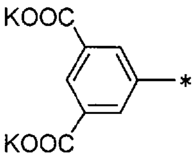
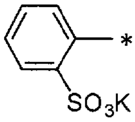
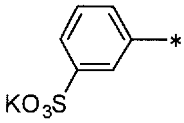
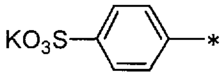
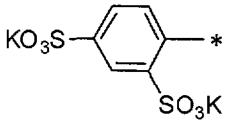

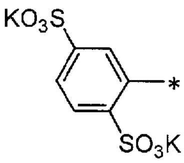
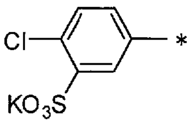
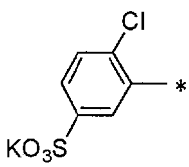
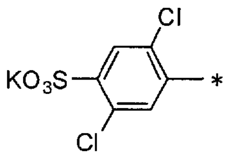
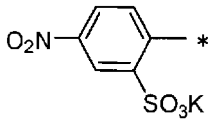

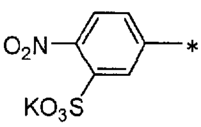
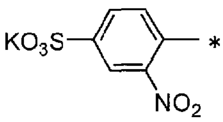
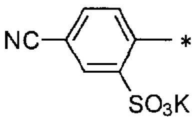
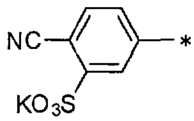
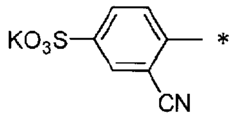

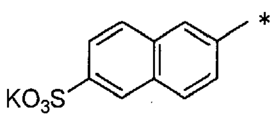 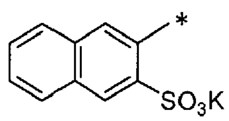 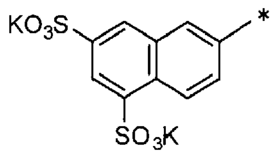 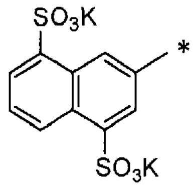

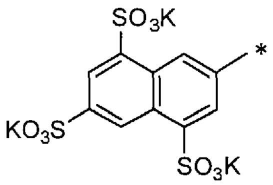 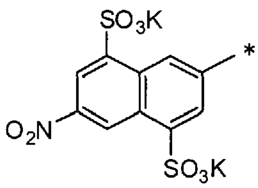 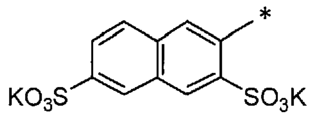 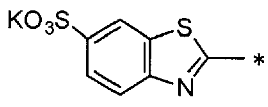

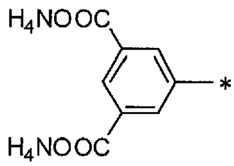 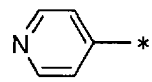

In formula (1), each of $R^{1a}$ to $R^{1k}$ independently represents a hydrogen atom or a monovalent substituent, and the substituents may combine with each other to form a ring.

In the case where each of $R^{1a}$ to $R^{1k}$ represents a monovalent substituent, the monovalent substituent includes a substituent selected from Substituent Group J and is preferably a halogen atom, an alkyl group, an alkoxy group, an amino group, a cyano group, an ionic hydrophilic group or an aryl group. These groups may further have a substituent.

In $R^{1a}$ to $R^{1k}$, the substituents may combine with each other to form a ring. The ring formed is not particularly limited but is preferably an aromatic ring, more preferably a benzene ring.

In view of ozone fastness, $R^{1i}$ is preferably a carboxy group, a substituted or unsubstituted carbamoyl group or a cyano group, more preferably a cyano group.

In view of light fastness, $R^{1j}$ is preferably a hydrogen atom, an aryl group or a methyl group, more preferably a methyl group.

In view of light fastness and hue, $R^{1a}$, $R^{1c}$, $R^{1d}$, $R^{1e}$, $R^{1f}$ and $R^{1h}$ are preferably a sulfo group, a halogen atom, an alkyl group or a hydrogen atom, more preferably a hydrogen atom.

From the standpoint that the absorption wavelength is sifted to the long wavelength side and a black ink with excellent color tone is obtained, each of $R^{1b}$ and $R^{1g}$ is independently preferably a halogen atom, an alkyl group or an alkoxy group, more preferably a halogen atom, an alkyl group having a carbon number of 1 to 10, or an alkoxy group having a carbon number of 1 to 10, still more preferably a chlorine atom, a methyl group or a methoxy group.

In view of light fastness, $R^{1k}$ is preferably a group represented by the following formula (7):

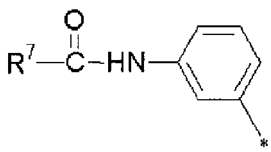

(7)

[In formula (7), $R^7$ represents a monovalent substituent, and * represents a bond.]

In formula (7), $R^7$ represents a monovalent substituent. The monovalent substituent represented by $R^7$ includes a substituent selected from Substituent Group A' and in view of light fastness, is preferably a substituted or unsubstituted alkyl group, a substituted or unsubstituted aryl group, a substituted or unsubstituted heterocyclic group, or a substituted or unsubstituted amino group, more preferably a substituted or unsubstituted alkyl group, a substituted or unsubstituted aryl group or a substituted or unsubstituted amino group. The alkyl group is preferably an alkyl group having a carbon number of 1 to 7, more preferably an alkyl group having a carbon number of 1 to 6, still more preferably an alkyl group

having a carbon number of 1 to 4, yet still more preferably a methyl group, an ethyl group, an n-propyl group, an isopropyl group, a tert-butyl group or a 2-ethylpentyl group, more preferably a methyl group, an ethyl group or an n-propyl group, still more preferably a methyl group or an ethyl group. The aryl group is preferably an aryl group having a carbon number of 6 to 12, more preferably an aryl group having a carbon number of 6 to 8, still more preferably a phenyl group or a naphthyl group, yet still more preferably a phenyl group. The heterocyclic group is preferably a thiophene ring or a pyridine ring.

The amino group is preferably an amino group substituted with a substituted or unsubstituted alkyl group, or an amino group substituted with a substituted or unsubstituted aryl group, more preferably an amino group substituted with a hydroxyl group-substituted alkyl group having a carbon number of 1 to 6, or an amino group substituted with a hydroxyl group-substituted phenyl group.

In the case where the alkyl group, aryl group heterocyclic group or amino group has a substituent, the substituent includes a monovalent substituent and is preferably an ionic hydrophilic group, an arylamino group, an alkylamino group or a halogen atom, more preferably an ionic hydrophilic group, still more preferably $-SO_3M$ or $-CO_2M$ (wherein M represents a hydrogen atom or a monovalent counter cation, and specific examples and preferred range thereof are the same as those of $M^{1a}$ and $M^{1b}$ above), yet still more preferably - $CO_2M$ or $-CO_2K$, and most preferably $-CO_2K$.

Above all, $R^7$ is preferably an alkyl group having $-CO_2M$ as a substituent, or a phenyl group having $-CO_2M$ as a substituent at least on the ortho-position.

Specific preferred examples of $R^7$ are illustrated below, but the present invention is not limited thereto. In the following specific examples, * represents a bond.

$LiOOC(H_2C)_2$—*

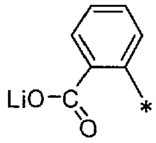

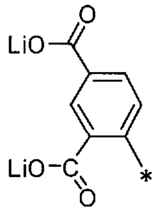

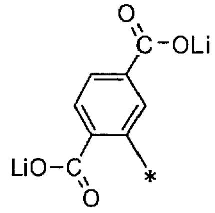

$LiO_3S(H_2C)_2$—*

$LiO_2C(H_2C)_2HN$—*

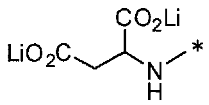

$LiO_3S(H_2C)_2HN$—*

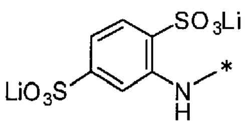

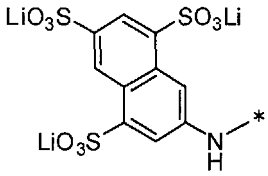

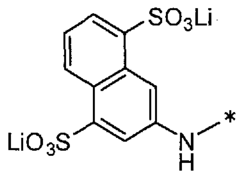

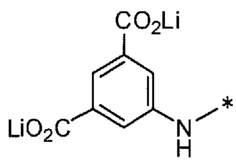

$NaOOC(H_2C)_2$—*

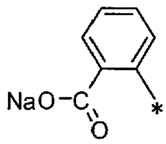

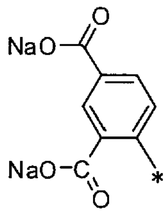

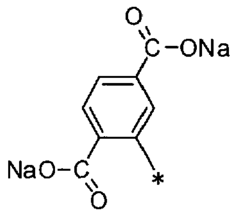

$NaO_3S(H_2C)_2$—*

$NaO_2C(H_2C)_2HN$—*

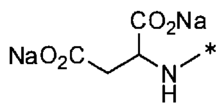

$NaO_3S(H_2C)_2HN$—*

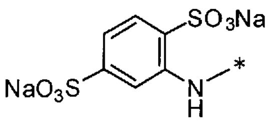

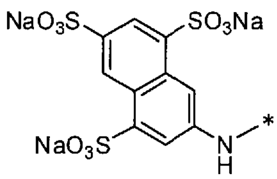

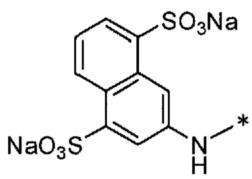

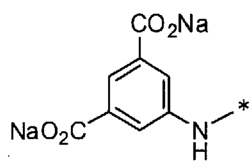

$KOOC(H_2C)_2$—*

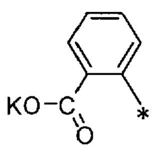

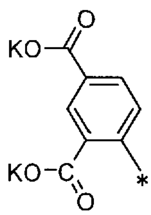

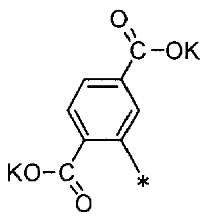

$KO_3S(H_2C)_2$—*

$KO_2C(H_2C)_2HN$—*

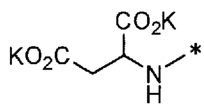

$KO_3S(H_2C)_2HN$—*

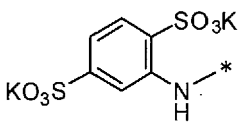

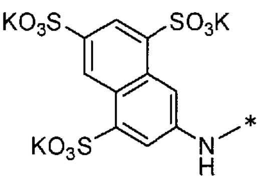

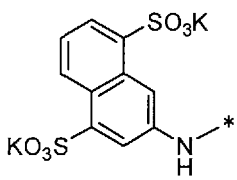

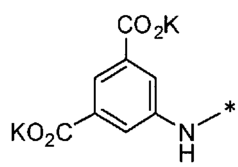

Me—*

$H_2N$—*

$H_3CHN$—*

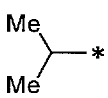

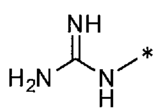

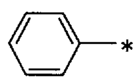

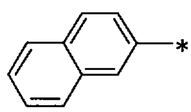

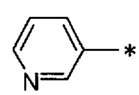

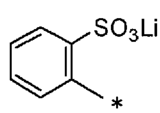

In view of light fastness, it is also preferred that $R^{1k}$ is a group represented by the following formula (8):

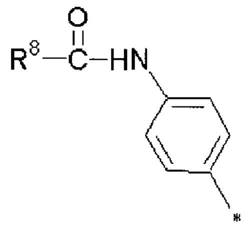

(8)

[In formula (8), $R^8$ represents a monovalent substituent, and * represents a bond.]

$R^8$ in formula (8) has the same meaning as $R^7$ in formula (7), and specific examples and preferred range are also the same.

In view of light fastness, it is also preferred that $R^{1k}$ is a group represented by the following formula (9):

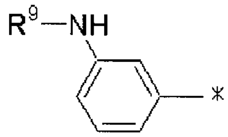

(9)

[In formula (9), $R^9$ represents a monovalent substituent, and * represents a bond.]

In formula (9), $R^9$ represents a monovalent substituent. The monovalent substituent represented by $R^9$ includes a substituent selected from Substituent Group A' and is preferably a substituted or unsubstituted alkyl group, a substituted or unsubstituted aryl group, a substituted or unsubstituted heterocyclic group, a substituted or unsubstituted alkylsulfonyl group, or a substituted or unsubstituted arylsulfonyl group, and in view of light fastness, more preferably a substituted or unsubstituted heterocyclic group or an alkylsulfonyl or arylsulfonyl group, still more preferably a substituted or unsubstituted heterocyclic group. The heterocyclic group is preferably a triazine ring group.

In the case where the alkyl group, aryl group, heterocyclic group, alkylsulfonyl group or arylsulfonyl group above has a substituent, the substituent includes a monovalent substituent and is preferably an ionic hydrophilic group, a hydroxyl group, an amino group, an arylamino group, an alkylamino group, an alkyl group or an alkylthio group, and such a substituent may be further substituted with a hydroxyl group or an ionic hydrophilic group.

The ionic hydrophilic group is preferably $-SO_3M$ or $-CO_2M$ (wherein M represents a hydrogen atom or a monovalent counter cation, and specific examples and preferred range thereof are the same as those of $M^{1a}$ and $M^{1b}$ above).

Specific preferred examples of $R^9$ are illustrated below, but the present invention is not limited thereto. In the following specific examples, * represents a bond.

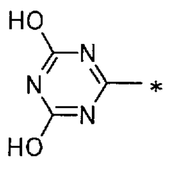

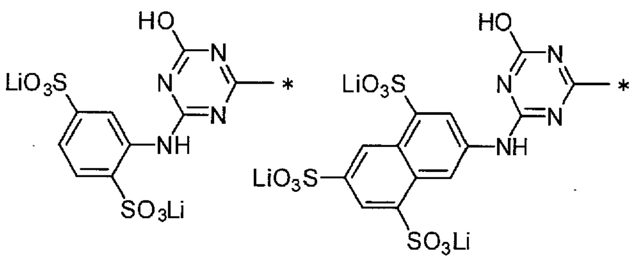

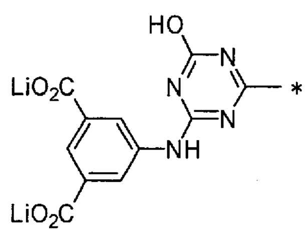

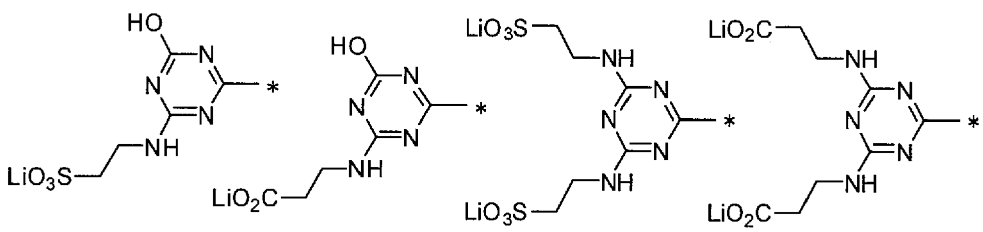

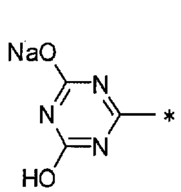
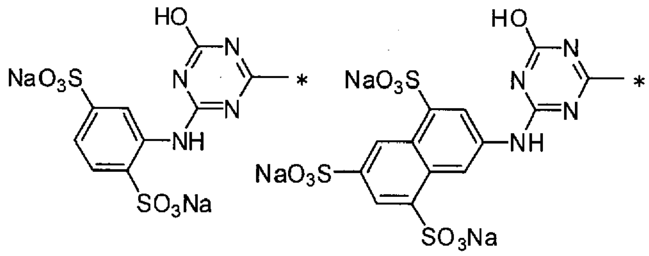
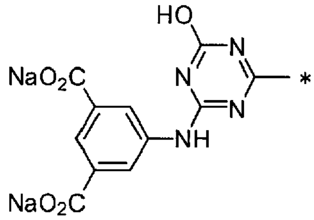

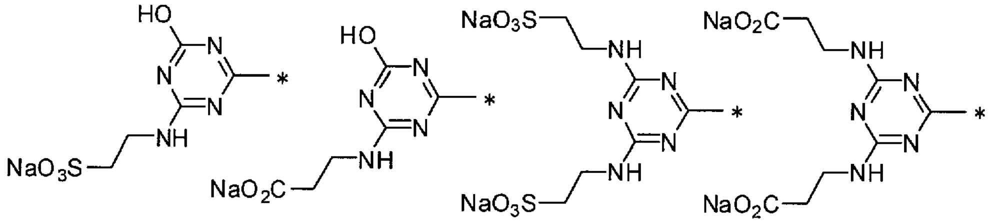

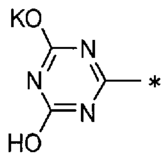
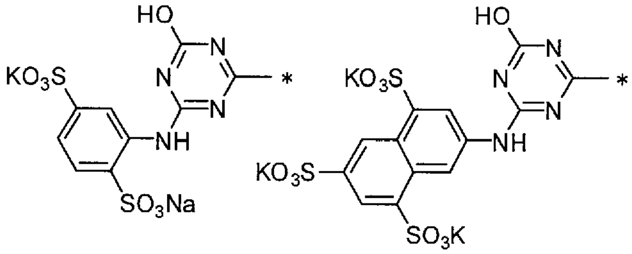
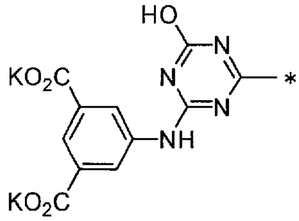

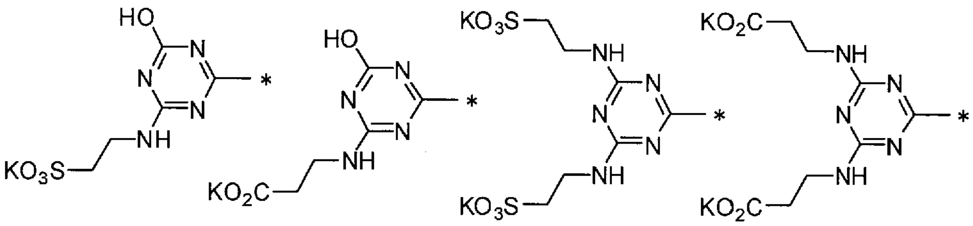

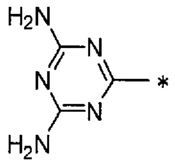
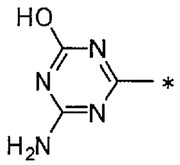
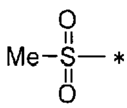
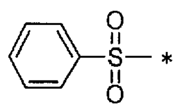

In view of light fastness, it is also preferred that $R^{1k}$ is a group represented by the following formula (10):

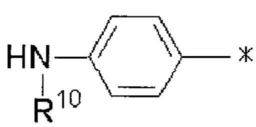

(10)

[In formula (10), $R^{10}$ represents a monovalent substituent, and * represents a bond.]

$R^{10}$ in formula (10) has the same meaning as $R^9$ in formula (9), and specific examples and preferred range are also the same.

In view of light fastness, it is also preferred that $R^{1k}$ is a group represented by the following formula (II):

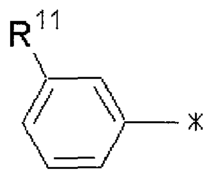

(11)

[In formula (11), $R^{11}$ represents a monovalent substituent, and * represents a bond.]

In formula (11), $R^{11}$ represents a monovalent substituent. The monovalent substituent represented by $R^{11}$ includes a substituent selected from Substituent Group A' and in view of light fastness, is preferably a nitro group, a hydroxyl group, a halogen atom, an alkyl group, an ionic hydrophilic group, an amino group, a substituted or unsubstituted alkoxy group or an alkylthio group, more preferably a nitro group, a hydroxyl group, a chlorine atom, a methyl group, an ionic hydrophilic group, an amino group, an ionic hydrophilic group-substituted or unsubstituted alkoxy group having a carbon number of 1 to 3, or a methylthio group. The ionic hydrophilic group is preferably $-SO_3M$ or $-CO_2M$ (wherein M represents a hydrogen atom or a monovalent counter cation, and specific examples and preferred range thereof are the same as those of $M^{1a}$ and $M^{1b}$ above).

Specific preferred examples of $R^{11}$ include a nitro group, a hydroxyl group, a chlorine atom, a methyl group, $-SO_3Na$, $-SO_3Li$, $-SO_3K$, an amino group, a methoxy group, a sulfo group-substituted propyloxy group, and a methylthio group.

In view of light fastness, it is also preferred that $R^{1k}$ is a group represented by the following formula (12):

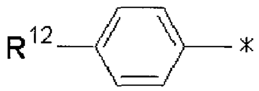

(12)

[In formula (12), $R^{12}$ represents a monovalent substituent, and * represents a bond.]

$R^{12}$ in formula (12) has the same meaning as $R^{11}$ in formula (11), and specific examples and preferred range are also the same.

In view of light fastness, it is also preferred that $R^{1k}$ is a naphthyl group. In the case where $R^{1k}$ is a naphthyl group, $R^{1k}$ is more preferably a 2-naphthyl group.

The compound represented by formula (1) is preferably a compound represented by the following formula (2):

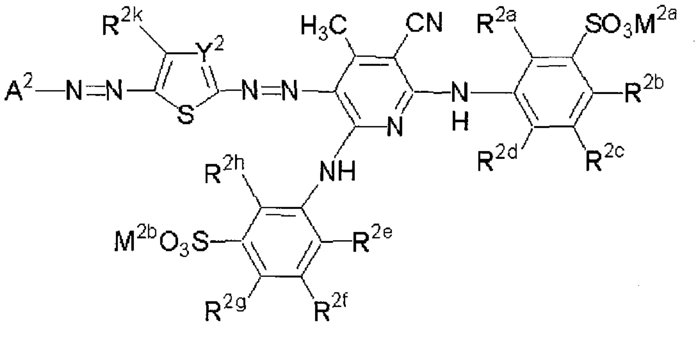

(2)

[In formula (2), each of $R^{2a}$ to $R^{2h}$ and $R^{2k}$ independently represents a hydrogen atom or a monovalent substituent, the substituents may combine with each other to form a ring, each of $M^{2a}$ and $M^{2b}$ independently represents a hydrogen atom or a monovalent counter cation, $Y^2$ represents a nitrogen atom or a carbon atom having a hydrogen atom or a monovalent substituent, $A^2$ represents an aromatic group, and the aromatic group represented by $A^2$ may contain a

heteroatom or may have a substituent.]

In formula (2), $R^{2a}$ to $R^{2h}$, $R^{2k}$, $M^{2a}$, $M^{2b}$, $Y^2$ and $A^2$ have the same meanings as $R^{1a}$ to $R^{1h}$, $R^{1k}$, $M^{1a}$, $M^{1b}$, $Y^1$ and $A^1$ in formula (1), and specific examples and preferred ranges are also the same.

The compound represented by formula (1) or (2) is preferably a compound represented by the following formula (3):

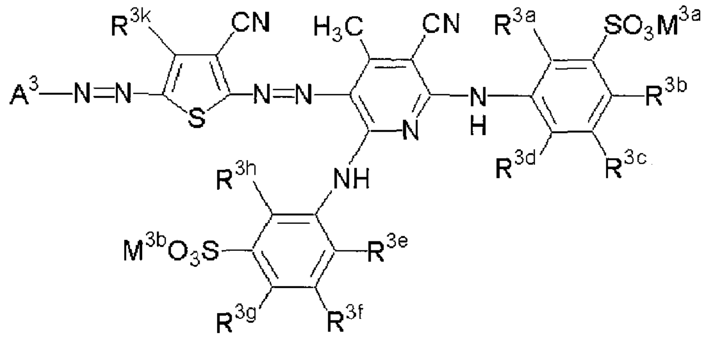

(3)

[In formula (3), each of $R^{3a}$ to $R^{3h}$ and $R^{3k}$ independently represents a hydrogen atom or a monovalent substituent, the substituents may combine with each other to form a ring, each of $M^{3a}$ and $M^{3b}$ independently represents a hydrogen atom or a monovalent counter cation, $A^3$ represents an aromatic group, and the aromatic group represented by $A^3$ may contain a heteroatom or may have a substituent.]

In formula (3), $R^{3a}$ to $R^{3h}$, $R^{3k}$, $M^{3a}$, $M^{3b}$ and $A^3$ have the same meanings as $R^{1a}$ to $R^{1h}$, $R^{1k}$, $M^{1a}$, $M^{1b}$ and $A^1$ in formula (1), and specific examples and preferred ranges are also the same.

The compound represented by any one of formulae (1) to (3) is preferably a compound represented by the following formula (4):

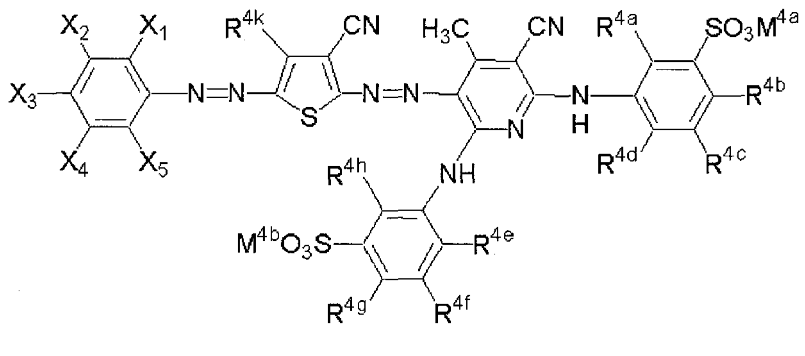

(4)

[In formula (4), each of $R^{4a}$ to $R^{4h}$ and $R^{4k}$ independently represents a hydrogen atom or a monovalent substituent, the substituents may combine with each other to form a ring, each of $M^{4a}$ and $M^{4b}$ independently represents a hydrogen atom or a monovalent counter cation, and each of $X_1$ to $X_5$ independently represents a hydrogen atom or a monovalent substituent.]

In formula (4), $R^{4a}$ to $R^{4h}$, $R^{4k}$, $M^{4a}$ and $M^{4b}$ have the same meanings as $R^{1a}$ to $R^{1h}$, $R^{1k}$, $M^{1a}$ and $M^{1b}$ in formula (1), and specific examples and preferred ranges are also the same.

In formula (4), each of $X_1$ to $X_5$ independently represents a hydrogen atom or a monovalent substituent. When each of $X_1$ to $X_5$ represents a monovalent substituent, the substituent includes a group selected from Substituent Group J.

Each of $X_1$, $X_2$, $X_3$, $X_4$ and $X_5$ is independently preferably a hydrogen atom, an ionic hydrophilic group, a cyano group, a substituted or unsubstituted alkylsulfonyl group, a substituted or unsubstituted arylsulfonyl group, a nitro group, a halogen atom, a substituted or unsubstituted alkoxycarbonyl group, a substituted or unsubstituted carbamoyl group or a substituted or unsubstituted sulfamoyl group, more preferably a hydrogen atom, an ionic hydrophilic group, a cyano group, a methanesulfonyl group, a phenylsulfonyl group, a nitro group, a halogen atom, a methoxycarbonyl group or a

carbamoyl group, still more preferably a hydrogen atom, an ionic hydrophilic group, a nitro group, a halogen atom or a cyano group, and most preferably a hydrogen atom, an ionic hydrophilic group, a nitro group or a halogen atom.

In formula (4), at least one of $X_1$, $X_2$, $X_3$, $X_4$ and $X_5$ is preferably an ionic hydrophilic group or an electron-withdrawing group having a Hammett's σp value of 0.3 or more, more preferably a halogen atom (preferably a chlorine atom), a nitro group, $-SO_3M$ or $-CO_2M$ (wherein M represents a hydrogen atom or a monovalent counter cation, and specific examples and preferred range thereof are the same as those of $M^{1a}$ and $M^{1b}$ above), still more preferably $-SO_3Li$ or $-CO_2Li$.

It is also preferred that the compound represented by any one of formulae (1) to (3) is a compound represented by the following formula (5):

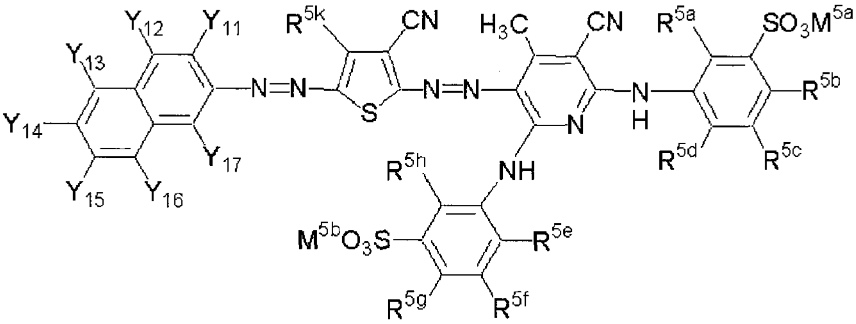

(5)

[In formula (5), each of $R^{5a}$ to $R^{5h}$ and $R^{5k}$ independently represents a hydrogen atom or a monovalent substituent, the substituents may combine with each other to form a ring, each of $M^{5a}$ and $M^{5b}$ independently represents a hydrogen atom or a monovalent counter cation, and each of $Y_{11}$ to $Y_{17}$ independently represents a hydrogen atom or a monovalent substituent.]

In formula (5), $R^{5a}$ to $R^{5h}$, $R^{5k}$, $M^{5a}$ and $M^{5b}$ have the same meanings as $R^{1a}$ to $R^{1h}$, $R^{1k}$, $M^{1a}$ and $M^{1b}$ in formula (1), and specific examples and preferred ranges are also the same.

In formula (5), each of $Y_{11}$ to $Y_{17}$ independently represents a hydrogen atom or a monovalent substituent. When each of $Y_{11}$ to $Y_{17}$ represents a monovalent substituent, the substituent includes a group selected from Substituent Group J.

Each of $Y_{11}$ to $Y_{17}$ is independently preferably a hydrogen atom, an ionic hydrophilic group, a cyano group, a substituted or unsubstituted alkylsulfonyl group, a substituted or unsubstituted arylsulfonyl group, a nitro group, a halogen atom, a substituted or unsubstituted alkoxycarbonyl group, a substituted or unsubstituted carbamoyl group or a substituted or unsubstituted sulfamoyl group, more preferably a hydrogen atom, an ionic hydrophilic group, a cyano group, a methanesulfonyl group, a phenylsulfonyl group, a nitro group, a halogen atom, a methoxycarbonyl group or a carbamoyl group, still more preferably a hydrogen atom, an ionic hydrophilic group, a nitro group, a halogen atom or a cyano group, and most preferably a hydrogen atom, an ionic hydrophilic group, a nitro group or a halogen atom.

In formula (5), at least one of $Y_{11}$ to $Y_{17}$ is preferably an ionic hydrophilic group or an electron-withdrawing group having a Hammett's σp value of 0.3 or more, more preferably a halogen atom (preferably a chlorine atom), a nitro group, $-SO_3M$ or $-CO_2M$ (wherein M represents a hydrogen atom or a monovalent counter cation, and specific examples and preferred range thereof are the same as those of $M^{1a}$ and $M^{1b}$ above), still more preferably $-SO_3Li$ or $-CO_2Li$.

It is also preferred that the compound represented by any one of formulae (1) to (3) is a compound represented by the following formula (6):

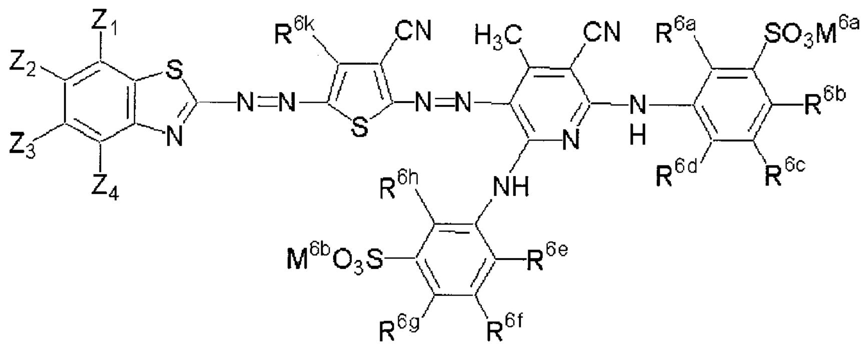

(6)

[In formula (6), each of $R^{6a}$ to $R^{6h}$ and $R^{6k}$ independently represents a hydrogen atom or a monovalent substituent, the substituents may combine with each other to form a ring, each of $M^{6a}$ and $M^{6b}$ independently represents a hydrogen atom or a monovalent counter cation, and each of $Z_1$ to $Z_4$ independently represents a hydrogen atom or a monovalent substituent.]

In formula (6), $R^{6a}$ to $R^{6h}$, $R^{6k}$, $M^{6a}$ and $M^{6b}$ have the same meanings as $R^{1a}$ to $R^{1h}$, $R^{1k}$, $M^{1a}$ and $M^{1b}$ in formula (1), and specific examples and preferred ranges are also the same.

In formula (6), each of $Z_1$, $Z_2$, $Z_3$ and $Z_4$ independently represents a hydrogen atom or a monovalent substituent. When each of $Z_1$, $Z_2$, $Z_3$ and $Z_4$ represents a monovalent substituent, the substituent includes a group selected from Substituent Group J.

Each of each of $Z_1$, $Z_2$, $Z_3$ and $Z_4$ is independently preferably a hydrogen atom, an ionic hydrophilic group, a cyano group, a substituted or unsubstituted alkylsulfonyl group, a substituted or unsubstituted arylsulfonyl group, a nitro group, a halogen atom, a substituted or unsubstituted alkoxycarbonyl group, a substituted or unsubstituted carbamoyl group or a substituted or unsubstituted sulfamoyl group, more preferably a hydrogen atom, an ionic hydrophilic group, a cyano group, a methanesulfonyl group, a phenylsulfonyl group, a nitro group, a halogen atom, a methoxycarbonyl group or a carbamoyl group, still more preferably a hydrogen atom, an ionic hydrophilic group, a nitro group, a halogen atom or a cyano group, and most preferably a hydrogen atom, an ionic hydrophilic group, a nitro group or a halogen atom.

In formula (6), at least one of $Z_1$, $Z_2$, $Z_3$ and $Z_4$ is preferably an ionic hydrophilic group or an electron-withdrawing group having a Hammett's σp value of 0.3 or more, more preferably a halogen atom (preferably a chlorine atom), a nitro group, $-SO_3M$ or $-CO_2M$ (wherein M represents a hydrogen atom or a monovalent counter cation, and specific examples and preferred range thereof are the same as those of $M^{1a}$ and $M^{1b}$ above), still more preferably $-SO_3Li$ or $-CO_2Li$.

The aqueous solution and the aqueous ink composition each using, as a colorant (coloring agent), the azo compound represented by any one of formulae (1) to (6) of the present invention mean a composition containing a color material such as dye or pigment and a dispersant (e.g., solvent) therefor and can be suitably used in particular for image formation.

The compound represented by any one of formulae (1) to (6) preferably has a maximum absorption wavelength (λmax) of 550 to 700 nm, more preferably from 550 to 650 nm, still more preferably from 570 to 650 nm, in the absorption spectrum measured using water as a solvent.

Also, the compound represented by any one of formulae (1) to (6) preferably has three or more ionic hydrophilic groups, more preferably from 3 to 6 ionic hydrophilic groups, still more preferably 4 or 5 ionic hydrophilic groups. This configuration produces an effect that water solubility of the azo compound of the present invention as well as storage stability of the aqueous solution are enhanced, the required performance as a water-soluble dye for black ink in inkjet recording is satisfied at a high level and when used as an ink for inkjet recording, the image quality of an inkjet printed matter can be more improved.

In the azo compound represented any one of formulae (1) to (6), at least one of $M^{1a}$ and $M^{1b}$, of $M^{2a}$ and $M^{2b}$, of $M^{3a}$ and $M^{3b}$, of $M^{4a}$ and $M^{4b}$, of $M^{5a}$ and $M^{5b}$, or of $M^{6a}$ and $M^{6b}$ is preferably a lithium ion, and it is more preferred that both of each pair are a lithium ion.

In the present invention, the compound represented by any one of formulae (1) to (6) can be applied even when an isotope (for example, $^{2}H$, $^{3}H$, $^{13}C$ or $^{15}N$) is contained therein.

Specific examples of the compound represented by any one of formulae (1) to (6) are illustrated below, but the present invention is not limited to these examples.

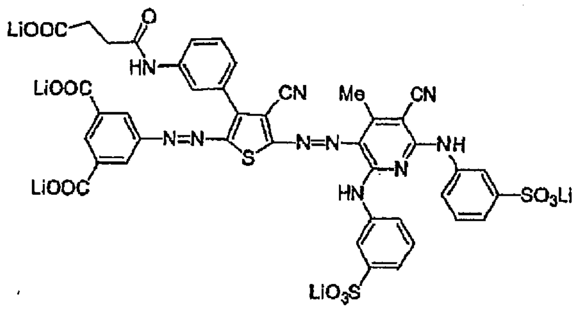

Compound 1

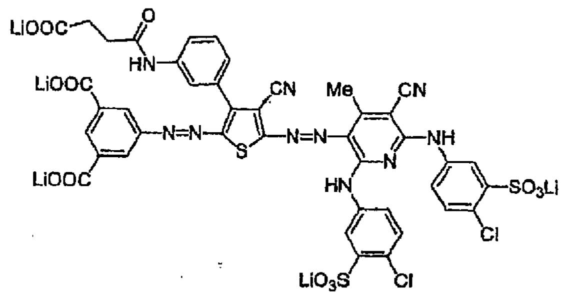

Compound 2

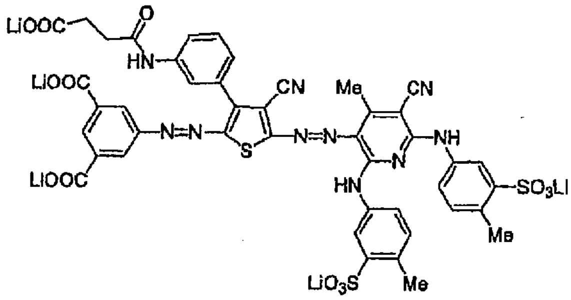

Compound 3

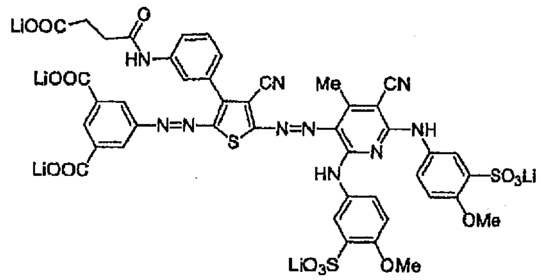

Compound 4

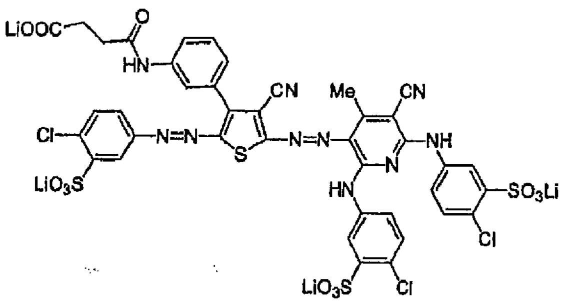

Compound 5

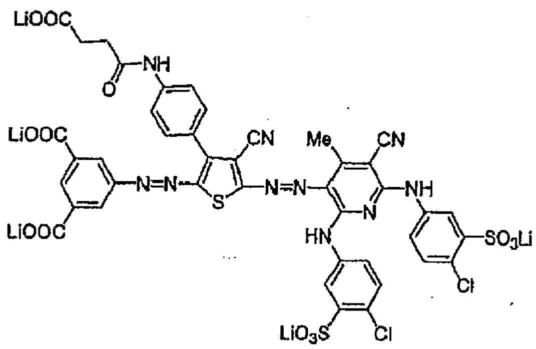

Compound 6

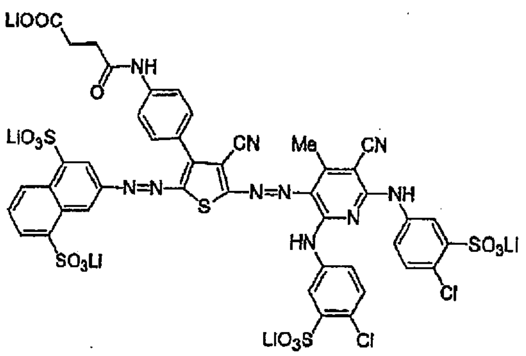

Compound 7

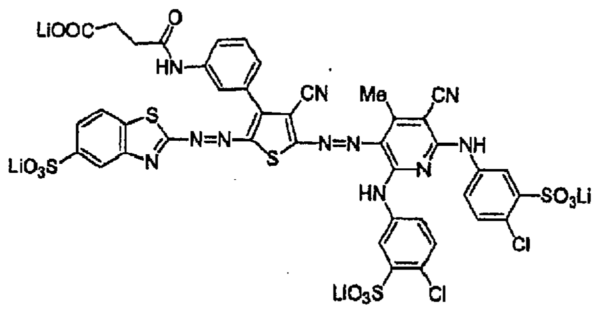

Compound 8

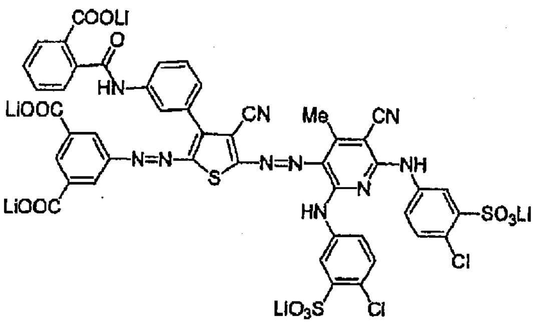

Compound 9

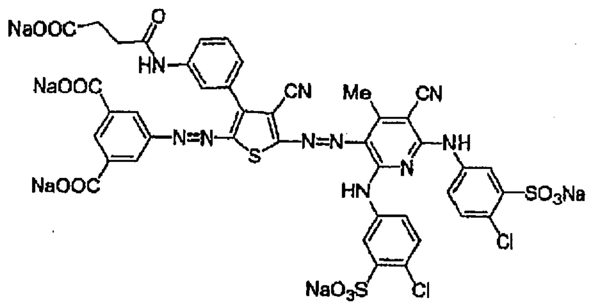

Compound 10

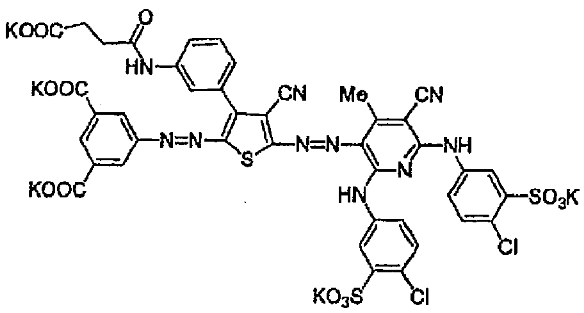

Compound 11

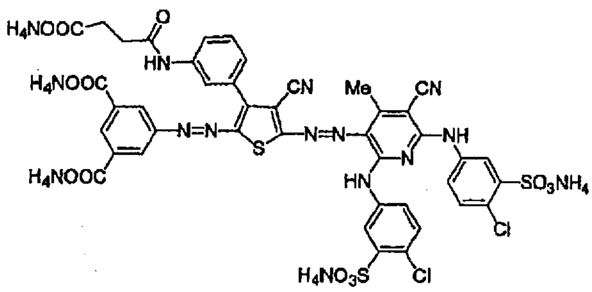

Compound 12

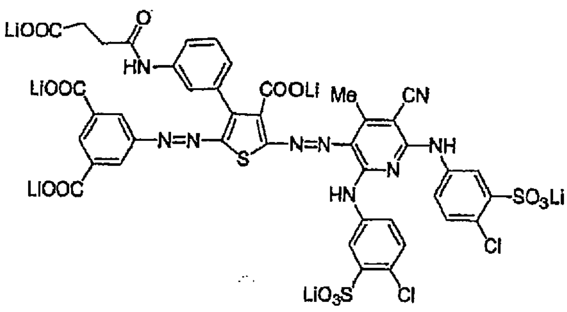

Compound 13

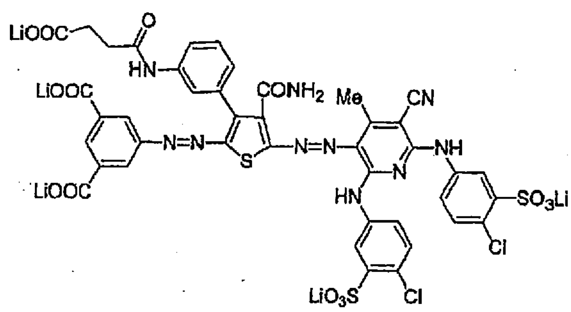

Compound 14

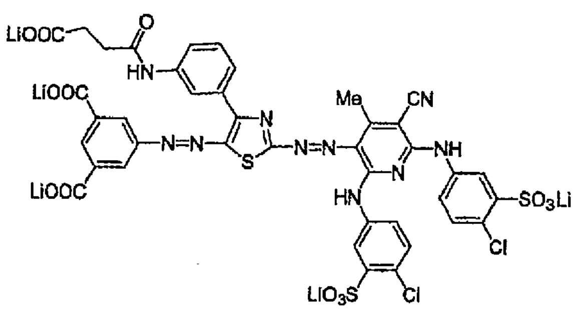

Compound 15

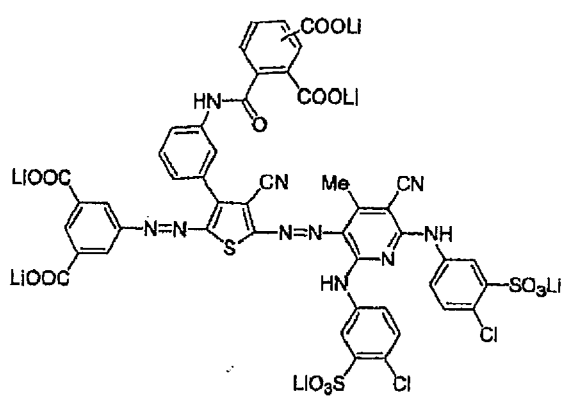

Compound 16

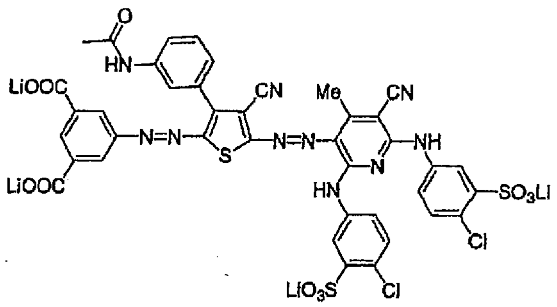

Compound 17

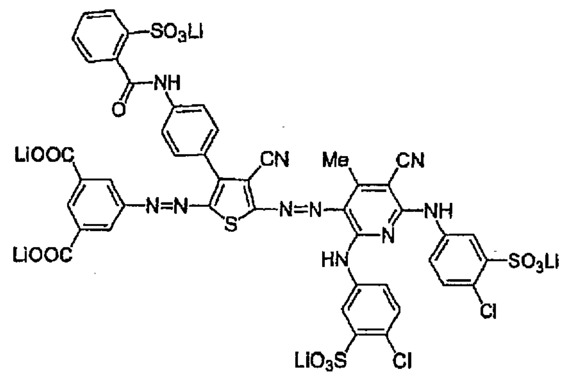

Compound 18

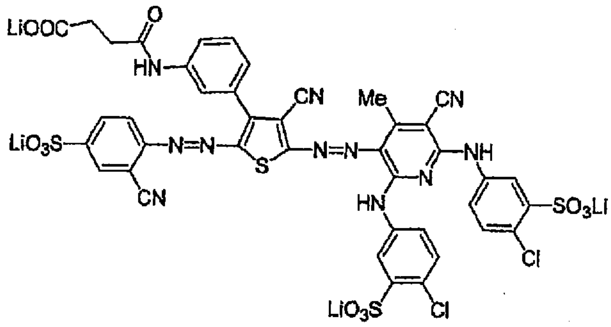

Compound 19

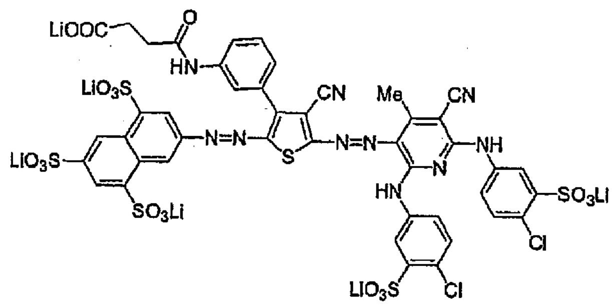

Compound 20

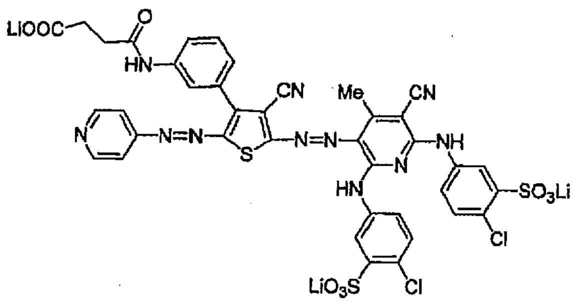

Compound 21

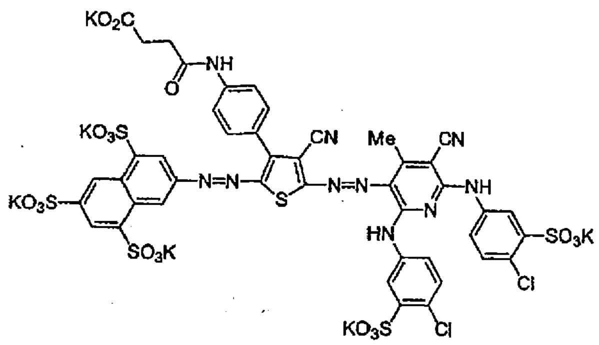

Compound 22

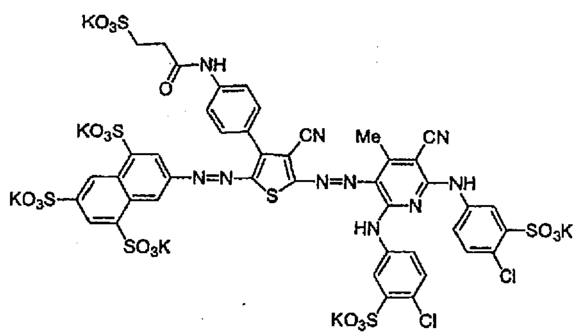

Compound 23

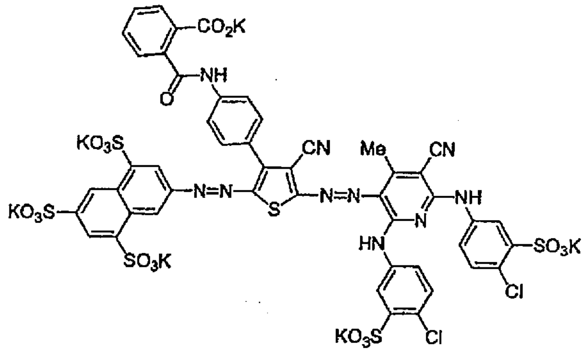

Compound 24

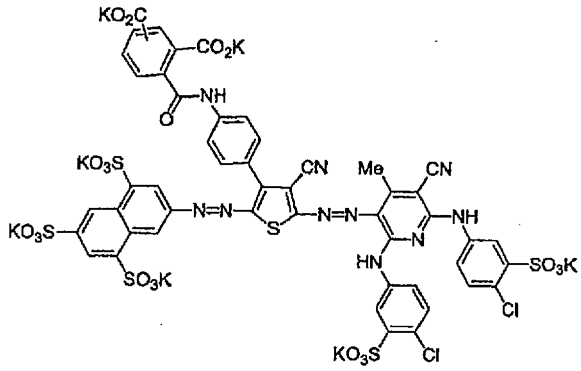

Compound 25

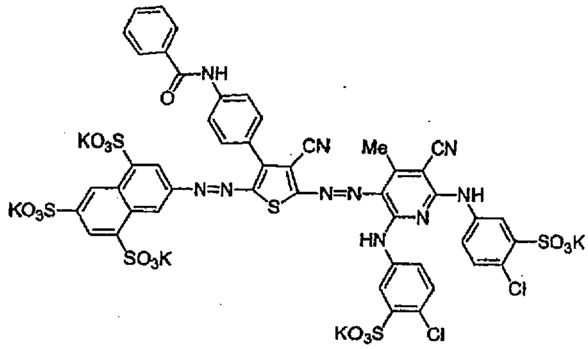

Compound 26

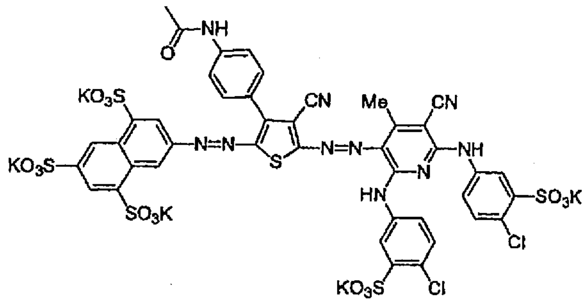

Compound 27

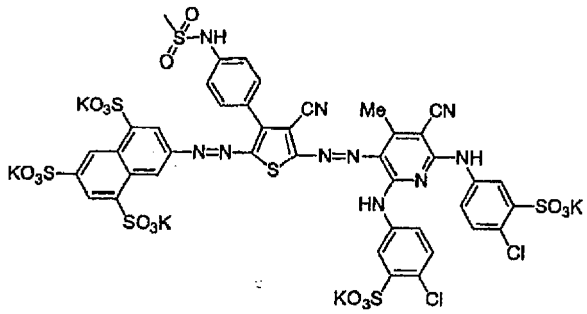

Compound 28

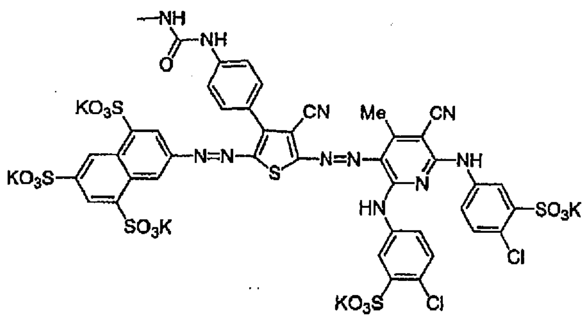

Compound 29

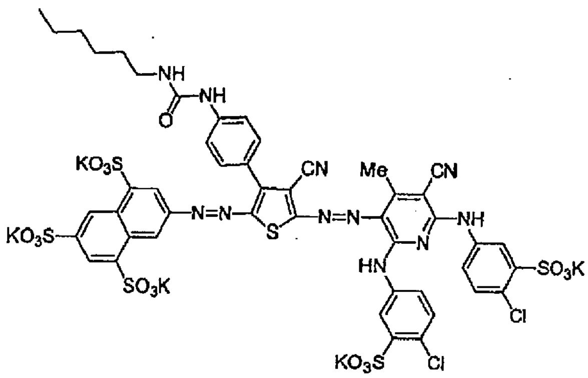

Compound 30

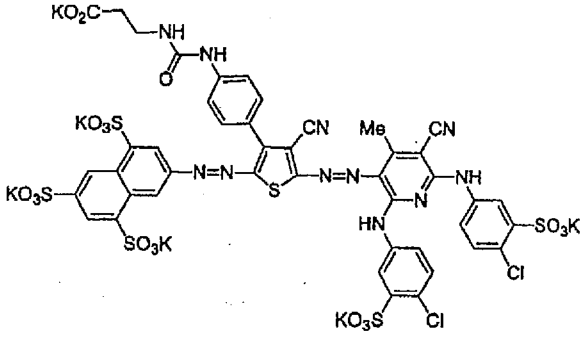

Compound 31

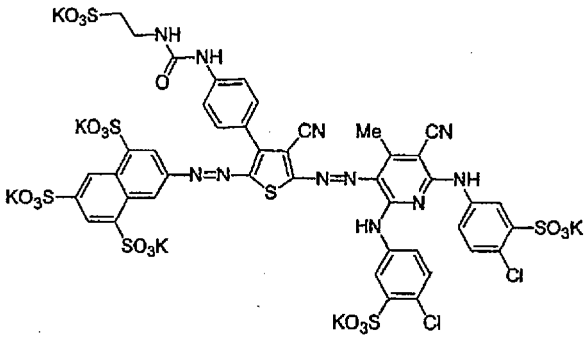

Compound 32

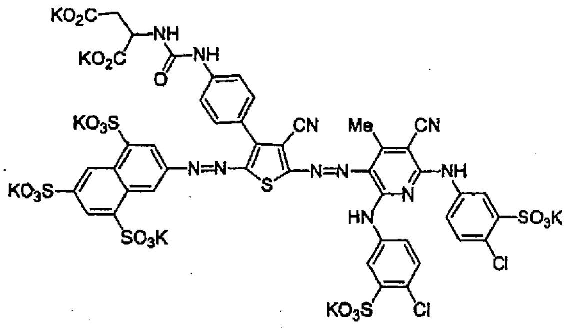

Compound 33

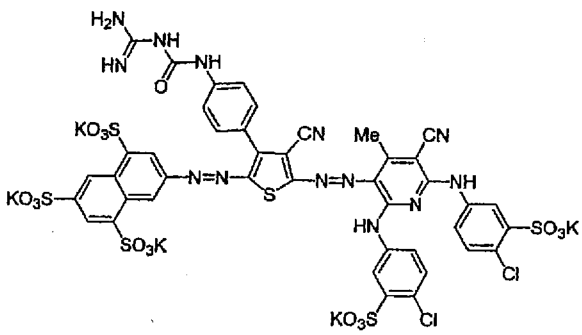

Compound 34

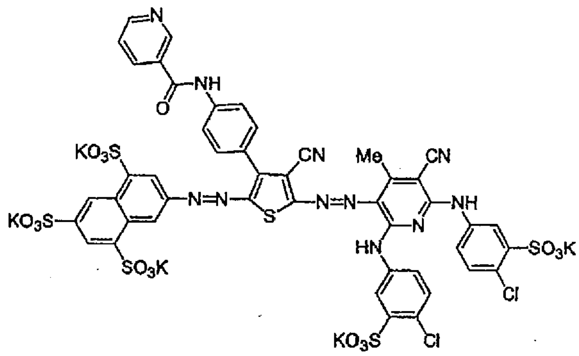

Compound 35

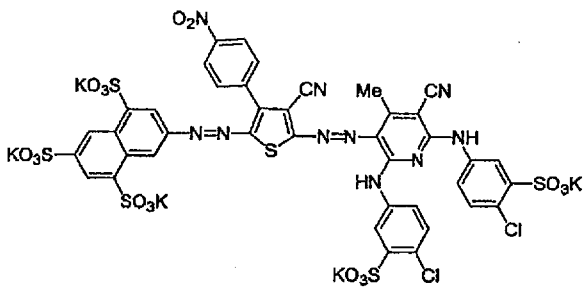

Compound 36

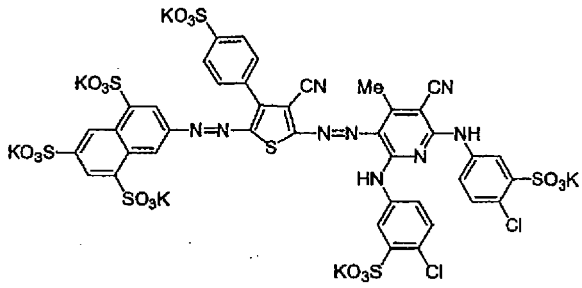

Compound 37

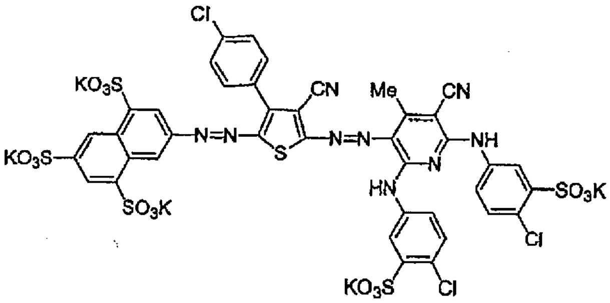

Compound 38

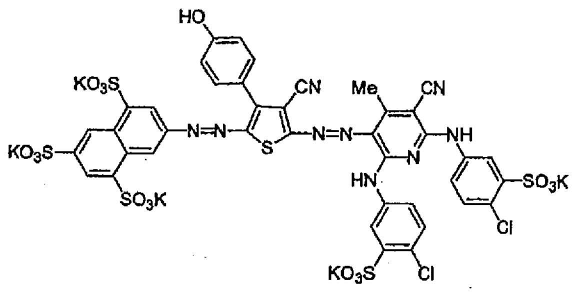

Compound 39

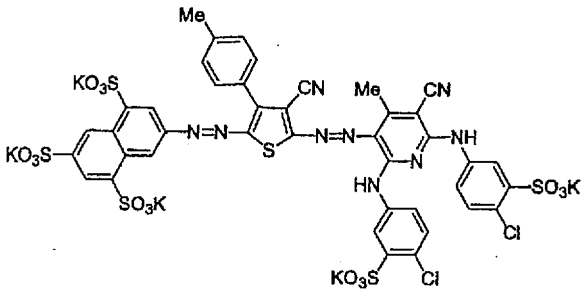

Compound 40

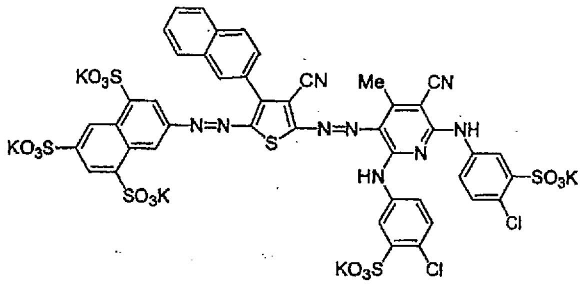

Compound 41

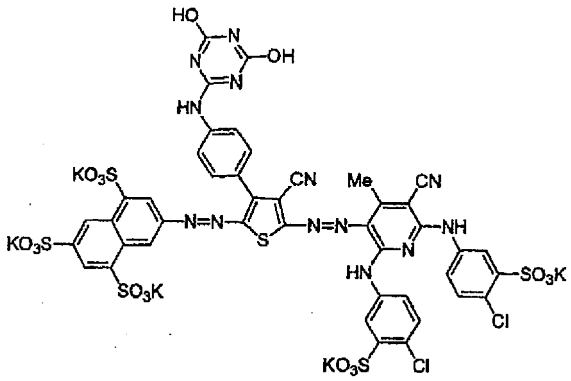

Compound 42

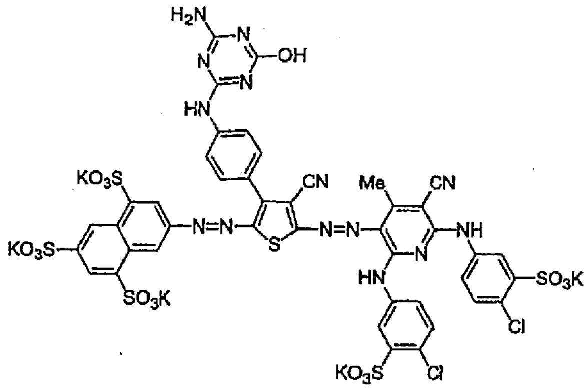

Compound 43

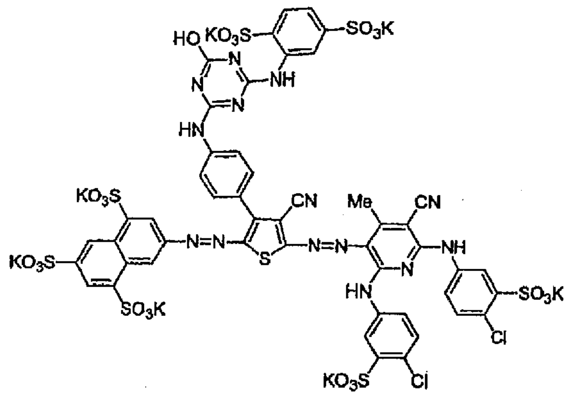

Compound 44

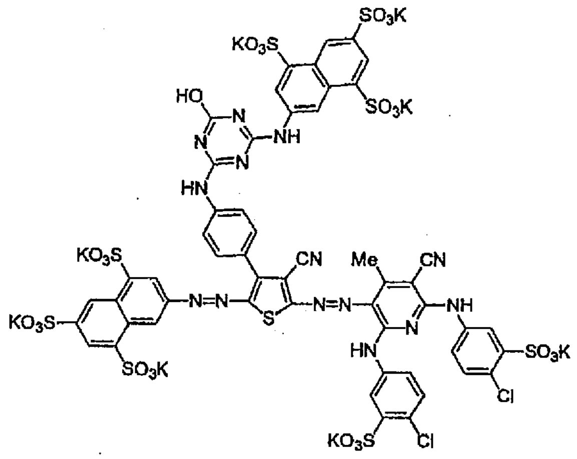

Compound 45

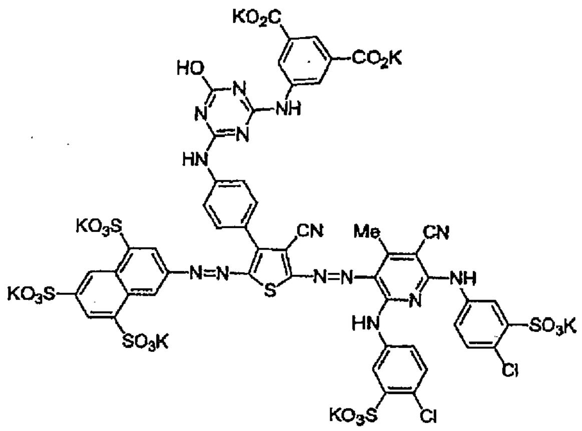

Compound 46

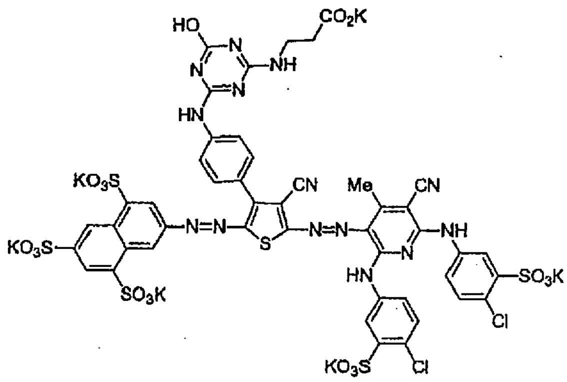

Compound 47

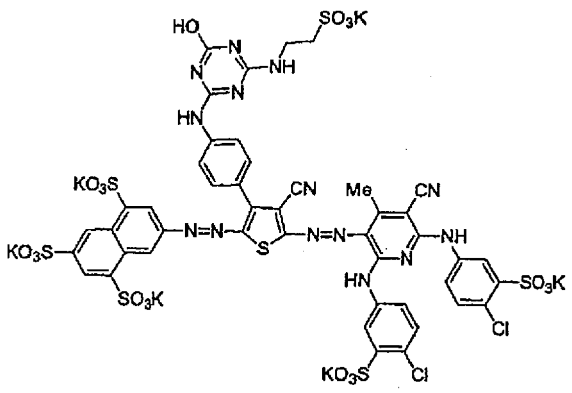

Compound 48

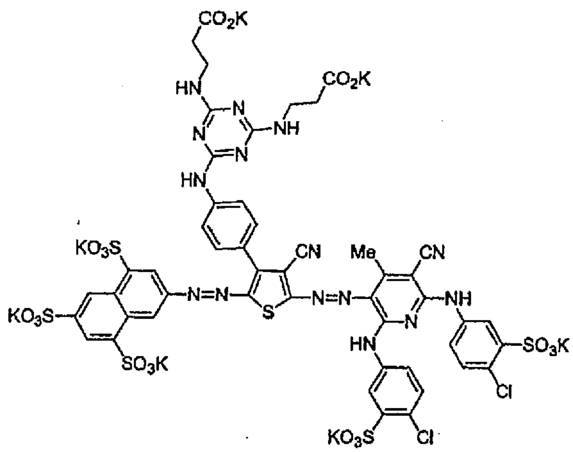

Compound 49

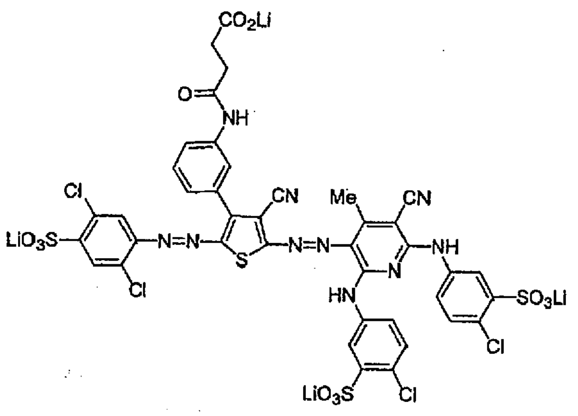

Compound 50

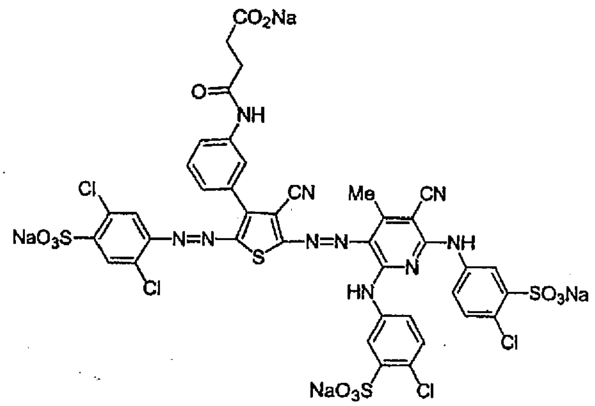

Compound 51

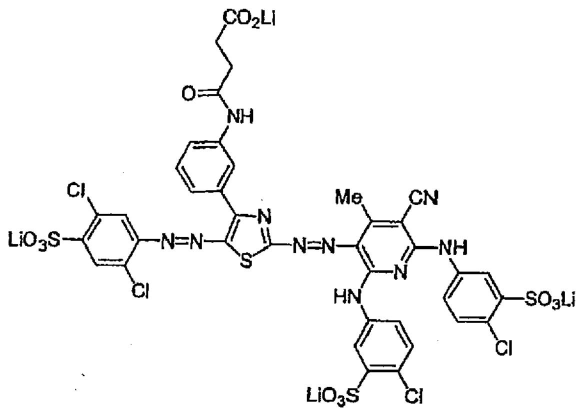

Compound 52

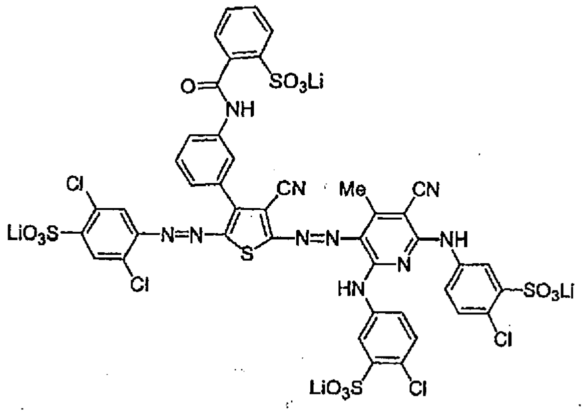

Compound 53

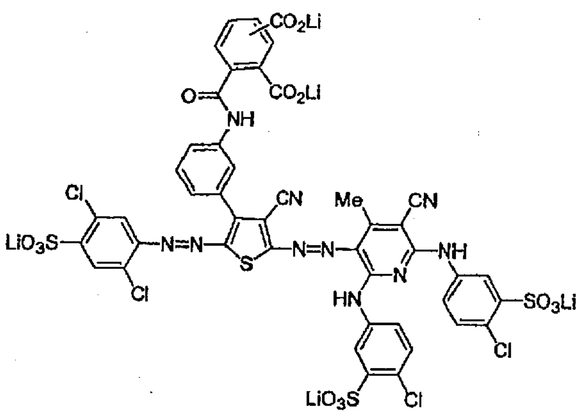

Compound 54

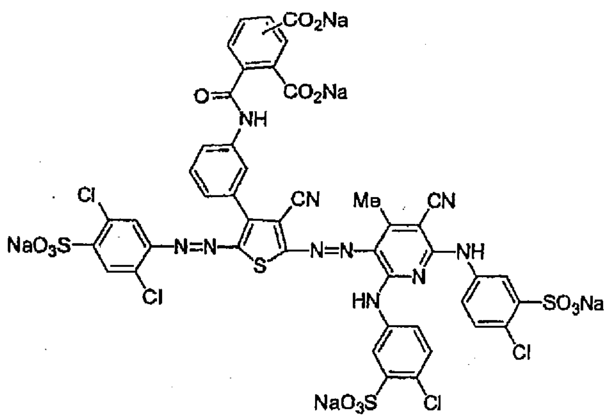

Compound 55

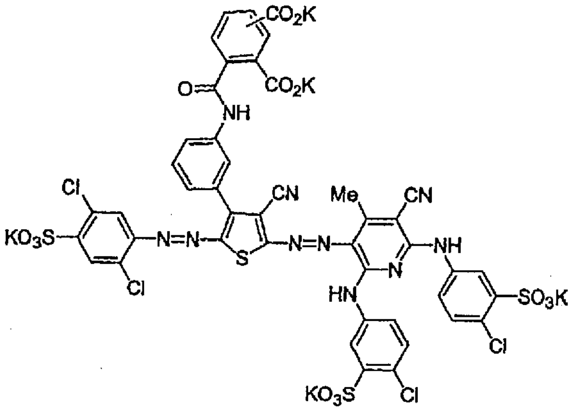

Compound 56

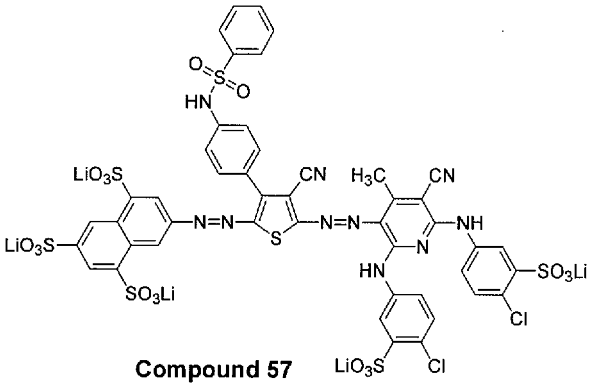

**Compound 57**

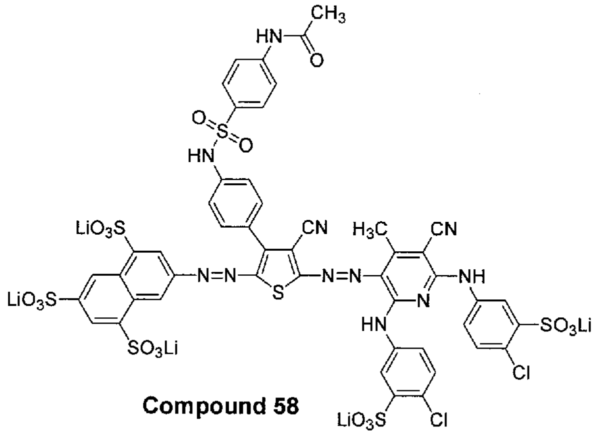

**Compound 58**

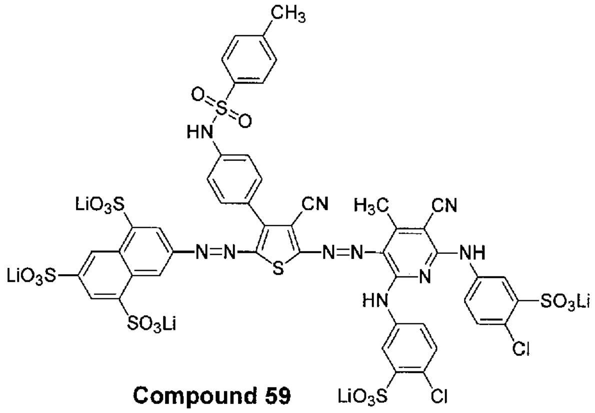

**Compound 59**

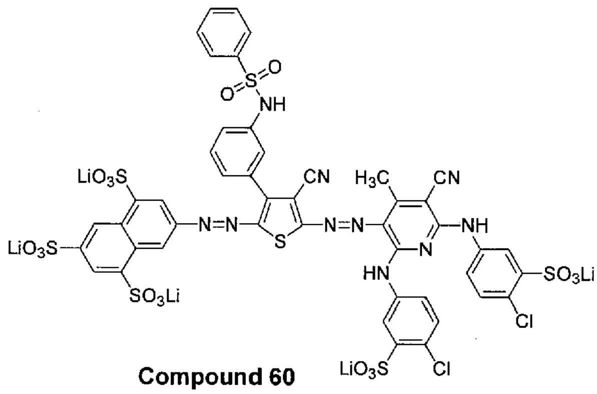

**Compound 60**

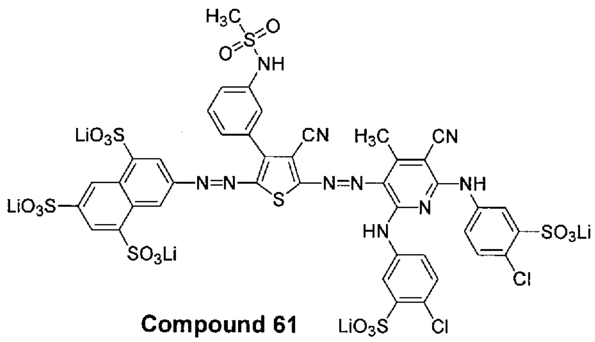

**Compound 61**

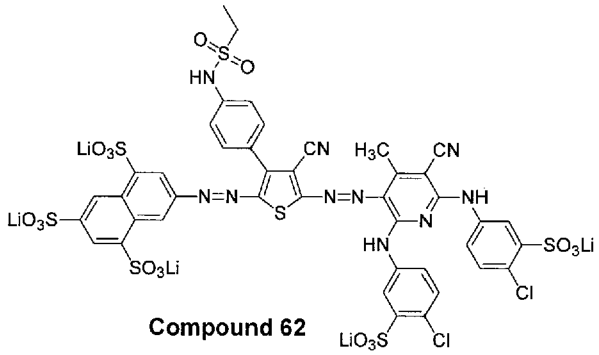

**Compound 62**

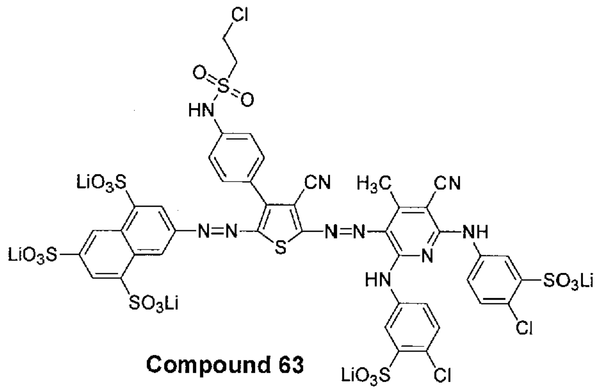

**Compound 63**

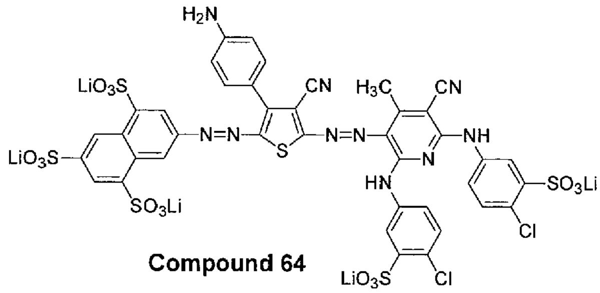

**Compound 64**

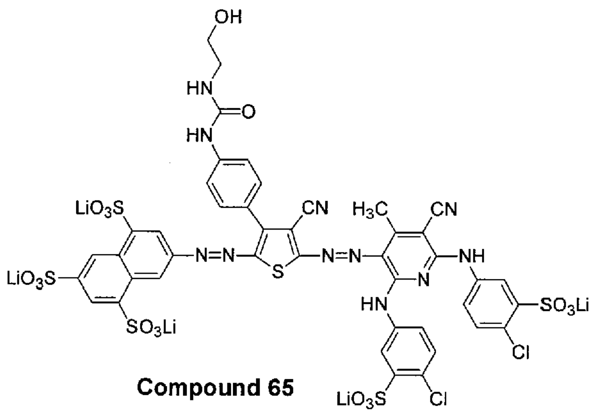

**Compound 65**

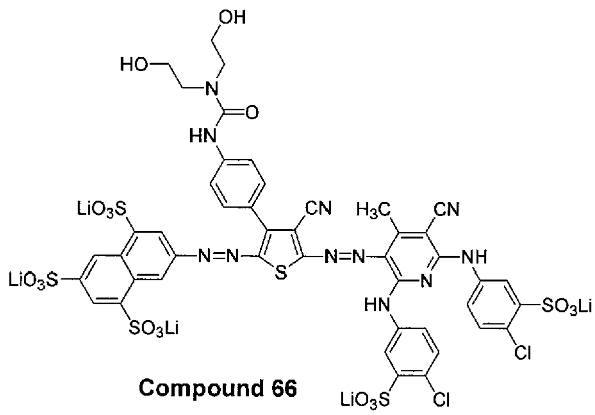

**Compound 66**

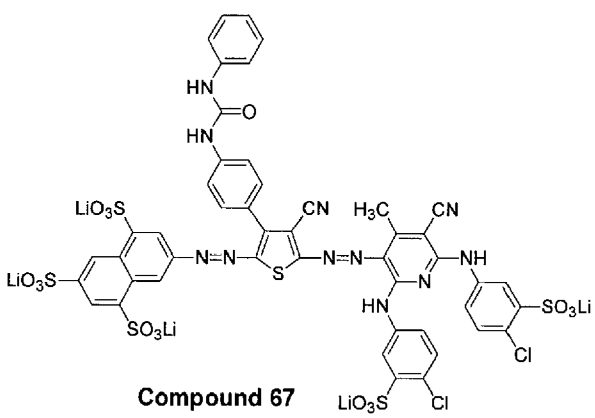

**Compound 67**

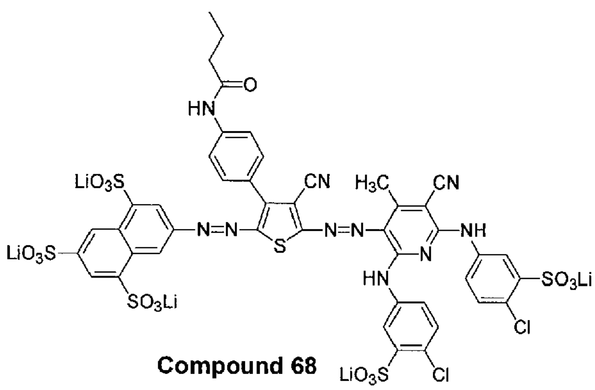

**Compound 68**

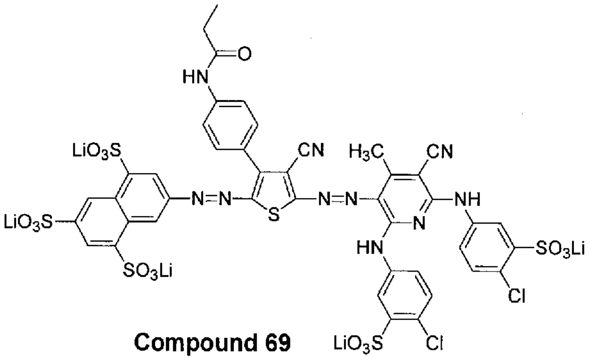

**Compound 69**

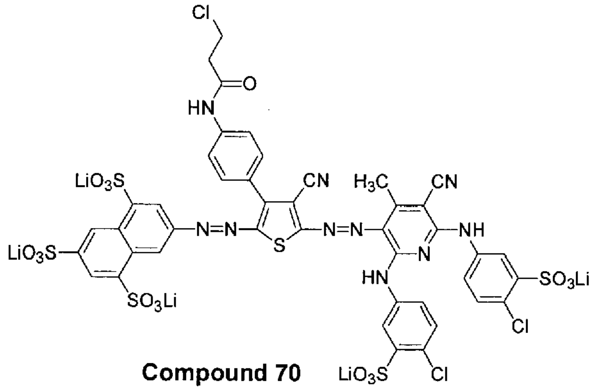

**Compound 70**

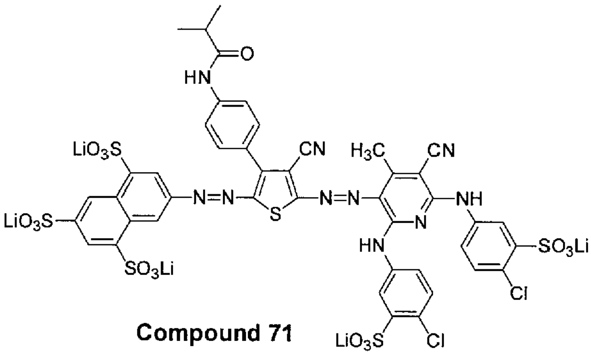

**Compound 71**

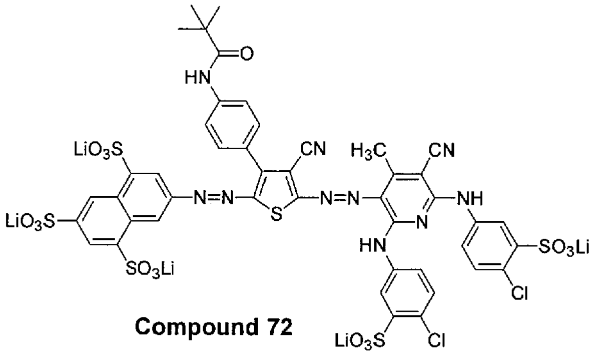

**Compound 72**

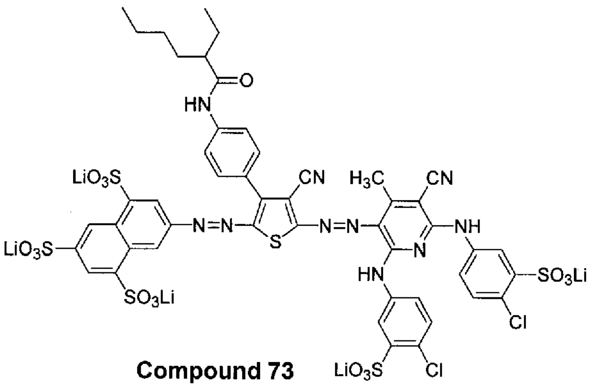

**Compound 73**

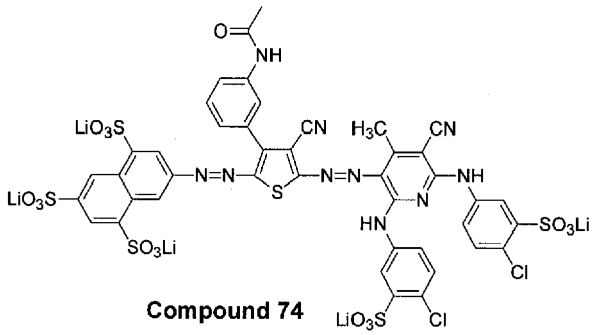

**Compound 74**

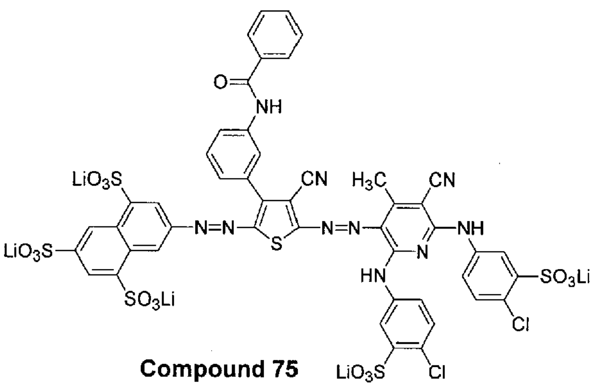

**Compound 75**

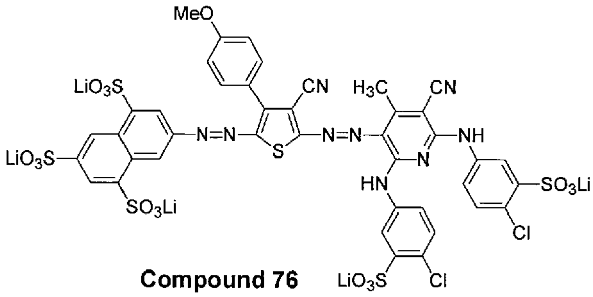

**Compound 76**

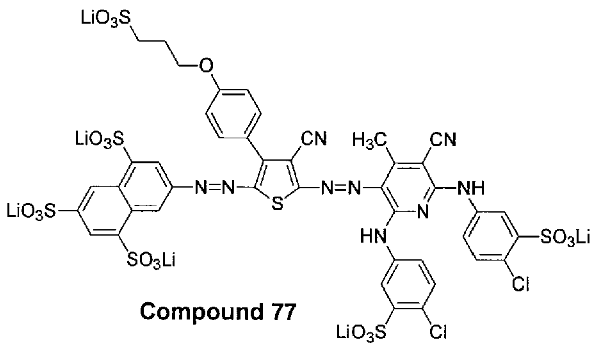

**Compound 77**

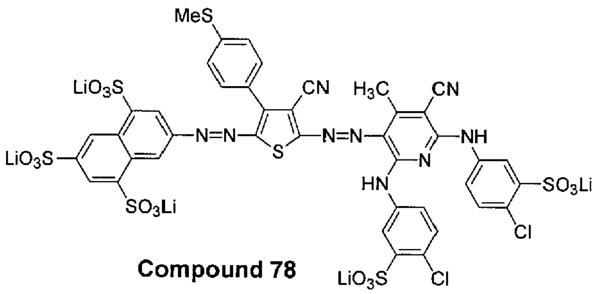

**Compound 78**

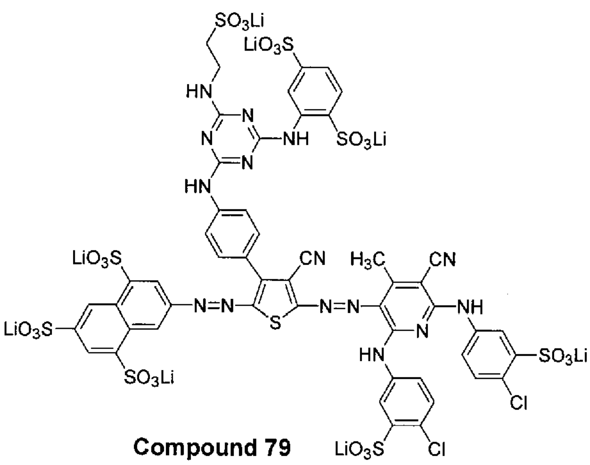

**Compound 79**

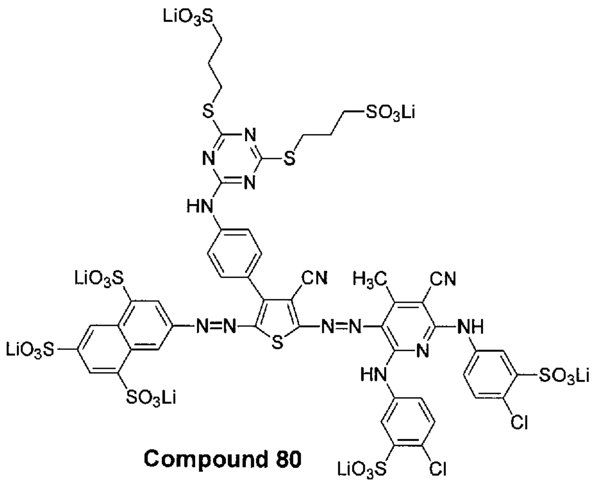

**Compound 80**

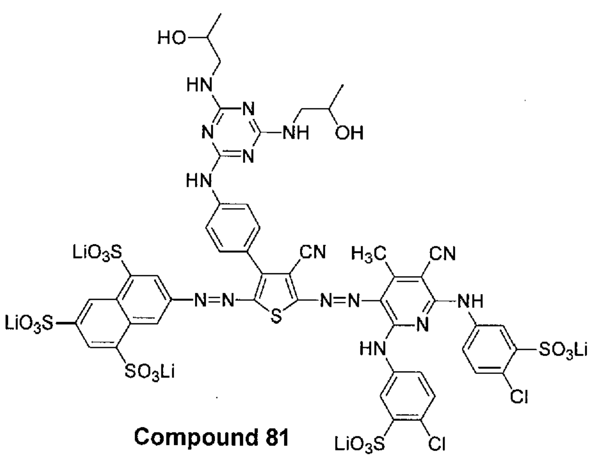

**Compound 81**

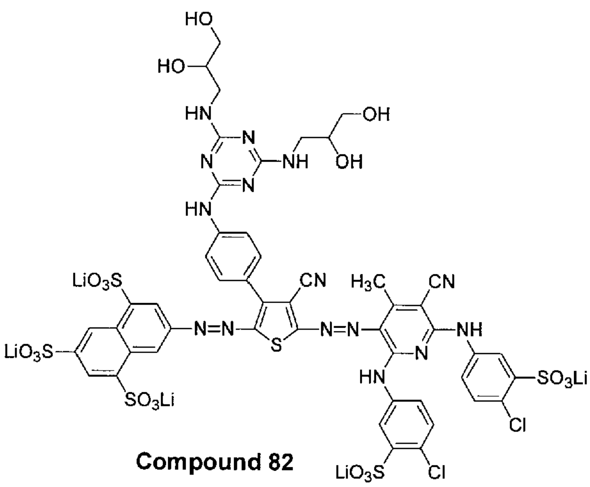

**Compound 82**

The azo compound represented by formula (1) can be synthesized by a coupling reaction of a diazo component and a coupler, and this is described in JP-A-2003-306623 and JP-A-2005-139427.

<Aqueous Solution>

The aqueous solution of the present invention comprises (a) a preservative and (b) at least one member of the compound represented by formula (1) and a salt thereof, wherein the content of (b) is from 1 to 25 mass%.

[(a) Preservative]

The aqueous solution may be subject to a problem of production of an insoluble material attributable to putrefaction. In order to prevent this problem, a preservative is added to the aqueous solution of the present invention.
As the preservative usable in the present invention, various preservatives can be used.
The preservative includes, first, a heavy metal ion-containing inorganic preservative (for example, a silver ion-containing preservative) and salts thereof. The organic preservative that can be used includes various preservatives such as quaternary ammonium salt (e.g., tetrabutylammonium chloride, cetylpyridinium chloride, benzyltrimethylammonium chloride), phenol derivative (e.g., phenol, cresol, butylphenol, xylenol, bisphenol), phenoxy ether derivative (e.g., phenoxyethanol), heterocyclic compound (e.g., benzotriazole, PROXEL, 1,2-benzoisothiazolin-3-one), acid amides, carbamic acid, carbamates, amidine/guanidines, pyridines (e.g., sodium pyridinethione-1-oxide), diazines, triazines, pyrrole/imidazoles, oxazole/oxazines, thiazole/thiadiazines, thioureas, thiosemicarbazides, dithiocarbamates, sulfides, sulfoxides, sulfones, sulfamides, antibiotics (e.g., penicillin, tetracycline), sodium dehydroacetate, sodium benzoate, ethyl p-hydroxybenzoate and salt thereof. Furthermore, those described, for example, in Bokin Bobai Handbook (Handbook of Microbicides and Fungicides) (Gihodo, 1986,) and Bokin Bobai Zai Jiten (Dictionary of Microbicides and Fungicides) (compiled by SAAAJ) may be also used as the preservative.
The preservative is preferably a phenol derivative or a heterocyclic compound, more preferably a heterocyclic compound, still more preferably a heterocyclic compound (PROXEL XL-II, PROXEL GXL (S)).
One preservative may be added alone, or two or more preservatives may be combined and added to the aqueous solution. Preservatives of various types such as oil-soluble structure and water-soluble structure may be used, but a water-soluble preservative is preferred.
Among them, at least one preservative is preferably a heterocyclic compound. In the present invention, when two or more preservatives are used in combination, the effect of the present invention is more successfully exerted. Preferred examples thereof include a combination of a heterocyclic compound and an antibiotic, and a combination of a heterocyclic compound and a phenol derivative. In the case of combining two preservatives, the content ratio therebetween is not particularly limited but is preferably preservative A (heterocyclic compound)/preservative B (phenol derivative) of 0.01 to 100 (by mass).
The amount of the preservative added to the aqueous solution may be set in a wide range but is preferably from 0.001 to 10 mass%, more preferably from 0.1 to 5 mass%. The preservative content in this range is effective for suppressing microbial growth in the aqueous solution.

[(b) Azo Compound Represented by Formula (1)]

The (b) azo compound represented by formula (1) in the aqueous solution of the present invention is as described above.

[(c) pH Adjusting Agent]

The aqueous solution of the present invention may further contain (c) a pH adjusting agent.
A neutralizing agent (organic base, inorganic alkali) may be used as the pH adjusting agent. For the purpose of enhancing the storage stability of the ink for inkjet recording, the pH adjusting agent is preferably added such that the ink for inkjet recording has a pH of 7.0 to 9.0, more preferably a pH of 7.5 to 8.5.
A desired pH can be achieved by adjusting the content of the pH adjusting agent.
The pH adjusting agent includes a basic compound such as organic base and inorganic base, and an acidic compound such as organic acid and inorganic acid.
As the basic compound, an inorganic compound such as sodium hydroxide, potassium hydroxide, lithium hydroxide, sodium carbonate, potassium carbonate, lithium carbonate, sodium hydrogencarbonate (sodium bicarbonate), potassium hydrogencarbonate, lithium hydrogencarbonate, sodium acetate, potassium acetate, sodium phosphate and sodium monohydrogenphosphate, an organic base such as aqueous ammonia, methylamine, ethylamine, diethylamine, triethylamine, ethanolamine, diethanolamine, triethanolamine, ethylenediamine, piperidine, diazabicyclooctane,

diazabicycloundecene, pyridine, quinoline, picoline, lutidine and collidine, and an alkali metal salt of an organic acid, such as lithium benzoate and potassium phthalate, may be also used.

As the acidic compound, an inorganic compound such as hydrochloric acid, sulfuric acid, phosphoric acid, boric acid, sodium hydrogensulfate, potassium hydrogensulfate, potassium dihydrogenphosphate and sodium dihydrogenphosphate, and an organic compound such as acetic acid, tartaric acid, benzoic acid, trifluoroacetic acid, methanesulfonic acid, ethanesulfonic acid, benzenesulfonic acid, p-toluenesulfonic acid, saccharic acid, phthalic acid, picolinic acid and quinolinic acid, may be also used.

The pH adjusting agent is preferably sodium hydrogencarbonate, potassium hydrogencarbonate or lithium hydrogencarbonate, more preferably sodium hydrogencarbonate or lithium hydrogencarbonate, still more preferably lithium hydrogencarbonate.

Also, in the present invention, another coloring agent may be used in combination with the azo compound represented by formula (1) so as to adjust the color to a more preferred hue. As the dye used in combination, an arbitrary dye (for example, a yellow dye, a magenta dye and a cyan dye) may be used. Examples of the yellow dye may include an aryl or heteryl azo dye having, as a coupling component (hereinafter, referred to as "coupler component"), heterocyclic rings such as substituted benzenes, substituted naphthalenes, pyrazolone and pyridone, or chain-opening active methylene compounds; an azomethine dye having chain-opening active methylene compounds as the coupler component; a methine dye such as benzylidene dye and monomethine oxonol dye; and a quinone-based dye such as naphthoquinone dye and anthraquinone dye. Other examples of the dye species include a quinophthalone dye, a nitro/ nitroso dye, an acridine dye, and an acridinone dye. Particularly, the dye that is preferably used in combination is a dye (S) having a $\lambda$max at 350 to 500 nm, and the yellow dyes described above and later may be used, but, among others, an azo dye having 2 to 6 azo groups per molecule is preferred. Incidentally, a yellow pigment may be also used in the present invention.

Examples of the magenta dye include an aryl or heteryl azo dye having, as the coupler component, heterocyclic rings such as phenols, naphthols, anilines, pyridines and pyrazines, or chain-opening active methylene compounds; an azomethine dye having chain-opening active methylene compounds as the coupler component; and an anthrapyridone dye. Among others, an azo dye or anthrapyridone dye having a heterocyclic ring in the chromophore is preferred.

Examples of the cyan dye include an aryl or heteryl azo dye having, as the coupler component, phenols, naphthols, anilines or the like; an azomethine dye having, as the coupler component, heterocyclic rings such as phenols, naphthols and pyrrolotriazole; a polymethine dye such as cyanine dye, oxonol dye and merocyanine dye; a carbonium dye such as diphenylmethane dye, triphenylmethane dye and xanthene dye; a phthalocyanine dye; an anthraquinone dye; and an indigo/thioindigo dyes. Among others, a phthalocyanine dye is preferred.

In particular, a dye having an oxidation potential higher than 1.0 V or an associative dye is preferred in view of fastness balance. Specific preferred examples of the dye used in combination include dyes described in JP-A-2005-146244.

As the heterocyclic azo dye, known yellow dyes and magenta dyes may be used, in addition to the compound represented by formula (1). These heterocyclic azo dyes, that is, yellow dyes and magenta dyes, preferably have at least one of the above-described features (oxidation potential, associative property), more preferably have all features. The oxidation potential of these dyes is more preferably higher than 1.1 V (vs SCE), still more preferably higher than 1.15 V (vs SCE).

Examples of the yellow dye that is the heterocyclic azo dye include those described in JP-A-2004-83903 (paragraphs [0048] to [0062]), JP-A-2003-277661 (paragraphs [0041] to [0050]), JP-A-2003-277662 (paragraphs [0042] to [0047]) and US Patent Application Publication US2003/0213405 (paragraph [0108]).

In the aqueous solution of the present invention, the main solvent is water, and the water content in all solvents is preferably from 50 to 100 mass%, more preferably from 60 to 100 mass%. Also, the aqueous solution of the present invention may contain a water-miscible organic solvent and a lipophilic medium, in addition to water.

In the aqueous solution of the present invention, the (b) azo compound represented by formula (1) or a salt thereof is dissolved or dispersed, preferably dissolved, in a solvent.

In the aqueous solution of the present invention, the content of the (b) azo compound represented by formula (1) or a salt thereof is from 1 to 25 mass%, preferably from 2 to 20 mass%, more preferably from 2 to 15 mass%, based on the total mass of the aqueous solution. When the content of (b) is in the range above, there is an effect that storage stability of the aqueous solution is good and at the same time, preparation of a water-soluble ink for inkjet recording is easy.

The aqueous solution of the present invention preferably has a pH at 25°C of 7.0 to 9.0, more preferably from 7.5 to 8.5. When the pH is in this range, there is an effect that high solution stability of the azo compound in the aqueous solution can be imparted and preparation of a water-soluble ink for inkjet recording is easy.

The aqueous solution of the present invention is sometimes referred to as "ink stock solution".

In the case where the azo compound represented by formula (1) is water-soluble, the aqueous solution of the present invention is preferably prepared by dissolving the compound in an aqueous medium, and in the case where the azo compound represented by formula (1) is oil-soluble, the aqueous solution is preferably prepared by dissolving and/or dispersing the compound in an aqueous medium and a lipophilic medium. The aqueous medium is a solvent mainly

composed of water and contains an organic solvent such as water-miscible organic solvent, if desired. The organic solvent may have a function as a viscosity reducing agent. The lipophilic medium is a solvent mainly composed of an organic solvent. The water-miscible organic solvent and the lipophilic medium are described later.

At the production of the aqueous solution, a step of removing dust as a solid matter by filtration (filtering step) is preferably added. This operation uses a barrier filter and as the barrier filter here, a filter having an effective diameter of 1 μm or less, preferably 0.3 μm or less, is used. As the material of the filter, various materials may be used, but particularly, in the case where the aqueous solution contains a water-soluble dye, a filter produced for an aqueous solvent is preferably used. Among others, it is preferred to use a jacket-type filter made of a polymer material that is less likely to produce a waste. As for the filtration method, the solution may be transferred to pass through a jacket, and either method of pressure filtration or vacuum filtration may be also utilized.

In the present invention, a viscosity reducing agent may be used and in this case, the filtration treatment may be performed without resistance.

In the step of preparing the aqueous solution or in the solution preparation step, as the method for dissolving the dye or other components, various methods such as dissolution by stirring, dissolution by radiation with ultrasonic wave and dissolution by shaking may be used. Among these, the stirring method is preferably used. In the case of performing stirring, various systems such as fluidization stirring and stirring through use of shearing force by utilizing a reversing agitator or a dissolver, which are known in this field, may be employed. On the other hand, a stirring method utilizing the shearing force with a vessel bottom, such as magnetic agitator, may be also preferably employed.

The aqueous solution of the present invention is not particularly limited in its use but is preferably used as an ink composition and more preferably used in an ink for inkjet recording.

[Ink Composition]

The ink composition of the present invention contains the above-described aqueous solution of the present invention.

The content of the compound represented by formula (1) in the ink composition is preferably from 0.2 to 20 mass%, more preferably from 0.5 to 10 mass%, still more preferably from 1.0 to 8.0 mass%.

The ink composition of the present invention preferably contains all dyes in an amount of 0.2 to 20 mass%, more preferably from 0.5 to 10 mass%, still more preferably from 1.0 to 8.0 mass%.

The ink composition of the present invention is preferably adjusted by a pH adjusting agent to a pH at 25°C of 7.0 to 10.0, more preferably a pH of 7.5 to 9.5. When the pH is 7.5 or more, solubility of the dye is enhanced and nozzle blocking can be prevented. Also, when the pH is 9.5 or less, there is a tendency that the long-term storage stability of the ink is excellent.

The pH adjusting agent for use in the ink composition includes those used for the aqueous solution of the present invention and is preferably lithium hydrogencarbonate, sodium hydrogencarbonate or potassium hydrogencarbonate, more preferably lithium hydrogencarbonate or sodium hydrogencarbonate.

The ink composition of the present invention is not particularly limited in its use and may be preferably used for the preparation of an ink composition for printing such as inkjet printing, an ink sheet in a heat-sensitive recording material, a color toner for electrophotography, a color filter used in a display such as LCD or PDP or in an imaging device such as CCD, or a dying solution for dying various fibers, but among others, is preferably an ink composition for inkjet recording.

As for the production method of the ink composition, the above-described aqueous solution can be used.

The ink composition is suitably a black ink but should not be limited to a black ink and may be used as an ink of an arbitrary color by mixing it with another dye or pigment.

The production method of the ink composition contains a step of producing a desired ink composition with a viscosity in the above-described range by using at least the aqueous solution (hereinafter, sometimes referred to as solution preparation step).

The solution preparation step is a step of preparing an ink composition having a specific viscosity and a desired use by using the aqueous solution obtained as above, and the ink composition may be a final product or an intermediate product. This solution preparation step includes at least a step of diluting the aqueous solution with a medium, preferably an aqueous medium. The aqueous solution containing an oil-soluble dye has no particular limitation on the medium used in this dilution step, but the aqueous solution is preferably emulsion-dispersed in an aqueous medium to prepare an aqueous ink composition. The medium may contain various components at required concentrations, the components may be added separately to the aqueous solution, or both may be combined.

The ink composition produced according to the present invention is produced using the aqueous solution having a high dye concentration and therefore, is more improved in the dye solubility than an ink composition produced by a conventional method and in turn, improved in the ejection stability.

The dye used in the aqueous solution and ink composition of the present invention is described below. The

dye is not particularly limited, but it is preferred to contain at least one azo compound represented by formula (1) wherein the λmax is present at 500 to 700 nm and the half-value width ($W\lambda,_{1/2}$) in an absorption spectrum of a dilute solution standardized to an absorbance of 1.0 is 100 nm or more, preferably from 120 to 500 nm, more preferably from 120 to 350 nm.

In the case where the azo compound represented by formula (1) can independently realize "(dense) black" of high image quality, that is, black which does not relay on a light source for observation and is less likely to emphasize any one color tone of B, G and R, the dye may be used alone as a material for the aqueous solution or ink composition, but usually, in the ink composition, the dye is generally used in combination with a dye capable of covering the region where the dye above has a low absorption. In the case of the ink composition using the azo compound represented by formula (1), it is usually preferred to use the compound in combination with another dye having a main absorption in a yellow region (λmax of 350 to 500 nm). The ink composition may be also produced by using still other dyes.

The another dye may be used in the aqueous solution but in view of storage stability, is preferably used by mixing it at the preparation of the ink composition.

Examples of dye that can be used in the present invention include the followings. A dye may be used alone, or a plurality of dyes are used in combination so as to control the color tone. Also, the ink composition obtained from each of ink stock solutions of yellow, magenta, cyan and black of the present invention may be used to form not only a simple image but also a full color image. Ink compositions of light and dark two colors may be also used for each color to form a full color image. Furthermore, an ink composition of intermediate color tone such as red, green, blue and violet may be also used. The ink composition of the present invention may make up an ink set to obtain a full color image. Alternatively, the ink composition may make up a part of an ink set. That is, an arbitrary ink composition other than that of the present invention may be combined with the ink composition of the present invention so as to make up an ink set.

In the ink composition of the present invention., another coloring agent may be used in combination together with the above-described dye for controlling the color tone so as to obtain a full color image.

As the coloring agent which can be used for the ink set of the present invention and the coloring agent which can be used in combination with the above-described dye, an arbitrary coloring agent can be used for each of those coloring agents. Examples of the dye that can be used in combination include the dyes described above and the following dyes.

Examples of the yellow dyes include an aryl or heteryl azo dye having, as a coupling component, phenols, naphthols, anilines, pyrazolones, pyridones or chain-opening active methylene compounds; an azomethine dye having chain-opening active methylene compounds as the coupling component; a methine dye such as benzylidene dye and monomethine oxonol dye; and a quinone-based dye such as naphthoquinone dye and anthraquinone dye. Other examples of the dye species include a quinophthalone dye, a nitro/ nitroso dye, an acridine dye, and an acridinone dye. These dyes may be a dye that takes on yellow for the first time when the chromophore is partially dissociated, and in this case, the counter cation may be an inorganic cation such as alkali metal and ammonium, or an organic cation such as pyridinium and quaternary ammonium salt or further may be a polymer cation having such a cation in a partial structure.

Examples of the magenta dye include an aryl or heteryl azo dye having, as the coupling component, phenols, naphthols or anilines; an azomethine dye having, as the coupling component, pyrazolones or pyrazolotriazole; a methine dye such as arylidene dye, styryl dye, merocyanine dye and oxonol dye; a carbonium dye such as diphenylmethane dye, triphenylmethane dye and xanthene dye; a quinone-based dye such as naphthoquinone, anthraquinone and anthrapyridone; and a condensed polycyclic dye such as dioxazine dye. These dye may be a dye that takes on magenta for the first time when the chromophore is partially dissociated, and in this case, the counter cation may be an inorganic cation such as an alkali metal and ammonium or an organic cation such as a pyridinium and quaternary ammonium salt or further may be a polymer cation having such a cation in a partial structure.

Examples of the cyan dye include an azomethine dye such as indoaniline dye and indophenol dye; a polymethine dye such as cyanine dye, oxonol dye and merocyanine dye; a carbonium dye such as diphenylmethane dye, triphenylmethane dye and xanthene dye; a phthalocyanine dye; an anthraquinone dye; an aryl or heteryl azo dye having, as a coupling agent, phenols, naphthols or anilines; and an indigo/thioindigo dye. These dyes may be a dye that taken on cyan for the first time when the chromophore is partially dissociated, and in this case, the counter cation may be an inorganic cation such as an alkali metal and ammonium or an organic cation such as pyridinium and quaternary ammonium salt or further may be a polymer cation having such a cation in a partial structure.

Also, a water-soluble dye such as direct dye, acidic dye, food dye, basic dye and reactive dye may be used in combination. Among others, preferred examples thereof include C.I. Direct Red 1, 2, 4, 9, 11, 23, 26, 31, 37, 39, 62, 63, 72, 75, 76, 79, 80, 81, 83, 84, 87, 89, 92, 95, 111, 173, 184, 207, 211, 212, 214, 218, 21, 223, 224, 225, 226, 227, 232, 233, 240, 241, 242, 243, 247, 254, C.I. Direct Violet 7, 9, 47, 48, 51, 66, 90, 93, 94, 95, 98, 100, 101, C.I. Direct Yellow 4, 8, 9, 11, 12, 27, 28, 29, 33, 35, 39, 41, 44, 50, 53, 58, 59, 68, 86, 87, 93, 95, 96, 98, 100, 106, 108, 109, 110, 120, 130, 132, 142, 144, 157, 161, 163, C.I. Direct Blue 1, 10, 15, 22, 25, 55, 67, 68, 71, 76, 77, 78, 80, 84, 86, 87, 90, 98, 106, 108, 109, 151, 156, 158, 159, 160, 168, 189, 192, 193, 194, 199, 200, 201, 202, 203, 207, 211, 213, 214, 218, 225, 229, 236, 237, 244, 248, 249, 251, 252, 264, 270, 280, 288, 289, 290, 291, C.I. Direct Black 9, 17, 19, 22, 32, 51, 56,

62, 69, 77, 80, 91, 94, 97, 108, 112, 113, 114, 117, 118, 121, 122, 125, 132, 146, 154, 166, 168, 173, 199, C.I. Acid Red 1, 8, 35, 42, 52, 57, 62, 80, 81, 82, 87, 94, 111, 114, 115, 118, 119, 127, 128, 131, 143, 144, 151, 152, 154, 158, 186, 245, 249, 254, 257, 261, 263, 266, 289, 299, 301, 305, 336, 337, 361, 396, 397, C.I. Acid Violet 5, 34, 43, 47, 48, 90, 103, 126, C.I. Acid Yellow 17, 19, 23, 25, 39, 40, 42, 44, 49, 50, 61, 64, 76, 79, 110, 127, 135, 143, 151, 159, 169, 174, 190, 195, 196, 197, 199, 218, 219, 222, 227, C.I. Acid Blue 9, 25, 40, 41, 62, 72, 76, 78, 80, 82, 87, 92, 106, 112, 113, 120, 127:1, 129, 138, 143, 175, 181, 185, 205, 207, 220, 221, 230, 232, 247, 249, 258, 260, 264, 271, 277, 278, 279, 280, 288, 290, 326, C.I. Acid Black 7, 24, 29, 48, 52:1, 172, C.I. Reactive Red 3, 6, 13, 17, 19, 21, 22, 23, 24, 29, 35, 37, 40, 41, 43, 45, 49, 55, 63, 106, 107, 112, 113, 114, 126, 127, 128, 129, 130, 131, 137, 160, 161, 174, 180, C.I. Reactive Violet 1, 3, 4, 5, 6, 7, 8, 9, 16, 17, 22, 23, 24, 26, 27, 33, 34, C.I. Reactive Yellow 2, 3, 13, 14, 15, 17, 18, 23, 24, 25, 26, 27, 29, 35, 37, 41, 42, C.I. Reactive Blue 2, 3, 5, 7, 8, 10, 13, 14, 15, 17, 18, 19, 21, 25, 26, 27, 28, 29, 38, 82, 89, 158, 182, 190, 203, 216, 220, 244, C.I. Reactive Black 4, 5, 8, 14, 21, 23, 26, 31, 32, 34, C.I. Basic Red 12, 13, 14, 15, 18, 22, 23, 24, 25, 27, 29, 35, 36, 38, 39, 45, 46, C.I. Basic Violet 1, 2, 3, 7, 10, 15, 16, 20, 21, 25, 27, 28, 35, 37, 39, 40, 48, C.I. Basic Yellow 1, 2, 4, 11, 13, 14, 15, 19, 21, 23, 24, 25, 28, 29, 32, 36, 39, 40, C.I. Basic Blue 1, 3, 5, 7, 9, 22, 26, 41, 45, 46, 47, 54, 57, 60, 62, 65, 66, 69, 71, and C.I. Basic Black 8.

In addition to the dye represented by formula above, dyes described in the following documents are also preferably used: JP-A-10-130557, JP-A-9-255906, JP-A-6-234944, JP-A-7-97541, EP 982371, WO 00/43450, WO 00/43451, WO 00/43452, WO 00/43453, WO 03/106572, WO 03/104332, JP-A-2003-238862, JP-A-2004-83609, JP-A-2002-302619, JP-A-2002-327131, JP-A-2002-265809, WO 01/48090, WO 04/087815, WO 02/90441, WO 03/027185, WO 04/085541, JP-A-2003-321627, JP-A-2002-332418, JP-2002-332419, WO 02/059215, WO 02/059216, WO 04/087814, WO 04/046252, WO 04/046265, U.S. Patent 6,652,637, WO 03/106572, WO 03/104332, WO 00/58407, Japanese Patent Nos. 3558213, 3558212, 3558211 and 2004-285351, WO 04/078860, JP-A-2004-323605, and WO 04/104108.

Furthermore, in the present invention, a pigment can be also used in combination with the dye.

As the pigment that can be used in the present invention, commercially available pigments as well as known pigments described in various documents can be utilized. Examples the document include Color Index (compiled by The Society of Dyers and Colourists), Kaitei Shinpan Ganryo Binran (Revised New Handbook of Pigments), compiled by Nippon Ganryo Gijutsu Kyokai (1989), Saishin Ganryo Oyo Gijutsu (Newest Pigment Application Technology), CMC (1986), Insatsu Ink Gijutsu (Printing Ink Technique), CMC (1984), and W. Herbst and K. Hunger, Industrial Organic Pigments, VCH Verlagsgesellschaft (1993). Specifically, examples of the organic pigment include an azo pigment (e.g., azo lake pigment, insoluble azo pigment, condensed azo pigment, chelate azo pigment), a polycyclic pigment (e.g., phthalocyanine-based pigment, anthraquinone-based pigment, perylene- or perynone-based pigment, indigo-based pigment, quinacridone-based pigment, dioxazine-based pigment, isoindolinone-based pigment, quinophthalone-based pigment, diketopyrrolopyrrole-based pigment), a dyeing lake pigment (e.g., lake pigment of an acid or basic dye), and an azine pigment; and examples of the inorganic pigment include a yellow pigment such as C.I. Pigment Yellow 34, 37, 42 and 53, a red-type pigment such as C.I. Pigment Red 101 and 108, a blue-type pigment such as C.I. Pigment Blue 27, 29 and 17:1, a black-type pigment such as C.I. Pigment Black 7 and magnetite, and a white-type pigment such as C.I. Pigment White 4, 6, 18 and 21.

As the pigment having a color tone preferred for image formation, the blue to cyan pigment is preferably a phthalocyanine pigment, an anthraquinone-type indanthrone pigment (for example, C.I. Pigment Blue 60) or a dyeing lake pigment-type triarylcarbonium pigment, and most preferably a phthalocyanine pigment (preferred examples thereof include copper phthalocyanine such as C.I. Pigment Blue 15:1, 15:2, 15:3, 15;4 and 15:6, monochloro or low chlorinated copper phthalocyanine, aluminum phthalocyanine including the pigments described in European Patent 860475, C.I. Pigment Blue 16 that is a nonmetallic phthalocyanine, and phthalocyanine with the center metal being Zn, Ni or Ti; and among these, C.I. Pigment Blue 15:3 and 15:4 and aluminum phthalocyanine are more preferred).

The red to violet pigment that is preferably used includes an azo pigment (preferred examples thereof include C.I. Pigment Red 3, 5, 11, 22, 38, 48:1, 48:2, 48:3, 48:4, 49:1, 52:1, 53:1, 57:1, 63:2, 144, 146 and 184; among these, C.1. Pigment Red 57:1, 146 and 184 are more preferred), a quinacridone-based pigment (preferred examples thereof include C.I. Pigment Red 122, 192, 202, 207 and 209 and C.I. Pigment Violet 19 and 42; among these, C.I. Pigment Red 122 is more preferred), a dyeing lake pigment-type triarylcarbonium pigment (preferred examples thereof include C.I. Pigment Red 81;1 and C.I. Pigment Violet 1, 2, 3, 27 and 39, which are a xanthene-based pigment), a dioxazine-based pigment (such as C.I. Pigment Violet 23 and 37), a diketopyrrolopyrrole-based pigment (such as C.I. Pigment Red 254), a perylene pigment (such as C.I. Pigment Violet 29), an anthraquinone-based pigment (such as C.I. Pigment Violet 5:1, 31 and 33), and a thioindigo-based pigment (such as C.I. Pigment Red 38 and 88).

The yellow pigment that is preferably used includes an azo pigment (preferred examples thereof include C.I. Pigment Yellow 1, 3, 74 and 98 which are of a monoazo pigment type, C.I. Pigment Yellow 12, 13, 14, 16, 17 and 83 which are of a disazo pigment type, C.I. Pigment Yellow 93, 94, 95, 128 and 155 which are of a general azo type, and C.I. Pigment Yellow 120, 151, 154, 156 and 180 which are of a benzimidazolone type; and among others, those using no benzidine-based compound as a raw material are more preferred), an isoindoline/isoindolinone-based pigment (preferred examples thereof include C.I. Pigment Yellow 109, 110, 137 and 139), a quinophthalone pigment (preferred examples thereof include C.I. Pigment Yellow 138), and a flavanthrone pigment (such as C.I. Pigment Yellow 24).

Preferred black pigments include an inorganic pigment (preferred examples thereof include carbon black and magnetite) and aniline black. In addition, an orange pigment (such as C.I. Pigment Orange 13 and 16) and a green pigment (such as C.I. Pigment Green 7) may be used.

The pigment that can be used in the present invention may be either the above-described uncoated pigment or a surface-treated pigment. Examples of the surface treatment method that may be conceived include a method of coating the surface with a resin or wax, a method of adhering a surfactant, and a method of binding a reactive substance (for example, a silane coupling agent, an epoxy compound, a polyisocyanate or a radical derived from a diazonium salt) to the pigment surface, and these are described in the following literatures and patents:

(1) Kinzoku Sekken no Seishitsu to Oyo (Properties and Applications of Metal Soap) (Saiwai Shobo Co., Ltd.);
(2) Insatsu Ink Insatsu (Printing Ink Printing) (CMC, 1984);
(3) Saishin Ganryo Oyo Giiutsu (Newest Pigment Application Technology) (CMC, 1986);
(4) U.S. Patents 5,554,739 and 5,571,311; and
(5) JP-A-9-151342, JP-A-10-140065, JP-A-10-292143 and JP-A-11-166145.

In particular, a self-dispersible pigment prepared by allowing a diazonium salt to act on carbon black, which is described in U.S. Patents of (4), and a capsulated pigment prepared by the method described in Japanese Patents of (5) are effective, because dispersion stability can be obtained without using an excess dispersant in the ink composition.

In the present invention, the pigment may be dispersed by further using a dispersant. Various known dispersants such as surfactant-type low molecular dispersant and polymer-type dispersant can be used according to the pigment used. Examples of the dispersant include those described in JP-A-3-69949 and European Patent 549,486. Also, at the time of using the dispersant, a pigment derivative called a synergist may be added so as to accelerate the adsorption of dispersant to the pigment. The particle size of the pigment that can be used in the present invention is preferably from 0.01 to 10 $\mu$m, more preferably from 0.05 to 1 $\mu$m, after the dispersion.

As the method of dispersing the pigment, a known dispersion technique used for the production of an ink or a toner may be used. Examples of the dispersing machines include a vertical or horizontal agitator mill, an attritor, a colloid mill, a ball mill, a three-roll mill, a pearl mill, a super mill, an impeller, a disperser, a KD mill, a dynatron and a pressure kneader. Details thereof are described in Saishin Ganryo Oyo Gijutsu (Newest Pigment Application Technology) (CMC, 1986).

As the water-soluble dye used in the present invention, it is also preferred to use dyes such as magenta dyes described in JP-A-2002-371214, phthalocyanine dyes described in JP-A-2002-309118 and water-soluble phthalocyanine dyes described in JP-A-2003-12952 and JP-A-2003-12956.

The ink composition of the present invention contains the dye in a medium, preferably in an aqueous medium. The aqueous medium is water or water to which a solvent such as water-miscible organic solvent is added, if desired. Incidentally, the water-miscible organic solvent may be a viscosity reducing agent in the ink stock solution, as described above.

The above-described water-miscible organic solvent that can be used in the present invention is, in this field, a material having a function as a drying inhibitor, a permeation accelerator, a wetting agent or the like of an ink composition for inkjet recording, and a high-boiling-point water-miscible organic solvent is mainly used. Such compounds include an alcohol (e.g., methanol, ethanol, propanol, isopropanol, butanol, isobutanol, sec-butanol, tert-butanol, pentanol, hexanol, cyclohexanol, benzyl alcohol), polyhydric alcohols (e.g., ethylene glycol, diethylene glycol, triethylene glycol, polyethylene glycol, propylene glycol, dipropylene glycol, polypropylene glycol, butylene glycol, hexanediol, pentanediol, glycerin, hexanetriol, thiodiglycol), a glycol derivative (e.g., ethylene glycol monomethyl ether, ethylene glycol monoethyl ether, ethylene glycol monobutyl ether, diethylene glycol monomethyl ether, diethylene glycol monobutyl ether, propylene glycol monomethyl ether, propylene glycol monobutyl ether, dipropylene glycol monomethyl ether, triethylene glycol monomethyl ether, ethylene glycol diacetate, ethylene glycol monomethyl ether acetate, triethylene glycol monomethyl ether, triethylene glycol monoethyl ether, ethylene glycol monophenyl ether), an amine (e.g., ethanolamine, diethanolamine, triethanolamine, N-methyldiethanolamine, N-ethyldiethanolamine, morpholine, N-ethylmorpholine, ethylenediamine, diethylenetriamine, triethylenetetramine, polyethyleneimine, tetramethylpropylenediamine), and other polar solvents (e.g., formamide, N,N-dimethylformamide, N,N-dimethylacetamide, dimethylsulfoxide, sulfolane, 2-pyrrolidone, N-methyl-2-pyrrolidone, N-vinyl-2-pyrrolidone, 2-oxazolidone, 1,3-dimethyl-2-imidazolidinone, acetonitrile, acetone). Two or more kinds of these water-miscible organic solvents may be used in combination.

Among these, an alcohol-based solvent is preferred. Also, the ink composition of the present invention preferably contains a water-miscible organic solvent having a boiling point of 150°C or more, and examples thereof include 2-pyrrolidone selected from the solvents described above. The water-miscible organic solvent is preferably contained in an amount of 5 to 60 mass%, more preferably from 10 to 45 mass%, in total.

By incorporating a surfactant into the ink composition of the present invention and thereby controlling the liquid properties of the ink composition, ejection stability of the ink composition can be enhanced and an excellent effect can be exerted on the improvement of water resistance of an image and prevention of blurring of the ink composition printed.

The surfactant includes, for example, an anionic surfactant such as sodium dodecylsulfate, sodium dodecyloxysulfonate and sodium alkylbenzenesulfonate, a cationic surfactant such as cetylpyridinium chloride, trimethylcetylammonium chloride and tetrabutylammonium chloride, and a nonionic surfactant such as polyoxyethylene nonylphenyl ether, polyoxyethylene naphthyl ether and polyoxyethylene octylphenyl ether. Among others, a nonionic surfactant is preferably used.

The content of the surfactant is from 0.001 to 20 mass%, preferably from 0.005 to 10 mass%, more preferably from 0.01 to 5 mass%, based on the ink composition.

In the case where the dye is an oil-soluble dye, the ink composition of the present invention can be prepared by dissolving the oil-soluble dye in a high-boiling-point organic solvent, and emulsion-dispersing the resulting ink stock solution in an aqueous medium. The boiling point of the high-boiling-point organic solvent used in the present invention is 150°C or more, preferably 170°C or more.

Examples thereof include phthalic acid esters (e.g., dibutyl phthalate, dioctyl phthalate, dicyclohexyl phthalate, di-2-ethylhexyl phthalate, decyl phthalate, bis(2,4-di-tert-amylphenyl) isophthalate, bis(1,1-diethylpropyl) phthalate), phosphoric acid or phosphone esters (e.g., diphenyl phosphate, triphenyl phosphate, tricresyl phosphate, 2-ethylhexyldiphenyl phosphate, dioctylbutyl phosphate, tricyclohexyl phosphate, tri-2-ethylhexyl phosphate, tridodecyl phosphate, di-2-ethylhexyldiphenyl phosphate), a benzoic acid ester (e.g., 2-ethylhexyl benzoate, 2,4-dichlorobenzoate, dodecyl benzoate, 2-ethylhexyl-p-hydroxybenzoate), amides (e.g., N,N-diethyldodecanamide, N,N-diethyllaurylamide), alcohol or phenols (e.g., isostearyl alcohol, 2,4-di-tert-amylphenol), aliphatic esters (e.g., dibutoxyethyl succinate, di-2-ethylhexyl succinate, 2-hexyldecyl tetradecanoate, tributyl citrate, diethyl azelate, isostearyl lactate, trioctyl citrate), an aniline derivative (e.g., N,N-dibutyl-2-butoxy-5-tert-octylaniline), chlorinated paraffins (e.g., paraffin having a chlorine content of 10 to 80%), trimesic acid esters (e.g., tributyl trimesate), dodecylbenzene, diisopropylnaphthalene, phenols (e.g., 2,4-di-tert-amylphenol, 4-dodecyloxyphenol, 4-dodecyloxycarbonylphenol, 4-(4-dodecyloxyphenylsulfonyl)phenol), carboxylic acids (e.g., 2-(2,4-di-tert-amylphenoxy)butyric acid, 2-ethoxyoctanedecanoic acid), and alkylphosophoric acids (e.g., di-2-(ethylhexyl)phosphoric acid, diphenylphosphoric acid). The high-boiling-point organic solvent may be used in an amount of, in terms of mass ratio to the oil-soluble dye, from 0.01 to 3 times, preferably from 0.01 to 1.0 times.

One of these high-boiling-point organic solvents may be used alone, or several kinds thereof [for example, tricresyl phosphate and dibutyl phthalate, trioctyl phosphate and di(2-ethylhexyl) sebacate, or dibutyl phthalate and poly(N-tert-butylacrylamide)] may be mixed and used.

Examples of the high-boiling-point organic solvent for use in the present invention other than the above-described compounds, and/or the synthesis method for the high-boiling-point organic solvent are described in U.S. Patents 2,322,027, 2,533,514, 2,772,163, 2,835,579, 3,594,171, 3,676,137, 3,689,271, 3,700,454, 3,748,141, 3,764,336, 3,765,897, 3,912,515, 3,936,303, 4,004,928, 4,080,209, 4,127,413, 4,193,802, 4,207,393, 4,220,711, 4,239,851, 4,278,757, 4,353,979, 4,363,873, 4,430,421, 4,430,422, 4,464,464, 4,483,918, 4,540,657, 4,684,606, 4,728,599, 4,745,049, 4,935,321 and 5,013,639, European Patents 276319A, 286253A, 289820A, 309158A, 309159A, 309160A, 509311A and 510576A, East German Patents 147,009, 157,147, 159,573 and 225,240A, British Patent 2,091,124A, JP-A-48-47335, JP-A-50-26530, JP-A-51-25133, JP-A-51-26036, JP-A-51-27921, JP-A-51-27922, JP-A-51-149028, JP-A-52-46816, JP-A-53-1520, JP-A-53-1521, JP-A-53-15127, JP-A-53-146622, JP-A-54-91325, JP-A-54-106228, JP-A-54-118246, JP-A-55-59464, JP-A-56-64333, JP-A-56-81836, JP-A-59-204041, JP-A-61-84641, JP-A-62-118345, JP-A-62-247364, JP-A-63-167357, JP-A-63-214744, JP-A-63-301941, JP-A-64-9452, JP-A-64-9454, JP-A-64-68745, JP-A-1-101543, JP-A-1-102454, JP-A-2-792, JP-A-2-4239, JP-A-2-43541, JP-A-4-29237, JP-A-4-30165, JP-A-4-232946, and JP-A-4-346338.

The high-boiling-point organic solvent is used in an amount of, in terms of the mass ratio to the oil-soluble dye, from 0.01 to 3.0 times, preferably from 0.01 to 1.0 times.

In the present invention, the oil-soluble dye or high-boiling-point organic solvent is preferably emulsion-dispersed in an aqueous medium. At the emulsion-dispersion, in view of emulsifiability, a low-boiling-point organic solvent may be used depending on the case. The low-boiling-point organic solvent is an organic solvent having a boiling point of about 30 to 150°C at atmospheric pressure. Preferred examples thereof include, but are not limited to, esters (e.g., ethyl acetate, butyl acetate, ethyl propionate, β-ethoxyethyl acetate, methylcellosolve acetate), alcohols (e.g., isopropyl alcohol, n-butyl alcohol, secondary butyl alcohol), ketones (e.g., methyl isobutyl ketone, methyl ethyl ketone, cyclohexanone), amides (e.g., dimethylformamide, N-methylpyrrolidone), and ethers (e.g., tetrahydrofuran, dioxane).

The emulsion dispersion is performed for dispersing an oil phase obtained by dissolving the dye in a high-boiling-point organic solvent or depending on the case, in a mixed solvent thereof with a low-boiling-point organic solvent, in an aqueous phase mainly composed of water to form fine oil droplets of the oil phase (this oil phase may be used as the ink stock solution, or the oil phase dispersed in the aqueous phase may be used as the ink stock solution). At this time, components such as surfactant, wetting agent, dye stabilizer, emulsification stabilizer, preservative and fungicide

may be added to either one or both of the aqueous phase and the oil phase, if desired. As the emulsification method, a method of adding the oil phase to the aqueous phase is generally employed, but a so-called phase inversion emulsification method of adding dropwise the aqueous phase to the oil phase may be also preferably used. Incidentally, the above-described emulsification method can be applied also when the dye is water-soluble and the component is oil-soluble.

At the emulsion dispersion, various surfactants can be used. Preferred examples thereof include an anionic surfactant such as fatty acid salt, alkylsulfuric ester salt, alkylbenzenesulfonate, alkylnaphthalenesulfonate, dialkylsulfosuccinate, alkylphosphoric ester salt, naphthalenesulfonic acid formalin condensate and polyoxyethylene alkylsulfuric ester salt, and a nonionic surfactant such as polyoxyethylene alkyl ether, polyoxyethylene alkylallyl ether, polyoxyethylene fatty acid ester, sorbitan fatty acid ester, polyoxyethylene sorbitan fatty acid ester, polyoxyethylene alkylamine, glycerin fatty acid ester and oxyethylene oxypropylene block copolymer. Also, SURFYNOLS (produced by Air Products & Chemicals), which is an acetylene-based polyoxyethylene oxide surfactant, is preferably used. Furthermore, for example, an amine oxide-type amphoteric surfactant such as N,N-dimethyl-N-alkylamine oxide is also preferred. In addition, surfactants described in JP-A-59-157636, pages (37) and (38), and Research Disclosure, No. 308119 (1989) can be also used.

For the purpose of stabilizing the dispersion immediately after emulsification, a water-soluble polymer may be added in combination with the surfactant above. As the water-soluble polymer, polyvinyl alcohol, polyvinylpyrrolidone, polyethylene oxide, polyacrylic acid, polyacrylamide and copolymers thereof are preferably used. In addition, it is also preferred to use a natural water-soluble polymer such as polysaccharides, casein and gelatin. Furthermore, for the stabilization of dye dispersion, a polymer substantially incapable of dissolving in an aqueous medium, such as polyvinyl, polyurethane, polyester, polyamide, polyurea and polycarbonate, which is obtained by the polymerization of acrylic acid esters, methacrylic acid esters, vinyl esters, acrylamides, methacrylamides, olefins, styrenes, vinyl ethers or acrylonitriles, may be also used in combination. This polymer preferably contains $-SO_3^-$ or $-COO^-$. In the case of using such a polymer substantially incapable of dissolving in an aqueous medium, the polymer is preferably used in an amount of 20 mass% or less, more preferably 10 mass% or less, based on the high-boiling-point organic solvent.

In preparing an aqueous ink composition by dispersing the oil-soluble dye or high-boiling-point organic solvent according to emulsion dispersion, control of the particle size is important. In order to elevate the color purity or density of an image formed by inkjet recording, it is essential to reduce the average particle size. The average particle size is, in terms of the volume average particle size, preferably 1 μm or less, more preferably from 5 to 100 nm.

The volume average particle size and particle size distribution of the dispersed particles can be easily measured by a known method such as static light scattering method, dynamic light scattering method, centrifugal precipitation method or the method described in Jikken Kagaku Koza (Lecture of Experimental Chemistry), 4th ed., pp. 417-418. For example, the ink composition is diluted with distilled water to have a particle concentration of 0.1 to 1 mass%, then, the particle size can be easily measured by a commercially available volume average particle size measuring apparatus (for example, Microtrac UPA (manufactured by Nikkiso K.K.)). The dynamic light scattering method utilizing the laser Doppler effect is particularly preferred because even a small particle size can be measured.

The volume average particle size is an average particle size weighted with the particle volume and is obtained by multiplying the diameter of individual particles in the gathering of particles by the volume of the particle and dividing the sum total of the obtained values by the total volume of particles. The volume average particle size is described in Soichi Muroi, Kobunshi Latex no Kagaku (Chemistry of Polymer Latex), page 119, Kobunshi Kanko Kai.

Also, it has been revealed that the presence of coarse particles plays a very great role in printing performance, that is, the coarse particle causes nozzle blocking of a head or even if the nozzle is not blocked, forms a dirt to bring about ejection failure or ejection slippage of the ink composition and thereby seriously affect the printing performance. In order to prevent this trouble, it is important to reduce the number of particles of 5 μm or more to 10 or less and the number of particles of 1 μm or more to 1,000 or less, in 1 μl of the ink composition when the ink composition prepared. As the method for removing these coarse particles, a known method such as centrifugal separation and microfiltration can be used. This separation operation may be performed immediately after the emulsion dispersion or may be performed immediately before filling an ink cartridge after various components such as wetting agent and surfactant are added to the emulsified dispersion. A mechanically emulsifying apparatus can be used as an effective device for reducing the average particle size and eliminating coarse particles.

As for the emulsifying apparatus, a known apparatus such as simple stirrer, impeller stirring system, in-line stirring system, mill system (e.g., colloid mill) and ultrasonic system can be used, but use of a high-pressure homogenizer is particularly preferred. The mechanism of the high-pressure homogenizer is described in detail in U.S. Patent 4,533,254 and JP-A-6-47264, and examples of the commercially available apparatus include Gaulin Homogenizer (manufactured by A.P.V GAULIN INC.), Microfluidizer (manufactured by MICROFLUIDEX INC.), and Altimizer (produced by Sugino Machine).

Also, a high-pressure homogenizer with a mechanism of pulverizing particles in an ultrahigh pressure jet stream, which is recently described in U.S. Patent 5,720,551, is particularly effective for the emulsion dispersion of the present invention. Examples of the emulsifying apparatus using this ultrahigh pressure jet stream include DeBEE2000 (manufactured by BEE INTERNATIONAL LTD.).

In performing the emulsification by a high-pressure emulsion-dispersing apparatus, the pressure is 50 MPa or more, preferably 60 MPa or more, more preferably 180 MPa or more.

For example, a method of using two or more emulsifying apparatuses in combination by performing the emulsification by a stirring emulsifier and then passing the resulting emulsion through a high-pressure homogenizer is particularly preferred. Also, a method of once performing the emulsion dispersion by such an emulsifying apparatus and after adding components such as wetting agent and surfactant, again passing the emulsion through a high-pressure homogenizer in the course of filling a cartridge with the ink composition, is preferred. In the case of containing a low-boiling-point organic solvent in addition to the high-boiling-point organic solvent, the low-boiling-point solvent is preferably removed in view of stability of the emulsified product, safety and hygiene. As for the method to remove the low-boiling-point solvent, various known methods can be used according to the kind of the solvent. That is, the methods are evaporation, vacuum evaporation, ultrafiltration and the like. This step of removing the low-boiling-point organic solvent is preferably performed as soon as possible immediately after the emulsification.

The preparation method for the composition for inkjet recording is described in detail in JP-A-5-148436, JP-A-5-295312, JP-A-7-97541, JP-A-7-82515 and JP-A-7-118584, and the methods described can be utilized also for the preparation of the ink composition of the present invention.

In the ink composition of the present invention, functional components for imparting various functions to the ink composition can be incorporated. Examples of the functional component include various solvents described above, a drying inhibitor for preventing blocking due to drying of the ink composition at the jetting orifice, a permeation accelerator for attaining better permeation of the ink composition into paper, an ultraviolet absorber, an antioxidant, a viscosity adjusting agent, a surface tension adjusting agent, a dispersant, a dispersion stabilizer, a fungicide, a rust inhibitor, a pH adjusting agent, an antifoaming agent, and a chelating agent, and these can be appropriately selected and used in an appropriate amount in the ink composition of the present invention. The functional component also include a component that is a kind of compound and exerts one function or two or more functions. Accordingly, with respect to the blending ratio of functional components in the following, the functional component having overlapping functions is dealt by independently counting the compound in each functional component.

The drying inhibitor for use in the present invention is preferably a water-soluble organic solvent having a vapor pressure lower than that of water. Specific examples thereof include polyhydric alcohols typified by ethylene glycol, propylene glycol, diethylene glycol, polyethylene glycol, thiodiglycol, dithiodiglycol, 2-methyl-1,3-propanediol, 1,2,6-hexanetriol, an acetylene glycol derivative, glycerin and trimethylolpropane; lower alkyl ethers of polyhydric alcohol, such as ethylene glycol monomethyl(or monoethyl) ether, diethylene glycol monomethyl(or monoethyl) ether and triethylene glycol monoethyl(or monobutyl) ether; heterocyclic rings such as 2-pyrrolidone, N-methyl-2-pyrrolidone, 1,3-dimethyl-2-imidazolidinone and N-ethylmorpholine; sulfur-containing compounds such as sulfolane, dimethylsulfoxide and 3-sulfolene; polyfunctional compounds such as diacetone alcohol and diethanolamine; and urea derivatives. Among these, polyhydric alcohols such as glycerin and diethylene glycol are preferred. One of these drying inhibitors may be used alone, or two or more thereof may be used in combination. The drying inhibitor is preferably contained in an amount of 10 to 50 mass% in the ink composition.

Examples of the permeation accelerator for use in the present invention include alcohols such as ethanol, isopropanol, butanol, di(tri)ethylene glycol monobutyl ether and 1,2-hexanediol, sodium laurylsulfate, sodium oleate, and a nonionic surfactant. Such a compound is sufficiently effective when contained in an amount of 10 to 30 mass% in the ink composition and is preferably used in an amount within the range causing no blurring of printed character or no print through.

As the ultraviolet absorber used for enhancing the preservability of an image in the present invention, benzotriazole-based compounds described in JP-A-58-185677, JP-A-61-190537, JP-A-2-782, JP-A-5-197075 and JP-A-9-34057, benzophenone-based compounds described in JP-A-46-2784, JP-A-5-194483 and U.S. Patent 3,214,463, cinnamic acid-based compounds described in JP-B-48-30492 (the term "JP-B" as used herein means an "examined Japanese patent publication"), JP-B-56-21141 and JP-A-10-88106, triazine-based compounds described in JP-A-4-298503, JP-A-8-53427, JP-A-8-239368, JP-A-10-182621 and JP-T-8-501291 (the term "JP-T" as used herein means a "published Japanese translation of a PCT patent application"), compounds described in Research Disclosure, No. 24239, and compounds capable of absorbing an ultraviolet ray and emitting fluorescence, so-called fluorescent brightening agents, typified by stilbene-based and benzoxazole-based compounds, can be used.

As the antioxidant used for enhancing the preservability of an image in the present invention, various organic or metal complex-based discoloration inhibitors can be used. Examples of the organic discoloration inhibitor include hydroquinones, alkoxyphenols, dialkoxyphenols, phenols, anilines, amines, indanes, chromans, alkoxyanilines, and heterocyclic rings, and examples of the metal complex include a nickel complex and a zinc complex. More specifically, compounds described in the patents cited in Research Disclosure, No. 17643, paragraphs VII-I and J, Research Disclosure, No. 15162, Research Disclosure, No. 18716, page 650, left column, Research Disclosure, No. 36544, page 527, Research Disclosure, No. 307105, page 872, and Research Disclosure, No. 15162, and compounds included in

formulae and examples of representative compounds described in JP-A-62-215272, pages 127 to 137, can be used.

Examples of the rust inhibitor for use in the present invention include an acidic sulfite, sodium thiosulfate, ammonium thiodiglycolate, diisopropylammonium nitrite, pentaerythritol tetranitrate, dicyclohexylammonium nitrite, and benzotriazole. Such a compound is preferably used in an amount of 0.02 to 5.00 mass% in the ink composition.

The conductivity of the ink composition of the present invention is from 0.01 to 10 S/m, preferably from 0.05 to 5 S/m.

As for the measuring method, the conductivity can be measured by an electrode method using a commercially available saturated potassium chloride. The conductivity can be controlled mainly by the ion concentration in an aqueous solution. In the case where the salt concentration is high, desalting can be performed using an ultrafiltration membrane or the like. Furthermore, in the case of adjusting the conductivity by adding a salt or the like, various organic salts or inorganic salts can be added for adjustment.

As the inorganic salt, an inorganic compound such as potassium halide, sodium halide, sodium sulfate, potassium sulfate, sodium hydrogensulfate, potassium hydrogensulfate, sodium nitrate, potassium nitrate, sodium hydrogencarbonate, potassium hydrogencarbonate, sodium phosphate, sodium monohydrogenphosphate, boric acid, potassium dihydrogenphosphate and sodium dihydrogenphosphate, and an organic compound such as sodium acetate, potassium acetate, potassium tartrate, sodium tartrate, sodium benzoate, potassium benzoate, sodium p-toluenesulfonate, potassium saccharinate, potassium phthalate and sodium picolinate, can be also used.

Furthermore, the conductivity can be also adjusted by selecting components of the later-described aqueous medium.

The viscosity of the ink composition of the present invention is, at 25°C, preferably from 1 to 30 mPa • s, more preferably from 2 to 15 mPa • s, still more preferably from 2 to 10 mPa • s. If the viscosity exceeds 30 mPa • s, the fixing rate of a recorded image is slowed down and the ejection performance is also reduced, whereas if the viscosity is less than 1 mPa • s, blurring of a recorded image occurs to deteriorate the quality.

The viscosity can be arbitrarily adjusted by the amount of the ink solvent added. Examples of the ink solvent include glycerin, diethylene glycol, triethanolamine, 2-pyrrolidone, diethylene glycol monobutyl ether, and triethylene glycol monobutyl ether.

Also, a viscosity adjusting agent may be used. Examples of the viscosity adjusting agent include a water-soluble polymer such as celluloses and polyvinyl alcohol, and a nonionic surfactant. These are described in more detail in Nendo Chosei Gijutsu (Viscosity Adjusting Technology), Chapter 9, Gijutsu Joho Kyokai (1999), and Chemicals for Inkjet Printers ('98 Enlarged Edition)-Zairyo no Kaihatsu Doko-Tenbo Chosa (Survey on Tendency Prospect of Development of Materials)-, pp. 162-174, CMC (1997).

The method for measuring the viscosity of a liquid is described in detail in JIS Z8803, but the viscosity can be simply and easily measured by a commercially available viscometer. Examples thereof include, as a rotary viscometer, a B-type viscometer and a E-type viscometer, which are manufactured by Tokyo Keiki Inc. In the present invention, the viscosity was measured at 25°C by a vibrating viscometer, Model VM-100A-L, manufactured by Yamaichi Electronics Co., Ltd. The unit of the viscosity is Pascal second (Pa•s), but usually, milli-Pascal second (mPa•s) is used.

The surface tension of the ink composition of the present invention is, at 25°C, preferably from 20 to 50 mN/m, more preferably from 20 to 40 mN/m, for both the dynamic surface tension and the static surface tension. If the surface tension exceeds 50 mN/m, ejection stability is reduced and blurring, feathering or the like occurs in color mixing to significantly deteriorate the print quality, whereas if the surface tension of the ink composition is less than 20 mN/m, a printing failure may be caused due to attachment or the like of the ink composition to the surface of a hardware.

For the purpose of adjusting the surface tension, the above-described various surfactants of cationic, anionic, nonionic and betaine types can be added. Also, two or more surfactants can be used in combination.

As the method for measuring the static surface tension, a capillary rise method, a dropping method, a hanging ring method and the like are known, but in the present invention, a vertical plate method is used as the method for measuring the static surface tension. When a thin plate of glass or platinum is vertically hung with a part thereof being immersed in a liquid, the surface tension of the liquid acts downward along the length of contact between the liquid and the plate. The surface tension can be measured by balancing this force with an upward force.

Also, as the method for measuring the dynamic surface tension, for example, a vibration jet method, a meniscus drop method and a maximum bubble pressure method are known as described in "Interface and Colloid" of Shin-Jikken Kagaku Koza (Lecture of New Experimental Chemistry), Vol. 18, , pp. 69-90, Maruzen (1977), and furthermore, a liquid membrane break method described in JP-A-3-2064 is known, but in the present invention, a differential bubble pressure method is used as the method for measuring the dynamic surface tension. The principle and method of this measurement are described below.

When an air bubble is formed in a solution homogenized by stirring, a new gas-liquid interface is produced, and surfactant molecules in the solution gather on the surface of water at a constant rate. When the bubble rate (bubble production rate) is changed, as the production rate decreases, more surfactant molecules gather on the surface of a bubble, as a result, the maximum bubble pressure immediately before bursting of the bubble is reduced, so that the

maximum bubble pressure (surface tension) based on the bubble rate can be detected. The preferred method for measuring the dynamic surface tension is a method of producing an air bubble in a solution by using large and small two probes, measuring the differential pressure between two probes in the maximum bubble pressure state, and then calculating the dynamic surface tension.

The content of a non-volatile component in the ink composition of the present invention is, based on the total amount of the ink composition, preferably from 10 to 70 mass% in view of ejection stability of the ink composition, print image quality, various fastnesses of an image, and reduction in blurring of an image and stickiness of a print surface after printing, more preferably from 20 to 60 mass% in view of ejection stability of the ink composition and reduction in blurring of an image after printing.

Here, the non-volatile component means a liquid, a solid component or a high-molecular weight component, having a boiling point of 150°C or more at 1 atm. Examples of the non-volatile component in the ink composition for inkjet recording include the dye and the high-boiling-point solvent as well as a polymer latex, a surfactant, a dye stabilizer, a fungicide and a buffer which are added, if desired. Many of these non-volatile components except for a dye stabilizer bring about reduction in the dispersion stability of the ink composition, and such a component exists on the inkjet image-receiving paper even after printing and develops a property of inhibiting stabilization due to association of the dye on the image-receiving paper and causing deterioration in various fastnesses of an image area and blurring of an image under high humidity conditions.

In the present invention, it is also possible to contain a high-molecular weight compound. The high-molecular weight compound as used herein indicates all polymer compounds having a number average molecular weight of 5,000 or more contained in the ink composition. The polymer compound includes, for example, a water-soluble polymer compound capable of substantially dissolving in an aqueous medium, a water-dispersible polymer compound such as polymer latex and polymer emulsion, and an alcohol-soluble polymer compound capable of dissolving in a polyhydric alcohol, which is used as an auxiliary solvent, but any polymer compound is encompassed by the polymer compound of the present invention as long as it can substantially undergo uniform dissolution or dispersion in the ink composition.

Specific examples of the water-soluble polymer compound include a water-soluble polymer such as polyvinyl alcohol, silanol-modified polyvinyl alcohol, carboxymethyl cellulose, hydroxyethyl cellulose, polyvinylpyrrolidone, polyalkylene oxide (e.g., polyethylene oxide, polypropylene oxide) and polyalkylene oxide derivative, a natural water-soluble polymer such as polysaccharide, starch, cationized starch, casein and gelatin, an aqueous acrylic resin such as polyacrylic acid, polyacrylamide and copolymer thereof, an aqueous alkyd resin, and a water-soluble polymer compound having a $-SO_3^-$ or $-COO^-$ group in the molecule and substantially dissolving in an aqueous medium.

Examples of the polymer latex include a styrene-butadiene latex, a styrene-acrylic latex, and a polyurethane latex. Examples of the polymer emulsion include an acrylic emulsion. One of these water-soluble polymer compounds may be used alone, or two or more thereof may be used in combination.

As already described, the water-soluble polymer compound is used as the viscosity adjusting agent for adjusting the viscosity of the ink composition to a viscosity region providing for good ejection characteristics, but if the amount added thereof is large, the viscosity of the ink composition increases to reduce the ejection stability of the ink composition and when the ink composition has aged, nozzle blocking is readily caused by a precipitate.

The amount of the polymer compound added as the viscosity adjusting agent is from 0 to 5 mass%, preferably from 0 to 3 mass%, more preferably from 0 to 1 mass%, based on the total amount of the ink composition, though this may vary depending on the molecular weight of the compound added (a compound having a higher molecular weight can be added in a smaller amount).

Furthermore, in the present invention, the above-described various surfactants of cationic, anionic, nonionic and betaine types can be used as the dispersant and the dispersion stabilizer, and fluorine-based and silicone-based compounds and a chelating agent typified by EDTA can be also used as the antifoaming agent, if desired.

A reflective medium that is a print medium suitably used in the present invention is described below. The reflective medium includes recording paper, recording film, and the like. As the support of the recording paper and recording film, those composed of, for example, a chemical pulp such as LBKP and NBKP, a mechanical pulp such as GP, PGW, RMP, TMP, CTMP, CMP and CGP, or a waste paper pulp such as DIP, and after mixing, if desired, conventionally known additives such as pigment, binder, sizing agent, fixing agent, cationic agent and paper strength increasing agent, produced by using various devices such as Fourdrinier paper machine and cylinder paper machine, can be used. In addition to these supports, the support may be either a synthetic paper or a plastic film sheet. The thickness of the support is preferably from 10 to 250 μm, and the basis weight thereof is preferably from 10 to 250 $g/m^2$.

An image-receiving layer and a backcoat layer may be directly provided on the support to produce an image-receiving material for the ink composition of the present invention and an ink set, or after providing a size press or an anchor coat layer by using starch, polyvinyl alcohol or the like, an image-receiving layer and a backcoat layer may be provided to produce an image-receiving material. Furthermore, the support may be subjected to a flattening treatment by a calendering device such as machine calender, TG calender and soft calender.

As the support, a paper of which both surfaces are laminated with a polyolefin (for example, polyethylene,

polystyrene, polybutene or a copolymer thereof) or polyethylene terephthalate, and a plastic film are more preferably used. A white pigment (e.g., titanium oxide, zinc oxide) or a tinting dye (e.g., cobalt blue, ultramarine, neodymium oxide) is preferably added to the polyolefin.

The image-receiving layer provided on the support contains a porous material or an aqueous binder. Also, the image-receiving layer preferably contains a pigment, and the pigment is preferably a white pigment. Examples of the white pigment include an inorganic white pigment such as calcium carbonate, kaolin, talc, clay, diatomaceous earth, synthetic amorphous silica, aluminum silicate, magnesium silicate, calcium silicate, aluminum hydroxide, alumina, lithopone, zeolite, barium sulfate, calcium sulfate, titanium dioxide, zinc sulfide and zinc carbonate, and an organic pigment such as styrene-based pigment, acrylic pigment, urea resin and melamine resin. In particular, a porous inorganic white pigment is preferred, and a synthetic amorphous silica having a large pore area is more preferred. As for the synthetic amorphous silica, both a silicic anhydride obtained by a dry production process (gas phase process) and a silicic acid hydrate obtained by a wet production process can be used.

As the recording paper containing the above-described pigment in the image-receiving layer, specifically, those disclosed in JP-A-10-81064, JP-A-10-119423, JP-A-10-157277, JP-A-10-217601, JP-A-11-348409, JP-A-2001-138621, JP-A-2000-43401, JP-A-2000-211235, JP-A-2000-309157, JP-A-2001-96897, JP-A-2001-138627, JP-A-11-91242, JP-A-8-2087, JP-A-8-2090, JP-A-8-2091, JP-A-8-2093, JP-A-8-174992, JP-A-11-192777 and JP-A-2001-301314 can be used.

Examples of the aqueous binder contained in the image-receiving layer include a water-soluble polymer such as polyvinyl alcohol, silanol-modified polyvinyl alcohol, starch, cationized starch, casein, gelatin, carboxymethyl cellulose, hydroxyethyl cellulose, polyvinylpyrrolidone, polyalkylene oxide and polyalkylene oxide derivative, and a water-dispersible polymer such as styrene-butadiene latex and acrylic emulsion. One of these aqueous binders may be used alone, or two or more thereof may be used in combination. In the present invention, among these, polyvinyl alcohol and silanol-modified polyvinyl alcohol are preferred in vie of adhesion to the pigment and peeling resistance of the ink-receiving layer.

The image-receiving layer may contain a mordant, a water-proofing agent, a light resistance improver, a gas resistance improver, a surfactant, a film hardening agent and other additives, in addition to the pigment and the aqueous binder.

The mordant added to the image-receiving layer is preferably immobilized and for such a purpose, a polymer mordant is preferably used.

The polymer mordant is described in JP-A-48-28325, JP-A-54-74430, JP-A-54,-124726, JP-A-55-22766, JP-A-55-142339, JP-A-60-23850, JP-A-60-23851, JP-A-60-23852, JP-A-60-23853, JP-A-60-57836, JP-A-60-60643, JP-A-60-118834, JP-A-60-122940, JP-A-60-122941, JP-A-60-122942, JP-A-60-235134, JP-A-1-161236 and U.S. Patents 2,484,430, 2,548,564, 3,148,061, 3,309,690, 4,115,124, 4,124,386, 4,193,800, 4,273,853, 4,282,305 and 4,450,224. An image-receiving material containing the polymer mordant described in JP-A-1-161236, pp. 212-215 is particularly preferred. When the polymer mordant described in this publication is used, an image with excellent image quality can be obtained and at the same time, the light resistance of the image is improved.

The water-proofing agent is effective in making an image be resistant to water. In particular, the water-proofing agent is preferably a cation resin. Examples of the cationic resin include polyamide polyamine epichlorohydrin, polyethyleneimine, polyaminesulfone, a polydimethyldiallylammonium chloride polymer, and cation polyacrylamide. The content of the cationic resin is preferably from 1 to 15 mass%, more preferably from 3 to 10 mass%, based on the total solid content of the ink-receiving layer.

Examples of the light resistance improver and gas resistance improver include a phenol compound, a hindered phenol compound, a thioether compound, a thiourea compound, a thiocyanic acid compound, an amine compound, a hindered amine compound, a TEMPO compound, a hydrazine compound, a hydrazide compound, an amidine compound, a vinyl group-containing compound, an ester compound, an amide compound, an ether compound, an alcohol compound, a sulfinic acid compound, a sugar, a water-soluble reducing compound, an organic acid, an inorganic acid, a hydroxy group-containing organic acid, a benzotriazole compound, a benzophenone compound, a triazine compound, a heterocyclic compound, a water-soluble metal salt, an organic metal compound, and a metal complex.

Specific examples of these compounds include those descried in JP-A-10-182621, JP-A-2001-260519, JP-A-2000-260519, JP-B-4-34953, JP-B-4-34513, JP-B-4-34512, JP-A-11-170686, JP-A-60-67190, JP-A-7-276808, JP-A-2000-94829, JP-T-8-512258 and JP-A-11-321090.

The surfactant functions as a coating aid, a releasability improver, a slipperiness improver or an antistatic agent. The surfactant is described in JP-A-62-173463 and JP-A-62-183457.

Instead of the surfactant, an organic fluoro compound may be used. The organic fluoro compound is preferably hydrophobic. Examples of the organic fluoro compound include a fluorine-containing surfactant, an oily fluorine-based compound (e.g., fluorine oil), and a solid fluorine compound resin (e.g., ethylene tetrafluoride resin). The organic fluoro compound is described in JP-B-57-9053 (columns 8 to 17), JP-A-61-20994 and JP-A-62-135826.

Examples of the film hardening agent that can be used include the materials described in JP-A-1-161236, page 222, JP-A-9-263036, JP-A-10-119423 and JP-A-2001-310547.

Other examples of the additive added to the image-receiving layer include a pigment dispersant, a thickener, an antifoaming agent, a dye, a fluorescent brightening agent, a preservative, a pH adjusting agent, and a matting agent. Incidentally, the ink-receiving layer may be composed of either one layer or two layers.

In the recording paper and recording film, a backcoat layer may be also provided, and the component which can be added to this layer includes a white pigment, an aqueous binder and other components.

Examples of the white pigment incorporated into the backcoat layer include a white inorganic pigment such as precipitated calcium carbonate, heavy calcium carbonate, kaolin, talc, calcium sulfate, barium sulfate, titanium dioxide, zinc oxide, zinc sulfide, zinc carbonate, satin white, aluminum silicate, diatomaceous earth, calcium silicate, magnesium silicate, synthetic amorphous silica, colloidal silica, colloidal alumina, pseudo-boehmite, aluminum hydroxide, alumina, lithopone, zeolite, hydrated halloysite, magnesium carbonate and magnesium hydroxide, and an organic pigment such as styrene-based plastic pigment, acrylic plastic pigment, polyethylene, microcapsule, urea resin and melamine resin.

Examples of the aqueous binder incorporated into the backcoat layer include a water-soluble polymer such as styrene/maleate copolymer, styrene/acrylate copolymer, polyvinyl alcohol, silanol-modified polyvinyl alcohol, starch, cationized starch, casein, gelatin, carboxymethyl cellulose, hydroxyethyl cellulose and polyvinylpyrrolidone, and a water-dispersible polymer such as styrene-butadiene latex and acrylic emulsion. Other components incorporated into the backcoat layer include an antifoaming agent, a foam inhibitor, a dye, a fluorescent brightening agent, a preservative and a water-proofing agent.

In a constituent layer (including the back layer) of the inkjet recording paper and recording film, a fine polymer particle dispersion may be added. The fine polymer particle dispersion is used for the purpose of improving film properties, for example, stabilizing the dimension or preventing the curling, adhesion or film cracking. The fine polymer particle dispersion is described in JP-A-62-245258, JP-A-62-136648 and JP-A-62-110066. When a fine polymer particle dispersion having a low glass transition temperature (40°C or less) is added to a layer containing the mordant, layer cracking or curling can be prevented. The curling can be also prevented even by adding a fine polymer particle dispersion having a high glass transition temperature to the back layer.

The ink composition of the present invention can be also used for uses other than inkjet recording, such as material for display image, image-forming material for interior decoration and image-forming material for outdoor decoration.

The material for display image indicates various materials such as poster, wall paper, ornamental article (e.g., decorative figurine, doll), handbill for commercial advertisement, wrapping paper, wrapping material, paper bag, vinyl bag, package material, billboard, image drawn on or attached to a side face of transportation facilities (such as automobile, bus and electric car), and logoed clothes. In the case of using the dye of the present invention as the material for forming a display image, the image encompasses not only an image in a narrow sense but also all patterns formed by the dye, which can be acknowledged by a human, such as abstract design, character and geometrical pattern.

The material for interior decoration indicates various materials such as wallpaper, ornamental article (e.g., decorative figurine, doll), luminaire member, furniture member and design member of floor or ceiling. In the case of using the dye of the present invention as the material for forming an image, the image encompasses not only an image in a narrow sense but also all patterns formed by the dye, which perceptible to a human, such as abstract design, character and geometrical pattern.

The material for outdoor decoration indicates various materials such as wall material, roofing material, billboard, gardening material, outdoor ornamental article (e.g., decorative figurine, doll) and outdoor luminaire member. In the case of using the dye of the present invention as the material for forming an image, the image encompasses not only an image in a narrow sense but also all patterns formed by the dye, which can be acknowledged by a human, such as abstract design, character and geometrical pattern.

In these uses, the medium on which the pattern is formed includes various materials such as paper, fiber, cloth (including non-woven fabric), plastic, metal and ceramic. As for the dyeing mode, the dye can be fixed by mordanting or printing or in the form of a reactive dye into which a reactive group is introduced. Among these, dyeing in the mordanting mode is preferred.

In the production of the ink stock solution or ink composition, ultrasonic vibration can be also applied in the process of dissolving additives such as dye.

The ultrasonic vibration is an action of previously applying an ultrasonic energy equivalent to or greater than the energy received by the ink head, in the production process of the ink composition so as to remove an air bubble and thereby prevent the ink composition from producing an air bubble due to pressure applied at the recording head.

The ultrasonic vibration is generated by an ultrasonic wave having a frequency of usually 20 KHz or more, preferably 40 KHz or more, more preferably 50 KHz. Also, the energy applied to the solution by the ultrasonic vibration is usually $2\times10^7$ J/m$^3$ or more, preferably $5\times10^7$ J/m$^3$ or more, more preferably $1\times10^8$ J/m$^3$ or more. Furthermore, the time for which the ultrasonic vibration is applied is usually on the order of 10 minutes to one hour.

The step of applying the ultrasonic vibration produces an effect whenever performed as long as it is after charging of the dye into the medium. The effect is exhibited even when the ultrasonic vibration is applied after the finished

ink composition is once stored. However, the ultrasonic vibration is preferably applied at the time of dissolving and/or dispersing the dye in the medium, because the effect of removing an air bubble is greater and dissolution and/or dispersion of the colorant in the medium is accelerated by the ultrasonic vibration.

That is, the step of applying at least ultrasonic vibration may be performed either during or after the process of dissolving and/or dispersing the dye in the medium. In other words, the step of applying at least ultrasonic vibration can be arbitrarily performed once or more between the preparation of the ink composition and the completion as a product.

As the mode for carrying out the present invention, the process of dissolving and/or dispersing the dye in a medium preferably includes a step of dissolving the dye in a part of the whole medium and a step of mixing the remaining medium therewith, and the ultrasonic vibration is preferably applied at least in either one of these steps. It is more preferred to apply the ultrasonic vibration at least in the step of dissolving the dye in a part of the whole medium.

The step of mixing the remaining medium may be of either single-step or multi-step. Also, in the production of the ink composition according to the present invention, deaeration by heating or deaeration under reduced pressure is preferably used in combination, because the effect of removing an air bubble in the ink composition is enhanced. The step for deaeration by heating or deaeration under reduced pressure is preferably performed simultaneously with or after the step of mixing the remaining medium. The device for generating ultrasonic vibration in the step of applying the ultrasonic vibration includes a known apparatus such as ultrasonic disperser.

At the production of the ink stock solution or ink composition of the present invention, a step of removing dust as a solid matter by filtration, which is further performed after the solution or composition preparation, is important. This operation uses a barrier filter, and as the barrier filter here, a filter having an effective diameter of 1 $\mu$m or less, preferably from 0.05 to 0.3 $\mu$m, more preferably from 0.25 to 0.3 $\mu$m, is used. As the material of the filter, various materials may be used, but particularly, in the case where the ink composition contains a water-soluble dye, a filter produced for an aqueous solvent is preferably used. Among others, it is preferred to use a filter made of a polymer material that is less likely to produce a waste. As for the filtration method, the solution may be transferred to pass through a filter, and either method of pressure filtration or vacuum filtration may be also utilized.

After the filtration, air is often entrained in the solution. In many cases, a bubble attributable to this air also gives rise to distortion of an image in the inkjet recording, and therefore, the above-described defoaming step is preferably provided separately. As for the defoaming method, the solution after filtration may be left standing still, or various methods using a commercially available device, such as ultrasonic defoaming and vacuum defoaming, can be utilized. In the case of ultrasonic defoaming, the defoaming operation is preferably performed for 30 seconds to 2 hours, more preferably for approximately from 5 minutes to one hour.

In order to prevent contamination with dust during operation, these operations are preferably performed by utilizing a space such as clean room or clean bench. In the present invention, the operation is preferably performed in a space having a cleanliness of class 1,000 or less. The "cleanliness" as used herein indicates a value measured by a dust counter.

In the present invention, the hitting volume of the ink composition on a recording material is from 0.1 to 100 pl. The hitting volume is preferably from 0.5 to 50 pl, more preferably from 2 to 50 pl.

[Ink for Inkjet Recording, Inkjet Recording Method, Ink Cartridge for Inkjet Recording, Inkjet Recording Device, and Inkjet Recorded Material]

The ink for inkjet recording of the present invention contains the above-described aqueous solution or ink composition of the present invention.

The inkjet recording method of the present invention is a method of forming a colored image (sometimes simply referred to as image) on a material on which an image is recorded (recording material) by using the ink for inkjet recording.

In the present invention, the inkjet recording method is not limited as long as it is a method of performing image recording by an inkjet printer by using the ink composition or ink set of the present invention, and the method is used for a known system, for example, an electric charge controlling system of jetting out the ink composition by utilizing the electrostatic induction force, a drop-on-demand system (pressure pulse system) of utilizing an oscillation pressure of a piezoelectric element, an acoustic inkjet system of converting electric signals into acoustic beams, irradiating the ink composition with the beams, and jetting out the ink composition by utilizing the radiation pressure, and a thermal inkjet (bubble jet (registered trademark)) system of heating the ink composition to form an air bubble and utilizing the generated pressure.

The inkjet recording system includes a system of ejecting a large number of small-volume droplets of a so-called photo ink that is an ink composition having a low concentration, a system of using a plurality of ink compositions having substantially the same hue but differing in the concentration and thereby improving the image quality, and a system using a colorless transparent ink composition. The hitting volume of the ink composition is controlled mainly by a printer head.

For example, in the case of a thermal inkjet system, the hitting volume can be controlled by the structure of the

printer head. That is, the ink composition can be hit in a desired size by changing the ink chamber, heating section and nozzle size. Also, even in a thermal inkjet system, hitting in a plurality of sizes can be realized by providing a plurality of printer heads differing in the heating section or nozzle size. In the case of a drop-on-demand system using a piezoelectric element, similarly to the thermal inkjet system, the hitting volume can be changed by the printer head structure, but, as described later, hitting in a plurality of sizes can be performed with printer heads having the same structure by controlling the waveform of driving signals for driving the piezoelectric element.

The ejection frequency on hitting the ink composition of the present invention on a recording material is preferably 1 KHz or more.

In order to record a high-quality image like a photograph, the hitting density must be 600 dpi (number of dots per inch) or more so that an image having high sharpness can be reproduced by a small ink droplet.

On the other hand, in hitting the ink composition by a head having a plurality of nozzles, the number of heads which can be driven at the same time is restricted, that is, from a few tens to about 200 in the type where a recording paper and a head are moved in the directions orthogonal to each other, and a few hundreds even in the type called a line head where the head is fixed. This is because the driving electric power is limited or due to the effect of heat generated in the head on the image, a large number of head nozzles cannot be simultaneously driven.

Here, the recording rate can be increased by raising the driving frequency. The hitting frequency can be controlled, in the case of a thermal inkjet system, by controlling the frequency of head-driving signal for heating the head.

In the case of a piezoelectric system, the hitting frequency can be controlled by controlling the frequency of signal for driving the piezoelectric head. The driving of the piezoelectric head is described. The hitting size, hitting speed and hitting frequency are determined in a printer control section based on the signal of an image to be printed, and a signal for driving a printer head is prepared. The driving signal is supplied to the printer head and by the signal for driving the piezoelectric head, the hitting size, hitting speed and hitting frequency are controlled. Here, the hitting size and hitting speed are determined by the shape and amplitude of the driving waveform, and the frequency is determined by the signal repetition period.

When the hitting frequency is set to 10 KHz, the head is driven every 100 micro-seconds and one-line recording is completed in 400 micro-seconds. When the travelling speed of recording paper is set such that the recording paper moves 1/600 inch, namely, about 42 microns, in 400 micro-seconds, the printing can be performed at a rate of one sheet per 1.2 seconds.

The present invention relates also to an ink cartridge for inkjet recording, which is filled with the above-described ink for inkjet recording.

As for the configurations of the printing device and printer for use in the present invention, the embodiments disclosed, for example, in JP-A-11-170527 are suitable. Also, as for the ink cartridge, those disclosed, for example, in JP-A-5-229133 are suitable. With respect to the suction and the configurations of, for example, a cap covering a printing head during suction, those disclosed, for example, in JP-A-7-276671 are suitable. Furthermore, a filter for removing an air bubble disclosed in JP-A-9-277552 is preferably provided in the vicinity of the head.

In addition, the nozzle surface is preferably subjected to a water repelling treatment described in JP-A-2002-292878. The application may be either a printer connected to a computer or a device specialized for printing a picture.

In the inkjet recording method applied to the present invention, the average hitting speed when hitting the ink composition on a recording material is preferably 2 m/sec or more, more preferably 5 m/sec or more. The hitting speed is controlled by controlling the shape and amplitude of the head driving waveform. Also, hitting in a plurality of sizes can be performed with the same head by appropriately using a plurality of driving waveforms according to the hitting size.

The recording paper and recording film, which are an example of the recording material used for performing inkjet printing by using the ink of the present invention, are described below.

As the support of the recording paper and recording film, those composed of, for example, a chemical pulp such as LBKP and NBKP, a mechanical pulp such as GP, PGW, RMP, TMP, CTMP, CMP and CGP, or a waste paper pulp such as DIP, and after mixing, if desired, conventionally known additives such as pigment, binder, sizing agent, fixing agent, cationic agent and paper strength increasing agent, produced by using various devices such as Fourdrinier paper machine and cylinder paper machine, can be used. In addition to these supports, the support may be either a synthetic paper or a plastic film sheet. The thickness of the support is preferably from 10 to 250 $\mu$m, and the basis weight thereof is preferably from 10 to 250 g/m$^2$. An ink-receiving layer and a backcoat layer may be directly provided on the support, or after providing a size press or an anchor coat layer by using starch, polyvinyl alcohol or the like, an ink-receiving layer and a backcoat layer may be provided. Furthermore, the support may be subjected to a flattening treatment by a calendering device such as machine calender, TG calender and soft calender. In the present invention, as the support, a paper of which both surfaces are laminated with a polyolefin (for example, polyethylene, polystyrene, polyethylene terephthalate, polybutene or a copolymer thereof), and a plastic film are more preferably used. A white pigment (e.g., titanium oxide, zinc oxide) or a tinting dye (e.g., cobalt blue, ultramarine, neodymium oxide) is preferably added to the polyolefin.

The inkjet recorded material of the present invention is obtained by forming a colored image on a recording

material with use of the ink for inkjet recording of the present invention. Here, formation of the image is suitably achieved by employing the inkjet recording method using the inkjet recording device described above.

## Examples

The present invention is described in greater detail by referring to Examples. The materials, reagents, ratios, devices, operations and the like described in the following Examples can be appropriately modified without departing from the spirit of the present invention. Accordingly, the scope of the present invention is not limited to the following specific examples. Incidentally, in the following Examples, unless otherwise indicated, "%" and "parts" indicate "mass%" and "parts by mass", respectively, and the molecular weight indicates the mass average molecular weight.

[Synthesis Example]

(Synthesis of Compound 1)

The synthesis scheme is shown below.

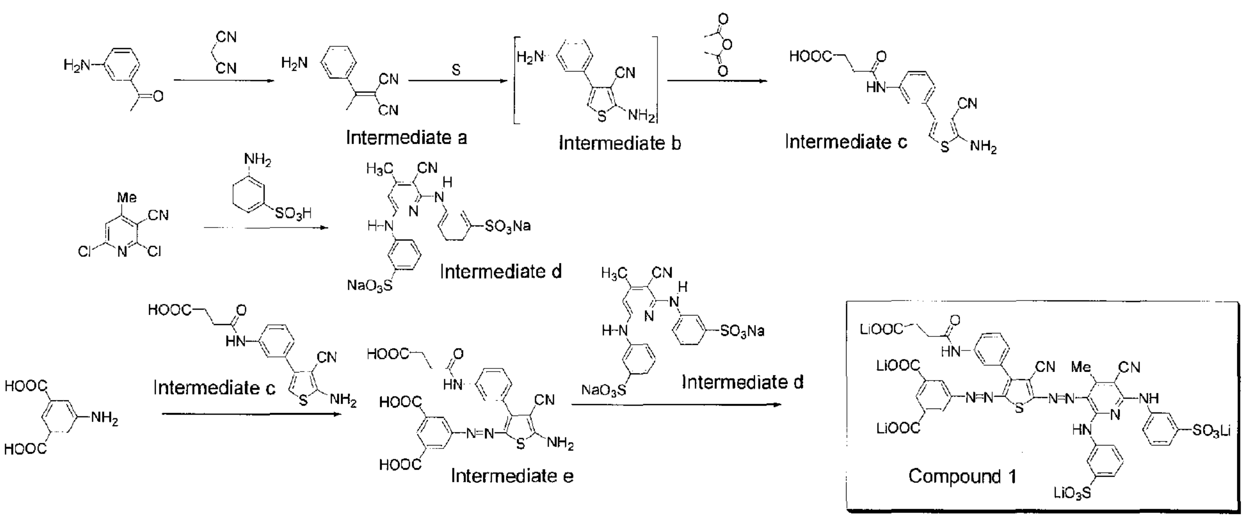


<Synthesis of Intermediate a>

In a three-neck flask, 135 g (1 mol) of 3-aminoacetophenone, 79 g (1.2 mol) of malonotrile, 77 g (1 mol) of ammonium acetate, 100 g of acetic acid and 350 mL of toluene were added and heated to an internal temperature of 100°C. After stirring for 2 hours, the contents were cooled to an internal temperature of 25°C, and 350 mL of methanol was added. The precipitated crystal was separated by filtration, and the crystal was washed with 70 mL of methanol twice and dried at 50°C to obtain 142 g of a yellow crystal of Intermediate a.

<Synthesis of Intermediate c>

In a three-neck flask, 92 g (0.5 mol) of Intermediate a, 16 g of sulfur and 300 mL of methanol were added, and to this suspension, 25 g of triethylamine was added to dropwise. The contents were heated to an internal temperature of 60°C and after stirring for 3 hours, cooled to an internal temperature of 15°C to obtain a methanol solution of Intermediate b. To the obtained methanol solution of Intermediate b, 50 g (0.5 mol) of succinic anhydride was added, followed by stirring at room temperature for 1 hour. The precipitated crystal was separated by filtration, and the crystal was washed with 200 mL of methanol twice and dried at 50°C to obtain 70 g of a yellow crystal of Intermediate c.

<Synthesis of Intermediate d>

56 g (0.3 mol) of 2,6-dichloro-3-cyano-4-methylpyridine, 121 g (0.7 mol) of 3-aminobenzenesulfonic acid and 64 g (0.6 mol) of sodium carbonate were dispersed in 150 mL of NMP (N-methylpyrrolidone) and stirred at 200°C for 8 hours. After cooling to 60°C, 600 mL of methanol was added dropwise. Thereafter, the crystal was separated by filtration, washed with methanol and dried at 50°C to obtain 120 g of a white crystal of Intermediate d.

<Synthesis of Intermediate e>

36 g (0.2 mol) of 5-aminoisophthalic acid was suspended in 200 mL of water, and 17 mL of 12 N hydrochloric acid was added dropwise at room temperature. After cooling in an ice bath to an internal temperature of 4°C, 30 mL of an aqueous solution of 14 g (0.2 mol) of sodium nitrite was added dropwise at an internal temperature of 5°C or less, followed by stirring for 30 minutes. Thereafter, 4 g of amidosulfuric acid was added, and the contents were continuously stirred at an internal temperature of 5°C for 10 minutes to obtain a diazonium solution. Separately, 63 g (0.2 mol) of Intermediate c was suspended in 1,000 mL of water, and the diazonium solution obtained above was added dropwise at room temperature over 20 minutes. The contents were heated to an internal temperature of 40°C, stirred at 40°C for 30 minutes and then cooled to room temperature, and the crystal was separated by filtration, washed with 120 mL of water and 60 mL of isopropyl alcohol and dried at 50°C to obtain 98 g of a brown crystal of Intermediate e.

<Synthesis of Compound 1>

To a suspension of 51 g (0.1 mol) of Intermediate e, 50 g (0.1 mol) of Intermediate d and 1,200 mL of water, 10 mL of an aqueous solution containing 7 g (0.1 mol) of sodium nitride was added dropwise at room temperature. After stirring at an internal temperature of 25°C for 2 hours, an aqueous 4 M lithium hydroxide solution was added dropwise until the pH reached 8.3. Subsequently, 3,000 mL of isopropyl alcohol was added dropwise, and the crystal was separated by filtration and then washed with isopropyl alcohol. The isolated crystal was added to 300 mL of water, and 900 mL of isopropyl alcohol was added dropwise thereto. After stirring for 5 minutes, the crystal was separated by filtration and washed with isopropyl alcohol. The obtained crystal was dissolved in water, and the solution was passed through a cation exchange resin (Amberlite IR-120, produced by Organo Corporation) filled with lithium ion, and then concentrated. The obtained crystal was dried at 50°C to obtain 80 g of a black crystal of Compound 1. As a result of LC-MS, a peak corresponding to Compound 1 was observed.

(Synthesis of Compound 2)

The synthesis scheme is shown below.

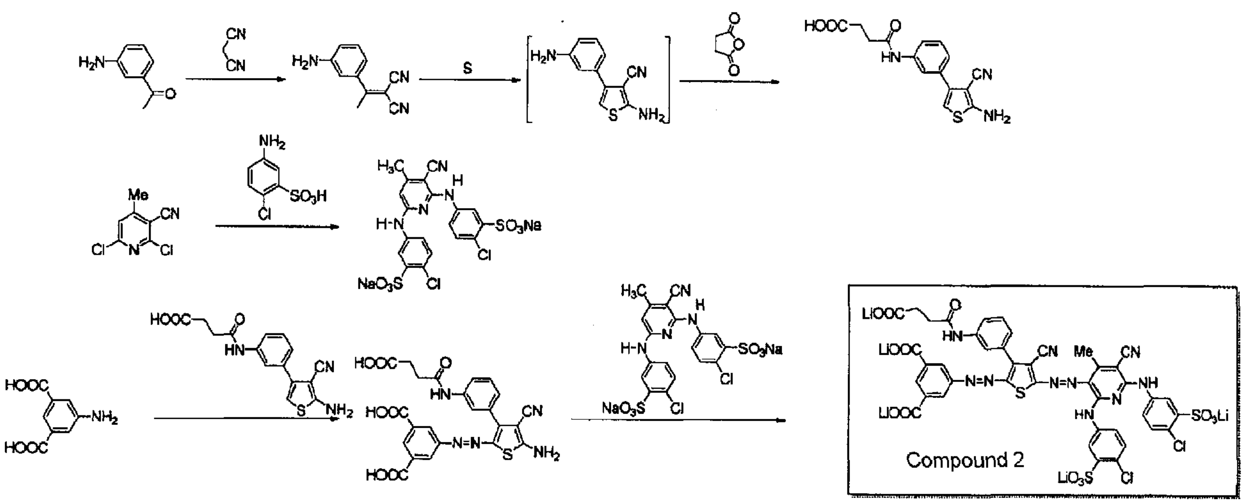

Compound 2

86 g of a black crystal of Compound 2 was obtained in the same manner as in the synthesis method of Compound 1 except for using 4-chloroaniline-3-sulfonic acid in place of 3-aminobenzenesulfonic acid. As a result of LC-MS, a peak corresponding to Compound 2 was observed.

(Synthesis of Compound 3)

The synthesis scheme is shown below.

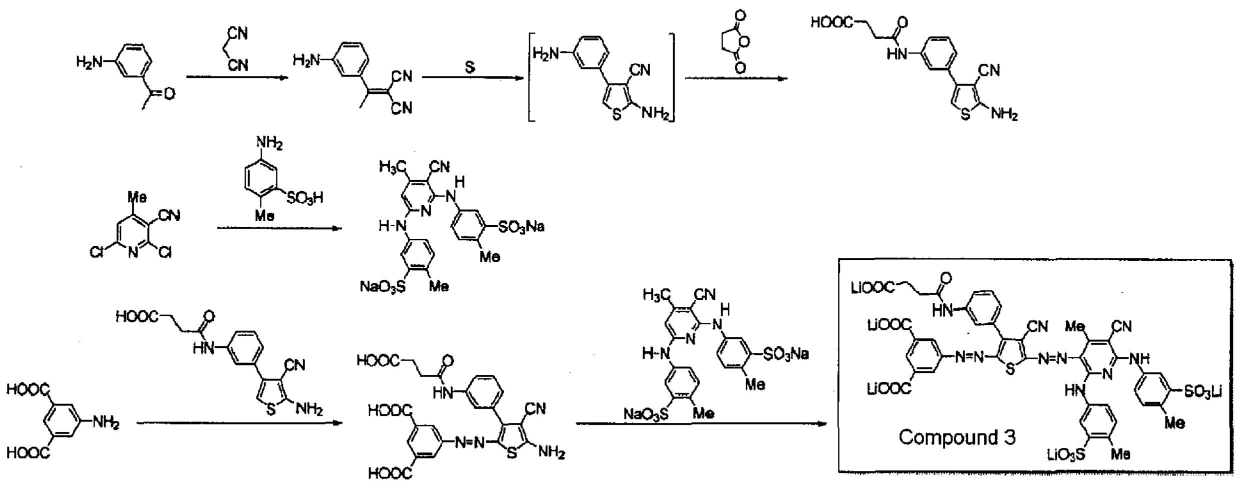


Compound 3

81 g of a black crystal of Compound 3 was obtained in the same manner as in the synthesis method of Compound 1 except for using 4-methylaniline-3-sulfonic acid in place of 3-aminobenzenesulfonic acid. As a result of LC-MS, a peak corresponding to Compound 3 was observed.

(Synthesis of Compound 4)

The synthesis scheme is shown below.

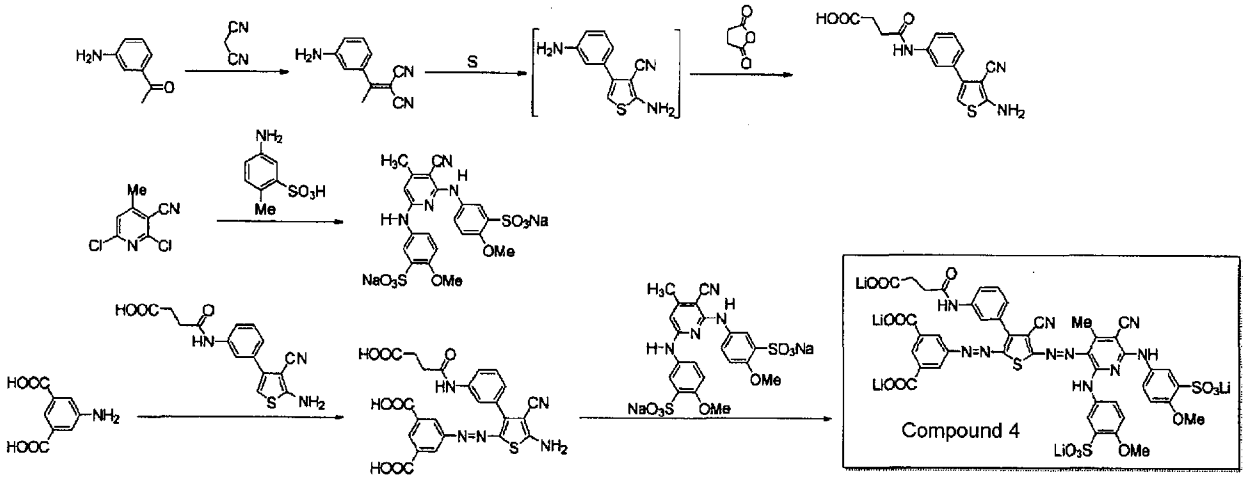


Compound 4

83 g of a black crystal of Compound 4 was obtained in the same manner as in the synthesis method of Compound 1 except for using 4-methoxyaniline-3-sulfonic acid in place of 3-aminobenzenesulfonic acid. As a result of LC-MS, a peak corresponding to Compound 4 was observed.

(Synthesis of Compound 5)

The synthesis scheme is shown below.

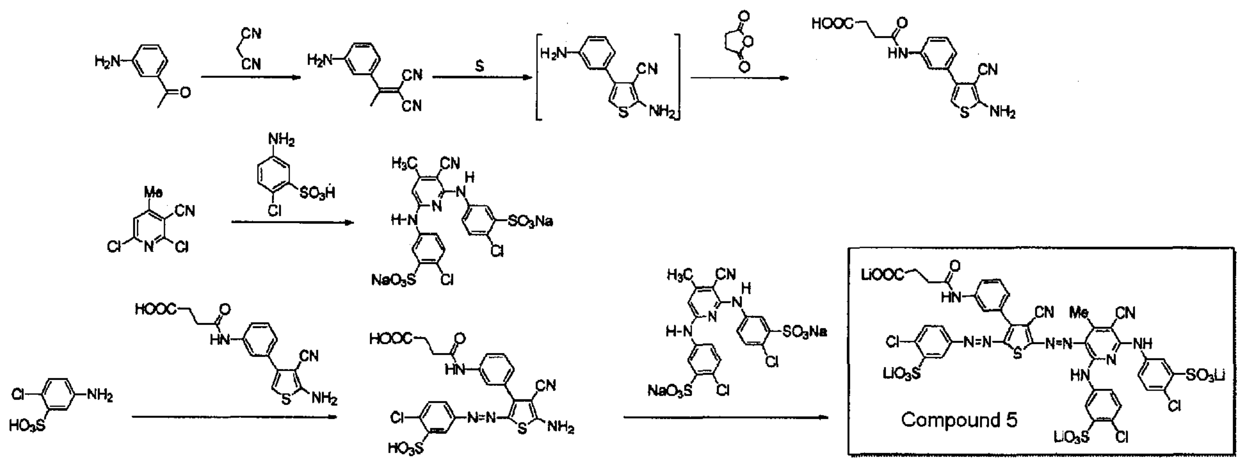


85 g of a black crystal of Compound 5 was obtained in the same manner as in the synthesis method of Compound 2 except for using 4-chloroaniline-3-sulfonic acid in place of 5-aminoisophthalic acid. As a result of LC-MS, a peak corresponding to Compound 5 was observed.

(Synthesis of Compound 6)

The synthesis scheme is shown below.

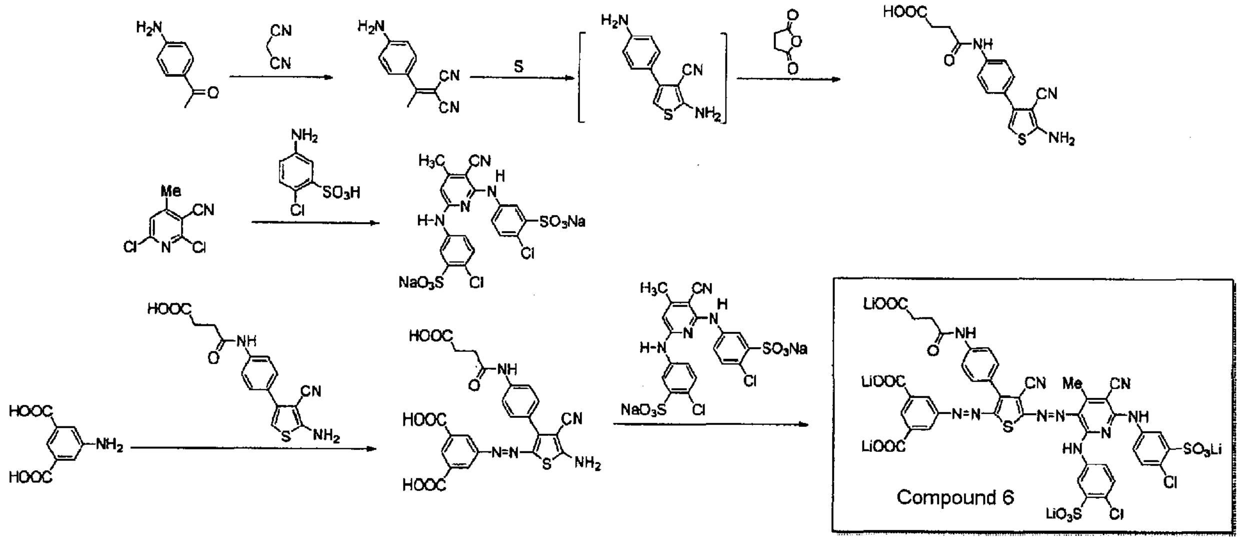


87 g of a black crystal of Compound 6 was obtained in the same manner as in the synthesis method of Compound 2 except for using 4-aminoacetophenone in place of 3-aminoacetophenone. As a result of LC-MS, a peak corresponding to Compound 6 was observed.

(Synthesis of Compound 7)

The synthesis scheme is shown below.

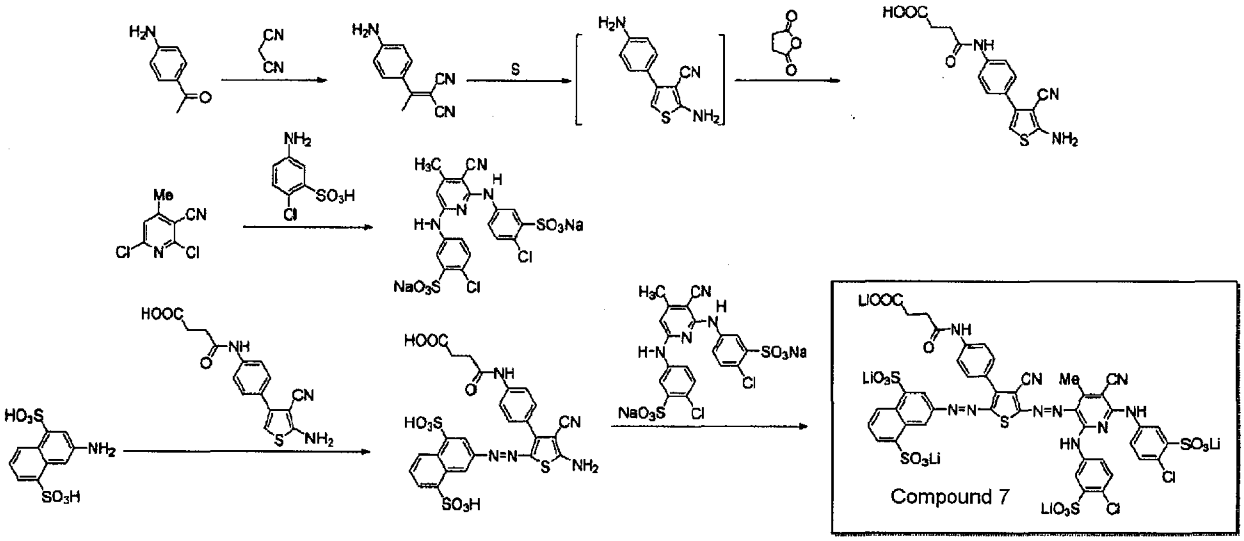


87 g of a black crystal of Compound 7 was obtained in the same manner as in the synthesis method of Compound 6 except for using 3-amino-1,5-naphthalenedisulfonic acid in place of 5-aminoisophthalic acid. As a result of LC-MS, a peak corresponding to Compound 7 was observed.

(Synthesis of Compound 8)

The synthesis scheme is shown below.

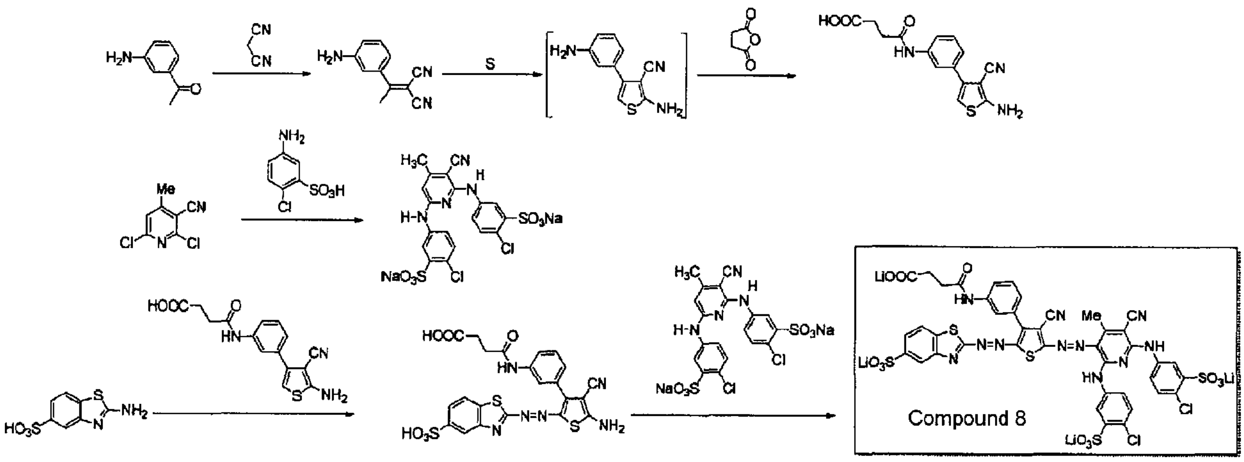


84 g of a black crystal of Compound 8 was obtained in the same manner as in the synthesis method of Compound 2 except for using 2-amino-benzothiazole-5-sulfonic acid in place of 5-aminoisophthalic acid. As a result of LC-MS, a peak corresponding to Compound 8 was observed.

(Synthesis of Compound 9)

The synthesis scheme is shown below.

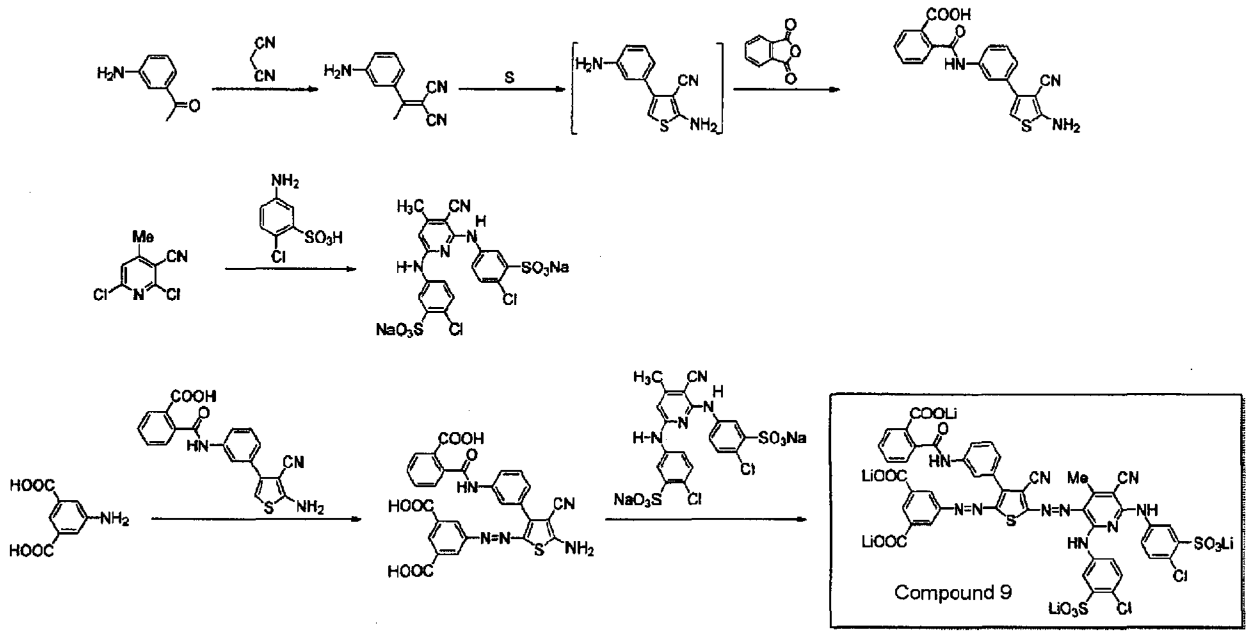

Compound 9

85 g of a black crystal of Compound 9 was obtained in the same manner as in the synthesis method of Compound 2 except for using phthalic anhydride in place succinic anhydride. As a result of LC-MS, a peak corresponding to Compound 9 was observed.

(Synthesis of Compound 10)

10 g of a black crystal of Compound 2 was dissolved in 100 mL of water, and the solution was passed through a cation exchange resin (Amberlite IR-120, produced by Organo Corporation) filled with sodium ion, and then concentrated. The obtained crystal was dried at 50°C to obtain 8 g of a black crystal of Compound 10.

(Synthesis of Compound 11)

10 g of a black crystal of Compound 2 was dissolved in 100 mL of water, and the solution was passed through a cation exchange resin (Amberlite IR-120, produced by Organo Corporation) filled with potassium ion, and then concentrated. The obtained crystal was dried at 50°C to obtain 8 g of a black crystal of Compound 11.

(Synthesis of Compound 12)

10 g of a black crystal of Compound 2 was dissolved in 100 mL of water, and the solution was passed through a cation exchange resin (Amberlite IR-120, produced by Organo Corporation) filled with ammonium ion, and then concentrated. The obtained crystal was dried at 50°C to obtain 7 g of a black crystal of Compound 12.

(Synthesis of Compound 16)

The synthesis scheme is shown below.

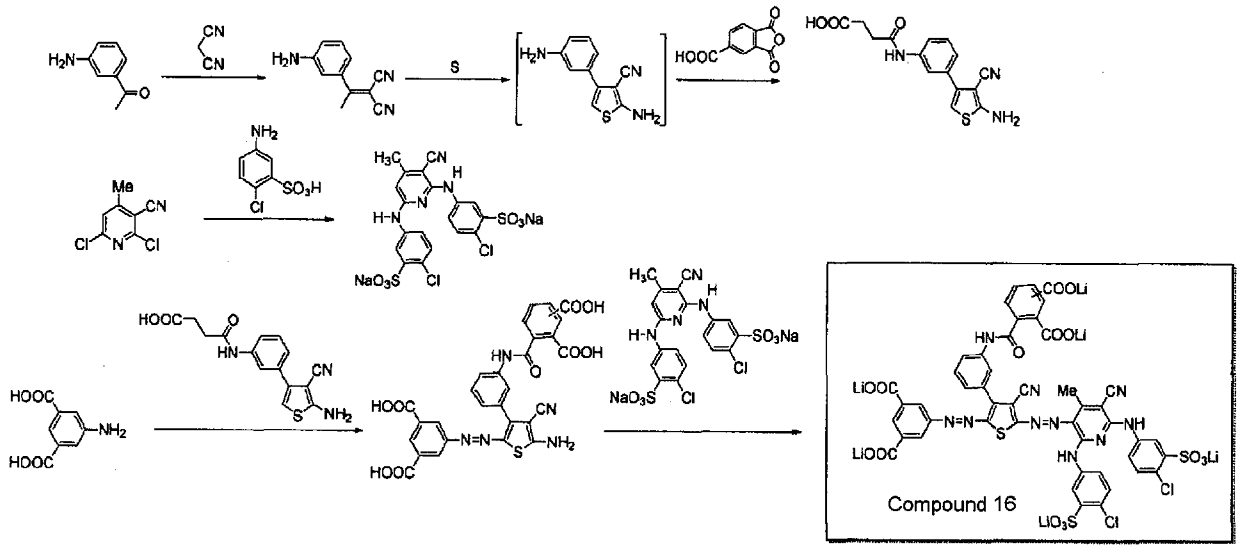


81 g of a black crystal of Compound 16 was obtained in the same manner as in the synthesis method of Compound 2 except for using trimellitic anhydride in place of succinic anhydride. As a result of LC-MS, a peak corresponding to Compound 16 was observed.

(Synthesis of Compound 17)

The synthesis scheme is shown below.

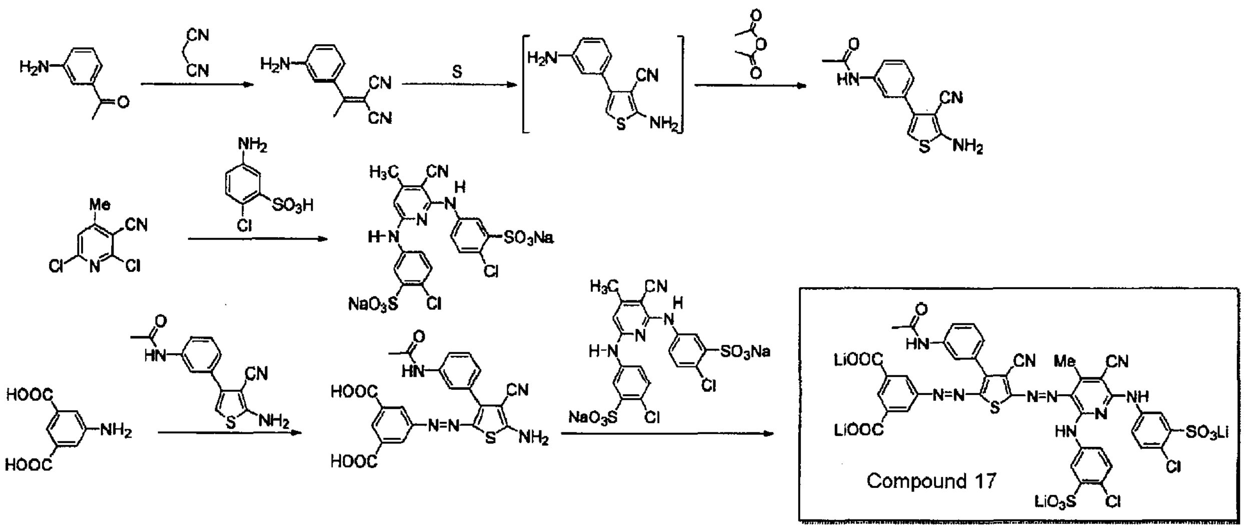


79 g of a black crystal of Compound 17 was obtained in the same manner as in the synthesis method of Compound 2 except for using acetic anhydride in place of succinic anhydride. As a result of LC-MS, a peak corresponding to Compound 17 was observed.

(Synthesis of Compound 18)

The synthesis scheme is shown below.

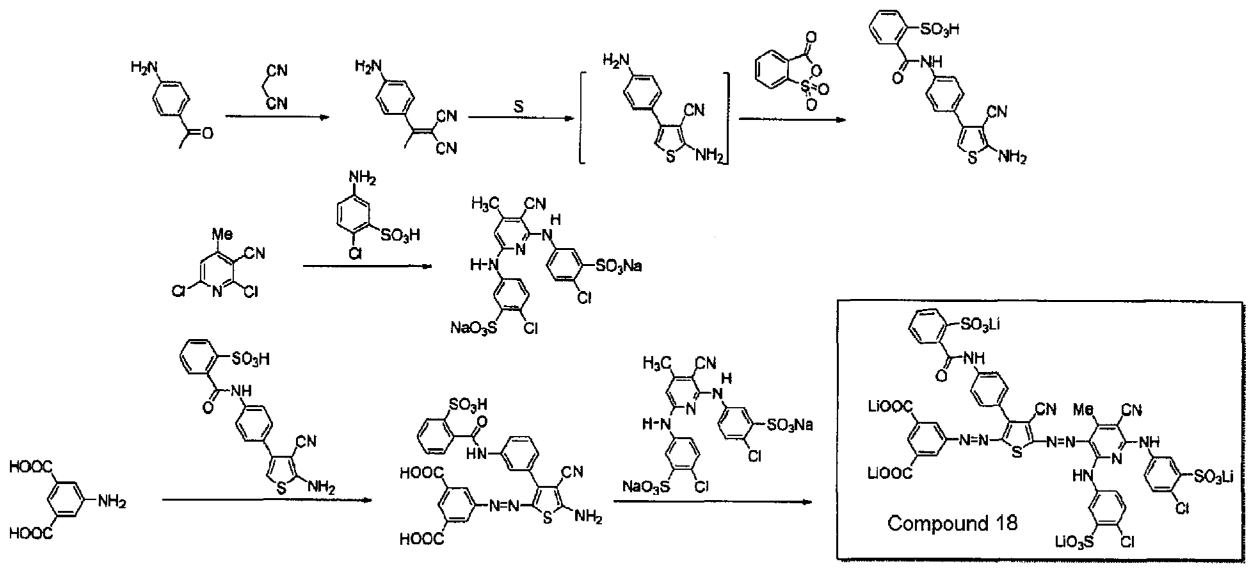


83 g of a black crystal of Compound 18 was obtained in the same manner as in the synthesis method of Compound 2 except for using 2-sulfobenzoic anhydride in place of succinic anhydride. As a result of LC-MS, a peak corresponding to Compound 18 was observed.

(Synthesis of Compound 19)

The synthesis scheme is shown below.

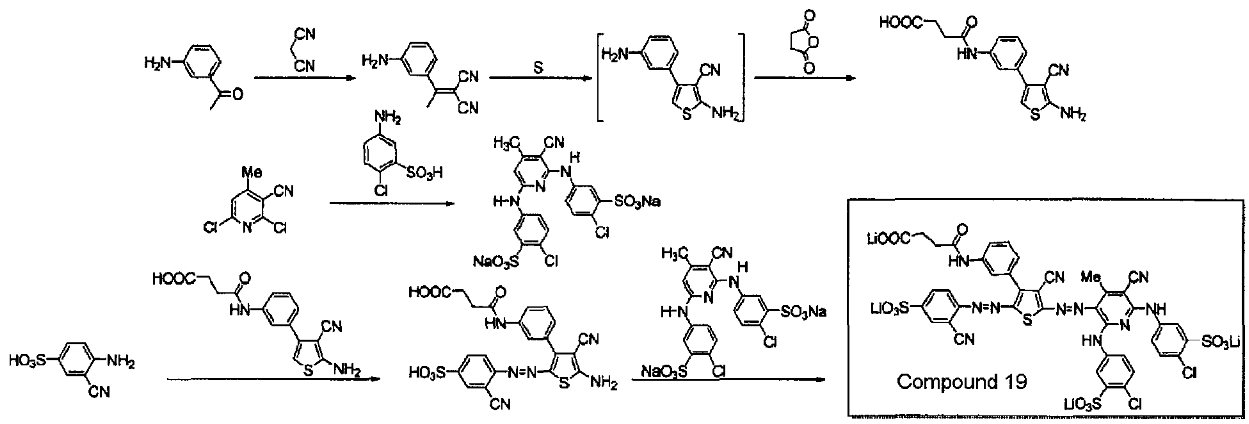


82 g of a black crystal of Compound 19 was obtained in the same manner as in the synthesis method of Compound 2 except for using 3-cyanosulfanyl acid in place of 5-aminoisophthalic acid. As a result of LC-MS, a peak corresponding to Compound 19 was observed.

(Synthesis of Compound 20)

The synthesis scheme is shown below.

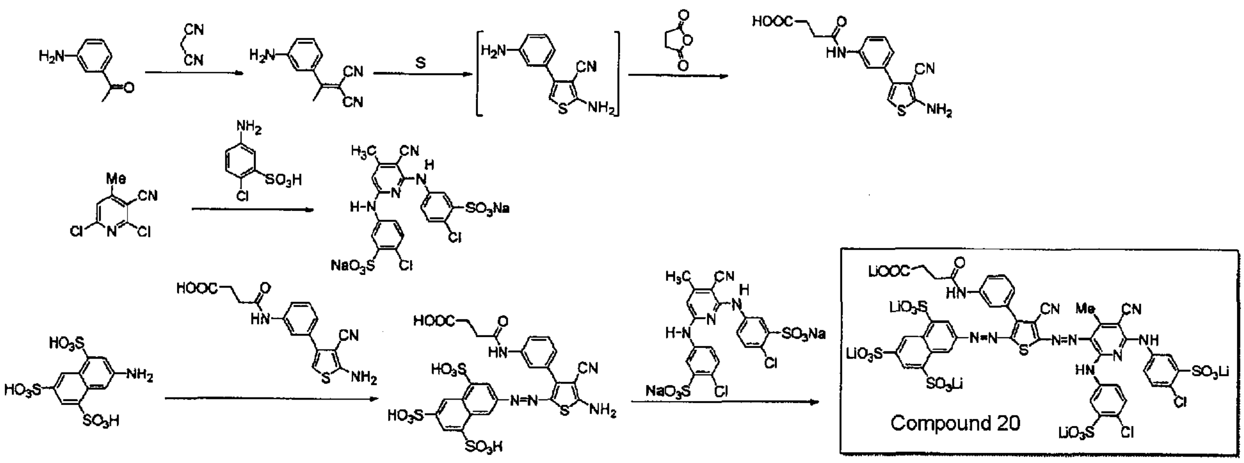

Compound 20

86 g of a black crystal of Compound 20 was obtained in the same manner as in the synthesis method of Compound 2 except for using 7-amino-1,3,5-naphthalenetrisulfonic acid in place of 5-aminoisophthalic acid. As a result of LC-MS, a peak corresponding to Compound 20 was observed.

(Synthesis of Compound 21)

The synthesis scheme is shown below.

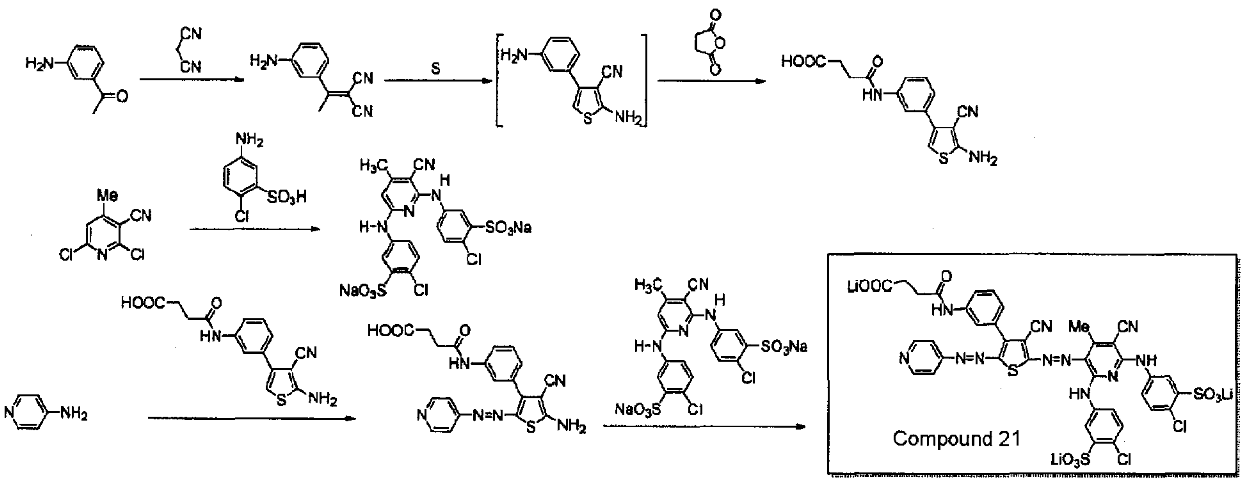

Compound 21

80 g of a black crystal of Compound 21 was obtained in the same manner as in the synthesis method of Compound 2 except for using 4-aminopyridine in place of 5-aminoisophthalic acid. As a result of LC-MS, a peak corresponding to Compound 21 was observed.

(Synthesis of Compound 27)

The synthesis scheme is shown below.

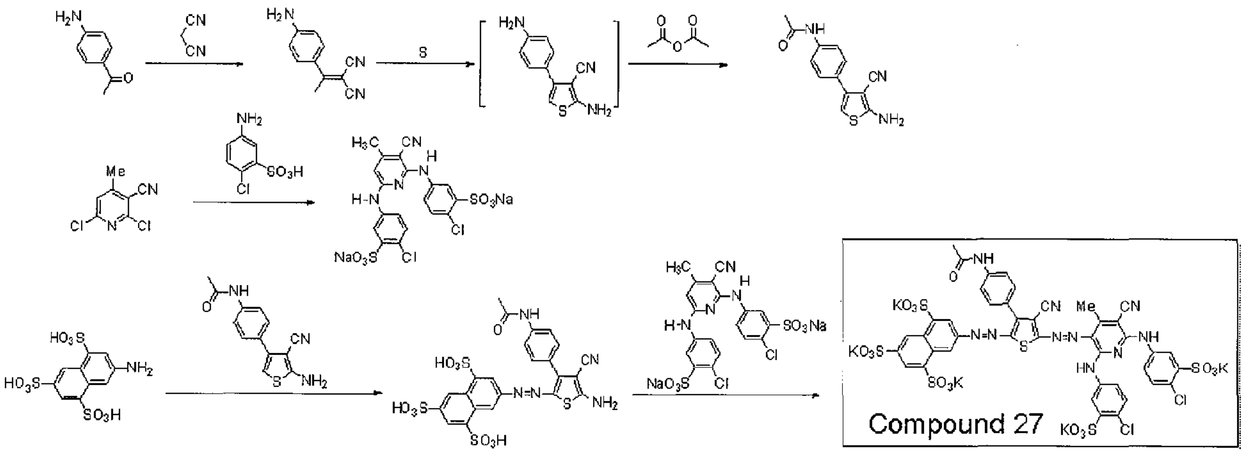


Compound 27 was obtained in the same manner as in the synthesis method of Compound 20 except for using 4-aminoacetophenone in place of 3-aminoacetophenone, using acetic anhydride in place of succinic anhydride, and passing the solution through a cation exchange resin (Amberlite IR-120, produced by Organo Corporation) filled with potassium ion. As a result of LC-MS, a peak corresponding to Compound 27 was observed.

(Synthesis of Compound 29)

The synthesis scheme is shown below.

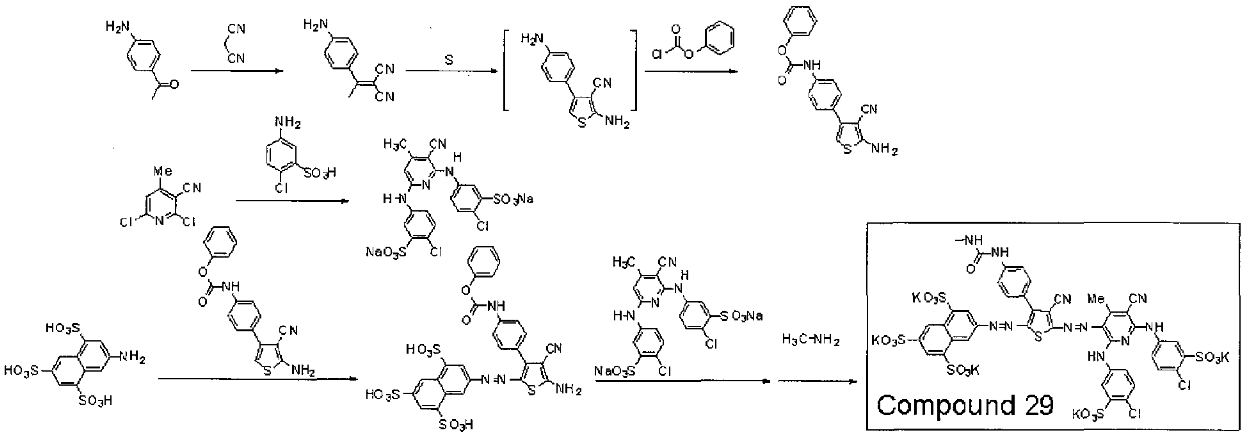


Compound 29 was obtained in the same manner as in the synthesis method of Compound 27 except for using phenyl chloroformate in place of acetic anhydride and reacting methylamine in the final step. As a result of LC-MS, a peak corresponding to Compound 29 was observed.

(Synthesis of Compound 31)

The synthesis scheme is shown below.

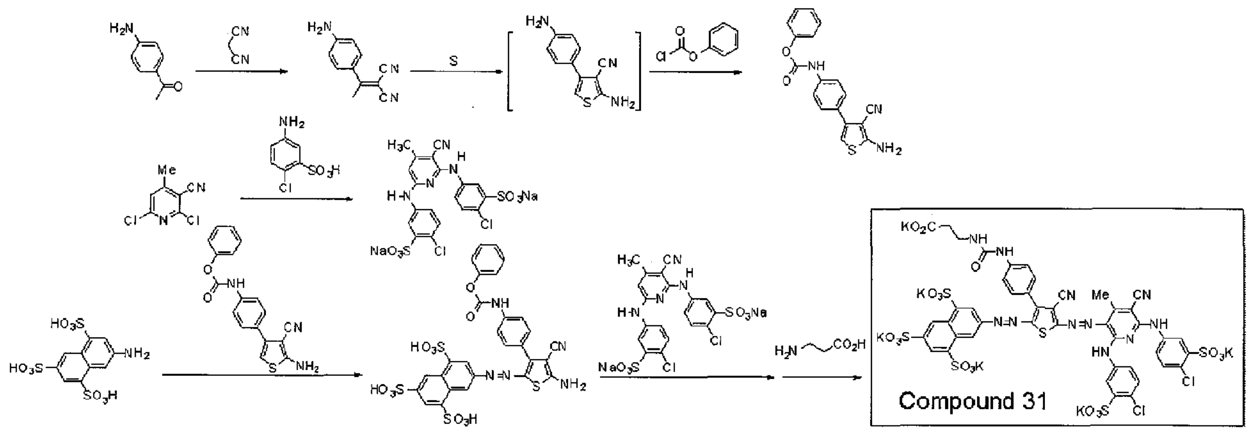

Compound 31

Compound 31 was obtained in the same manner as in the synthesis method of Compound 29 except for using β-alanine in place of methylamine. As a result of LC-MS, a peak corresponding to Compound 31 was observed.

(Synthesis of Compound 32)

The synthesis scheme is shown below.

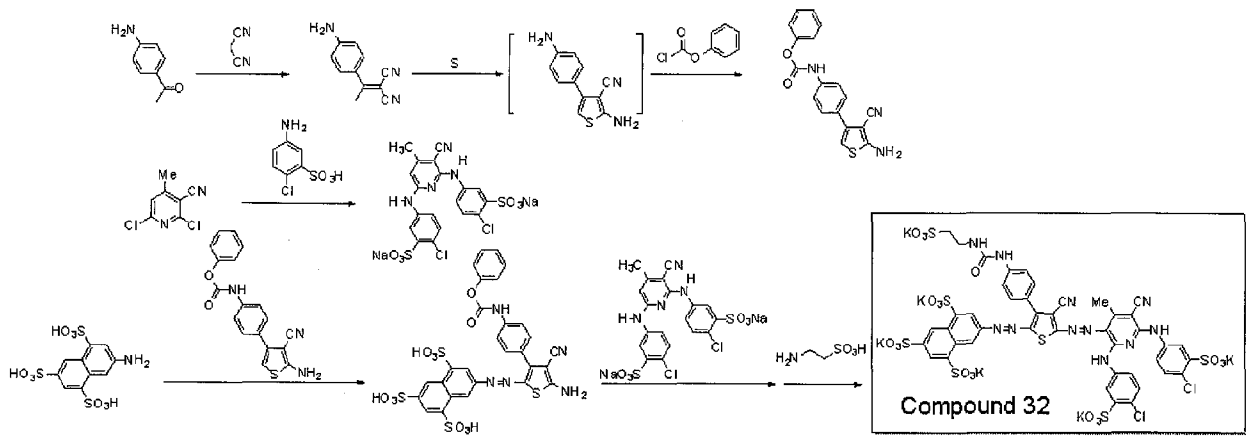

Compound 32

Compound 32 was obtained in the same manner as in the synthesis method of Compound 29 except for using taurine in place of methylamine. As a result of LC-MS, a peak corresponding to Compound 32 was observed.

(Synthesis of Compound 33)

The synthesis scheme is shown below.

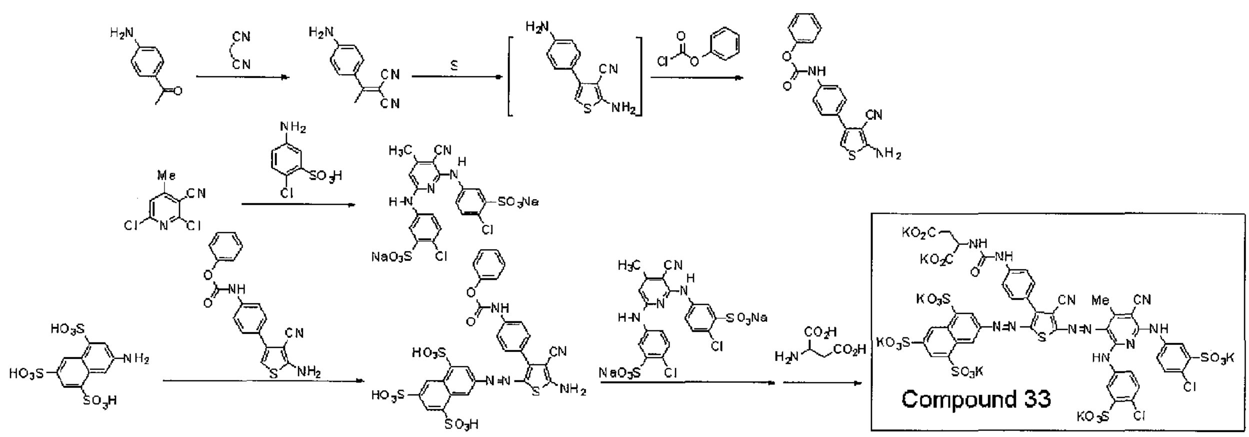

Compound 33

Compound 33 was obtained in the same manner as in the synthesis method of Compound 29 except for using aspartic acid in place of methylamine. As a result of LC-MS, a peak corresponding to Compound 33 was observed.

(Synthesis of Compound 36)

The synthesis scheme is shown below.

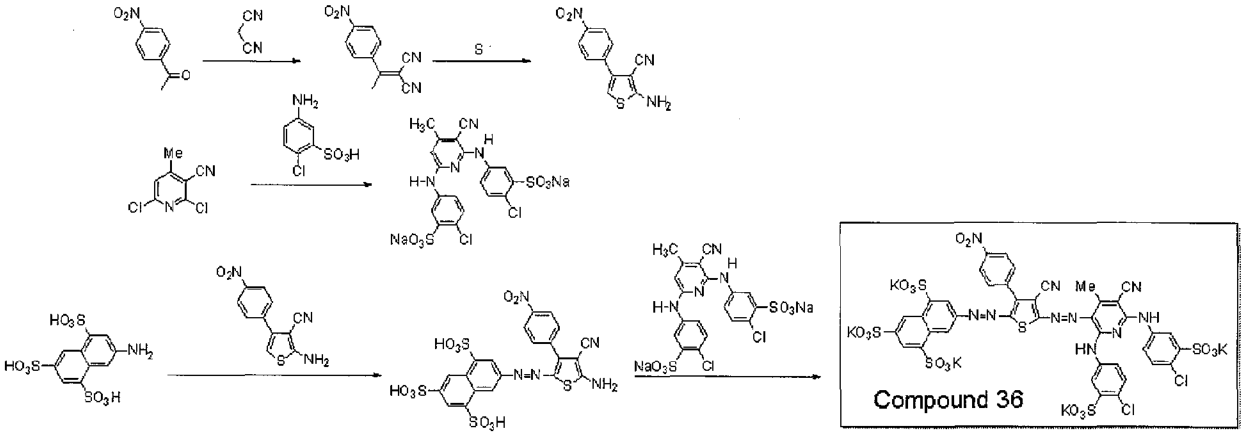


Compound 36 was obtained in the same manner as in the synthesis method of Compound 27 except for using 4-nitroacetophenone in place of 4-aminoacetophenone and performing the synthesis without using acetic anhydride. As a result of LC-MS, a peak corresponding to Compound 36 was observed.

(Synthesis of Compound 38)

The synthesis scheme is shown below.

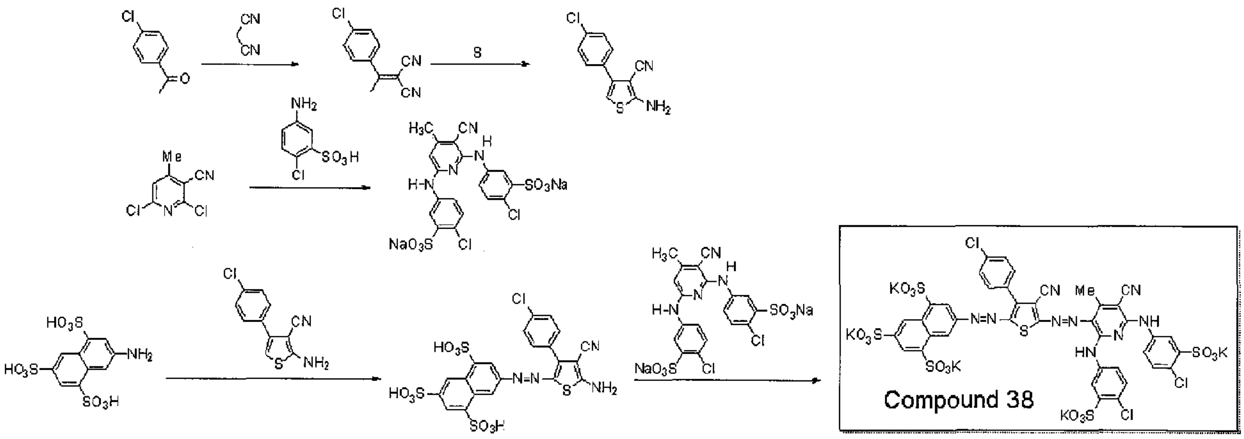


Compound 38 was obtained in the same manner as in the synthesis method of Compound 36 except for using 4-chloroacetophenone in place of 4-nitroacetophenone. As a result of LC-MS, a peak corresponding to Compound 38 was observed.

(Synthesis of Compound 41)

The synthesis scheme is shown below.

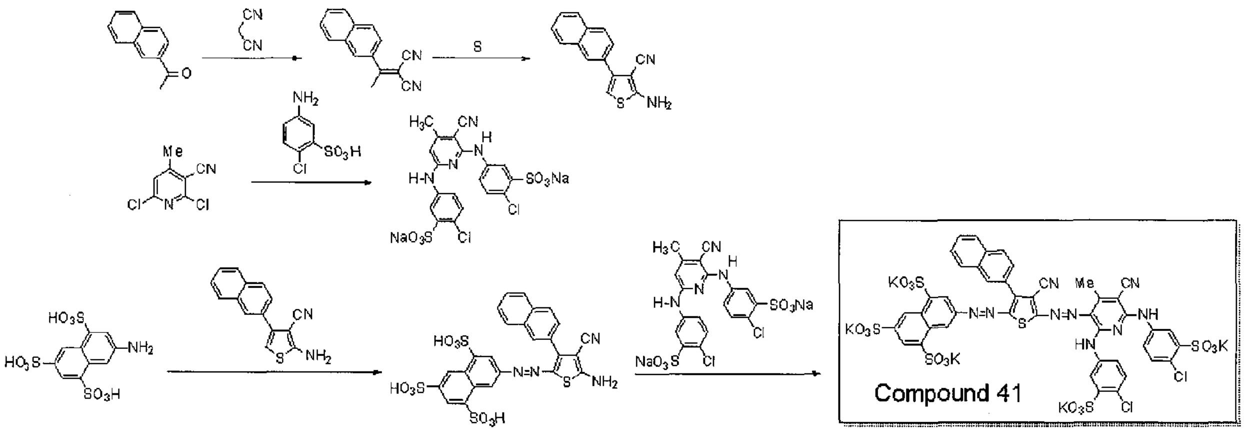


Compound 41

Compound 41 was obtained in the same manner as in the synthesis method of Compound 36 except for using 2-acetonaphthone in place of 4-nitroacetophenone. As a result of LC-MS, a peak corresponding to Compound 41 was observed.

(Synthesis of Compound 42)

The synthesis scheme is shown below.

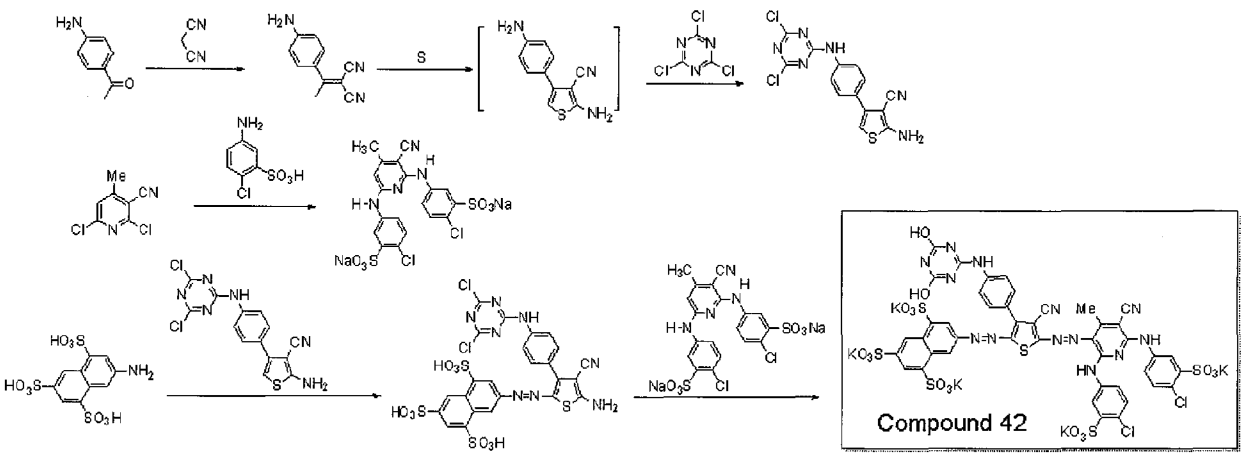


Compound 42

Compound 42 was obtained in the same manner as in the synthesis method of Compound 27 except for using cyanuric chloride in place of acetic anhydride. As a result of LC-MS, a peak corresponding to Compound 42 was observed.

(Synthesis of Compound 50)

The synthesis scheme is shown below.

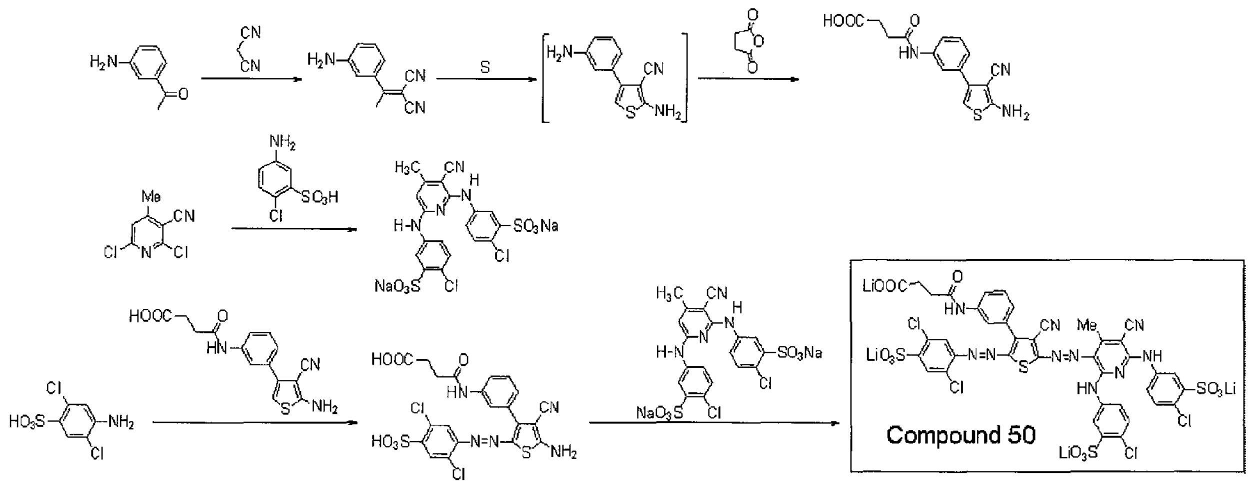


Compound 50 was obtained in the same manner as in the synthesis method of Compound 2 except for using 3,5-dichlorosulfanilic acid in place of 5-aminoisophthalic acid. As a result of LC-MS, a peak corresponding to Compound 50 was observed.

(Synthesis of Compound 51)

A crystal of Compound 50 was dissolved in water, and the solution was passed through a cation exchange resin (Amberlite IR-120, produced by Organo Corporation) filled with sodium ion, and concentrated to obtain Compound 51.

(Synthesis of Compound 57)

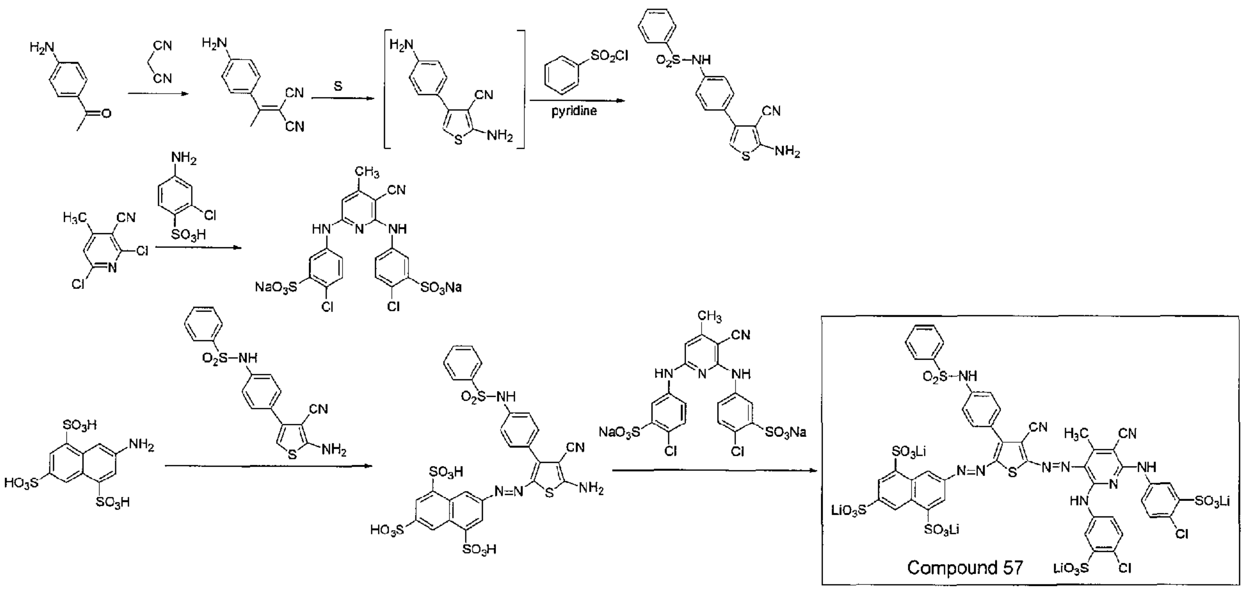


Compound 57 was obtained in the same manner as in the synthesis method of Compound 27 except that benzenesulfonic acid chloride and pyridine in an amount equal to that of the acid chloride were used in place of acetic anhydride and a cation exchange resin filled with lithium ion in place of potassium ion was used. As a result of LC-MS, a peak corresponding to Compound 57 was observed.

(Synthesis of Compound 58)

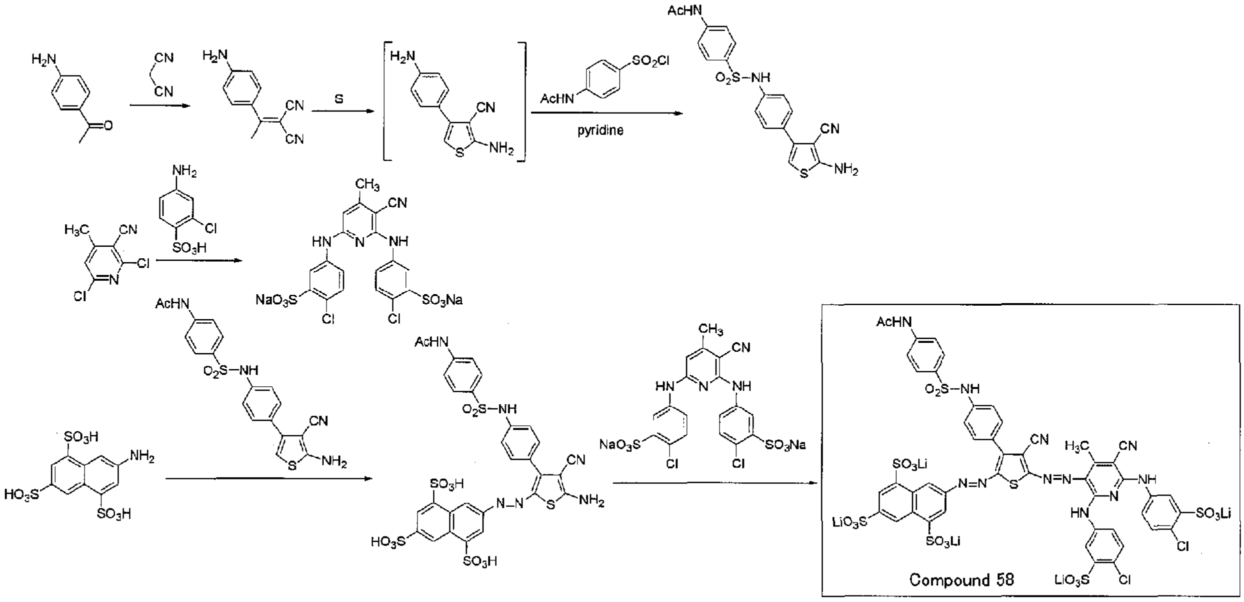


Compound 58

Compound 58 was obtained in the same manner as in the synthesis method of Compound 57 except for using 4-acetaminobenzenesulfonic acid chloride in place of benzenesulfonic acid chloride. As a result of LC-MS, a peak corresponding to Compound 58 was observed.

(Synthesis of Compound 59)

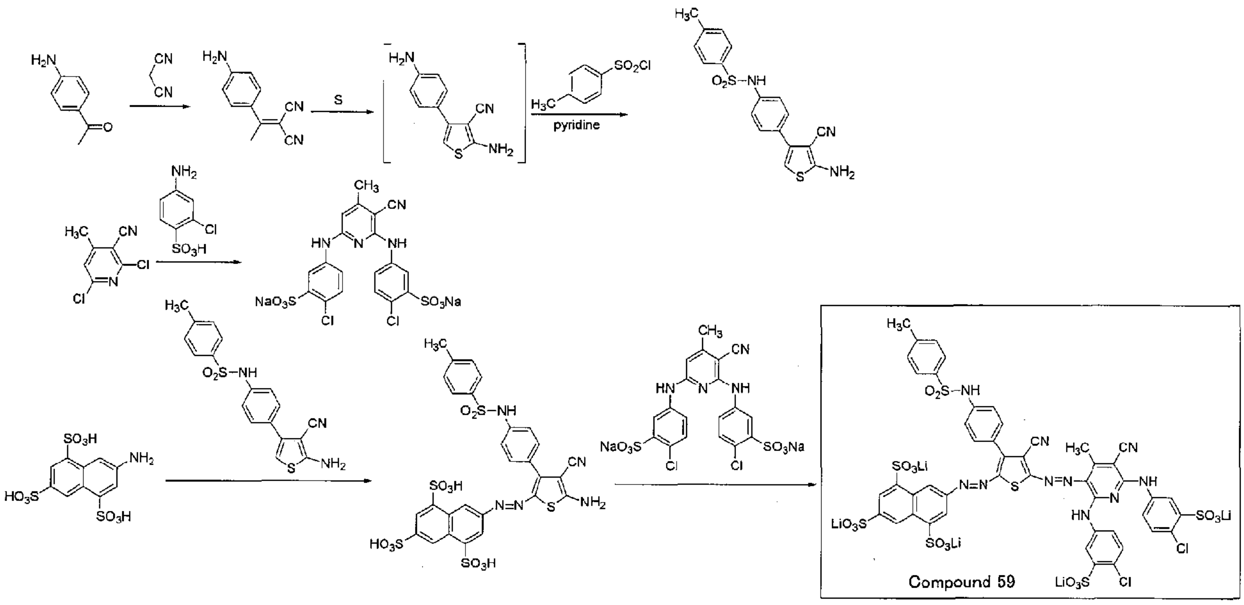


Compound 59

Compound 59 was obtained in the same manner as in the synthesis method of Compound 57 except for using p-toluenesulfonic acid chloride in place of benzenesulfonic acid chloride. As a result of LC-MS, a peak corresponding to Compound 59 was observed.

(Synthesis of Compound 60)

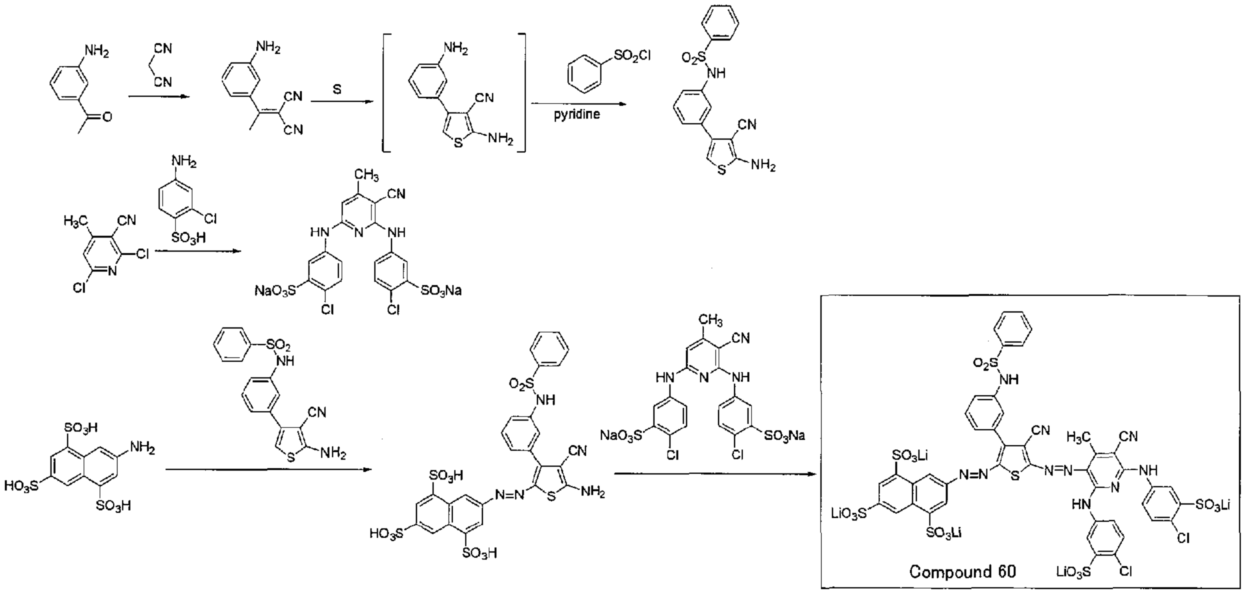


Compound 60

Compound 60 was obtained in the same manner as in the synthesis method of Compound 57 except for using 3-aminoacetophenone in place of 4-aminoacetophenone. As a result of LC-MS, a peak corresponding to Compound 60 was observed.

(Synthesis of Compound 61)

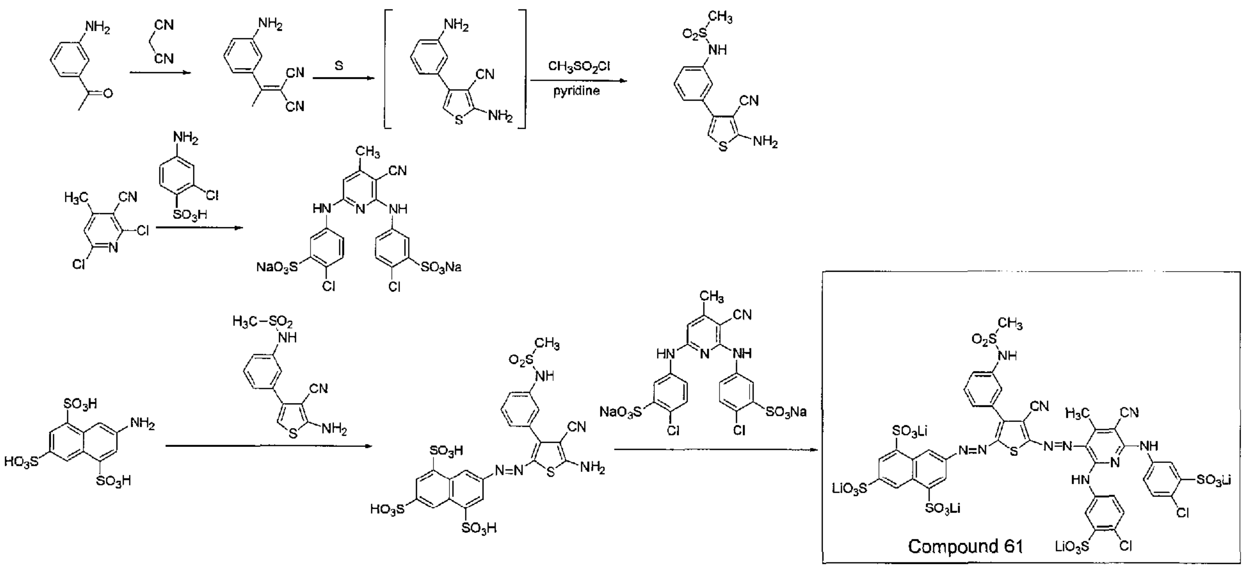


Compound 61

Compound 61 was obtained in the same manner as in the synthesis method of Compound 60 except for using mesyl chloride in place of benzenesulfonic acid chloride. As a result of LC-MS, a peak corresponding to Compound 61 was observed.

(Synthesis of Compound 62)

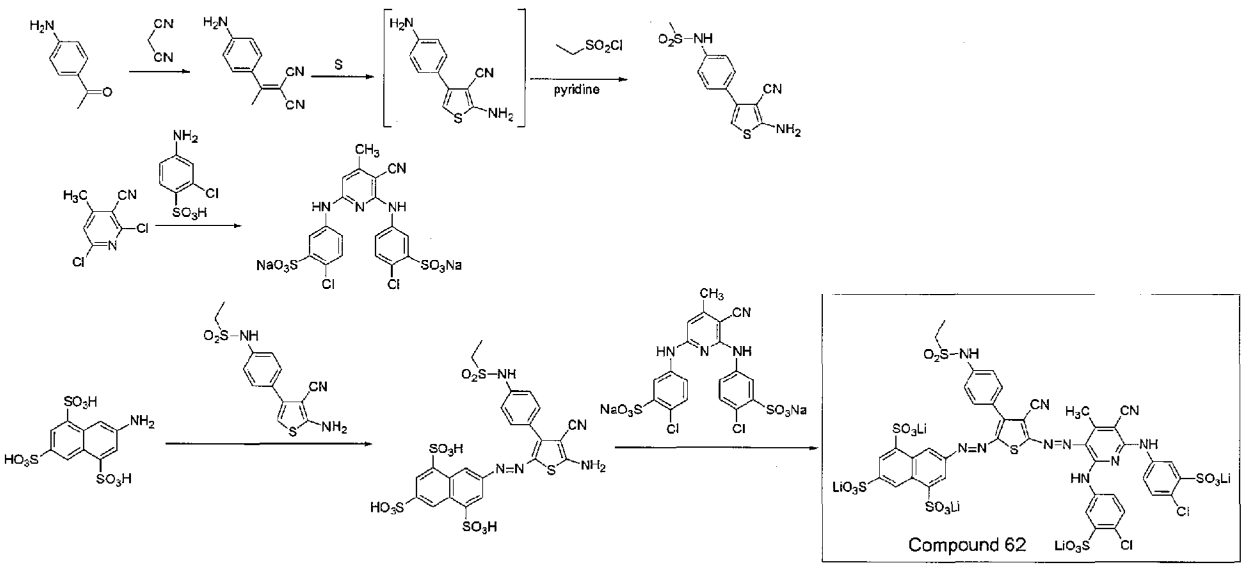


Compound 62 was obtained in the same manner as in the synthesis method of Compound 57 except for using ethanesulfonic acid chloride in place of benzenesulfonic acid chloride. As a result of LC-MS, a peak corresponding to Compound 62 was observed.

(Synthesis of Compound 63)

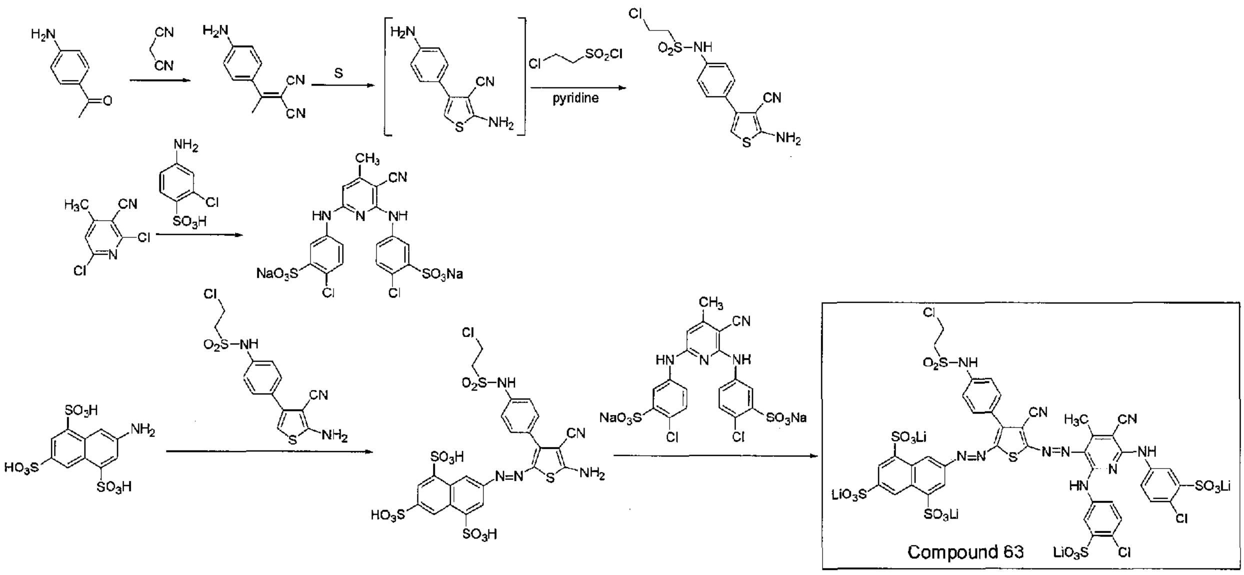


Compound 63 was obtained in the same manner as in the synthesis method of Compound 57 except for using 2-chloroethanesulfonic acid chloride in place of benzenesulfonic acid chloride. As a result of LC-MS, a peak corresponding to Compound 63 was observed.

(Synthesis of Compound 64)

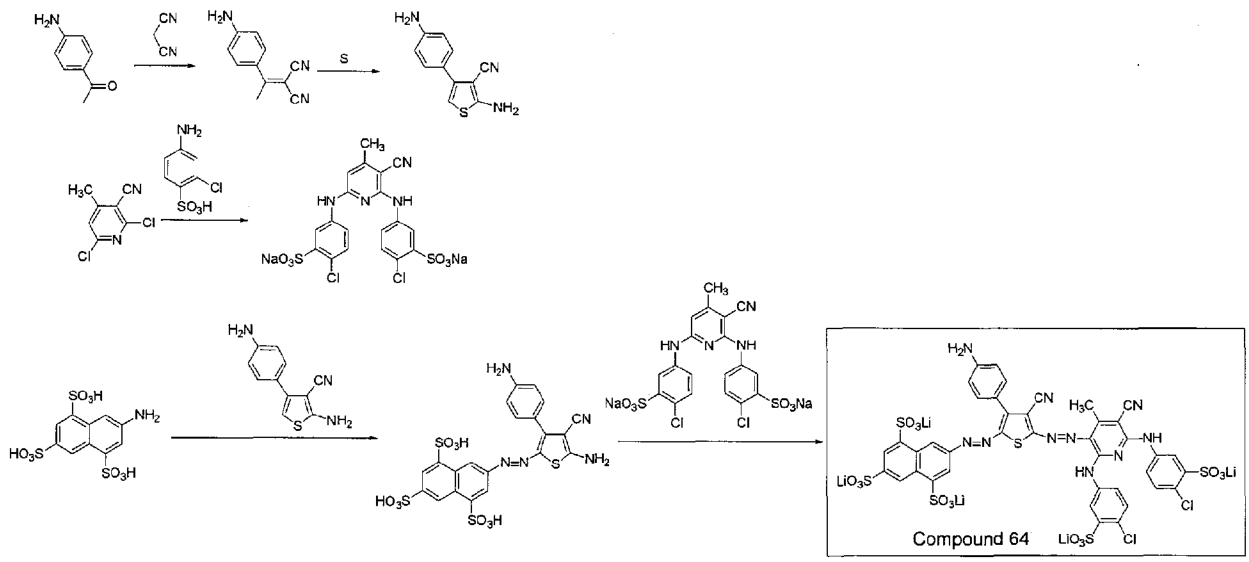

Compound 64

Compound 64 was obtained in the same manner as in the synthesis method of Compound 57 except for not using benzenesulfonic acid chloride and pyridine. As a result of LC-MS, a peak corresponding to Compound 64 was observed.

(Synthesis of Compound 65)

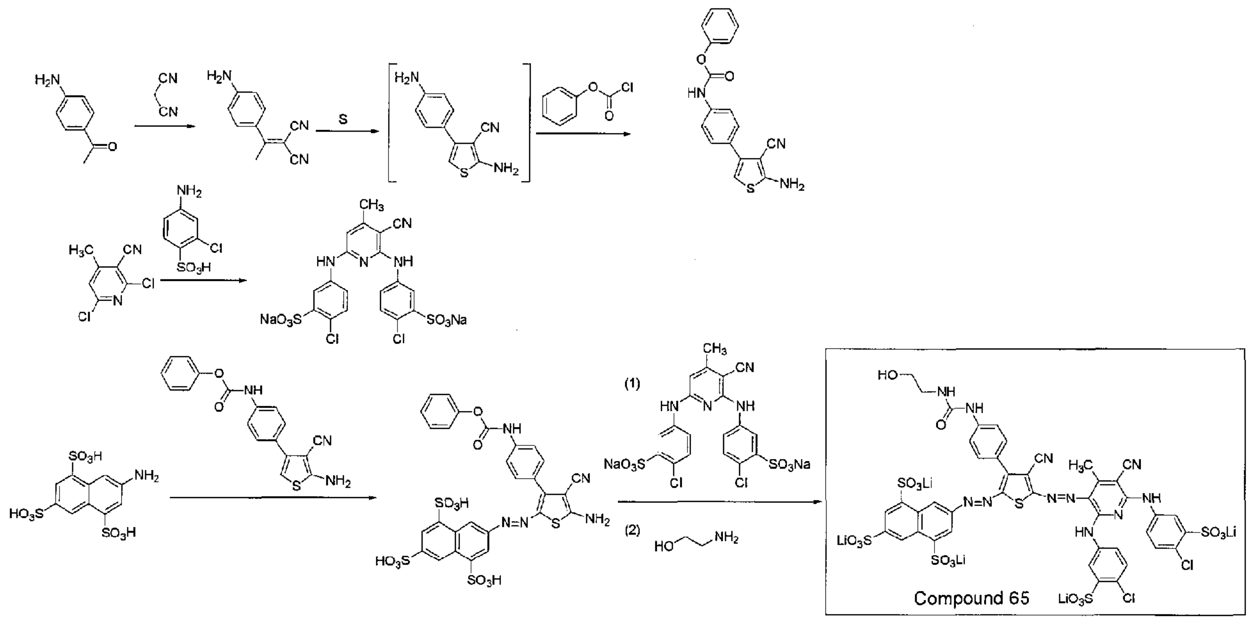

Compound 65

Compound 65 was obtained in the same manner as in the synthesis method of Compound 29 except that 2-aminoethanol was used in place of methylamine after the azo coupling in the final step and a cation exchange resin filled with lithium ion in place of potassium ion was used. As a result of LC-MS, a peak corresponding to Compound 65 was observed.

(Synthesis of Compound 66)

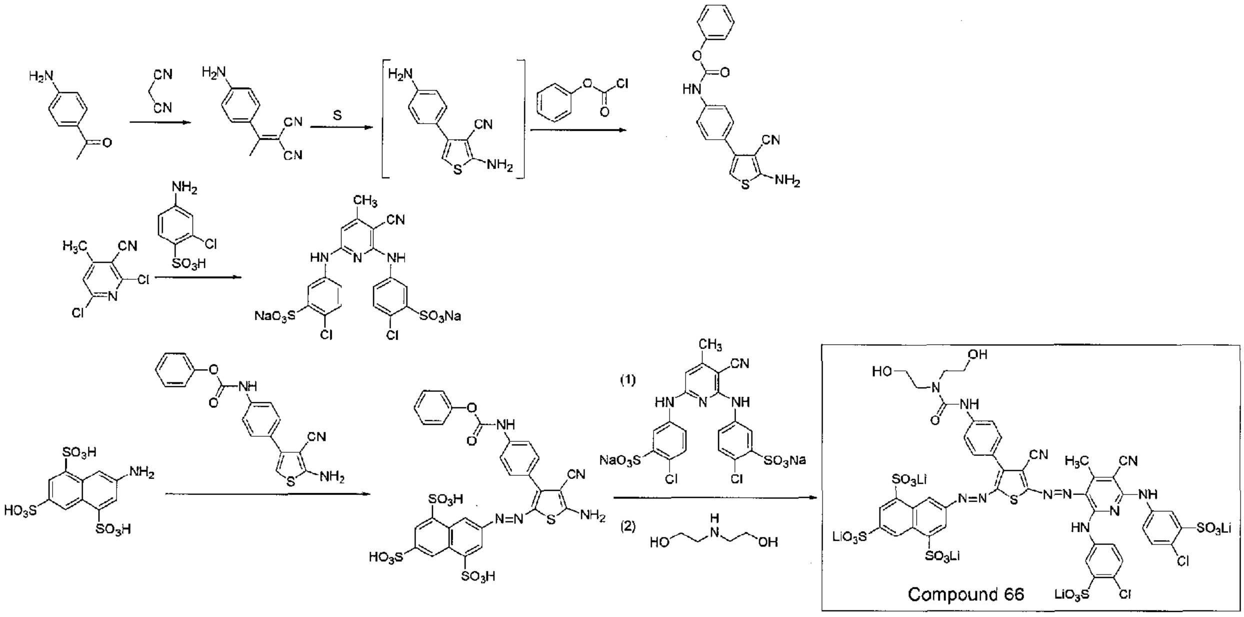


Compound 66

Compound 66 was obtained in the same manner as in the synthesis method of Compound 64 except that di(2-hydroxyethyl)amine was used in place of 2-aminoethanol after the azo coupling in the final step. As a result of LC-MS, a peak corresponding to Compound 66 was observed.

(Synthesis of Compound 67)

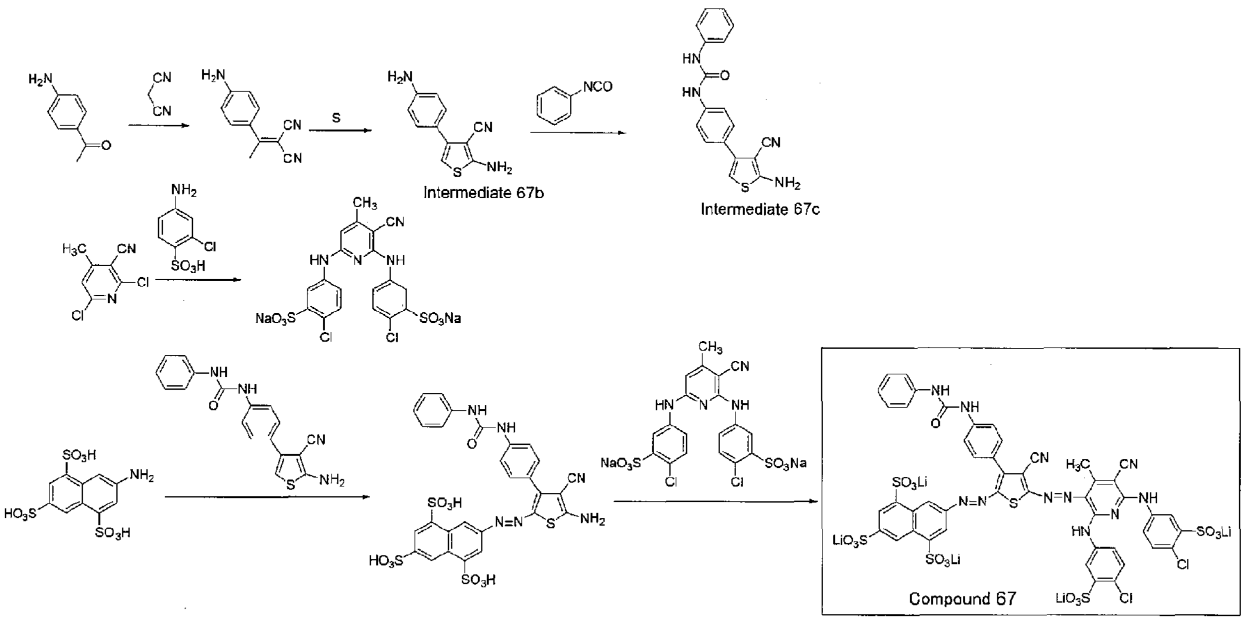

Intermediate 67b

Intermediate 67c

Compound 67

To a methanol solution of Intermediate 67b obtained by the same synthesis method as that for Compound 7, a 5-fold amount of water was added to crystallize Intermediate 67b, which was subsequently collected by filtration. Intermediate 67b was dissolved in a 10-fold amount of acetonitrile, and an equimolar amount of phenyl isocyanate was added. After allowing the reaction to proceed for 1 hour, water at 5 times the amount of the reaction solution was added,

and the precipitated crystal was collected by filtration to obtain Intermediate 67c. The subsequent procedure was performed in the same manner as in the synthesis method of Compound 57 to obtain Compound 67. As a result of LC-MS, a peak corresponding to Compound 67 was observed.

(Synthesis of Compound 68)

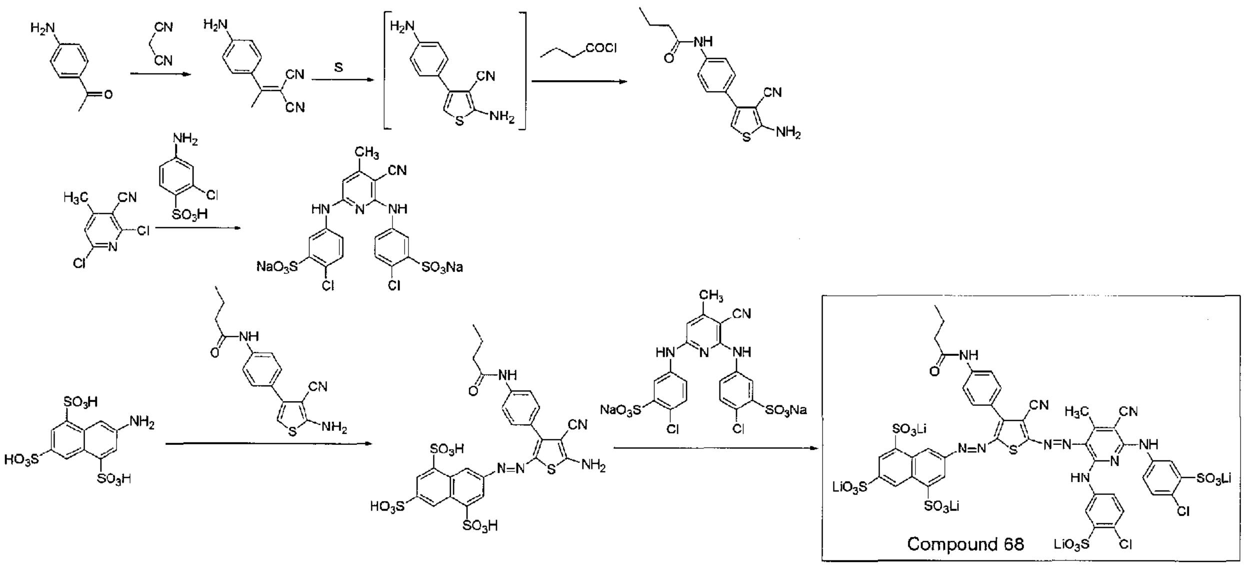

Compound 68

Compound 68 was obtained in the same manner as in the synthesis method of Compound 27 except that butyric acid chloride was used in place of acetic anhydride and a cation exchange resin filled with lithium ion in place of potassium ion was used. As a result of LC-MS, a peak corresponding to Compound 68 was observed.

(Synthesis of Compound 69)

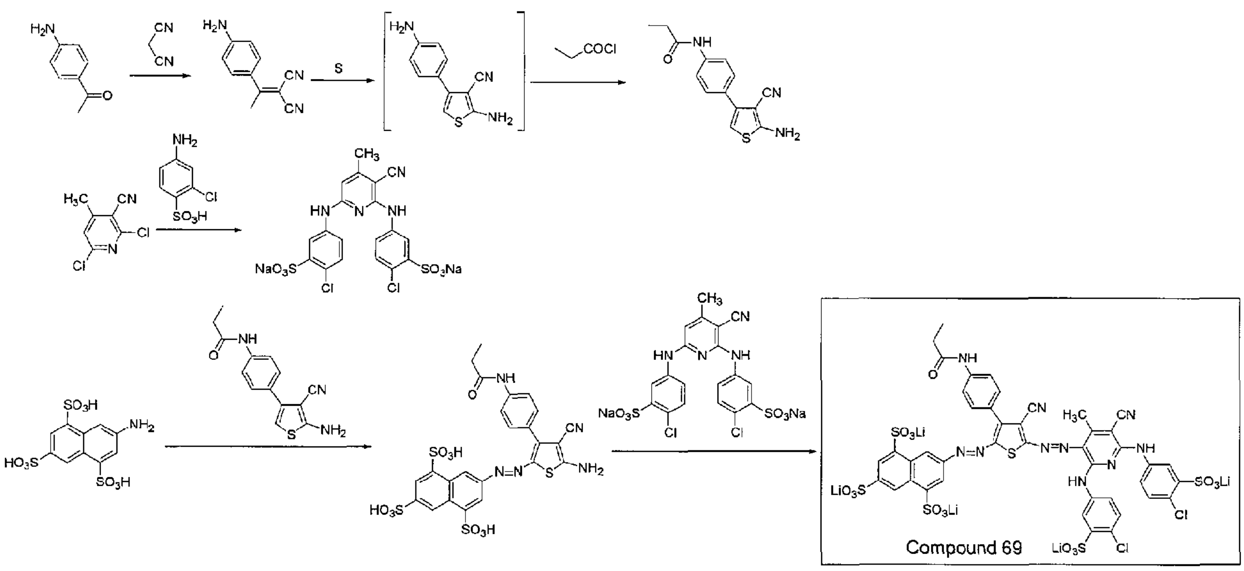

Compound 69

Compound 69 was obtained in the same manner as in the synthesis method of Compound 68 except for using propionic acid chloride in place of butyric acid chloride. As a result of LC-MS, a peak corresponding to Compound 69

was observed.

(Synthesis of Compound 70)

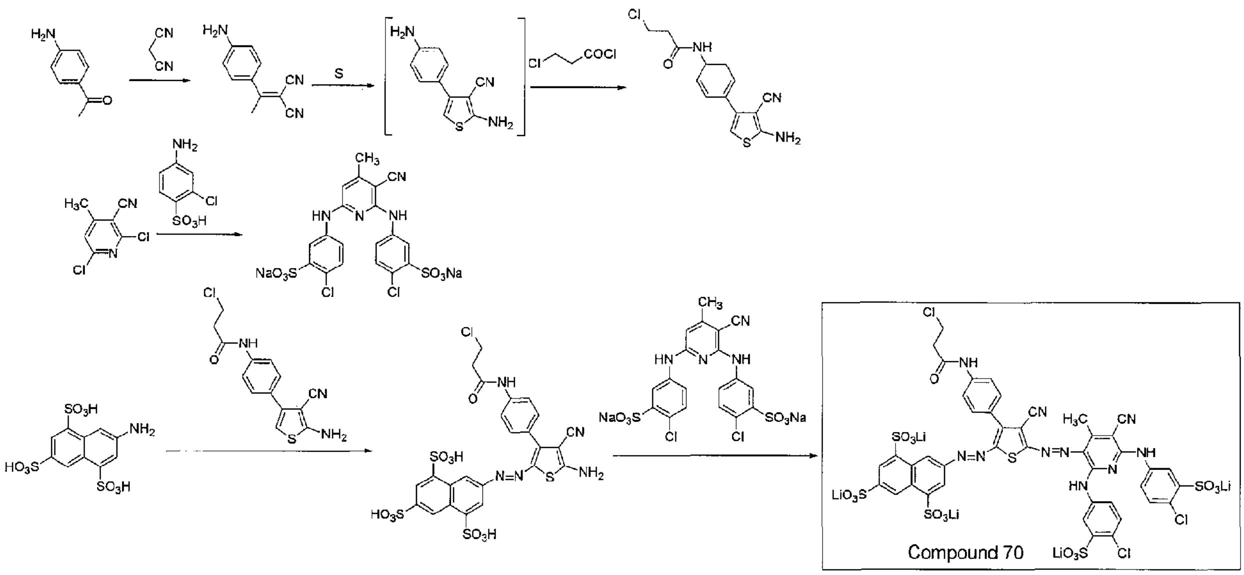

Compound 70

Compound 70 was obtained in the same manner as in the synthesis method of Compound 68 except for using 3-chloropropionic acid chloride in place of butyric acid chloride. As a result of LC-MS, a peak corresponding to Compound 70 was observed.

(Synthesis of Compound 71)

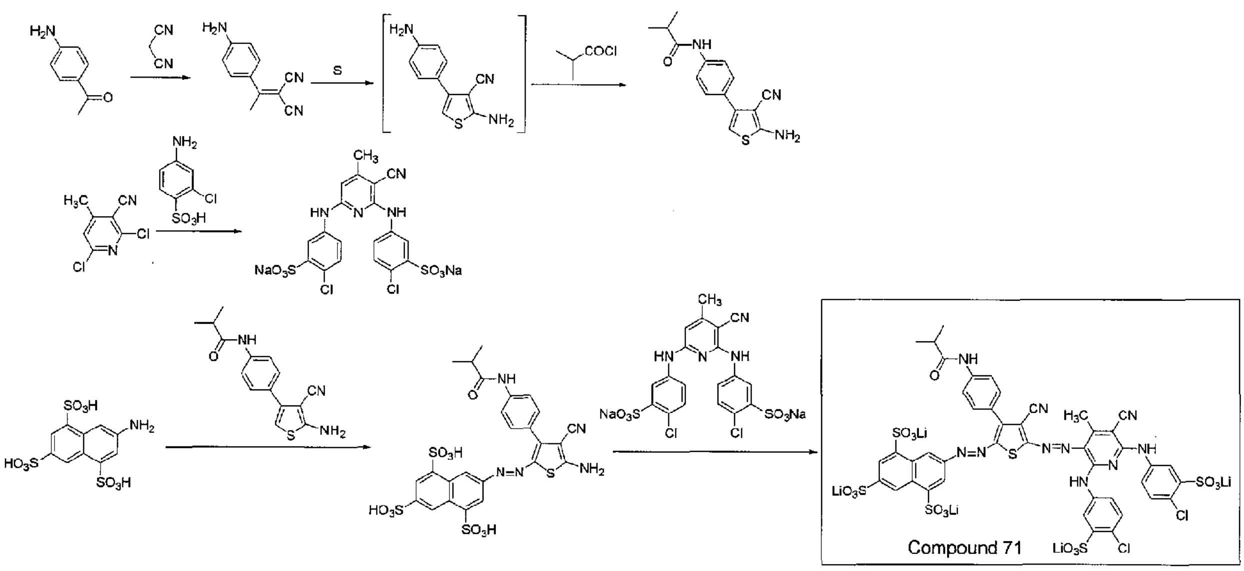

Compound 71

Compound 71 was obtained in the same manner as in the synthesis method of Compound 68 except for using 2-methylpropionic acid chloride in place of butyric acid chloride. As a result of LC-MS, a peak corresponding to Compound 71 was observed.

(Synthesis of Compound 72)

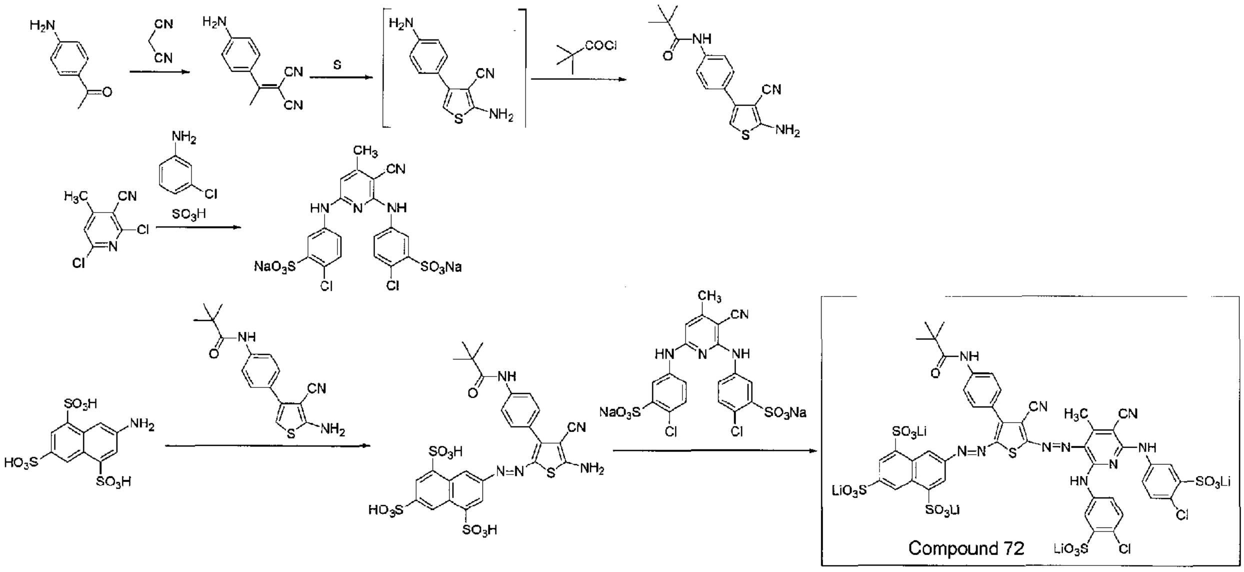


Compound 72 was obtained in the same manner as in the synthesis method of Compound 68 except for using pivaloyl chloride in place of butyric acid chloride. As a result of LC-MS, a peak corresponding to Compound 72 was observed.

(Synthesis of Compound 73)

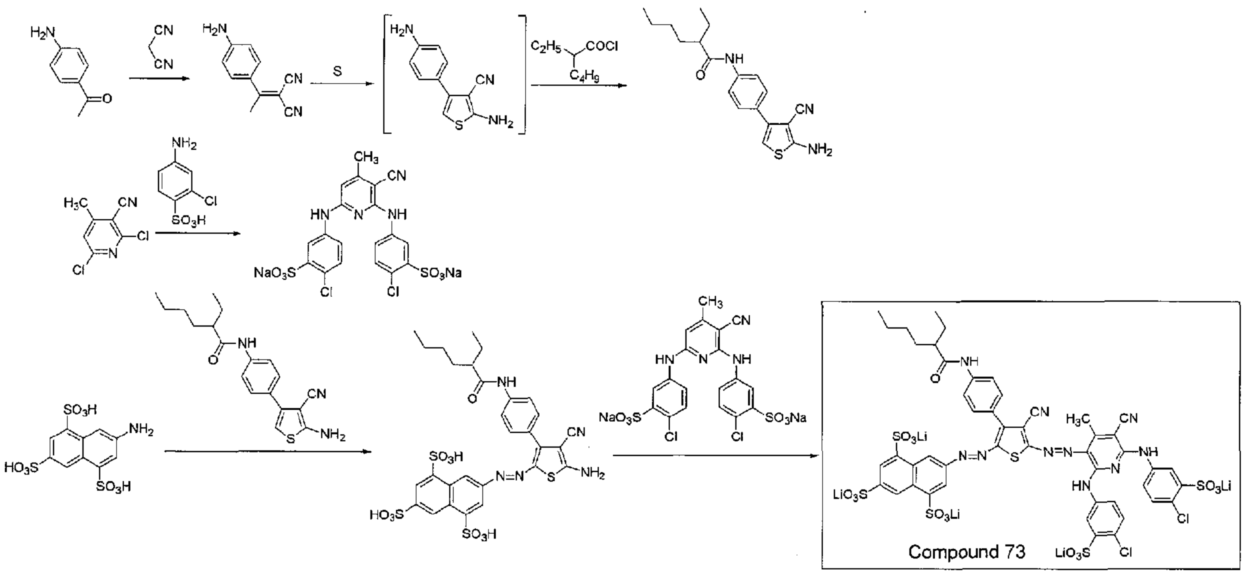


Compound 73 was obtained in the same manner as in the synthesis method of Compound 68 except for using 2-ethylhexanoic acid chloride in place of butyric acid chloride. As a result of LC-MS, a peak corresponding to Compound 73 was observed.

(Synthesis of Compound 74)

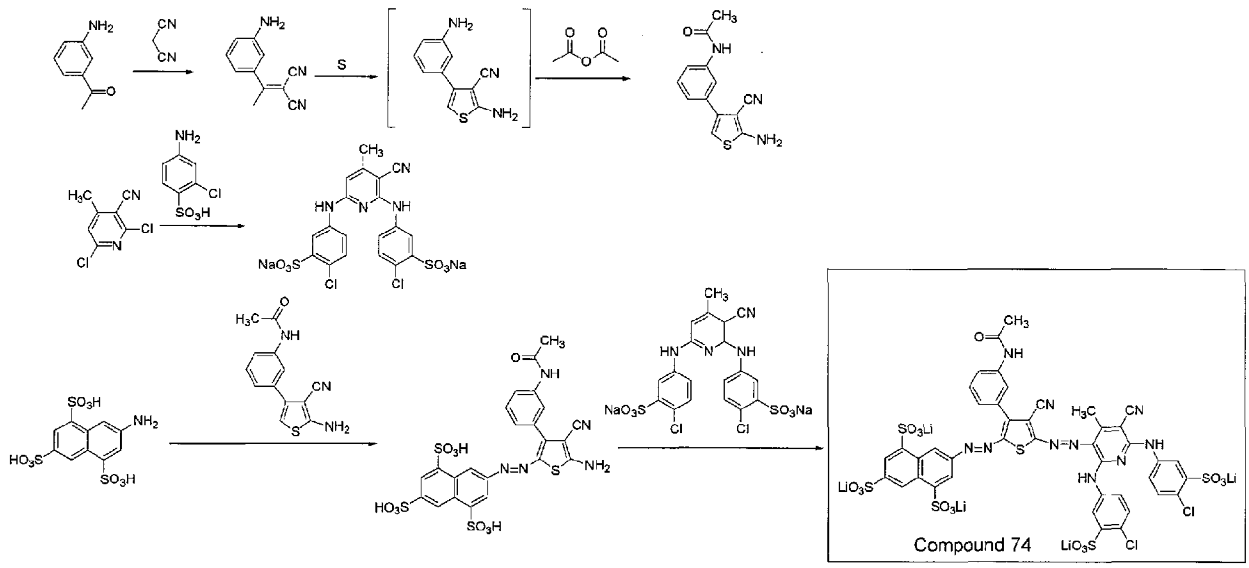


Compound 74

Compound 74 was obtained in the same manner as in the synthesis method of Compound 27 except that 3-aminoacetophenone was used in place of 4-aminoacetophenone and a cation exchange resin filled with lithium ion in place of potassium ion was used. As a result of LC-MS, a peak corresponding to Compound 74 was observed.

(Synthesis of Compound 75)

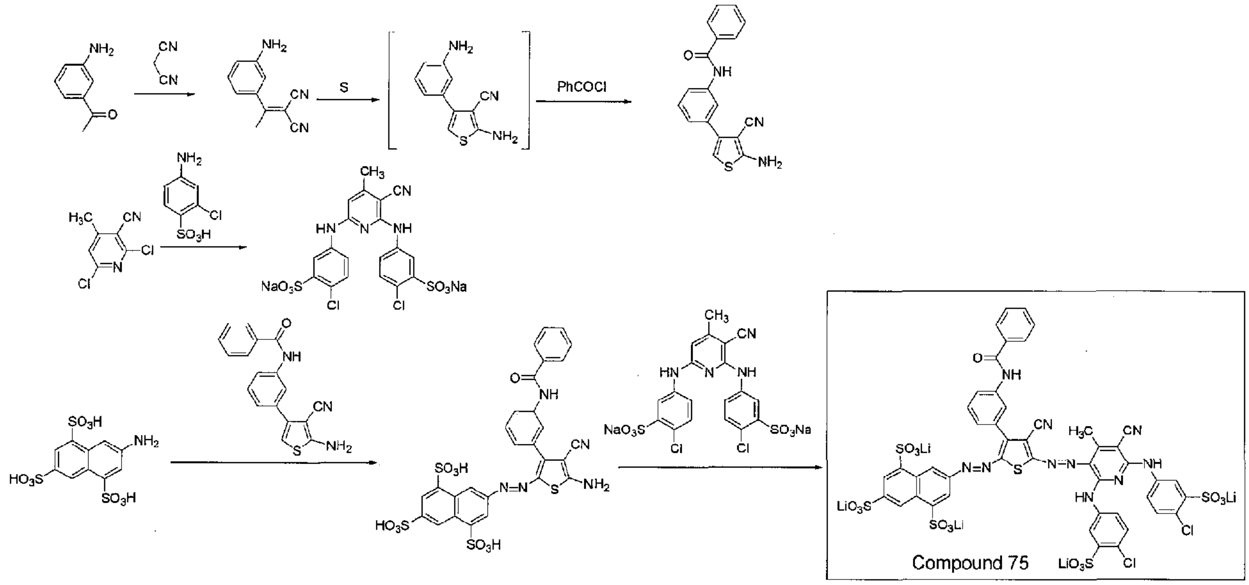


Compound 75

Compound 75 was obtained in the same manner as in the synthesis method of Compound 74 except for using benzoyl chloride in place of acetic anhydride. As a result of LC-MS, a peak corresponding to Compound 75 was observed.

(Synthesis of Compound 76)

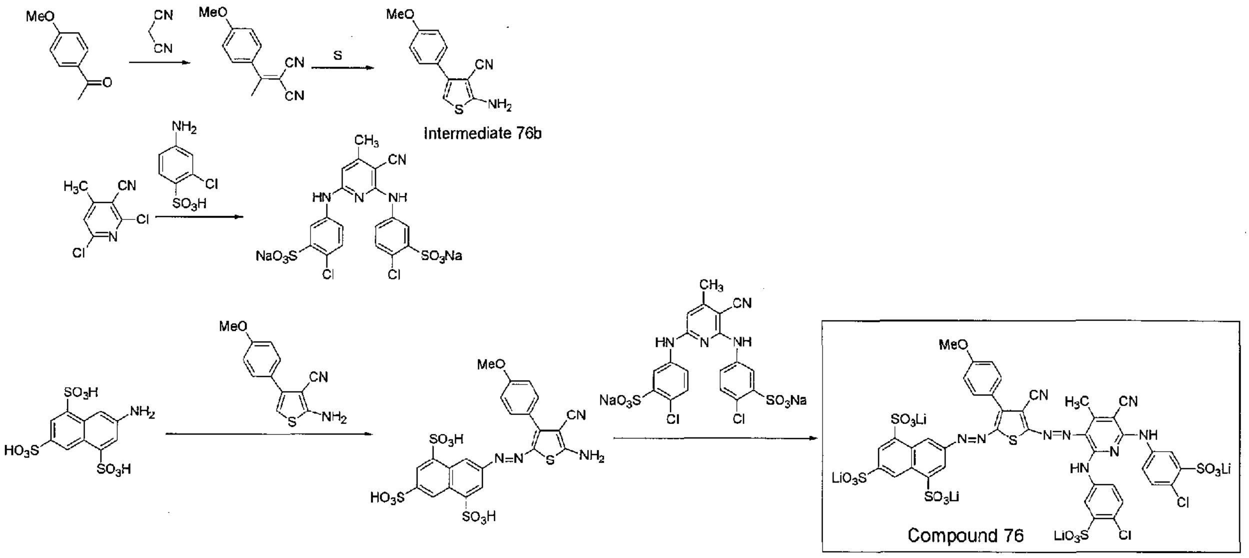


A methanol solution of Intermediate 76b corresponding to Intermediate b was obtained by the same synthesis method as that for Compound 1 by using 4-methoxyacetophenone in place of 3-aminoacetophenone. Compound 76 was obtained in the same manner as in the synthesis method of Compound 1 by using the methanol solution of Intermediate 76b in place of Intermediate c. As a result of LC-MS, a peak corresponding to Compound 76 was observed.

(Synthesis of Compound 77)

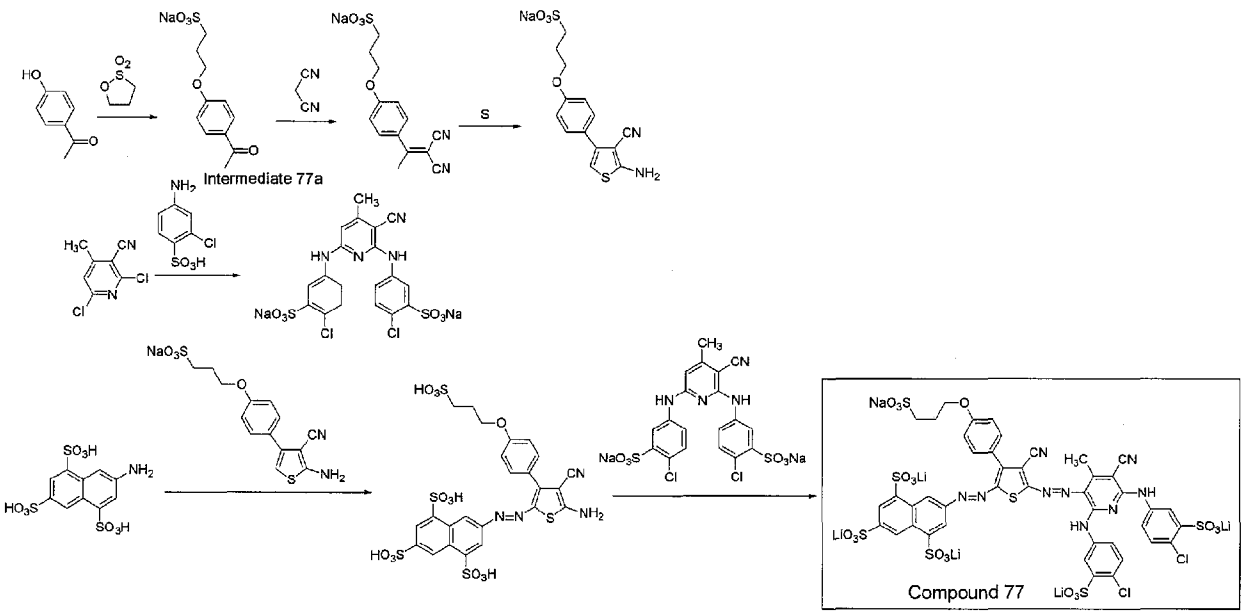


4-Hydroxyacetophenone, one molar equivalent of propanesultone and one molar equivalent of sodium carbonate were reacted under reflux in an isopropyl alcohol at 10 times the weight of 4-hydroxyacetophenone, and the precipitated crystal was collected by filtration to obtain Intermediate 77a. Compound 77 was obtained in the same manner as in the synthesis method of Compound 76 by using Intermediate 77a in place of 4-methoxyacetophenone. As a result of LC-MS, a peak corresponding to Compound 77 was observed.

(Synthesis of Compound 78)

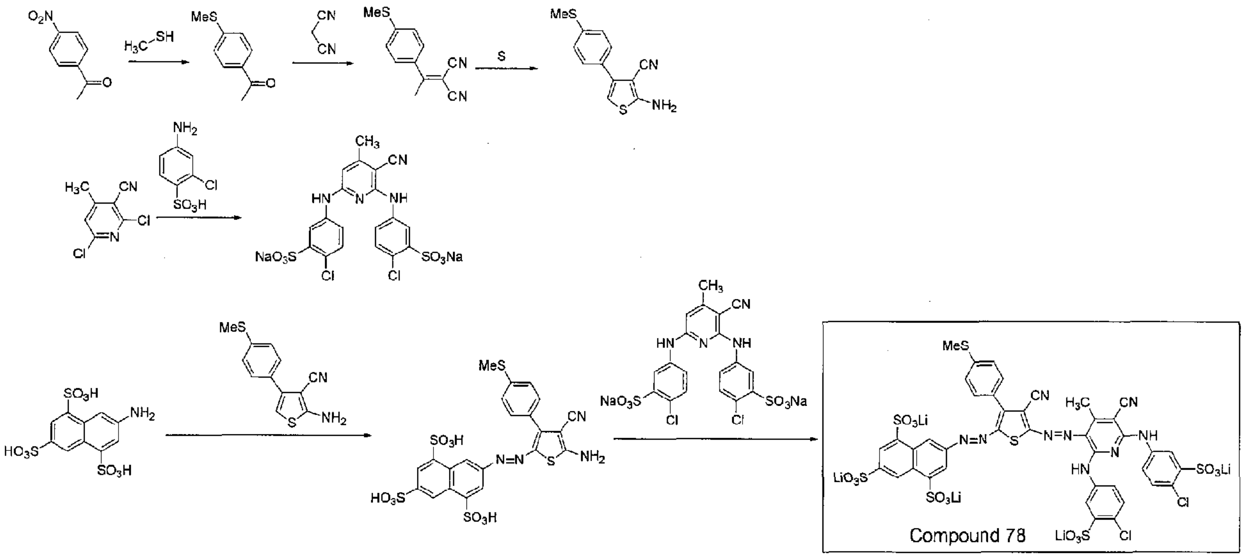

Compound 78

To a solution obtained by dissolving 4-nitroacetophenone in 10 times its weight of dimethylsulfoxide, one equivalent of an aqueous methylmercaptane sodium solution (15%) was added and reacted at 100°C, and after adding water at 10 times the volume of the reaction solution, the precipitated crystal was collected by filtration to obtain 4-methylthioxyacetophenone. Compound 78 was obtained in the same manner as in the synthesis method of Compound 76 by using 4-methylthioxyacetophenone in place of 4-methoxyacetophenone. As a result of LC-MS, a peak corresponding to Compound 78 was observed.

(Synthesis of Compound 79)

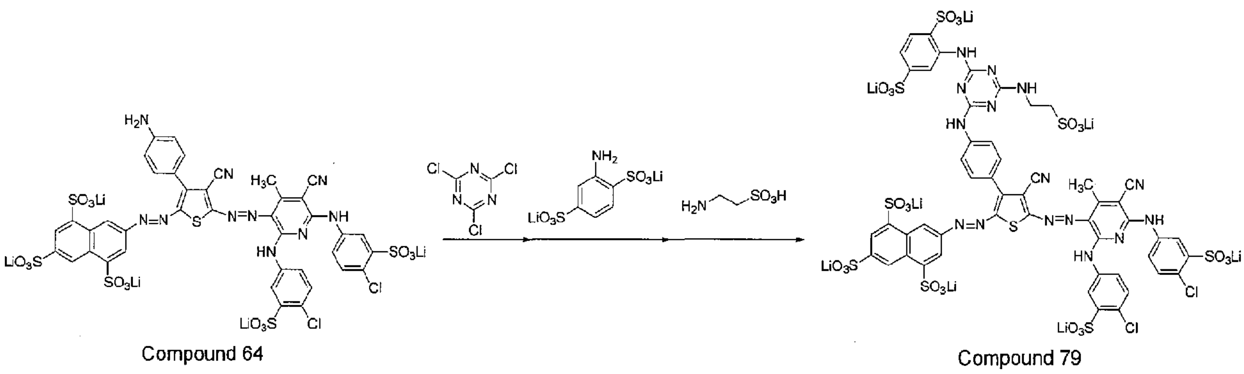

Compound 64 Compound 79

A 10% aqueous solution of Compound 64 was cooled to 0°C and the pH thereof was adjusted to 2 to 3 with aqueous hydrochloric acid. Thereto, an equimolar amount of cyanuric chloride was added and after stirring for 1 hour, the liquid temperature was raised to 40°C. The pH was adjusted to 6 to 8 with an aqueous lithium hydroxide solution, and an equimolar amount of 2,5-disulfoaniline was added thereto and stirred for 3 hours. Thereafter, the reaction solution was heated to 70°C and the pH was adjusted to 6 to 10 with an aqueous lithium hydroxide solution. Furthermore, an equimolar amount of taurine was added and reacted for 3 hours, and the reaction solution was crystallized by adding isopropyl alcohol at 3 times the volume of the reaction solution. The crystal obtained by filtration was dissolved in water, and the solution was passed through a cation exchange resin (Amberlite IR-120, produced by Organo Corporation) filled with lithium ion, and then concentrated. The obtained crystal was dried at 50°C to obtain a black crystal of Compound 79. As a result of LC-MS, a peak corresponding to Compound 79 was observed.

(Synthesis of Compound 80)

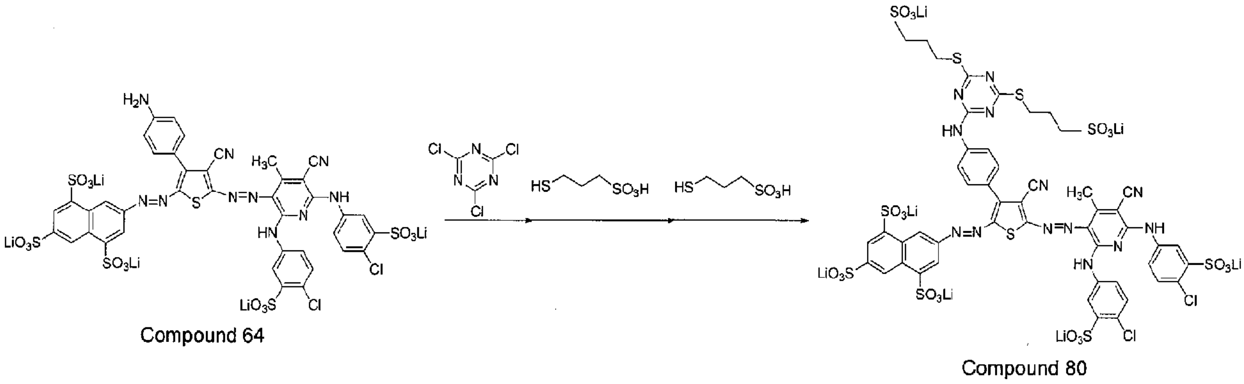

Compound 64

Compound 80

Compound 80 was obtained in the same manner as in the synthesis method of Compound 79 by replacing 2,5-disulfoaniline and taurine by sodium 3-thiopropanesulfonate and sodium 3-thiopropanesulfonate, respectively. As a result of LC-MS, a peak corresponding to Compound 80 was observed.

(Synthesis of Compound 81)

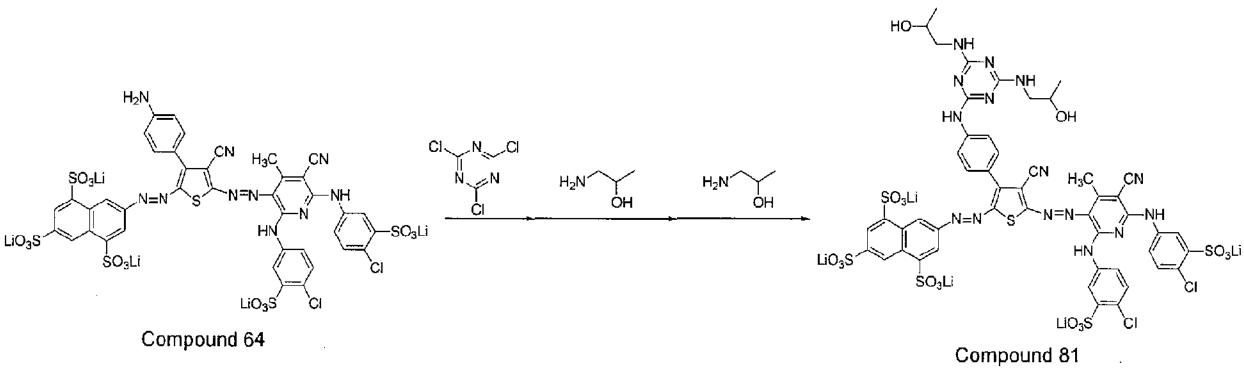

Compound 64

Compound 81

Compound 81 was obtained in the same manner as in the synthesis method of Compound 79 by replacing 2,5-disulfoaniline and taurine by 2-hydroxypropylamine and 2-hydroxypropylamine, respectively. As a result of LC-MS, a peak corresponding to Compound 81 was observed.

(Synthesis of Compound 82)

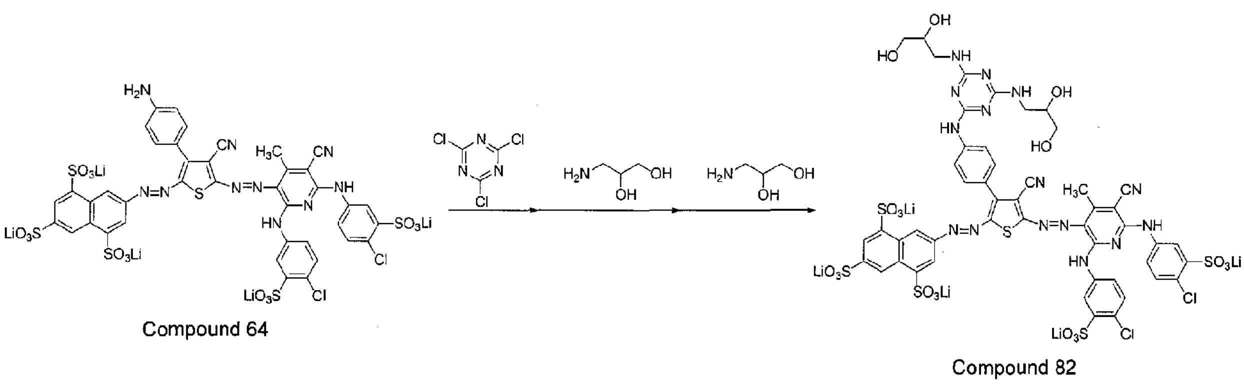

Compound 64

Compound 82

Compound 82 was obtained in the same manner as in the synthesis method of Compound 79 by replacing 2,5-disulfoaniline and taurine by 2,3-dihydroxypropylamine and 2,3-dihydroxypropylamine, respectively. As a result of LC-MS, a peak corresponding to Compound 82 was observed.

[Preparation of Aqueous Solution]

Here, the aqueous solution of the present invention is referred to as "ink stock solution).

Incidentally, the aqueous solution was adjusted to a pH of 8.1 to 8.3 by using an aqueous 4 mol/L lithium hydroxide solution.

[Example 1]

100 g of Compound 1 was dissolved in 900 g of ultrapure water with stirring at room temperature and thereafter, 0.1 g as a solid content of a preservative (Proxel XL-II, produced by Fujifilm Imaging Colorants) was added. The pH was adjusted to 8.2 by using an aqueous 4 mol/L lithium hydroxide solution, and unnecessary matters were removed by filtration through a membrane filter having an effective diameter of 0.2 μm to obtain Ink Stock Solution-1.

[Example 2]

Ink Stock Solution-2 was obtained by performing the same operation as in Example 1 except for using Compound 2 in place of Compound 1.

[Example 3]

Ink Stock Solution-3 was obtained by performing the same operation as in Example 1 except for using Compound 3 in place of Compound 1.

[Example 4]

Ink Stock Solution-4 was obtained by performing the same operation as in Example 1 except for using Compound 4 in place of Compound 1.

[Example 5]

Ink Stock Solution-5 was obtained by performing the same operation as in Example 1 except for using Compound 5 in place of Compound 1.

[Example 6]

Ink Stock Solution-6 was obtained by performing the same operation as in Example 1 except for using Compound 6 in place of Compound 1.

[Example 7]

Ink Stock Solution-7 was obtained by performing the same operation as in Example 1 except for using Compound 7 in place of Compound 1.

[Example 8]

Ink Stock Solution-8 was obtained by performing the same operation as in Example 1 except for using Compound 8 in place of Compound 1.

[Example 9]

Ink Stock Solution-9 was obtained by performing the same operation as in Example 1 except for using Compound 9 in place of Compound 1.

[Example 10]

Ink Stock Solution-10 was obtained by performing the same operation as in Example 1 except for using Compound 10 in place of Compound 1.

[Example 11]

Ink Stock Solution-11 was obtained by performing the same operation as in Example 1 except for using Compound 11 in place of Compound 1.

[Example 12]

Ink Stock Solution-12 was obtained by performing the same operation as in Example 1 except for using Compound 12 in place of Compound 1.

[Example 13]

Ink Stock Solution-13 was obtained by performing the same operation as in Example 1 except for using Compound 13 in place of Compound 1.

[Example 14]

Ink Stock Solution-14 was obtained by performing the same operation as in Example 1 except for using Compound 14 in place of Compound 1.

[Example 15]

Ink Stock Solution-15 was obtained by performing the same operation as in Example 1 except for using Compound 15 in place of Compound 1.

[Example 16]

Ink Stock Solution-16 was obtained by performing the same operation as in Example 1 except for using Compound 16 in place of Compound 1.

[Example 17]

Ink Stock Solution-17 was obtained by performing the same operation as in Example 1 except for using Compound 17 in place of Compound 1.

[Example 18]

Ink Stock Solution-18 was obtained by performing the same operation as in Example 1 except for using Compound 18 in place of Compound 1.

[Example 19]

Ink Stock Solution-19 was obtained by performing the same operation as in Example 1 except for using Compound 19 in place of Compound 1.

[Example 20]

Ink Stock Solution-20 was obtained by performing the same operation as in Example 1 except for using Compound 20 in place of Compound 1.

[Example 21]

Ink Stock Solution-21 was obtained by performing the same operation as in Example 1 except for using Compound 21 in place of Compound 1.

[Example 22]

Ink Stock Solution-22 was obtained by performing the same operation as in Example 1 except for using Compound 27 in place of Compound 1.

[Example 23]

Ink Stock Solution-23 was obtained by performing the same operation as in Example 1 except for using Compound 29 in place of Compound 1.

[Example 24]

Ink Stock Solution-24 was obtained by performing the same operation as in Example 1 except for using Compound 31 in place of Compound 1.

[Example 25]

Ink Stock Solution-25 was obtained by performing the same operation as in Example 1 except for using Compound 32 in place of Compound 1.

[Example 26]

Ink Stock Solution-26 was obtained by performing the same operation as in Example 1 except for using Compound 33 in place of Compound 1.

[Example 27]

Ink Stock Solution-27 was obtained by performing the same operation as in Example 1 except for using Compound 36 in place of Compound 1.

[Example 28]

Ink Stock Solution-28 was obtained by performing the same operation as in Example 1 except for using Compound 38 in place of Compound 1.

[Example 29]

Ink Stock Solution-29 was obtained by performing the same operation as in Example 1 except for using Compound 41 in place of Compound 1.

[Example 30]

Ink Stock Solution-30 was obtained by performing the same operation as in Example 1 except for using Compound 42 in place of Compound 1.

[Example 31]

Ink Stock Solution-31 was obtained by performing the same operation as in Example 1 except for using Compound 50 in place of Compound 1.

[Example 32]

Ink Stock Solution-32 was obtained by performing the same operation as in Example 1 except for using Compound 51 in place of Compound 1.

[Comparative Example 1]

Comparative Ink Stock Solution-01 was obtained by performing the same operation as in Example 1 except for using the following compound (Comparative Compound 1) in place of Compound 1.

Comparative Compound 1:

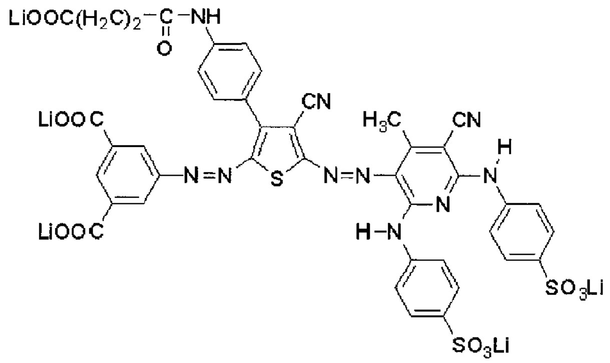

[Examples 33 to 58]

Ink Stock Solution-33 to Ink Stock Solution-58 were obtained by performing the same operation as in Example 1 except for using Compounds 57 to 82 respectively in place of Compound 1.

[Preparation of Ink Composition]

10.0 g of Ink Stock Solution-1 obtained in Example 1, 5.0 g of ultrapure water, 1.6 g of glycerin, 0.2 g of triethylene glycol, 0.1 g of propylene glycol, 0.4 g of 1,2-hexanediol, 1.6 g of triethylene glycol monobutyl ether, 0.3 g of Olfine E1010 (produced by Nissin Chemical Industry Co., Ltd.), 0.8 g of 2-pyrrolidone, and 0.02 g of lithium hydrogencarbonate were added and stirred at ordinary temperature for 30 minutes, and thereafter, pH was adjusted to 8.2 by using an aqueous 4 mol/L lithium hydroxide solution. The obtained solution was filtered through a membrane filter having an effective opening size of 1.0 μm to obtain Ink Composition A-1.

Ink Composition A-2 to Ink Composition A-58 and Comparative Ink Composition B-1 were obtained in the same manner as Ink Composition A-1 except for replacing Ink Stock Solution-1 by Ink Stock Solution-2 to Ink Stock Solution-58 and Comparative Ink Stock Solution-01, respectively.

[Evaluation of Suppression of Bronze Gloss]

Black solid printing was performed to give a hitting amount of 1.5 to 2.2 mg per square inch on an inkjet exclusive recording medium (Exclusive Paper A: Photo Crispia <Ko-Kotaku> (trade name, produced by Seiko Epson Corporation), Exclusive Paper B: Canon Photo Paper Glossy Pro [Platinum Grade] (trade name, produced by Canon, Inc.), Exclusive Paper C: GASAI Photo Finish Pro (trade name, produced by Fujifilm Corporation)) by using an inkjet printer, Stylus Color 880 (trademark) (trade name, manufactured by Seiko Epson Corporation) and using each of Ink Composition A-1 to Ink Composition A-58 and Comparative Ink Composition B-1, and the obtained printed matter was measured (measuring angle: 60°) using a gloss meter (PG-1M, manufactured by Nippon Denshoku Industries Co., Ltd.) to determine the glossiness. The printing was performed in two environments of 20°C-40% RH and 35°C-60% RH. The obtained glossiness and the elevation value calculated according to the following formula were used as the standard for judging the degree of bronze phenomenon generation, and judgment was performed based on the following criteria for judgment.

$$\text{Elevation value} = \text{glossiness (printed matter)} - \text{glossiness (recording medium)}$$

[Criteria for Judgment]

Rank A: less than 15

Rank B: from 15 to less than 35
Rank C: from 35 to less than 55
Rank D: 55 or more

[Evaluation of Color Tone]

The color tone was evaluated as follows.

With respect to Exclusive Paper C having formed thereon an image, the OD value was measured using a reflection densitometer (X-Rite 310TR), and the printing density was evaluated by the maximum value of OD. The results are shown in the Table below. Incidentally, when the evaluation of color tone was performed in the same manner also for Exclusive Papers A and B, the same results as in Exclusive Paper C were obtained.

(Criteria for Judgment)

Rank A: 2.2 or more
Rank B: from 1.5 to less than 2.2
Rank C: less than 1.5

Table 1

| | Colorant | Ink Composition | Bronze Gloss | | | Color Tone |
|---|---|---|---|---|---|---|
| | | | Exclusive Paper A | Exclusive Paper B | Exclusive Paper C | |
| Example 1 | Compound 1 | A-1 | A | A | A | B |
| Example 2 | Compound 2 | A-2 | A | A | A | A |
| Example 3 | Compound 3 | A-3 | A | A | A | A |
| Example 4 | Compound 4 | A-4 | A | A | A | A |
| Example 5 | Compound 5 | A-5 | A | A | A | A |
| Example 6 | Compound 6 | A-6 | A | A | A | A |
| Example 7 | Compound 7 | A-7 | A | A | A | A |
| Example 8 | Compound 8 | A-8 | A | A | A | A |
| Example 9 | Compound 9 | A-9 | A | A | A | A |
| Example 10 | Compound 10 | A-10 | A | A | A | A |
| Example 11 | Compound 11 | A-11 | A | A | A | A |
| Example 12 | Compound 12 | A-12 | A | A | A | A |
| Example 13 | Compound 13 | A-13 | A | A | A | B |
| Example 14 | Compound 14 | A-14 | B | A | A | B |
| Example 15 | Compound 15 | A-15 | A | A | A | B |
| Example 16 | Compound 16 | A-16 | A | A | A | A |
| Example 17 | Compound 17 | A-17 | A | A | A | A |
| Example 18 | Compound 18 | A-18 | A | A | A | A |
| Example 19 | Compound 19 | A-19 | A | A | A | A |
| Example 20 | Compound 20 | A-20 | A | A | A | A |
| Example 21 | Compound 21 | A-21 | B | B | A | A |
| Example 22 | Compound 27 | A-22 | A | A | A | A |

(continued)

| | Colorant | Ink Composition | Bronze Gloss | | | Color Tone |
|---|---|---|---|---|---|---|
| | | | Exclusive Paper A | Exclusive Paper B | Exclusive Paper C | |
| Example 23 | Compound 29 | A-23 | A | A | A | A |
| Example 24 | Compound 31 | A-24 | A | A | A | A |
| Example 25 | Compound 32 | A-25 | A | A | A | A |
| Example 26 | Compound 33 | A-26 | A | A | A | A |
| Example 27 | Compound 36 | A-27 | A | A | A | A |
| Example 28 | Compound 38 | A-28 | A | A | A | A |
| Example 29 | Compound 41 | A-29 | A | A | A | A |
| Example 30 | Compound 42 | A-30 | A | A | A | A |
| Example 31 | Compound 50 | A-31 | A | A | A | A |
| Example 32 | Compound 51 | A-32 | A | A | A | A |
| Comparative Example 1 | Comparative Compound 1 | B-1 | B | B | B | C |
| Example 33 | Compound 57 | A-33 | A | A | A | A |
| Example 34 | Compound 58 | A-34 | A | A | A | A |
| Example 35 | Compound 59 | A-35 | A | A | A | A |
| Example 36 | Compound 60 | A-36 | A | A | A | A |
| Example 37 | Compound 61 | A-37 | A | A | A | A |
| Example 38 | Compound 62 | A-38 | A | A | A | A |
| Example 39 | Compound 63 | A-39 | A | A | A | A |
| Example 40 | Compound 64 | A-40 | A | A | A | A |
| Example 41 | Compound 65 | A-41 | A | A | A | A |
| Example 42 | Compound 66 | A-42 | A | A | A | A |
| Example 43 | Compound 67 | A-43 | A | A | A | A |
| Example 44 | Compound 68 | A-44 | A | A | A | A |
| Example 45 | Compound 69 | A-45 | A | A | A | A |
| Example 46 | Compound 70 | A-46 | A | A | A | A |
| Example 47 | Compound 71 | A-47 | A | A | A | A |
| Example 48 | Compound 72 | A-48 | A | A | A | A |
| Example 49 | Compound 73 | A-49 | A | A | A | A |
| Example 50 | Compound 74 | A-50 | A | A | A | A |
| Example 51 | Compound 75 | A-51 | A | A | A | A |
| Example 52 | Compound 76 | A-52 | A | A | A | A |
| Example 53 | Compound 77 | A-53 | A | A | A | A |
| Example 54 | Compound 78 | A-54 | A | A | A | A |
| Example 55 | Compound 79 | A-55 | A | A | A | A |
| Example 56 | Compound 80 | A-56 | A | A | A | A |

(continued)

| | Colorant | Ink Composition | Bronze Gloss | | | Color Tone |
|---|---|---|---|---|---|---|
| | | | Exclusive Paper A | Exclusive Paper B | Exclusive Paper C | |
| Example 57 | Compound 81 | A-57 | A | A | A | A |
| Example 58 | Compound 82 | A-58 | A | A | A | A |

It is seen from the results shown above that the ink composition of Comparative Example exhibited practically problem-free performances with respect to suppression of the bronze gloss and print density of the obtained print matter, but the ink composition of the present invention exerted very excellent effects on the suppression of bronze gloss and print density of the obtained print matter in all cases of using Exclusive Papers A to C as the recording medium and is understood to satisfy high levels of performances.

[Evaluation of Dependency on Observation Light Source]

(Preparation of Ink Stock Solution of Complementary Dye)

100 g of Complementary Dye 1 shown below and 100 g of Compound 1 were dissolved in 900 g of ultrapure water with stirring at room temperature, and 0.1 g as a solid content of a preservative (Proxel XL-II, produced by Fujifilm Imaging Colorants) was added. The pH was adjusted to 8.2 by using an aqueous 4 mol/L sodium hydroxide solution, and unnecessary matters were removed by filtration through a membrane filter having an effective size of 0.2 μm to obtain Complementary Dye Stock Solution-1. Complementary Dye Stock Solution-2 to Complementary Dye Stock Solution-4 were obtained in the same manner by using Complementary Dyes 2 to 4 shown below.

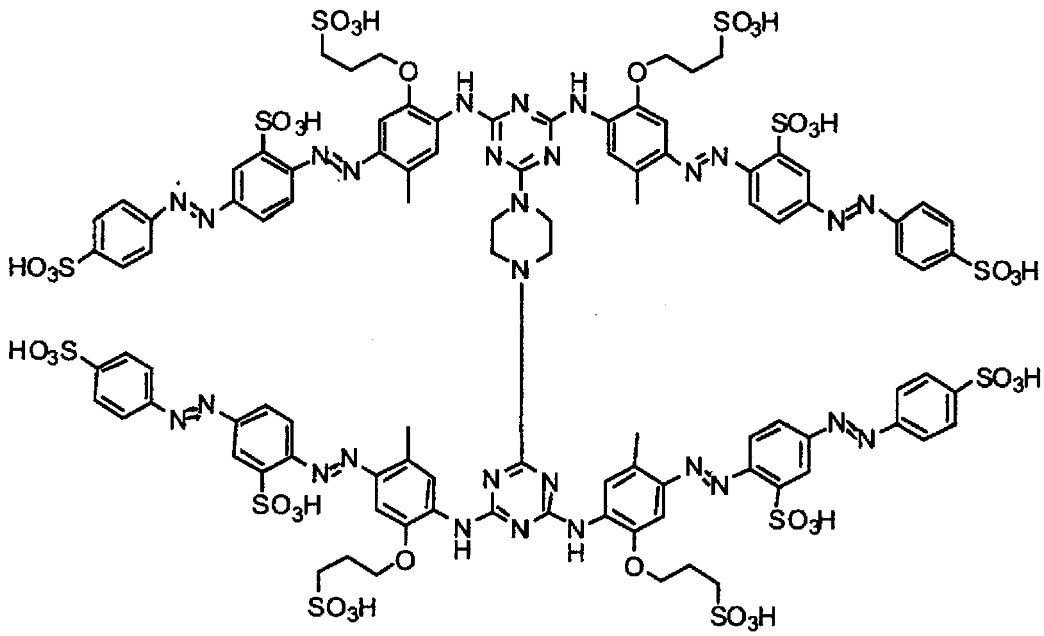

Complementary Dye 1

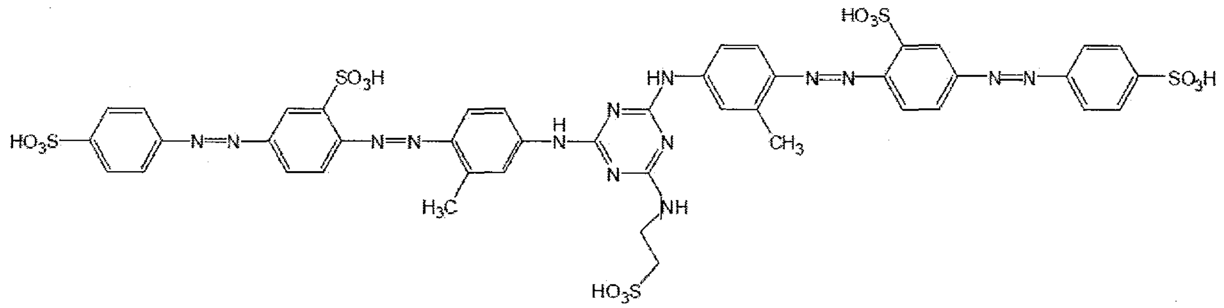

Complementary Dye 2

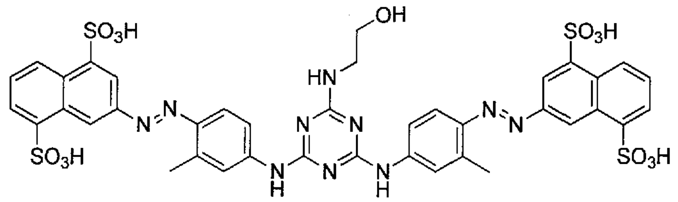

Complementary Dye 3

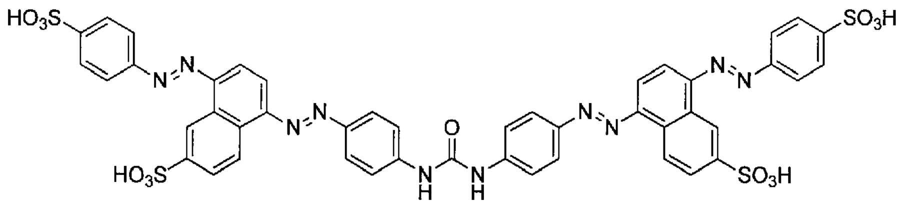

Complementary Dye 4

(Preparation of Ink Composition)

7.0 g of Ink Stock Solution-1 obtained in Example 1, X g of Complementary Dye Stock Solution-1, (8.0-X) g of ultrapure water, 1.6 g of glycerin, 0.2 g of triethylene glycol, 0.1 g of propylene glycol, 0.4 g of 1,2-hexanediol, 1.6 g of triethylene glycol monobutyl ether, 0.3 g of Olfine E1010 (produced by Nissin Chemical Industry Co., Ltd.), 0.8 g of 2-pyrrolidone, and 0.02 g of lithium hydrogencarbonate were added and stirred at ordinary temperature for 30 minutes, and thereafter, pH was adjusted to 8.2 by using an aqueous 4 mol/L sodium hydroxide solution. The obtained solution was filtered through a membrane filter having an effective opening size of 1.0 μm to obtain Ink Composition 101-1. Incidentally, as for X, the value of X was adjusted such that in a printed matter, the maximum value of absorption at 570 to 700 nm becomes equal to the maximum value of absorption at 400 to 570 nm. Ink Compositions 101-2 to 101-4 using Complementary Dye Stock Solution-2 to Complementary Dye Stock Solution-4 were obtained in the same manner.

Black solid printing was performed to give a hitting amount of 1.5 to 2.2 mg per square inch on an inkjet exclusive recording medium (photo paper Crispia <Ko-Kotaku> (trade name, produced by Seiko Epson Corporation)) by using an inkjet printer, Stylus Color 880 (trademark) (trade name, manufactured by Seiko Epson Corporation) and using each of Ink Compositions 101-1 to 101-4, whereby printed matters were obtained. The obtained printed matter was measured for the reflection spectrum by a spectral absorption measuring device (UV-2400, manufactured by Shimadzu Corporation). As the reflection spectrum R(λ), a spectrum measured at 380 to 780 nm in steps of 5 nm was used.

As the observation light source, the following 15 light sources were used.

D50, A, D65, F1, F2, F3, F4, F5, F6, F7, F8, F9, F10, F11 and F12.

As for the spectrum P(λ) of such a light source, the spectra shown in Table T.1. and Table T6.1. of Technical Report Colorimetry, 3rd Edition (CIE 15:2004) were used as the emission spectrum.

As the color-matching functions (CIE 1986) x (λ), y (λ) and z (λ) in the XYZ color system, those shown in Table T.5. of Technical Report Colorimetry, 3rd Edition (CIE 15:2004) were used.

Tristimulus values X, Y and Z of the printed matter were calculated according to the following formulae:

$$X = \frac{100 \cdot \sum R(\lambda) \cdot P(\lambda) \cdot \bar{x}(\lambda)}{\sum P(\lambda) \cdot \bar{y}(\lambda)}$$

$$Y = \frac{100 \cdot \sum R(\lambda) \cdot P(\lambda) \cdot \bar{y}(\lambda)}{\sum P(\lambda) \cdot \bar{y}(\lambda)}$$

$$Z = \frac{100 \cdot \sum R(\lambda) \cdot P(\lambda) \cdot \bar{z}(\lambda)}{\sum P(\lambda) \cdot \bar{y}(\lambda)}$$

Tristimulus values $X_n$, $Y_n$ and $Z_n$ at the white point of the light source were defined as follows.

$$X_n = \frac{100 \cdot \sum P(\lambda) \cdot \bar{x}(\lambda)}{\sum P(\lambda) \cdot \bar{y}(\lambda)}$$

$$Y_n = 100$$

$$Z_n = \frac{100 \cdot \sum P(\lambda) \cdot \bar{z}(\lambda)}{\sum P(\lambda) \cdot \bar{y}(\lambda)}$$

Colorimetric values L*, a*, b* in the CIE LAB with respect to the reflection spectrum of each coloring matter and the light source were calculated according to the following formulae:

$$L^* = 116 \cdot f(Y/Y_n) - 16$$

$$a^* = 500 \cdot (f(X/X_n) - f(Y/Y_n))$$

$$b^* = 200 \cdot (f(Y/Y_n) - f(Z/Z_n))$$

Here, the function f used in the formulae above is defined as follows.

$$f(t)=\begin{cases} t^{1/3} & t>(6/29)^3 \\ \frac{1}{3}\left(\frac{29}{6}\right)^2 t+\frac{4}{29} & otherwise \end{cases}$$

By defining the color difference ΔE between the colorimetric values ($L_1$*,$a_1$*,$b_1$*) (L*, a*, b*) of a printed matter to light source 1 and the colorimetric values ($L_2$*,$a_2$*,$b_2$*) of a printed matter to light source 2 as follows, the color difference ΔE between respective light sources (light source 2) (A, D65, F1, F2, F3, F4, F5, F6, F7, F8, F9, F10, F11 and F12) with respect to the standard light source D50 (light source 1) was calculated, and the maximum value of calculated color differences was designated as $\Delta E_{max}$.

Dependency on Observation Light Source was evaluated by the following judgment ctiteria.

Each Ink Composition was obtained by performing the same operation as in the Ink Composition 101-1 to 101-4 except for using Ink Stock Solutions as described in the following

Table 2 (Ink Stock Solutions-1 to 57, and Comparative Ink Stock Solution-01) respectively in place of Ink Stock Solution-1. These were evaluated in the same manner.

(Judgment Criteria)

A: $\Delta E_{max}<3$
B: $3\leq\Delta E_{max}<5$
C: $5\leq\Delta E_{max}$

Table 2

| Ink Composition | Ink Stock Solution Used | Colorant Contained | Complementary Dye Contained | Dependency on Observation Light Source | Remarks |
|---|---|---|---|---|---|
| Ink Composition 101-1 | Ink Stock Solution -1 | Compound 1 | Complementary Dye 1 | B | Example |
| Ink Composition 101-2 | Ink Stock Solution -1 | Compound 1 | Complementary Dye 2 | B | Example |
| Ink Composition 101-3 | Ink Stock Solution -1 | Compound 1 | Complementary Dye 3 | B | Example |
| Ink Composition 101-4 | Ink Stock Solution -1 | Compound 1 | Complementary Dye 4 | B | Example |
| Ink Composition 102-1 | Ink Stock Solution -2 | Compound 2 | Complementary Dye 1 | A | Example |
| Ink Composition 102-2 | Ink Stock Solution -2 | Compound 2 | Complementary Dye 2 | A | Example |
| Ink Composition 102-3 | Ink Stock Solution -2 | Compound 2 | Complementary Dye 3 | A | Example |
| Ink Composition 102-4 | Ink Stock Solution -2 | Compound 2 | Complementary Dye 4 | A | Example |
| Ink Composition 103-1 | Ink Stock Solution -3 | Compound 3 | Complementary Dye 1 | A | Example |
| Ink Composition 103-2 | Ink Stock Solution -3 | Compound 3 | Complementary Dye 2 | A | Example |

(continued)

| Ink Composition | Ink Stock Solution Used | Colorant Contained | Complementary Dye Contained | Dependency on Observation Light Source | Remarks |
|---|---|---|---|---|---|
| Ink Composition 103-3 | Ink Stock Solution -3 | Compound 3 | Complementary Dye 3 | A | Example |
| Ink Composition 103-4 | Ink Stock Solution -3 | Compound 3 | Complementary Dye 4 | A | Example |
| Ink Composition 104-1 | Ink Stock Solution -4 | Compound 4 | Complementary Dye 1 | A | Example |
| Ink Composition 104-2 | Ink Stock Solution -4 | Compound 4 | Complementary Dye 2 | A | Example |
| Ink Composition 104-3 | Ink Stock Solution -4 | Compound 4 | Complementary Dye 3 | A | Example |
| Ink Composition 104-4 | Ink Stock Solution -4 | Compound 4 | Complementary Dye 4 | A | Example |
| Ink Composition 105-1 | Ink Stock Solution -5 | Compound 5 | Complementary Dye 1 | A | Example |
| Ink Composition 105-2 | Ink Stock Solution -5 | Compound 5 | Complementary Dye 2 | A | Example |
| Ink Composition 105-3 | Ink Stock Solution -5 | Compound 5 | Complementary Dye 3 | A | Example |
| Ink Composition 105-4 | Ink Stock Solution -5 | Compound 5 | Complementary Dye 4 | A | Example |
| Ink Composition 106-1 | Ink Stock Solution -6 | Compound 6 | Complementary Dye 1 | A | Example |
| Ink Composition 106-2 | Ink Stock Solution -6 | Compound 6 | Complementary Dye 2 | A | Example |
| Ink Composition 106-3 | Ink Stock Solution -6 | Compound 6 | Complementary Dye 3 | A | Example |
| Ink Composition 106-4 | Ink Stock Solution -6 | Compound 6 | Complementary Dye 4 | A | Example |
| Ink Composition 107-1 | Ink Stock Solution -7 | Compound 7 | Complementary Dye 1 | A | Example |
| Ink Composition 107-2 | Ink Stock Solution -7 | Compound 7 | Complementary Dye 2 | A | Example |
| Ink Composition 107-3 | Ink Stock Solution -7 | Compound 7 | Complementary Dye 3 | A | Example |
| Ink Composition 107-4 | Ink Stock Solution -7 | Compound 7 | Complementary Dye 4 | A | Example |
| Ink Composition 108-1 | Ink Stock Solution -8 | Compound 8 | Complementary Dye 1 | A | Example |
| Ink Composition 108-2 | Ink Stock Solution -8 | Compound 8 | Complementary Dye 2 | A | Example |
| Ink Composition 108-3 | Ink Stock Solution -8 | Compound 8 | Complementary Dye 3 | A | Example |

(continued)

| Ink Composition | Ink Stock Solution Used | Colorant Contained | Complementary Dye Contained | Dependency on Observation Light Source | Remarks |
|---|---|---|---|---|---|
| Ink Composition 108-4 | Ink Stock Solution -8 | Compound 8 | Complementary Dye 4 | A | Example |
| Ink Composition 109-1 | Ink Stock Solution -9 | Compound 9 | Complementary Dye 1 | A | Example |
| Ink Composition 109-2 | Ink Stock Solution -9 | Compound 9 | Complementary Dye 2 | A | Example |
| Ink Composition 109-3 | Ink Stock Solution -9 | Compound 9 | Complementary Dye 3 | A | Example |
| Ink Composition 109-4 | Ink Stock Solution -9 | Compound 9 | Complementary Dye 4 | A | Example |
| Ink Composition 110-1 | Ink Stock Solution -10 | Compound 10 | Complementary Dye 1 | A | Example |
| Ink Composition 110-2 | Ink Stock Solution -10 | Compound 10 | Complementary Dye 2 | A | Example |
| Ink Composition 110-3 | Ink Stock Solution -10 | Compound 10 | Complementary Dye 3 | A | Example |
| Ink Composition 110-4 | Ink Stock Solution -10 | Compound 10 | Complementary Dye 4 | A | Example |
| Ink Composition 111-1 | Ink Stock Solution -11 | Compound 11 | Complementary Dye 1 | A | Example |
| Ink Composition 111-2 | Ink Stock Solution -11 | Compound 11 | Complementary Dye 2 | A | Example |
| Ink Composition 111-3 | Ink Stock Solution -11 | Compound 11 | Complementary Dye 3 | A | Example |
| Ink Composition 111-4 | Ink Stock Solution -11 | Compound 11 | Complementary Dye 4 | A | Example |
| Ink Composition 112-1 | Ink Stock Solution -12 | Compound 12 | Complementary Dye 1 | A | Example |
| Ink Composition 112-2 | Ink Stock Solution -12 | Compound 12 | Complementary Dye 2 | A | Example |
| Ink Composition 112-3 | Ink Stock Solution -12 | Compound 12 | Complementary Dye 3 | A | Example |
| Ink Composition 112-4 | Ink Stock Solution -12 | Compound 12 | Complementary Dye 4 | A | Example |
| Ink Composition 113-1 | Ink Stock Solution -13 | Compound 13 | Complementary Dye 1 | B | Example |
| Ink Composition 113-2 | Ink Stock Solution -13 | Compound 13 | Complementary Dye 2 | B | Example |
| Ink Composition 113-3 | Ink Stock Solution -13 | Compound 13 | Complementary Dye 3 | B | Example |
| Ink Composition 113-4 | Ink Stock Solution -13 | Compound 13 | Complementary Dye 4 | B | Example |

(continued)

| Ink Composition | Ink Stock Solution Used | Colorant Contained | Complementary Dye Contained | Dependency on Observation Light Source | Remarks |
|---|---|---|---|---|---|
| Ink Composition 114-1 | Ink Stock Solution -14 | Compound 14 | Complementary Dye 1 | B | Example |
| Ink Composition 114-2 | Ink Stock Solution -14 | Compound 14 | Complementary Dye 2 | B | Example |
| Ink Composition 114-3 | Ink Stock Solution -14 | Compound 14 | Complementary Dye 3 | B | Example |
| Ink Composition 114-4 | Ink Stock Solution -14 | Compound 14 | Complementary Dye 4 | B | Example |
| Ink Composition 115-1 | Ink Stock Solution -15 | Compound 15 | Complementary Dye 1 | B | Example |
| Ink Composition 115-2 | Ink Stock Solution -15 | Compound 15 | Complementary Dye 2 | B | Example |
| Ink Composition 115-3 | Ink Stock Solution -15 | Compound 15 | Complementary Dye 3 | B | Example |
| Ink Composition 115-4 | Ink Stock Solution -15 | Compound 15 | Complementary Dye 4 | B | Example |
| Ink Composition 116-1 | Ink Stock Solution -16 | Compound 16 | Complementary Dye 1 | A | Example |
| Ink Composition 116-2 | Ink Stock Solution -16 | Compound 16 | Complementary Dye 2 | A | Example |
| Ink Composition 116-3 | Ink Stock Solution -16 | Compound 16 | Complementary Dye 3 | A | Example |
| Ink Composition 116-4 | Ink Stock Solution -16 | Compound 16 | Complementary Dye 4 | A | Example |
| Ink Composition 117-1 | Ink Stock Solution -17 | Compound 17 | Complementary Dye 1 | A | Example |
| Ink Composition 117-2 | Ink Stock Solution -17 | Compound 17 | Complementary Dye 2 | A | Example |
| Ink Composition 117-3 | Ink Stock Solution -17 | Compound 17 | Complementary Dye 3 | A | Example |
| Ink Composition 117-4 | Ink Stock Solution -17 | Compound 17 | Complementary Dye 4 | A | Example |
| Ink Composition 118-1 | Ink Stock Solution -18 | Compound 18 | Complementary Dye 1 | A | Example |
| Ink Composition 118-2 | Ink Stock Solution -18 | Compound 18 | Complementary Dye 2 | A | Example |
| Ink Composition 118-3 | Ink Stock Solution -18 | Compound 18 | Complementary Dye 3 | A | Example |
| Ink Composition 118-4 | Ink Stock Solution -18 | Compound 18 | Complementary Dye 4 | A | Example |
| Ink Composition 119-1 | Ink Stock Solution -19 | Compound 19 | Complementary Dye 1 | A | Example |

(continued)

| Ink Composition | Ink Stock Solution Used | Colorant Contained | Complementary Dye Contained | Dependency on Observation Light Source | Remarks |
|---|---|---|---|---|---|
| Ink Composition 119-2 | Ink Stock Solution -19 | Compound 19 | Complementary Dye 2 | A | Example |
| Ink Composition 119-3 | Ink Stock Solution -19 | Compound 19 | Complementary Dye 3 | A | Example |
| Ink Composition 119-4 | Ink Stock Solution -19 | Compound 19 | Complementary Dye 4 | A | Example |
| Ink Composition 120-1 | Ink Stock Solution -20 | Compound 20 | Complementary Dye 1 | A | Example |
| Ink Composition 120-2 | Ink Stock Solution -20 | Compound 20 | Complementary Dye 2 | A | Example |
| Ink Composition 120-3 | Ink Stock Solution -20 | Compound 20 | Complementary Dye 3 | A | Example |
| Ink Composition 120-4 | Ink Stock Solution -20 | Compound 20 | Complementary Dye 4 | A | Example |
| Ink Composition 121-1 | Ink Stock Solution-21 | Compound 21 | Complementary Dye 1 | A | Example |
| Ink Composition 121-2 | Ink Stock Solution-21 | Compound 21 | Complementary Dye 2 | A | Example |
| Ink Composition 121-3 | Ink Stock Solution-21 | Compound 21 | Complementary Dye 3 | A | Example |
| Ink Composition 121-4 | Ink Stock Solution-21 | Compound 21 | Complementary Dye 4 | A | Example |
| Ink Composition 122-1 | Ink Stock Solution-22 | Compound 27 | Complementary Dye 1 | A | Example |
| Ink Composition 122-2 | Ink Stock Solution-22 | Compound 27 | Complementary Dye 2 | A | Example |
| Ink Composition 122-3 | Ink Stock Solution-22 | Compound 27 | Complementary Dye 3 | A | Example |
| Ink Composition 122-4 | Ink Stock Solution-22 | Compound 27 | Complementary Dye 4 | A | Example |
| Ink Composition 123-1 | Ink Stock Solution-23 | Compound 29 | Complementary Dye 1 | A | Example |
| Ink Composition 123-2 | Ink Stock Solution-23 | Compound 29 | Complementary Dye 2 | A | Example |
| Ink Composition 123-3 | Ink Stock Solution-23 | Compound 29 | Complementary Dye 3 | A | Example |
| Ink Composition 123-4 | Ink Stock Solution-23 | Compound 29 | Complementary Dye 4 | A | Example |
| Ink Composition 124-1 | Ink Stock Solution-24 | Compound 31 | Complementary Dye 1 | A | Example |
| Ink Composition 124-2 | Ink Stock Solution-24 | Compound 31 | Complementary Dye 2 | A | Example |

(continued)

| Ink Composition | Ink Stock Solution Used | Colorant Contained | Complementary Dye Contained | Dependency on Observation Light Source | Remarks |
|---|---|---|---|---|---|
| Ink Composition 124-3 | Ink Stock Solution-24 | Compound 31 | Complementary Dye 3 | A | Example |
| Ink Composition 124-4 | Ink Stock Solution-24 | Compound 31 | Complementary Dye 4 | A | Example |
| Ink Composition 125-1 | Ink Stock Solution-25 | Compound 32 | Complementary Dye 1 | A | Example |
| Ink Composition 125-2 | Ink Stock Solution-25 | Compound 32 | Complementary Dye 2 | A | Example |
| Ink Composition 125-3 | Ink Stock Solution-25 | Compound 32 | Complementary Dye 3 | A | Example |
| Ink Composition 125-4 | Ink Stock Solution-25 | Compound 32 | Complementary Dye 4 | A | Example |
| Ink Composition 126-1 | Ink Stock Solution-26 | Compound 33 | Complementary Dye 1 | A | Example |
| Ink Composition 126-2 | Ink Stock Solution-26 | Compound 33 | Complementary Dye 2 | A | Example |
| Ink Composition 126-3 | Ink Stock Solution-26 | Compound 33 | Complementary Dye 3 | A | Example |
| Ink Composition 126-4 | Ink Stock Solution-26 | Compound 33 | Complementary Dye 4 | A | Example |
| Ink Composition 127-1 | Ink Stock Solution-27 | Compound 36 | Complementary Dye 1 | A | Example |
| Ink Composition 127-2 | Ink Stock Solution-27 | Compound 36 | Complementary Dye 2 | A | Example |
| Ink Composition 127-3 | Ink Stock Solution-27 | Compound 36 | Complementary Dye 3 | A | Example |
| Ink Composition 127-4 | Ink Stock Solution-27 | Compound 36 | Complementary Dye 4 | A | Example |
| Ink Composition 128-1 | Ink Stock Solution-28 | Compound 38 | Complementary Dye 1 | A | Example |
| Ink Composition 128-2 | Ink Stock Solution-28 | Compound 38 | Complementary Dye 2 | A | Example |
| Ink Composition 128-3 | Ink Stock Solution-28 | Compound 38 | Complementary Dye 3 | A | Example |
| Ink Composition 128-4 | Ink Stock Solution-28 | Compound 38 | Complementary Dye 4 | A | Example |
| Ink Composition 129-1 | Ink Stock Solution-29 | Compound 41 | Complementary Dye 1 | A | Example |
| Ink Composition 129-2 | Ink Stock Solution-29 | Compound 41 | Complementary Dye 2 | A | Example |
| Ink Composition 129-3 | Ink Stock Solution-29 | Compound 41 | Complementary Dye 3 | A | Example |

(continued)

| Ink Composition | Ink Stock Solution Used | Colorant Contained | Complementary Dye Contained | Dependency on Observation Light Source | Remarks |
|---|---|---|---|---|---|
| Ink Composition 129-4 | Ink Stock Solution-29 | Compound 41 | Complementary Dye 4 | A | Example |
| Ink Composition 130-1 | Ink Stock Solution-30 | Compound 42 | Complementary Dye 1 | A | Example |
| Ink Composition 130-2 | Ink Stock Solution-30 | Compound 42 | Complementary Dye 2 | A | Example |
| Ink Composition 130-3 | Ink Stock Solution-30 | Compound 42 | Complementary Dye 3 | A | Example |
| Ink Composition 130-4 | Ink Stock Solution-30 | Compound 42 | Complementary Dye 4 | A | Example |
| Ink Composition 131-1 | Ink Stock Solution-31 | Compound 50 | Complementary Dye 1 | A | Example |
| Ink Composition 131-2 | Ink Stock Solution-31 | Compound 50 | Complementary Dye 2 | A | Example |
| Ink Composition 131-3 | Ink Stock Solution-31 | Compound 50 | Complementary Dye 3 | A | Example |
| Ink Composition 131-4 | Ink Stock Solution-31 | Compound 50 | Complementary Dye 4 | A | Example |
| Ink Composition 132-1 | Ink Stock Solution-32 | Compound 51 | Complementary Dye 1 | A | Example |
| Ink Composition 132-2 | Ink Stock Solution-32 | Compound 51 | Complementary Dye 2 | A | Example |
| Ink Composition 132-3 | Ink Stock Solution-32 | Compound 51 | Complementary Dye 3 | A | Example |
| Ink Composition 132-4 | Ink Stock Solution-32 | Compound 51 | Complementary Dye 4 | A | Example |
| Ink Composition 133-1 | Ink Stock Solution-33 | Compound 57 | Complementary Dye 1 | A | Example |
| Ink Composition 133-2 | Ink Stock Solution-33 | Compound 57 | Complementary Dye 2 | A | Example |
| Ink Composition 133-3 | Ink Stock Solution-33 | Compound 57 | Complementary Dye 3 | A | Example |
| Ink Composition 133-4 | Ink Stock Solution-33 | Compound 57 | Complementary Dye 4 | A | Example |
| Ink Composition 134-1 | Ink Stock Solution-34 | Compound 58 | Complementary Dye 1 | A | Example |
| Ink Composition 134-2 | Ink Stock Solution-34 | Compound 58 | Complementary Dye 2 | A | Example |
| Ink Composition 134-3 | Ink Stock Solution-34 | Compound 58 | Complementary Dye 3 | A | Example |
| Ink Composition 134-4 | Ink Stock Solution-34 | Compound 58 | Complementary Dye 4 | A | Example |

(continued)

| Ink Composition | Ink Stock Solution Used | Colorant Contained | Complementary Dye Contained | Dependency on Observation Light Source | Remarks |
|---|---|---|---|---|---|
| Ink Composition 135-1 | Ink Stock Solution-35 | Compound 59 | Complementary Dye 1 | A | Example |
| Ink Composition 135-2 | Ink Stock Solution-35 | Compound 59 | Complementary Dye 2 | A | Example |
| Ink Composition 135-3 | Ink Stock Solution-35 | Compound 59 | Complementary Dye 3 | A | Example |
| Ink Composition 135-4 | Ink Stock Solution-35 | Compound 59 | Complementary Dye 4 | A | Example |
| Ink Composition 136-1 | Ink Stock Solution-36 | Compound 60 | Complementary Dye 1 | A | Example |
| Ink Composition 136-2 | Ink Stock Solution-36 | Compound 60 | Complementary Dye 2 | A | Example |
| Ink Composition 136-3 | Ink Stock Solution-36 | Compound 60 | Complementary Dye 3 | A | Example |
| Ink Composition 136-4 | Ink Stock Solution-36 | Compound 60 | Complementary Dye 4 | A | Example |
| Ink Composition 137-1 | Ink Stock Solution-37 | Compound 61 | Complementary Dye 1 | A | Example |
| Ink Composition 137-2 | Ink Stock Solution-37 | Compound 61 | Complementary Dye 2 | A | Example |
| Ink Composition 137-3 | Ink Stock Solution-37 | Compound 61 | Complementary Dye 3 | A | Example |
| Ink Composition 137-4 | Ink Stock Solution-37 | Compound 61 | Complementary Dye 4 | A | Example |
| Ink Composition 138-1 | Ink Stock Solution-38 | Compound 62 | Complementary Dye 1 | A | Example |
| Ink Composition 138-2 | Ink Stock Solution-38 | Compound 62 | Complementary Dye 2 | A | Example |
| Ink Composition 138-3 | Ink Stock Solution-38 | Compound 62 | Complementary Dye 3 | A | Example |
| Ink Composition 13 8-4 | Ink Stock Solution-38 | Compound 62 | Complementary Dye 4 | A | Example |
| Ink Composition 139-1 | Ink Stock Solution-39 | Compound 63 | Complementary Dye 1 | A | Example |
| Ink Composition 139-2 | Ink Stock Solution-39 | Compound 63 | Complementary Dye 2 | A | Example |
| Ink Composition 139-3 | Ink Stock Solution-39 | Compound 63 | Complementary Dye 3 | A | Example |
| Ink Composition 139-4 | Ink Stock Solution-39 | Compound 63 | Complementary Dye 4 | A | Example |
| Ink Composition 140-1 | Ink Stock Solution-40 | Compound 64 | Complementary Dye 1 | A | Example |

(continued)

| Ink Composition | Ink Stock Solution Used | Colorant Contained | Complementary Dye Contained | Dependency on Observation Light Source | Remarks |
|---|---|---|---|---|---|
| Ink Composition 140-2 | Ink Stock Solution-40 | Compound 64 | Complementary Dye 2 | A | Example |
| Ink Composition 140-3 | Ink Stock Solution-40 | Compound 64 | Complementary Dye 3 | A | Example |
| Ink Composition 140-4 | Ink Stock Solution-40 | Compound 64 | Complementary Dye 4 | A | Example |
| Ink Composition 141-1 | Ink Stock Solution-41 | Compound 65 | Complementary Dye 1 | A | Example |
| Ink Composition 141-2 | Ink Stock Solution-41 | Compound 65 | Complementary Dye 2 | A | Example |
| Ink Composition 141-3 | Ink Stock Solution-41 | Compound 65 | Complementary Dye 3 | A | Example |
| Ink Composition 141-4 | Ink Stock Solution-41 | Compound 65 | Complementary Dye 4 | A | Example |
| Ink Composition 142-1 | Ink Stock Solution-42 | Compound 66 | Complementary Dye 1 | A | Example |
| Ink Composition 142-2 | Ink Stock Solution-42 | Compound 66 | Complementary Dye 2 | A | Example |
| Ink Composition 142-3 | Ink Stock Solution-42 | Compound 66 | Complementary Dye 3 | A | Example |
| Ink Composition 142-4 | Ink Stock Solution-42 | Compound 66 | Complementary Dye 4 | A | Example |
| Ink Composition 143-1 | Ink Stock Solution-43 | Compound 67 | Complementary Dye 1 | A | Example |
| Ink Composition 143-2 | Ink Stock Solution-43 | Compound 67 | Complementary Dye 2 | A | Example |
| Ink Composition 143-3 | Ink Stock Solution-43 | Compound 67 | Complementary Dye 3 | A | Example |
| Ink Composition 143-4 | Ink Stock Solution-43 | Compound 67 | Complementary Dye 4 | A | Example |
| Ink Composition 144-1 | Ink Stock Solution-44 | Compound 68 | Complementary Dye 1 | A | Example |
| Ink Composition 144-2 | Ink Stock Solution-44 | Compound 68 | Complementary Dye 2 | A | Example |
| Ink Composition 144-3 | Ink Stock Solution-44 | Compound 68 | Complementary Dye 3 | A | Example |
| Ink Composition 144-4 | Ink Stock Solution-44 | Compound 68 | Complementary Dye 4 | A | Example |
| Ink Composition 145-1 | Ink Stock Solution-45 | Compound 69 | Complementary Dye 1 | A | Example |
| Ink Composition 145-2 | Ink Stock Solution-45 | Compound 69 | Complementary Dye 2 | A | Example |

(continued)

| Ink Composition | Ink Stock Solution Used | Colorant Contained | Complementary Dye Contained | Dependency on Observation Light Source | Remarks |
|---|---|---|---|---|---|
| Ink Composition 145-3 | Ink Stock Solution-45 | Compound 69 | Complementary Dye 3 | A | Example |
| Ink Composition 145-4 | Ink Stock Solution-45 | Compound 69 | Complementary Dye 4 | A | Example |
| Ink Composition 146-1 | Ink Stock Solution-46 | Compound 70 | Complementary Dye 1 | A | Example |
| Ink Composition 146-2 | Ink Stock Solution-46 | Compound 70 | Complementary Dye 2 | A | Example |
| Ink Composition 146-3 | Ink Stock Solution-46 | Compound 70 | Complementary Dye 3 | A | Example |
| Ink Composition 146-4 | Ink Stock Solution-46 | Compound 70 | Complementary Dye 4 | A | Example |
| Ink Composition 147-1 | Ink Stock Solution-47 | Compound 71 | Complementary Dye 1 | A | Example |
| Ink Composition 147-2 | Ink Stock Solution-47 | Compound 71 | Complementary Dye 2 | A | Example |
| Ink Composition 147-3 | Ink Stock Solution-47 | Compound 71 | Complementary Dye 3 | A | Example |
| Ink Composition 147-4 | Ink Stock Solution-47 | Compound 71 | Complementary Dye 4 | A | Example |
| Ink Composition 148-1 | Ink Stock Solution-48 | Compound 72 | Complementary Dye 1 | A | Example |
| Ink Composition 148-2 | Ink Stock Solution-48 | Compound 72 | Complementary Dye 2 | A | Example |
| Ink Composition 148-3 | Ink Stock Solution-48 | Compound 72 | Complementary Dye 3 | A | Example |
| Ink Composition 148-4 | Ink Stock Solution-48 | Compound 72 | Complementary Dye 4 | A | Example |
| Ink Composition 149-1 | Ink Stock Solution-49 | Compound 73 | Complementary Dye 1 | A | Example |
| Ink Composition 149-2 | Ink Stock Solution-49 | Compound 73 | Complementary Dye 2 | A | Example |
| Ink Composition 149-3 | Ink Stock Solution-49 | Compound 73 | Complementary Dye 3 | A | Example |
| Ink Composition 149-4 | Ink Stock Solution-49 | Compound 73 | Complementary Dye 4 | A | Example |
| Ink Composition 150-1 | Ink Stock Solution-50 | Compound 74 | Complementary Dye 1 | A | Example |
| Ink Composition 150-2 | Ink Stock Solution-50 | Compound 74 | Complementary Dye 2 | A | Example |
| Ink Composition 150-3 | Ink Stock Solution-50 | Compound 74 | Complementary Dye 3 | A | Example |

(continued)

| Ink Composition | Ink Stock Solution Used | Colorant Contained | Complementary Dye Contained | Dependency on Observation Light Source | Remarks |
|---|---|---|---|---|---|
| Ink Composition 150-4 | Ink Stock Solution-50 | Compound 74 | Complementary Dye 4 | A | Example |
| Ink Composition 151-1 | Ink Stock Solution-51 | Compound 75 | Complementary Dye 1 | A | Example |
| Ink Composition 151-2 | Ink Stock Solution-51 | Compound 75 | Complementary Dye 2 | A | Example |
| Ink Composition 151-3 | Ink Stock Solution-51 | Compound 75 | Complementary Dye 3 | A | Example |
| Ink Composition 151-4 | Ink Stock Solution-51 | Compound 75 | Complementary Dye 4 | A | Example |
| Ink Composition 152-1 | Ink Stock Solution-52 | Compound 76 | Complementary Dye 1 | A | Example |
| Ink Composition 152-2 | Ink Stock Solution-52 | Compound 76 | Complementary Dye 2 | A | Example |
| Ink Composition 152-3 | Ink Stock Solution-52 | Compound 76 | Complementary Dye 3 | A | Example |
| Ink Composition 152-4 | Ink Stock Solution-52 | Compound 76 | Complementary Dye 4 | A | Example |
| Ink Composition 153-1 | Ink Stock Solution-53 | Compound 77 | Complementary Dye 1 | A | Example |
| Ink Composition 153-2 | Ink Stock Solution-53 | Compound 77 | Complementary Dye 2 | A | Example |
| Ink Composition 153-3 | Ink Stock Solution-53 | Compound 77 | Complementary Dye 3 | A | Example |
| Ink Composition 153-4 | Ink Stock Solution-53 | Compound 77 | Complementary Dye 4 | A | Example |
| Ink Composition 151-1 | Ink Stock Solution-54 | Compound 78 | Complementary Dye 1 | A | Example |
| Ink Composition 154-2 | Ink Stock Solution-54 | Compound 78 | Complementary Dye 2 | A | Example |
| Ink Composition 154-3 | Ink Stock Solution-54 | Compound 78 | Complementary Dye 3 | A | Example |
| Ink Composition 154-4 | Ink Stock Solution-54 | Compound 78 | Complementary Dye 4 | A | Example |
| Ink Composition 155-1 | Ink Stock Solution-55 | Compound 79 | Complementary Dye 1 | A | Example |
| Ink Composition 155-2 | Ink Stock Solution-55 | Compound 79 | Complementary Dye 2 | A | Example |
| Ink Composition 155-3 | Ink Stock Solution-55 | Compound 79 | Complementary Dye 3 | A | Example |
| Ink Composition 155-4 | Ink Stock Solution-55 | Compound 79 | Complementary Dye 4 | A | Example |

(continued)

| Ink Composition | Ink Stock Solution Used | Colorant Contained | Complementary Dye Contained | Dependency on Observation Light Source | Remarks |
|---|---|---|---|---|---|
| Ink Composition 156-1 | Ink Stock Solution-56 | Compound 80 | Complementary Dye 1 | A | Example |
| Ink Composition 156-2 | Ink Stock Solution-56 | Compound 80 | Complementary Dye 2 | A | Example |
| Ink Composition 156-3 | Ink Stock Solution-56 | Compound 80 | Complementary Dye 3 | A | Example |
| Ink Composition 156-4 | Ink Stock Solution-56 | Compound 80 | Complementary Dye 4 | A | Example |
| Ink Composition 157-1 | Ink Stock Solution-57 | Compound 81 | Complementary Dye 1 | A | Example |
| Ink Composition 157-2 | Ink Stock Solution-57 | Compound 81 | Complementary Dye 2 | A | Example |
| Ink Composition 157-3 | Ink Stock Solution-57 | Compound 81 | Complementary Dye 3 | A | Example |
| Ink Composition 157-4 | Ink Stock Solution-57 | Compound 81 | Complementary Dye 4 | A | Example |
| Comparative Ink Composition 1-1 | Comparative Ink Stock Solution-01 | Comparative Compound 1 | Complementary Dye 1 | C | Comparative Example |
| Comparative Ink Composition 1-2 | Comparative Ink Stock Solution-01 | Comparative Compound 1 | Complementary Dye 2 | C | Comparative Example |
| Comparative Ink Composition 1-3 | Comparative Ink Stock Solution-01 | Comparative Compound 1 | Complementary Dye 3 | C | Comparative Example |
| Comparative Ink Composition 1-4 | Comparative Ink Stock Solution-01 | Comparative Compound 1 | Complementary Dye 4 | C | Comparative Example |

This application is based on a Japanese patent application filed on September 26, 2012 (Application No. 2012-212754), a Japanese patent application filed on March 14, 2013 (Application No. 2013-051807), a Japanese patent application filed on September 20, 2013 (Application No. 2013-196181), the contents thereof being incorporated herein by reference.

## Claims

**1.** A compound represented by the following formula (1):

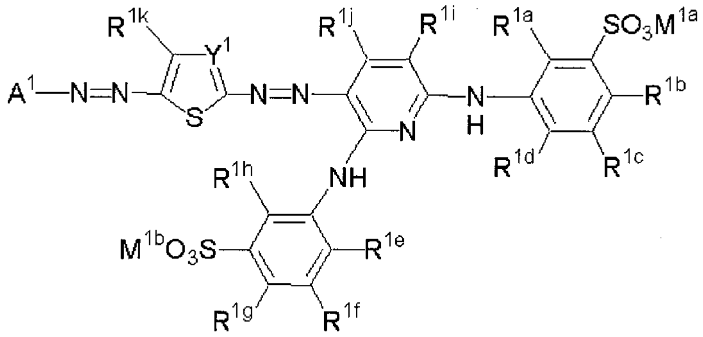

(1)

wherein
each of $R^{1a}$ to $R^{1k}$ independently represents a hydrogen atom or a monovalent substituent, the substituents may combine with each other to form a ring,
each of $M^{1a}$ and $M^{1b}$ independently represents a hydrogen atom or a monovalent counter cation,
$Y^1$ represents a nitrogen atom or a carbon atom having a hydrogen atom or monovalent substituent,
$A^1$ represents an aromatic group, and the aromatic group represented by $A^1$ may contain a heteroatom or may have a substituent.

2. The compound as claimed in claim 1,
wherein the compound represented by formula (1) is a compound represented by the following formula (2):

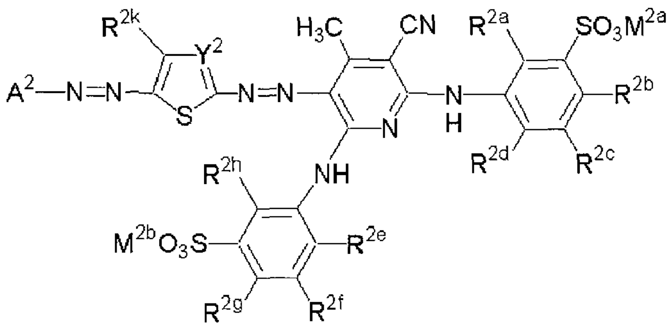

(2)

wherein
each of $R^{2a}$ to $R^{2h}$ and $R^{2k}$ independently represents a hydrogen atom or a monovalent substituent, the substituents may combine with each other to form a ring,
each of $M^{2a}$ and $M^{2b}$ independently represents a hydrogen atom or a monovalent counter cation,
$Y^2$ represents a nitrogen atom or a carbon atom having a hydrogen atom or a monovalent substituent,
$A^2$ represents an aromatic group, and the aromatic group represented by $A^2$ may contain a heteroatom or may have a substituent.

3. The compound as claimed in claim 2,
wherein the compound represented by formula (2) is a compound represented by the following formula (3):

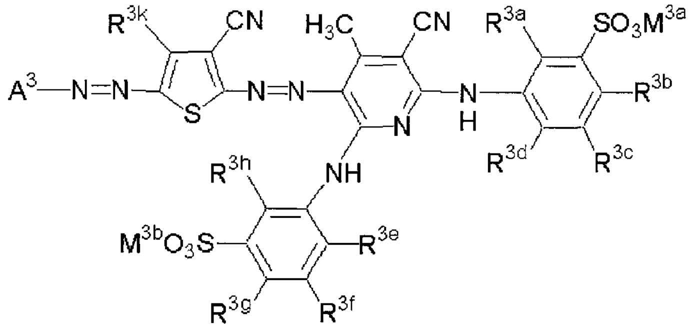

(3)

wherein
each of $R^{3a}$ to $R^{3h}$ and $R^{3k}$ independently represents a hydrogen atom or a monovalent substituent, the substituents may combine with each other to form a ring,
each of $M^{3a}$ and $M^{3b}$ independently represents a hydrogen atom or a monovalent counter cation,
$A^3$ represents an aromatic group, and the aromatic group represented by $A^3$ may contain a heteroatom or may have a substituent.

**4.** The compound as claimed in claim 3,
wherein the compound represented by formula (3) is a compound represented by the following formula (4):

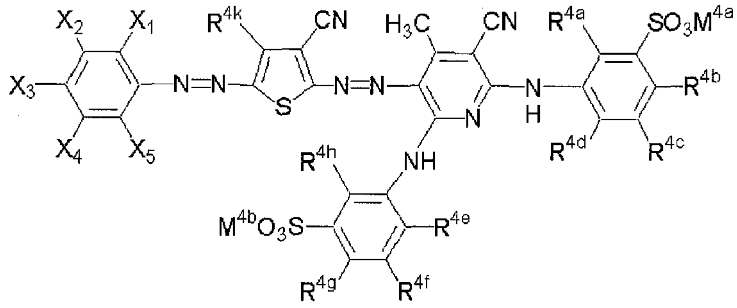

(4)

wherein
each of $R^{4a}$ to $R^{4h}$ and $R^{4k}$ independently represents a hydrogen atom or a monovalent substituent, the substituents may combine with each other to form a ring,
each of $M^{4a}$ and $M^{4b}$ independently represents a hydrogen atom or a monovalent counter cation, and
each of $X_1$ to $X_5$ independently represents a hydrogen atom or a monovalent substituent.

**5.** The compound as claimed in claim 3,
wherein the compound represented by formula (3) is a compound represented by the following formula (5):

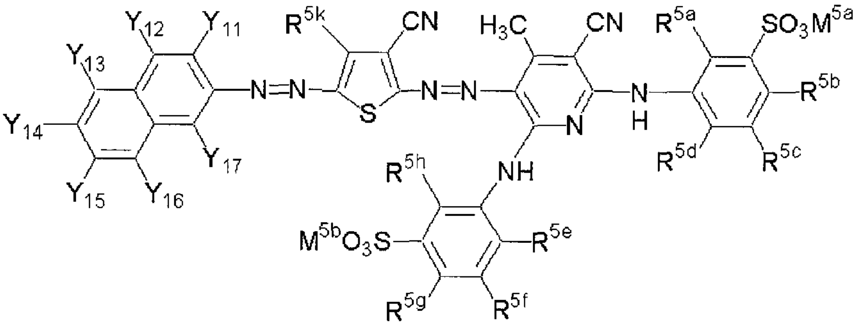

(5)

wherein
each of $R^{5a}$ to $R^{5h}$ and $R^{5k}$ independently represents a hydrogen atom or a monovalent substituent, the substituents may combine with each other to form a ring,
each of $M^{5a}$ and $M^{5b}$ independently represents a hydrogen atom or a monovalent counter cation, and
each of $Y_{11}$ to $Y_{17}$ independently represents a hydrogen atom or a monovalent substituent.

**6.** The compound as claimed in claim 3,
wherein the compound represented by formula (3) is a compound represented by the following formula (6):

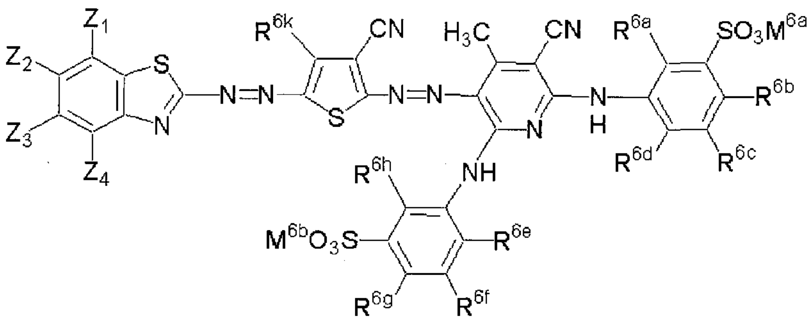

(6)

wherein
each of $R^{6a}$ to $R^{6h}$ and $R^{6k}$ independently represents a hydrogen atom or a monovalent substituent, the substituents may combine with each other to form a ring,
each of $M^{6a}$ and $M^{6b}$ independently represents a hydrogen atom or a monovalent counter cation, and
each of $Z_1$ to $Z_4$ independently represents a hydrogen atom or a monovalent substituent.

**7.** The compound as claimed in any one of claims 1 to 6,
wherein $R^{1k}$, $R^{2k}$, $R^{3k}$, $R^{4k}$, $R^{5k}$ or $R^{6k}$ is a group represented by the following formula (7):

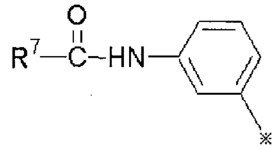

(7)
wherein $R^7$ represents a monovalent substituent, and
* represents a bond.

**8.** The compound as claimed in any one of claims 1 to 6, wherein $R^{1k}$, $R^{2k}$, $R^{3k}$, $R^{4k}$, $R^{5k}$ or $R^{6k}$ is a group represented by the following formula (8):

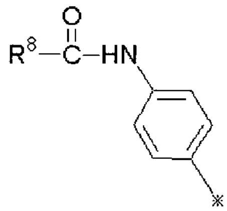

(8)

wherein $R^8$ represents a monovalent substituent, and
* represents a bond.

**9.** The compound as claimed in any one of claims 1 to 6, wherein $R^{1k}$, $R^{2k}$, $R^{3k}$, $R^{4k}$, $R^{5k}$ or $R^{6k}$ is a group represented by the following formula (9):

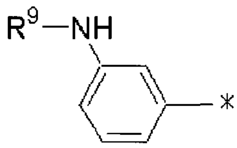

(9)

wherein $R^9$ represents a monovalent substituent, and
* represents a bond.

**10.** The compound as claimed in any one of claims 1 to 6, wherein $R^{1k}$, $R^{2k}$, $R^{3k}$, $R^{4k}$, $R^{5k}$ or $R^{6k}$ is a group represented by the following formula (10):

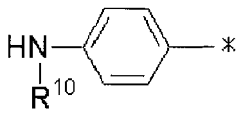

(10)

wherein $R^{10}$ represents a monovalent substituent, and
* represents a bond.

**11.** The compound as claimed in any one of claims 1 to 6, wherein $R^{1k}$, $R^{2k}$, $R^{3k}$, $R^{4k}$, $R^{5k}$ or $R^{6k}$ is a group represented by the following formula (11):

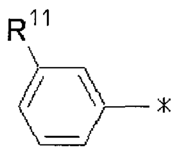

(11)

wherein $R^{11}$ represents a monovalent substituent, and
* represents a bond.

**12.** The compound as claimed in any one of claims 1 to 6,
wherein $R^{1k}$, $R^{2k}$, $R^{3k}$, $R^{4k}$, $R^{5k}$ or $R^{6k}$ is a group represented by the following formula (12):

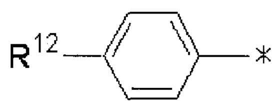

(12)

wherein $R^{12}$ represents a monovalent substituent, and
* represents a bond.

**13.** The compound as claimed in any one of claims 1 to 12, having three or more ionic hydrophilic groups.

**14.** An aqueous solution comprising:

(a) a preservative and
(b) at least one member of the compound claimed in any one of claims 1 to 12 and a salt thereof,

wherein the content of (b) is from 1 to 25 mass%.

**15.** The aqueous solution as claimed in claim 14, further comprising:

(c) a pH adjusting agent.

**16.** The aqueous solution as claimed in claim 14 or 15,
wherein the pH at 25°C is from 7.0 to 9.0.

**17.** An ink composition comprising the aqueous solution claimed in any one of claims 14 to 16.

**18.** An ink for inkjet recording, comprising the aqueous solution claimed in any one of claims 14 to 16 or the ink composition claimed in claim 17.

**19.** An inkjet recording method, comprising:

forming a colored image on a recording material by using the ink for inkjet recording claimed in claim 18.

**20.** An ink cartridge for inkjet recording, which is filled with the ink for inkjet recording claimed in claim 18.

**21.** An inkjet recorded material, wherein a colored image is formed on a recording material by using the ink for inkjet recording claimed in claim 18.

Europäisches Patentamt
European Patent Office
Office européen des brevets

# EUROPEAN SEARCH REPORT

Application Number
EP 13 18 5960

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 495 080 A1 (FUJI PHOTO FILM CO LTD [JP]) 12 January 2005 (2005-01-12) * compounds b-6,h-4,n-5 * ----- | 1-6, 13-21 | INV. C09B31/153 C09D11/00 |
| A,P | EP 2 604 656 A1 (FUJIFILM CORP [JP]) 19 June 2013 (2013-06-19) * examples * ----- | 1-21 | |
| X,D | EP 1 437 388 A1 (FUJI PHOTO FILM CO LTD [JP] FUJIFILM CORP [JP]) 14 July 2004 (2004-07-14) * page 24; table 8 * * page 25; table 9 * * page 26; table 10 * * page 28; table 12 * ----- | 1-21 | |
| X,D | WO 2004/113463 A1 (FUJI PHOTO FILM CO LTD [JP]; CHINO TOMOHIRO [JP]; FUJIWARA TOSHIKI [JP) 29 December 2004 (2004-12-29) * page 18 * * page 27 - page 32 * ----- | 1,8,12 | |
| | | | TECHNICAL FIELDS SEARCHED (IPC) |
| A,D,P | EP 2 599 837 A1 (FUJIFILM CORP [JP]) 5 June 2013 (2013-06-05) * claims; examples * ----- | 1-21 | C09B C09D |
| A,D | EP 1 391 488 A1 (FUJI PHOTO FILM CO LTD [JP] FUJIFILM CORP [JP]) 25 February 2004 (2004-02-25) * tables 1-4 * ----- | 1-21 | |

The present search report has been drawn up for all claims

1

| Place of search | Date of completion of the search | Examiner |
|---|---|---|
| Munich | 4 November 2013 | Ketterer, Michael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 18 5960

This annex lists the patent family members relating to the patent documents cited in the above-mentioned European search report.
The members are as contained in the European Patent Office EDP file on
The European Patent Office is in no way liable for these particulars which are merely given for the purpose of information.

04-11-2013

| Patent document cited in search report | Publication date | Patent family member(s) | Publication date |
|---|---|---|---|
| EP 1495080 A1 | 12-01-2005 | AT 310780 T | 15-12-2005 |
| | | AU 2003223116 A1 | 27-10-2003 |
| | | CA 2482895 A1 | 23-10-2003 |
| | | CN 1646643 A | 27-07-2005 |
| | | DE 60302448 D1 | 29-12-2005 |
| | | DE 60302448 T2 | 08-06-2006 |
| | | EP 1495080 A1 | 12-01-2005 |
| | | US 2005243151 A1 | 03-11-2005 |
| | | WO 03087238 A1 | 23-10-2003 |
| EP 2604656 A1 | 19-06-2013 | CA 2807052 A1 | 02-02-2012 |
| | | CN 103052688 A | 17-04-2013 |
| | | EP 2604656 A1 | 19-06-2013 |
| | | JP 2012177073 A | 13-09-2012 |
| | | TW 201211162 A | 16-03-2012 |
| | | US 2013139723 A1 | 06-06-2013 |
| | | WO 2012014954 A1 | 02-02-2012 |
| EP 1437388 A1 | 14-07-2004 | EP 1437388 A1 | 14-07-2004 |
| | | JP 4486810 B2 | 23-06-2010 |
| | | JP 2005146244 A | 09-06-2005 |
| | | US 2004187232 A1 | 30-09-2004 |
| WO 2004113463 A1 | 29-12-2004 | AT 485346 T | 15-11-2010 |
| | | AU 2004250006 A1 | 29-12-2004 |
| | | CA 2514815 A1 | 29-12-2004 |
| | | EP 1633824 A1 | 15-03-2006 |
| | | KR 20060021285 A | 07-03-2006 |
| | | US 2006162616 A1 | 27-07-2006 |
| | | WO 2004113463 A1 | 29-12-2004 |
| EP 2599837 A1 | 05-06-2013 | CA 2807057 A1 | 02-02-2012 |
| | | CN 103038290 A | 10-04-2013 |
| | | EP 2599837 A1 | 05-06-2013 |
| | | JP 2012177074 A | 13-09-2012 |
| | | TW 201209103 A | 01-03-2012 |
| | | US 2013139724 A1 | 06-06-2013 |
| | | WO 2012014955 A1 | 02-02-2012 |
| EP 1391488 A1 | 25-02-2004 | DE 60317278 T2 | 28-08-2008 |
| | | EP 1391488 A1 | 25-02-2004 |
| | | JP 4226860 B2 | 18-02-2009 |
| | | JP 2004083609 A | 18-03-2004 |
| | | US 2004053988 A1 | 18-03-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012014954 A
- WO 2012014955 A
- JP 2003306623 A
- JP 2005139427 A
- JP 2005146244 A
- JP 2004083903 A
- JP 2003277661 A
- JP 2003277662 A
- US 20030213405 A
- JP 10130557 A
- JP 9255906 A
- JP 6234944 A
- JP 7097541 A
- EP 982371 A
- WO 0043450 A
- WO 0043451 A
- WO 0043452 A
- WO 0043453 A
- WO 03106572 A
- WO 03104332 A
- JP 2003238862 A
- JP 2004083609 A
- JP 2002302619 A
- JP 2002327131 A
- JP 2002265809 A
- WO 0148090 A
- WO 04087815 A
- WO 0290441 A
- WO 03027185 A
- WO 04085541 A
- JP 2003321627 A
- JP 2002332418 A
- JP 2002332419 A
- WO 02059215 A
- WO 02059216 A
- WO 04087814 A
- WO 04046252 A
- WO 04046265 A
- US 6652637 B
- WO 0058407 A
- JP 3558213 B
- JP 3558212 B
- JP 3558211 B
- JP 2004285351 A
- WO 04078860 A
- JP 2004323605 A
- WO 04104108 A
- EP 860475 A
- US 5554739 A
- US 5571311 A
- JP 9151342 A
- JP 10140065 A
- JP 10292143 A
- JP 11166145 A
- JP 3069949 A
- EP 549486 A
- JP 2002371214 A
- JP 2002309118 A
- JP 2003012952 A
- JP 2003012956 A
- US 2322027 A
- US 2533514 A
- US 2772163 A
- US 2835579 A
- US 3594171 A
- US 3676137 A
- US 3689271 A
- US 3700454 A
- US 3748141 A
- US 3764336 A
- US 3765897 A
- US 3912515 A
- US 3936303 A
- US 4004928 A
- US 4080209 A
- US 4127413 A
- US 4193802 A
- US 4207393 A
- US 4220711 A
- US 4239851 A
- US 4278757 A
- US 4353979 A
- US 4363873 A
- US 4430421 A
- US 4430422 A
- US 4464464 A
- US 4483918 A
- US 4540657 A
- US 4684606 A
- US 4728599 A
- US 4745049 A
- US 4935321 A
- US 5013639 A
- DE 276319 A
- DE 286253 A
- DE 289820 A
- DE 309158 A
- DE 309159 A

- DE 309160 A
- DE 509311 A
- DE 510576 A
- DE 147009
- DE 157147
- DE 159573
- DE 225240 A
- DE 2091124 A
- JP 48047335 A
- JP 50026530 A
- JP 51025133 A
- JP 51026036 A
- JP 51027921 A
- JP 51027922 A
- JP 51149028 A
- JP 52046816 A
- JP 53001520 A
- JP 53001521 A
- JP 53015127 A
- JP 53146622 A
- JP 54091325 A
- JP 54106228 A
- JP 54118246 A
- JP 55059464 A
- JP 56064333 A
- JP 56081836 A
- JP 59204041 A
- JP 61084641 A
- JP 62118345 A
- JP 62247364 A
- JP 63167357 A
- JP 63214744 A
- JP 63301941 A
- JP 649452 A
- JP 649454 A
- JP 6468745 A
- JP 1101543 A
- JP 1102454 A
- JP 2000792 A
- JP 2004239 A
- JP 2043541 A
- JP 4029237 A
- JP 4030165 A
- JP 4232946 A
- JP 4346338 A
- JP 59157636 A
- US 4533254 A
- JP 6047264 A
- US 5720551 A
- JP 5148436 A
- JP 5295312 A
- JP 7082515 A
- JP 7118584 A
- JP 58185677 A
- JP 61190537 A
- JP 2000782 A
- JP 5197075 A
- JP 9034057 A
- JP 46002784 A
- JP 5194483 A
- US 3214463 A
- JP 48030492 B
- JP 56021141 B
- JP 10088106 A
- JP 4298503 A
- JP 8053427 A
- JP 8239368 A
- JP 10182621 A
- JP 8501291 T
- JP 62215272 A
- JP 3002064 A
- JP 10081064 A
- JP 10119423 A
- JP 10157277 A
- JP 10217601 A
- JP 11348409 A
- JP 2001138621 A
- JP 2000043401 A
- JP 2000211235 A
- JP 2000309157 A
- JP 2001096897 A
- JP 2001138627 A
- JP 11091242 A
- JP 8002087 A
- JP 8002090 A
- JP 8002091 A
- JP 8002093 A
- JP 8174992 A
- JP 11192777 A
- JP 2001301314 A
- JP 48028325 A
- JP 54074430 A
- JP 54124726 A
- JP 55022766 A
- JP 55142339 A
- JP 60023850 A
- JP 60023851 A
- JP 60023852 A
- JP 60023853 A
- JP 60057836 A
- JP 60060643 A
- JP 60118834 A
- JP 60122940 A
- JP 60122941 A
- JP 60122942 A
- JP 60235134 A
- JP 1161236 A
- US 2484430 A
- US 2548564 A
- US 3148061 A
- US 3309690 A
- US 4115124 A
- US 4124386 A
- US 4193800 A
- US 4273853 A
- US 4282305 A

- US 4450224 A
- JP 2001260519 A
- JP 2000260519 A
- JP 4034953 B
- JP 4034513 B
- JP 4034512 B
- JP 11170686 A
- JP 60067190 A
- JP 7276808 A
- JP 2000094829 A
- JP 8512258 T
- JP 11321090 A
- JP 62173463 A
- JP 62183457 A
- JP 57009053 B
- JP 61020994 A
- JP 62135826 A
- JP 9263036 A
- JP 2001 A
- JP 310547 A
- JP 62245258 A
- JP 62136648 A
- JP 62110066 A
- JP 11170527 A
- JP 5229133 A
- JP 7276671 A
- JP 9277552 A
- JP 2002292878 A

**Non-patent literature cited in the description**


- Shin Jikken Kagaku Koza 9, Bunseki Kagaku. Maruzen, 1977
- Dai 4 Han, Jikken Kagaku Koza 15, Bunseki. Maruzen, 1991
- **J.A. DEAN.** Lange's Handbook of Chemistry. McGraw-Hill, 1979
- Kagakuno Ryoiki. Nankodo, 1979, 96-103
- Color Index
- Kaitei Shinpan Ganryo Binran. 1989
- Saishin Ganryo Oyo Gijutsu. CMC, 1986
- Insatsu Ink Gijutsu. CMC, 1984
- **W. HERBST ; K. HUNGER.** Industrial Organic Pigments. VCH Verlagsgesellschaft, 1993
- Kinzoku Sekken no Seishitsu to Oyo. Saiwai Shobo Co., Ltd,
- Insatsu Ink Insatsu. CMC, 1984
- Saishin Ganryo Oyo Giiutsu. CMC, 1986
- *Research Disclosure,* 1989
- Jikken Kagaku Koza. 417-418
- *Research Disclosure, No. 24239*
- *Research Disclosure, No. 17643,* VII-I
- *J, Research Disclosure, No. 15162*
- *Research Disclosure, No. 18716,* 650
- *Research Disclosure, No. 36544,* 527
- *Research Disclosure, No. 307105,* 872
- *Research Disclosure, No. 15162*
- Nendo Chosei Gijutsu. Gijutsu Joho Kyokai, 1999
- Chemicals for Inkjet Printers. Zairyo no Kaihatsu Doko-Tenbo Chosa. CMC, 1997, 162-174
- Interface and Colloid. Shin-Jikken Kagaku Koza. Maruzen, 1977, vol. 18, 69-90
- Technical Report Colorimetry. 2004